(19) Europäisches Patentamt
European Patent Office
Office européen des brevets

(11) Veröffentlichungsnummer : **0 092 520**
**B1**

(12) # EUROPÄISCHE PATENTSCHRIFT

(45) Veröffentlichungstag der Patentschrift : **12.11.86**

(51) Int. Cl.[4] : **C 09 B 44/02**, C 07 D401/00, C 07 D213/00, D 06 P 1/41

(21) Anmeldenummer : **83810152.5**

(22) Anmeldetag : **13.04.83**

(54) **Basische Verbindungen, deren Herstellung und Verwendung.**

(30) Priorität : **15.04.82 DE 3213826**
**27.01.83 DE 3302614**
**29.01.83 DE 3302950**

(43) Veröffentlichungstag der Anmeldung : **26.10.83 Patentblatt 83/43**

(45) Bekanntmachung des Hinweises auf die Patenterteilung : **12.11.86 Patentblatt 86/46**

(84) Benannte Vertragsstaaten :
**AT BE CH DE FR GB IT LI NL SE**

(56) Entgegenhaltungen :
**EP-A- 0 041 040**
**DE-A- 2 752 282**
**FR-A- 2 383 997**
**Die Akte enthält technische Angaben, die nach dem Eingang der Anmeldung eingereicht wurden und die nicht in dieser Patentschrift enthalten sind.**

(73) Patentinhaber : **SANDOZ AG**
**Lichtstrasse 35**
**CH-4002 Basel (CH)**
**BE CH FR GB IT LI NL SE**
**SANDOZ-PATENT-GMBH**
**Humboldtstrasse 3**
**D-7850 Lörrach (DE)**
**DE**
**SANDOZ-ERFINDUNGEN Verwaltungsgesellschaft m.b.H.**
**Brunner Strasse 59**
**A-1235 Wien (AT)**
**AT**

(72) Erfinder : **Greve, Manfred**
**Niklaus Konrad Weg 5**
**CH-4143 Dornach (CH)**
Erfinder : **Moser, Helmut**
**Libellenstrasse 25**
**CH-4104 Oberwil (CH)**
Erfinder : **Pedrazzi, Reinhard**
**Grabenmattweg 59/7**
**CH-4123 Allschwil (CH)**
Erfinder : **Wald, Roland**
**10 rue Wilson**
**F-68330 Huningue (FR)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Gegenstand der Erfindung sind basische Verbindungen bzw. Azoverbindungen, die in einer der möglichen tautomeren Form der Formel

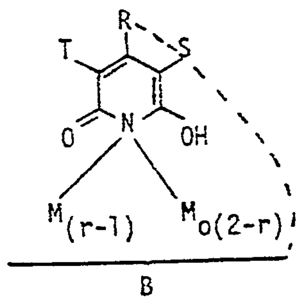

(I)

entsprechen, worin

R unabhängig voneinander Wasserstoff, einen (1-4C)-Alkyl-, (5-6C)-Cycloalkyl- oder Phenylrest, Benzyl oder Phenyläthyl,

T unabhängig voneinander Wasserstoff,

i) $-CN$

$i_1$) 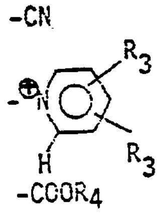 $A^{\ominus}$.

$i_2$) $-COOR_4$

$i_3$) $-CONR_5R_6$.

$i_4$) $-SO_2NR_5R_6$

$i_5$) 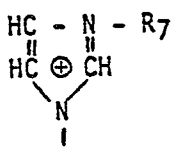 $A^{\ominus}$ oder

$i_6$) 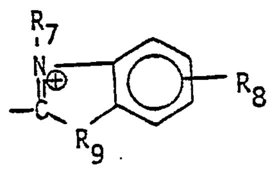 $A^{\ominus}$

r 1 oder 2,

M unabhängig voneinander einen Rest $-R_1-NH-R_0$,

$M_0$ unabhängig voneinander einen Rest $-R_1-NH-R_0$, Wasserstoff, $-NR_{11}R_{12}$, (1-4C)-Alkyl, Hydroxy-(2-4C)-alkyl, (1-4C)-Alkoxy-(1-4C)-alkyl, gegebenenfalls durch 1 bis 3 (1-4C)-Alkyl substituiertes (5-6C)-Cycloalkyl, Phenyl-(1-3C)-alkyl, $-V_1-NR_{13}R_{14}$ oder $-V_2-$

$$\overset{\oplus}{N}R_{13}R_{14}R_{15}A^{\ominus},$$

$A^{\ominus}$ ein Anion, bedeuten,

S falls r für 2 steht,

a) Wasserstoff,

b) einen Rest der Formel

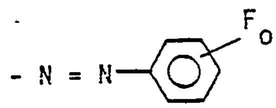 oder

c) —N = N—A—N = N—K

und $F_0$ einen in der Azochemie üblichen Substituenten, vorzugsweise Halogen, $NO_2$, $NH_2$, Alkyl, Alkoxy, CN, Trifluoralkyl, Phenyl, Anilin, Benzoyl, Carbamoyl, Phenoxy, Halogenphenoxy, Dihalogenphenoxy, Alkylsulfonyl, Phenylsulfonyl, Alkylsulfonylamine, Di-N-alkylaminosulfonyl oder Alkylsulfonyl, bedeuten oder, falls r für 1 steht,

d) einen Rest der Formel

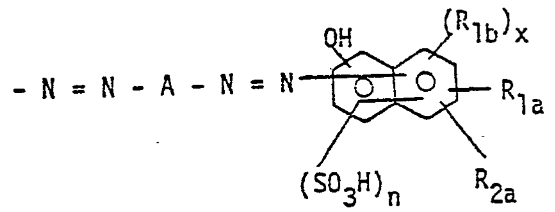

bedeutet, worin

n 0, 1 oder 2,

x 0 oder 1,

A unabhängig voneinander den Rest einer Tetrazokomponente,

K den Rest einer beliebigen, vorzugsweise sulfonsäuregruppenfreien Diazo- oder Kupplungskomponente aus der Reihe Pyrazolon-5, 5-Aminopyrazol, Anilin, Phenol, Acetoacetylalkyl- oder -arylamid, Barbitursäure, Dimedon, Dimedoncarbonsäureester, Pyridon (z. B. des Restes B, worin r 1 oder 2 ist) oder 2,6-Diaminopyridin,

$R_{1b}$, falls x für 1 steht, einen Rest der Formel

e) —N = N—$K_1$

f) —N = N—$A_1$—N = N—$K_1$

g) 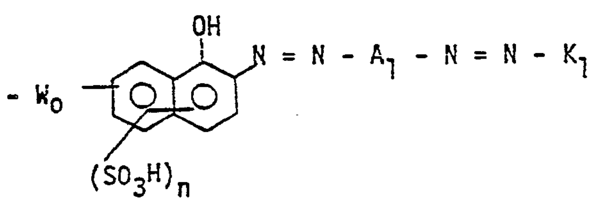

$K_1$ einen Rest K oder ein α- oder β-Naphthol, ein α- oder β-Aminonaphthalin oder ein Aminonaphthol,

$R_1$ unabhängig voneinander einen gegebenenfalls durch ein oder 2 Heteroatome unterbrochenen Alkylen- oder Alkenylenrest, einen Phenylen- oder Cyclohexylenrest,

$R_0$ oder einen Rest der Formel

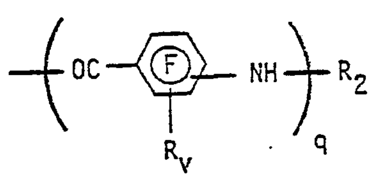 (Ia)

q 0 oder 1 und

$R_v$ Wasserstoff, Halogen, Nitro, (1-4C)-Alkyl oder (1-4C)-Alkoxy, bedeuten,

$R_2$ steht für einen Rest der Formel

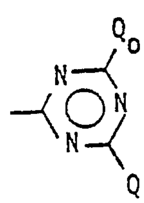 $h_1$)

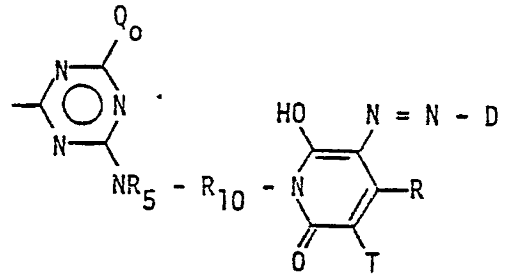

$h_3$) oder

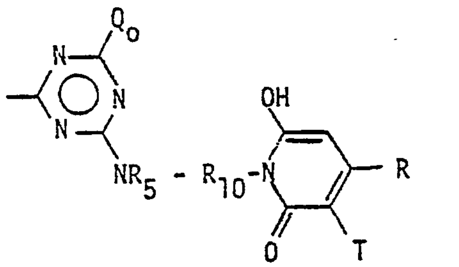

$h_2$)

$$- CO - (CH_2)_{1-3} - Z \qquad h_4)$$

oder auch für Wasserstoff, worin

D unabhängig voneinander den Rest einer Diazokomponente, die durch in der Azochemie übliche Substituenten substituiert sein kann, vorzugsweise einen Rest

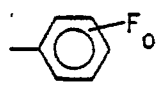

- A - N = N - K oder - A - N = N 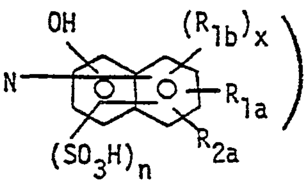

$R_3$ unabhängig voneinander Wasserstoff, einen (1-4C)-Alkylrest, $—NR_5R_6$ oder $—CONR_5R_6$,
$R_4$ unabhängig voneinander (1-6C)-Alkyl oder Phenyl-(1-3C)-alkyl,
$R_5$ und $R_6$ unabhängig voneinander Wasserstoff oder (1-4C)-Alkyl,
$R_7$ unabhängig voneinander (1-4C)-Alkyl,
$R_8$ unabhängig voneinander Wasserstoff oder (1-4C)-Alkyl,
$R_9$ unabhängig voneinander —S—, —O— oder

$R_{10}$ unabhängig voneinander einen (2-6C)-Alkylenrest oder

$$-\overset{*}{N}HCOCH_2-$$

wobei das mit *bezeichnete Atom am $—\dot{N}R_5—$ Rest gebunden ist,

$Q_0$ einen aliphatischen, cycloaliphatischen, aromatischen oder heterocyclischen Aminrest, in welchen das N-Atom an das C-Atom des Triazinylrestes gebunden ist,

Q Halogen, OH, $NH_2$, (1-4C)-Alkoxy, Phenyl oder $Q_0$,

Z unabhängig voneinander einen Rest der Formel $—NR_{11}R_{12}$ oder

$$- \overset{\oplus}{N}R_{13}R_{14}R_{15} \quad A^{\ominus}$$

$R_{11}$ und $R_{12}$ unabhängig voneinander Wasserstoff, unsubstituiertes (1-6C)-Alkyl, durch OH, CN oder Halogen substituiertes (2-6C)-Alkyl, Phenyl-(1-3C)-Alkyl, dessen Phenylrest durch 1 bis 3 Substituenten aus der Reihe Chlor, (1-4C)-Alkyl und (1-4C)-Alkoxy substituiert sein kann, unsubstituiertes oder durch 1 bis 3 (1-4C)-Alkylgruppen substituiertes (5-6C)-Cycloalkyl,

$R_{13}$ und $R_{14}$ unabhängig voneinander eine der Bedeutungen von $R_{11}$ und $R_{12}$, wobei einer der Reste $R_{13}$ und $R_{14}$ und vorzugsweise beide Reste von Wasserstoff verschieden sind,

$R_{15}$ unabhängig voneinander einen gegebenenfalls durch Phenyl substituierten (1-4C)-Alkylrest,

$V_1$ unabhängig voneinander (1-6C)-Alkylen oder (2-6C)-Alkenylen,

$V_2$ unabhängig voneinander (2-6C)-Alkylen oder (2-6C)-Alkenylen bedeuten,

die Reste $R_5$ und $R_6$ bzw. $R_{11}$ und $R_{12}$, bzw. $R_{13}$ und $R_{14}$ zusammen mit dem an sie gebundenen N-Atom einen gesättigten heterocyclischen Ring und $R_{13}$, $R_{14}$ und $R_{15}$ zusammen mit dem an sie gebundenen N-Atom einem Pyridinring oder einen gesättigten heterocyclischen Ring bilden können und der Pyridin- und der heterocyclische Ring durch eine, zwei oder drei (1-4C)-Alkylgruppen substituiert sein können,

$R_{1a}$ Wasserstoff,

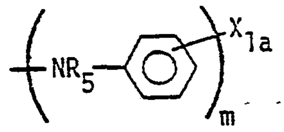

oder

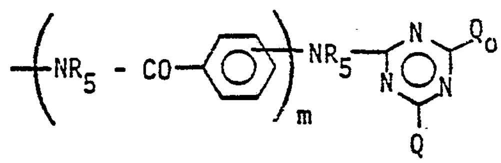

wobei für x = 1, $R_{1a}$ Wasserstoff bedeutet,

$$X_{1a} - NR_5 - Q_{10} - NR_{11}R_{12}, - COCH_2 - \overset{\oplus}{N}R_{13}R_{14}R_{15}\ A^{\ominus},$$
$$- NR_5 - COCH_2 - NR_{11}R_{12}, - CO - Q_{10} - \overset{\oplus}{N}R_{13}R_{14}R_{15}\ A^{\ominus},$$
$$- NR_5 - Q_{10} - \overset{\oplus}{N}R_{13}R_{14}R_{15}\ A^{\ominus}, -NR_5-COCH_2\overset{\oplus}{N}R_{13}R_{14}R_{15}A^{\ominus}$$
oder $CO-Q_{10}-\overset{\oplus}{N}(CH_3)_2-(CH_2)_4-Z\ A^{\ominus},$ )

m 0 oder 1,

$Q_{10}$

$$-\overset{*}{N}HCOCH_2-$$

oder lineares oder verzweigtes (2-6C)-Alkylen,

$R_{2a}$ Wasserstoff oder, wenn $R_{1a}$ für Wasserstoff und $R_{1b}$ für einen Rest der Formel e) oder f) steht, auch OH, $NH_2$, (1-4C)-Alkylcarbonylamino, Benzoylamino oder Phenylamino, deren Phenylrest durch 1 oder 2 Substituenten aus der Reihe Halogen, $NO_2$, $NH_2$ (1-4C)-Alkyl und (1-4C)-Alkoxy substituiert sein kann,

$A_1$ unabhängig voneinander den Rest einer Tetrazokomponente oder den Rest einer Diazo-/-Kupplungskomponente und

$W_0$ ein zweiwertiges Brückenglied bedeuten,

Gemische der Verbindungen der Formel I, deren Säureadditionssalze oder Salze von Säuren sowie Metallkomplexe, wie 1 : 1- oder 1 : 2-Metallkomplexe davon, mit der Massgabe, dass sie,

α) falls die Monoazoverbindungen b) der Formel I sulfogruppenfrei sind, eine und vorzugsweise zwei und, falls die Dis- und Polyazoverbindungen der Formel I sulfogruppenfrei sind, mindestens zwei wasserlöslich machende kationische oder mindestens zwei wasserlöslichmachende protonierbare basische Gruppen oder mindestens eine kationische und mindestens eine protonierbare basische Gruppen enthalten und, falls die Verbindungen der Formel I Sulfogruppen enthalten, die Anzahl der kationischen und/oder protonierbaren basischen Gruppen die Anzahl der Sulfogruppen und der eventuell zusätzlich vorhandenen anionischen Gruppen um mindestens 1 übersteigt, und

β) falls r für 2, S für Wasserstoff und $R_0$ für Wasserstoff stehen, $R_1$ verschieden ist von Alkylen, T verschieden von CN und der Rest M von Sulfonsäuregruppen frei.

Bevorzugte basische Verbindungen entsprechen in einer der möglichen tautomeren Form der Formel

(Siehe Formel Seite 6 f.)

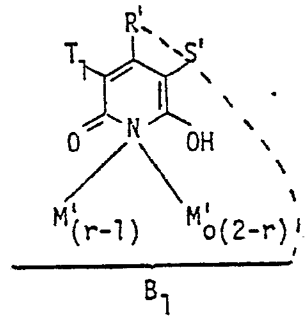

(II)

worin

R' unabhängig voneinander Methyl, Aethyl, Phenyl, Benzyl, oder Cyclohexyl,

$T_1$ unabhängig voneinander Wasserstoff,

i) - CN

$i_1'$) 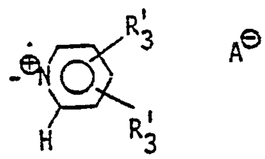 oder

$i_3'$) - $CONR_5'R_6'$,

M' unabhängig voneinander einen Rest $—R_1'—NH—R_0'$,

$M_0'$ unabhängig voneinander einen Rest $—R_1'—NHR_0'$, Wasserstoff, $—CH_3$, $—C_2H_5$, $—C_2H_4OH$, Cyclohexyl, Benzyl, $—(CH_2)_{1\text{-}3}—NR_{13}'R_{14}'$ oder $—(CH_2)_{2\text{-}3}$

$- \overset{\oplus}{N}R_{13}'R_{14}'R_{15}'A^{\ominus}$

S' falls r für 2 steht,

a) Wasserstoff,

b') einen Rest der Formel

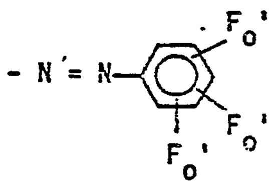

$c_1$) 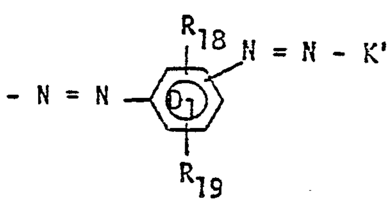 oder

$c_2$) 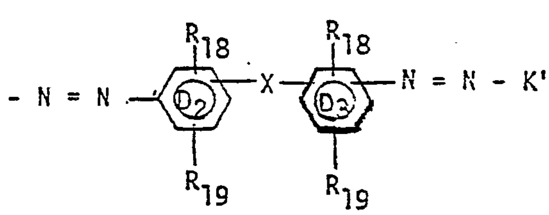

bedeutet,

oder S' falls r für 1 steht,

$d_1$) einen Rest der Formel

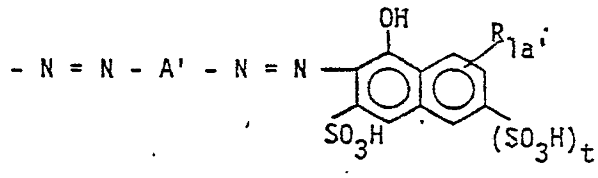

d2) 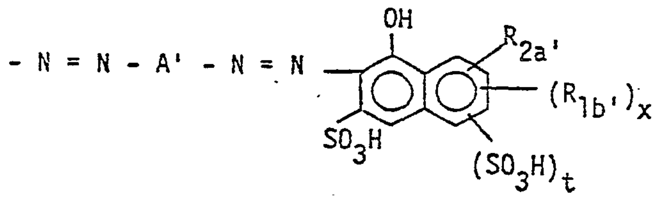

d3) 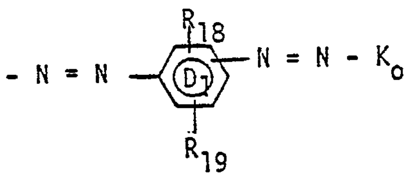 oder

d4) 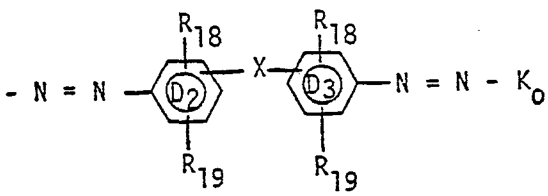

bedeutet, worin

$K_0$ einen Rest der Formel

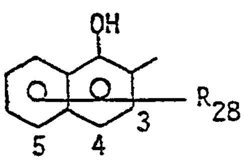

aa),

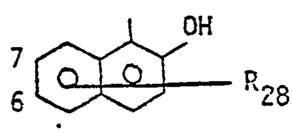

$aa_1$),

A' unabhängig voneinander einen Rest der Formel

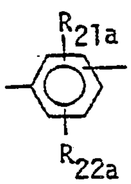

(m)

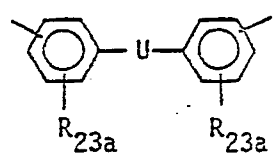

(n)

oder 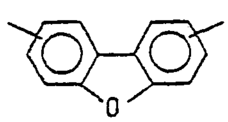 (o),

$R_{1b}$' falls x für 1 steht, einen Rest der Formel

e') $-N=N-K_1'$

f') $-N=N-A'-N=N-K_1'$

(Siehe Formel Seite 8 f.)

g') 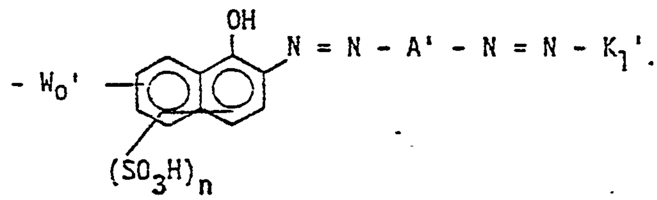

K' einen Rest der Formel
aa$_4$) $B_1$, worin r sowohl 1 als auch 2 sein kann,
aa$_5$) ein Phenol,

aa$_6$) 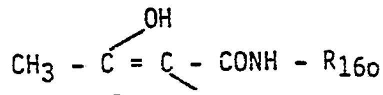

aa$_7$) 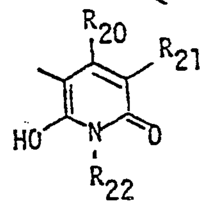

aa$_8$) 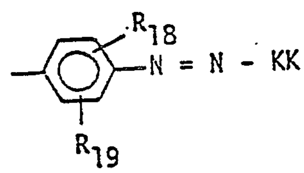

aa$_9$) 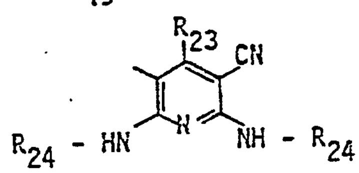

aa$_{10}$) 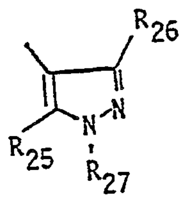

aa$_{11}$) 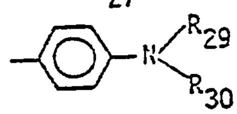

aa$_{12}$) 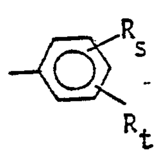

$R_1'$ unabhängig voneinander einen geradkettigen oder verzweigten (2-8C)-Alkylen oder meta- oder para-Phenylenrest,
$R_0'$ einen Rest der Formel

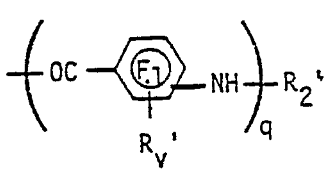 (Ib)

$R_v'$ unabhängig voneinander Wasserstoff, Chlor, Nitro, Methyl oder Methoxy bedeuten,
$R_2'$ steht für einen Rest der Formel

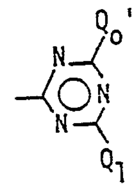

$h_1'$ oder

$- CO -(CH_2)_{1-2} - Z_1$ $\qquad h_4'$)

oder auch für Wasserstoff,

$R_3'$ unabhängig voneinander Wasserstoff, Methyl, Aethyl, $—NH_2$ oder $—N(CH_3)_2$,

$R_5'$ und $R_6'$ unabhängig voneinander Wasserstoff, Methyl oder Aethyl,

$Q_1$ Chlor, OH, $NH_2$,

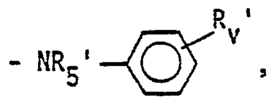 , 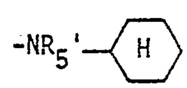 , $OCH_3$

Mono-(1-4C)-alkylamino, Di-(1-2C)-alkylamino, Monohydroxy-(2-4C)-alkylamino, Bis [hydroxy-(2-4C)-alkyl]-amino oder $Q_0'$ und

$$Q_0' \quad - \underset{\substack{| \\ R_5'}}{N} - R_x$$

bedeuten, worin

$R_x$ einen durch eine und/oder mehrere Gruppen der Formel $—NR_5'—$ oder

$$- \overset{\oplus}{N}R_7R_7 - A^{\ominus}$$

unterbrochenen, gegebenenfalls durch OH substituierten (1-12C)-Alkylrest,

$- NHCOCH_2 - Z_1$,

$- CH_2CONH - V - Z_1$, $- V - Z_1$, $- V -$ Arylen $- Z_0$,

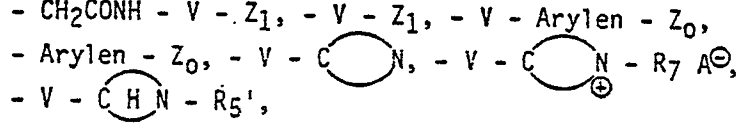

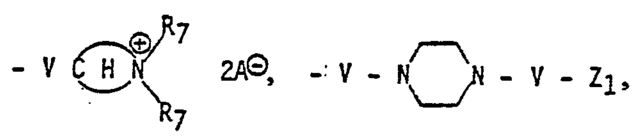

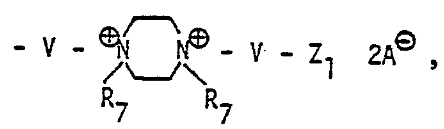

oder $R_x$ und $R_5'$ zusammen mit dem an sie gebundenen N-Atom einen Rest

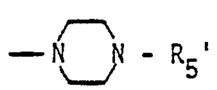 oder 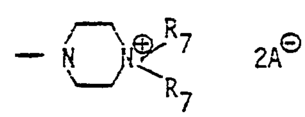

bilden können,

V unabhängig voneinander einen (1-8C)-Alkylenrest oder einen (3-8)-Alkenylenrest,

$Z_0$ $—N(CH_3)_2$, $—N(C_2H_5)_2$,

$$- \overset{\oplus}{N}(CH_3)_3\ A^{\ominus} - \overset{\oplus}{N}(C_2H_5)_3\ A^{\ominus}$$

oder einen Rest $—CONH—V_0—Z_1$, $—NHCO—V_0—Z_1$, $—CO—V_0—Z_1$, $—SO_2—NH—V_0—Z_1$, $—V_0—Z_1$ oder $—NHNHCOCH_2—Z_1$,

und der Arylenrest durch Halogen, OH, $NO_2$, (1-4C)-Alkyl oder (1-4C)-Alkoxy substituiert sein kann, $V_0$ für einen (1-8C)-Alkylenrest steht und die Gruppe der Formel

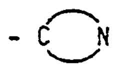 oder 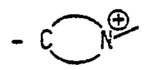

für einen ungesättigten Heterocyclus und die Gruppe der Formel

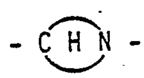 oder 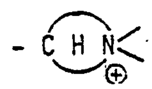

für einen gesättigten Heterocyclus stehen,

$Z_1$ unabhängig voneinander einen Rest der Formel $-NR_{11}'R_{12}'$ oder

$$-\overset{\oplus}{N}R_{13}'R_{14}'R_{15}'\ A^{\ominus}$$

$R_{11}'$ und $R_{12}'$ unabhängig voneinander Wasserstoff, lineares oder verzweigtes (1-6C)-Alkyl, unverzweigtes Hydroxy (2-3C)-alkyl, Benzyl, 2-Cyanäthyl oder 2-Chloräthyl,

$R_{13}'$ und $R_{14}'$ unabhängig voneinander lineares oder verzweigtes (1-6C)-Alkyl, unverzweigtes Hydroxy (2-3C)-alkyl, Benzyl, 2-Cyanoäthyl oder 2-Chloräthyl,

$R_{15}'$ Methyl, Aethyl, Propyl oder Benzyl

bedeuten,

die Reste $R_5'$ und $R_6'$ bzw. $R_{11}'$ und $R_{12}'$ zusammen mit dem an sie gebundenen N-Atom einen Morpholin-, Piperidin-, Pyrrolidin-, Piperazin- oder N-Methylpiperazinring, oder die Reste $R_{13}'$, $R_{14}'$ und $R_{15}'$ zusammen mit dem an sie gebundenen N-Atom einen Pyridin, Picolin- oder Lutidinring oder einen Ring der Formel

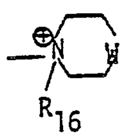 $A^{\ominus}$

worin $R_{16}$ Methyl oder Aethyl und W—$CH_2$, —O—, —S—, —$SO_2$—, —SO—,

$$-NH,\ -N-R_{16}\ \text{oder}\ -\overset{}{\underset{\oplus}{N}}(R_{16})_2\ A^{\ominus}$$

bedeuten, bilden können, worin

$F_0'$ unabhängig voneinander Wasserstoff, Halogen, —$NO_2$, —$NH_2$, (1-4C)-Alkyl, (1-4C)-Alkoxy, CN, Trifluormethyl, Phenyl, Anilino, Benzoyl, Carbamoyl, Phenoxy, Halogenphenoxy, Dihalogenphenoxy, (1-4C)-Alkylsulfonyl, Phenylsulfonyl, (1-4C)-Alkylsulfonylamino, Di-N(1-4C)-alkylaminosulfonyl oder (1-4C)-Alkylsulfonyl bedeuten,

X, falls die Ringe $D_2$ und $D_3$ unsubstituiert sind, für $X_1$ die direkte Bindung,

$X_2$ einen geradkettigen oder verzweigten Alkylenrest mit 1 bis 4 Kohlenstoffatomen,

(Siehe Formeln Seite 11 ff.)

$R_{42}$ unabhängig voneinander Halogen, (1-4C)-Alkyl oder (1-4C)-Alkoxy,

$R_{43}$ unabhängig voneinander einen geradkettigen oder verzweigten (1-4C)-Alkylenrest,

$R_{44}$ unabhängig voneinander Wasserstoff oder (1-4C)-Alkyl,

$R_{45}$ unabhängig voneinander Halogen,

$-NH-CH_2-CH_2-OH$,

$-N(CH_2-CH_2-OH)_2$, $NH_2$, OH, $NH-(CH_2)_{2-3}N(C_2H_5)_2$,

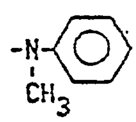, 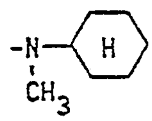, 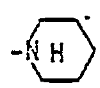, $-OCH_3$, $OC_2H_5$,

$X_3$ - CO -, $X_4$ 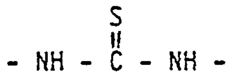, $X_5$ - S -, $X_6$ - O -,

$X_7$ - CH = CH -, $X_8$ - S - S -, $X_9$ - $SO_2$ -,

$X_{10}$ - NH -, $X_{11}$ - NH - CO -, $X_{12}$ 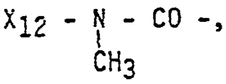

$X_{13}$ 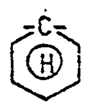 , $X_{14}$ 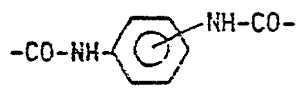 ,

$X_{15}$ 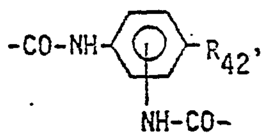 $X_{16}$ 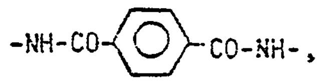

$X_{17}$ -$SO_2$-NH-, $X_{18}$ 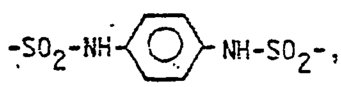

$X_{19}$ 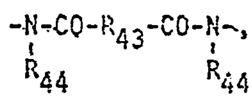 $X_{20}$ 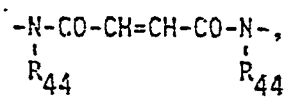

$X_{21}$ 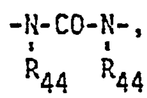 $X_{22}$ -CO-NH-NH-CO-,

$X_{23}$ -$CH_2$CO-NH-NH-CO-$CH_2$-, $X_{24}$ -CH=CH-CO-NH-NH-CO-CH=CH-,

$X_{25}$ 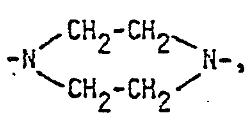 $X_{26}$ 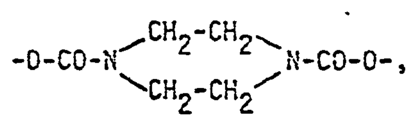

$X_{27}$ 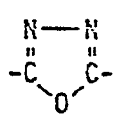 , $X_{28}$ 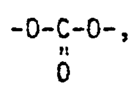 $X_{29}$ 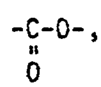 $X_{30}$ 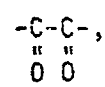

$X_{31}$ -O-$(CH_2)_g$-O-,

$X_{32}$ 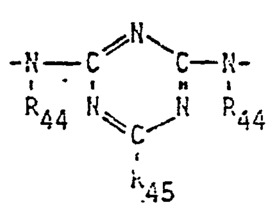 , $X_{33}$ 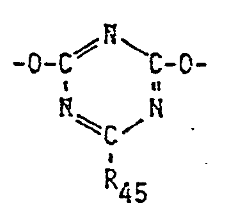 ,

(Fortsetzung)

$X_{34}$ $-CO-\underset{R_{44}}{N}-R_{43}-\underset{R_{44}}{N}-CO-$, $X_{35}$ $-CO-\underset{R_{44}}{N}-(CH_2)_g-O-(CH_2)_g-\underset{R_{44}}{N}-CO-$,

$X_{36}$ $-CO-\underset{R_{44}}{N}-(CH_2)_g-\underset{R_{44}}{N}-(CH_2)_g-\underset{R_{44}}{N}-CO-$,

$X_{37}$ $-CO-\underset{R_{44}}{N}-(CH_2)_g-O-(CH_2)_g-O-(CH_2)_g-\underset{R_{44}}{N}-CO-$,

$R_{38}$ $-CH_2-CO-\underset{R_{44}}{N}-$, $R_{39}$ $-CH{=}CH-CO-\underset{R_{44}}{N}-$,

$X_{40}$ $-N{=}N-$, $X_{41}$ $-CH_2-S-CH_2-$, $X_{42}$ $-SO-$,

$X_{43}$ $-CH_2-SO-CH_2-$,

$X_{44}$ $-CH_2-SO_2-CH_2-$, $X_{45}$ $-CH_2-NH-CO-NH-CH_2-$,

$X_{46}$ $-CH_2-NH-CS-NH-CH_2-$,

$X_{47}$ 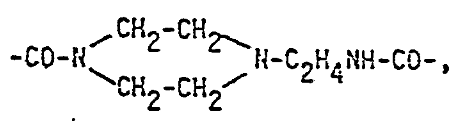

$X_{48}$ $-CH_2-CH_2-CO-\underset{R_{44}}{N}-$, $X_{49}$ $-CH_2-CO-CH_2-$,

$X_{50}$ $-CH{=}CH-CH{=}CH-$, $X_{51}$ 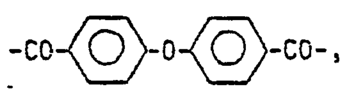

$X_{52}$ 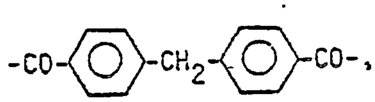 $X_{53}$ 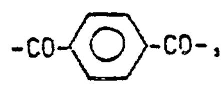

$X_{54}$ 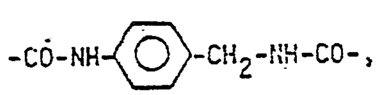

$X_{55}$ 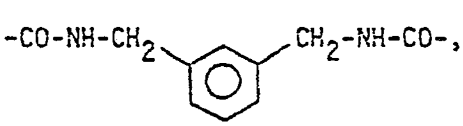

(Fortsetzung)

$X_{56}$ 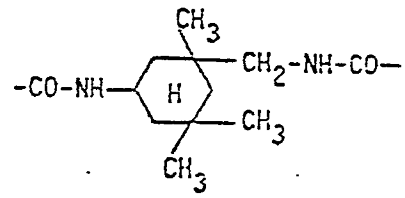 ,

$X_{57}$ 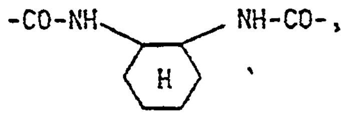 , $X_{58}$ $-CH_2-CO-$

$X_{59}$ $-CH=CH-CO-CH=CH-$, $X_{60}$ 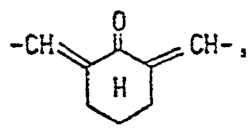

$X_{61}$ 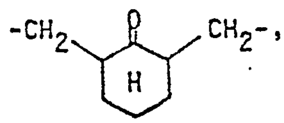 $X_{62}$ $-O-CH_2-CH_2-O-$,

$X_{63}$ $-O-CH_2-O-$, $X_{64}$ $-CO-NH-R_{43}-CO-NH-$,

$X_{100}$ $-CO-NH-R_{43}-CO-NH-R_{43}-NH-CO-$,

$X_{101}$ $-CO-NH-R_{43}-NH-CO-CH_2-CH_2-CO-NH-R_{43}-NH-CO-$,

$X_{102}$ $-CO-NH-R_{43}-NH-CO-CH=CH-CO-NH-R_{43}-NH-CO-$,

$X_{103}$ 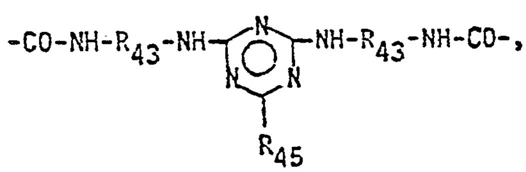

$X_{104}$ $-SO_2-NR_{44}-(CH_2)_g-NR_{44}-SO_2-$,

$X_{105}$ $-CO-NR_{44}-R_{43}-O-CO-$,

$X_{106}$ 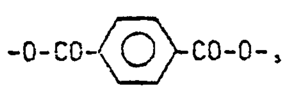

$X_{107}$ 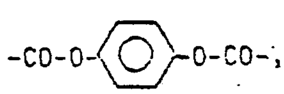

$X_{108}$ $-CONH-R_{43}-NH-CO-NH-R_{43}-NH-CO-$,

g unabhängig voneinander 1, 2, 3 oder 4,

X falls die Ringe $D_2$ und/oder $D_3$ substituiert sind, insbesondere in ortho-Stellung zu den beiden Azobrücken, für die direkte Bindung $X_1$ oder für $X_2$, $X_{14}$, $X_{21}$, $X_{32}$, $X_{34}$ oder $X_{40}$ steht,

$R_s$ und $R_t$ unabhängig voneinander Wasserstoff, (1-4C)-Alkyl oder (1-4C)-Alkoxy,

$R_{160}$ einen Rest

$$-(CH_2)_v-Z_1,$$

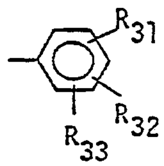
,
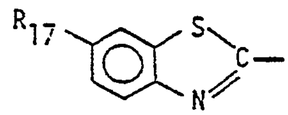
,

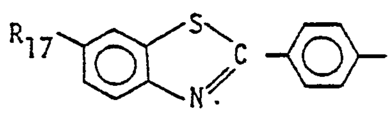
,
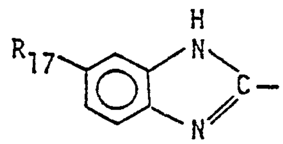
,

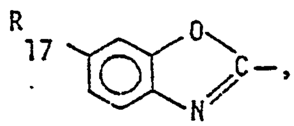
,
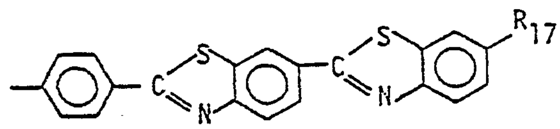

oder
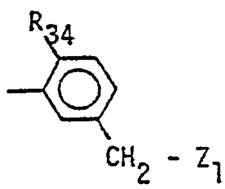

$R_{17}$ unabhängig voneinander H oder Methyl,

$R_{18}$ und $R_{19}$ unabhängig voneinander Wasserstoff, Halogen, (1-4C)-Alkyl oder (1-4C)-Alkoxy,

$R_{20}$ H, Methyl, Aethyl, Phenyl, Benzyl oder Cyclohexyl,

$R_{21}$ einen der Reste $T_1$ oder

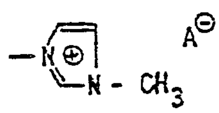

$R_{22}$ einen Rest der Formel $-R_1'-NH-R_0'$, Wasserstoff, einen gegebenenfalls durch Phenyl substituierten (1-4C)-Alkylrest, $-(CH_2)-CN$, $-(CH_2)_v-Z_1$, $-(CH_2)_2-OH$, $-(CH_2)_2OCH_3$ oder

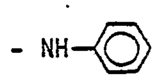

$R_{23}$ (1-4C)-Alkyl oder Phenyl,

$R_{24}$ unabhängig voneinander Wasserstoff, (1-4C)-Alkyl, $-(CH_2)_{2\text{-}3}-OCH_3$, $-(CH_2)_2-OH$, $-(CH_2)_{2\text{-}3}-N(CH_3)_2$ oder

$$-(CH_2)_{2-3}-\overset{\oplus}{N}(CH_3)_3\ A^{\ominus}$$

$R_{25}$ OH oder $NH_2$,

$R_{26}$ (1-4C)-Alkyl oder $-COR_{35}$,

$R_{27}$ Wasserstoff,

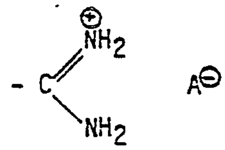

oder
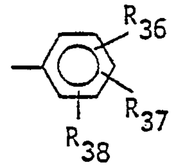
,

$R_{28}$ in einer der Stellungen 3, 4, 5, 6 oder 7 unabhängig voneinander Wasserstoff,

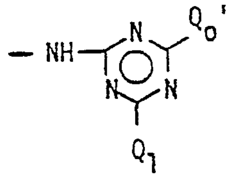 , $-NHCO(CH_2)_{1-3}-Z_1$

$-SO_2-NH-V_0-Z_1$, $-CONH-V_0-Z_1$, $-CONHNH_2$, $-NH-V_0-Z_1$, $-CH_2-Z_1$, $-NHNHCOCH_2-Z_1$ oder

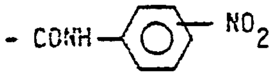

$R_{29}$ (1-4C)-Alkyl,

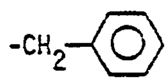

oder $-(CH_2)_2-CN$,

$R_{30}$ (1-4C)-Alkyl oder $-(CH_2)_v-Z_1$,

v unabhängig voneinander 1, 2, 3, 4, 5 oder 6,

$R_{31}$ und $R_{32}$ unabhängig voneinander Wasserstoff, Halogen, (1-4C)-Alkyl, (1-4C)-Alkoxy, $NO_2$ oder CN,

$R_{33}$ Wasserstoff,

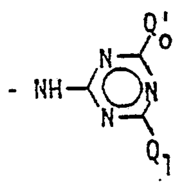

$-NHCO-(CH_2)_{2-3}-Z_1$, $-SO_2-NH-(CH_2)_{2-3}-Z_1$ oder $-CONH-V_0-Z_1$,

$R_{34}$ (1-4C)-Alkoxy,

$R_{35}$ $-O-R_{39}$, $-NR_{40}R_{41}$ oder $-NH-V_0-Z_1$,

$R_{36}$ und $R_{37}$ unabhängig voneinander Wasserstoff, Halogen, (1-4C)-Alkyl, (1-4C)-Alkoxy, $-NO_2$ oder $-NH_2$,

$R_{38}$ Wasserstoff, $-CONH-V_0-Z_1$, $-SO_2NH-V_0-Z_1$, $-NHCO(CH_2)_{2-3}-Z_1$ oder

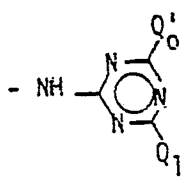

$R_{39}$ (1-4C)-Alkyl,

$R_{40}$ und $R_{41}$ unabhängig voneinander Wasserstoff oder (1-4C)-Alkyl,

$R_{21a}$ H, OH, Halogen, (1-4C)-Alkyl, (1-4C)-Alkoxy, CN, $-CONH_2$, $-NHCO(1\text{-}4C)\text{-}Alkyl$, $-NHCONH_2$, COOH oder $-SO_3H$,

$R_{22a}$ H, Halogen, (1-4C)-Alkyl oder (1-4C)-Alkoxy und die Reste $R_{21a}$ und $R_{22a}$ paraständig zueinander stehen,

$R_{23a}$ unabhängig voneinander H, OH, Halogen, CN, (1-4C)-Alkyl, (1-4C)-Alkoxy, $-COOH$ oder $-SO_3H$,

U die direkte Bindung, $-(CH_2)_{1-3}$, $-O-$, $-S-$, $-SO_2-$, $-NHCO-$, $-NHCONH-$, $-NHCO(CH_2)_{2-3}-CONH-$, $-CONH(CH_2)_{2-3}-NHCO-$, $-O(CH_2)_{2-3}-O-$, $-N=N-$, $-CH=CH-CO-CH=CH-$,

t 0 oder 1,

$R_{2a}'$ H, OH oder $NH_2$,

$R_{1a}'$ Wasserstoff,

$- NR_5' - Q_{10}' - NR_{11}'R_{12}'$,

$- NR_5' - Q_{10}' - \overset{\oplus}{N}R_{13}'R_{14}'R_{15}'\ A^{\ominus}$,

$- NR_5'COCH_2NR_{11}'R_{12}'$, $- NR_5'$ 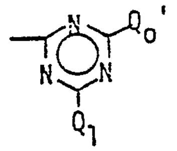 ,

$- NR_5' - COCH_2\overset{\oplus}{N}R_{13}'R_{14}'R_{15}'\ A^{\ominus}$ oder

$- CO - Q_{10}' - \overset{\oplus}{N}(CH_3)_2 - (CH_2)_{1-4} - Z_1\ A^{\ominus}$,

$Q_{10}'$ $-(CH_2)_{2-6}-$, $-CH_2-CH(CH_3)-$ oder $-CH_2-CH(CH_3)-CH_2-$,

$W_0'$ $-NH-$, $-NHCONH-$, $-NHCO-CH=CH-CO-NH-$,

$-NHCO(CH_2)_{2-3}-CONH-$, $-NHCO$ 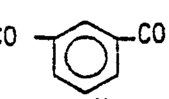 $CO-NH-$,

$-NH-CO$ 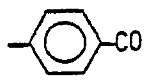 $CO-NH-$ oder 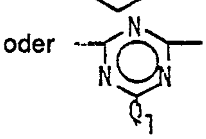

$K_1'$ einen Rest der Formel aa), $aa_1$), $aa_2$), $aa_3$), $aa_4$), $aa_5$), $aa_6$), $aa_7$), $aa_8$), $aa_9$), $aa_{10}$), $aa_{11}$), $aa_{12}$) oder

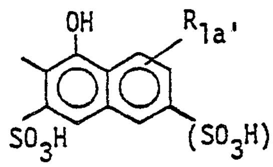

$aa_{13}$) oder

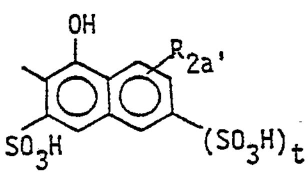

$aa_{14}$)

und KK einen Rest der Formel $aa_1$), $aa_2$), $aa_3$), $aa_4$), $aa_5$), $aa_6$), $aa_7$), $aa_8$), $aa_9$), $aa_{10}$), $aa_{11}$), $aa_{12}$), $aa_{13}$) oder $aa_{14}$) bedeuten,

Gemische der Verbindungen der Formel II, deren Säureadditionssalze oder Salze von Säuren sowie 1 : 1- oder 1 : 2-Metallkomplexe davon, mit der Massgabe, dass

$\alpha_1$) die Monoazoverbindungen b' der Formel II eine und vorzugsweise zwei und, falls die Dis- und Polyazoverbindungen der Formel II von sulfonsäuregruppenfrei sind [Formeln $c_1$), $c_2$), $d_3$), $d_4$)], sie mindestens zwei wasserlöslich machende kationische oder mindestens zwei protonierbare basische Gruppen oder mindestens eine kationische und mindestens eine protonierbare Gruppe enthalten und, falls die Verbindungen der Formel II Sulfogruppen enthalten, die Anzahl der kationischen und/oder protonierbaren basischen Gruppen die Anzahl der Sulfogruppen und die eventuell zusätzlich vorhandenen —COOH-Gruppen um mindestens 1 übersteigt und

$\beta_1$) falls r für 2, S' für Wasserstoff, $R_0'$ für Wasserstoff stehen, $R_1'$ verschieden ist von Alkylen, $T_1$ verschieden von CN und der Rest M' von Sulfonsäuregruppen frei.

Besonders bevorzugte basische Verbindungen entsprechen in einer der möglichen tautomeren Form der Formel

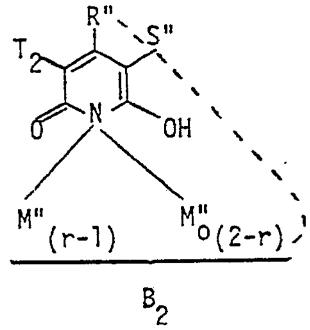

(III)

worin

R'' Methyl oder Phenyl,

$T_2$ unabhängig voneinander

- CN oder $i_1$'')- 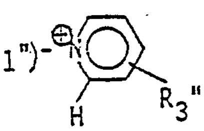 $A^{\ominus}$

M'' unabhängig voneinander $—R_1''—NH—R_0''$,

$M_0''$ unabhängig voneinander $—R_1''—NH—R_0''$, Wasserstoff, $CH_3$, $—C_2H_5$, Benzyl, $—(CH_2)_{2\text{-}3}—NR_{13}''R_{14}''$ oder $—(CH_2)_{2\text{-}3}—NR_{13}''R_{14}''R_{15}''$ $A^{\ominus}$,

S'' falls r für 2 steht,

a) Wasserstoff,

b'') einen Rest der Formel

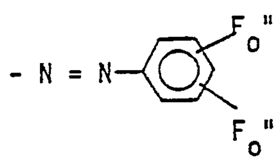

,

$c_1$') 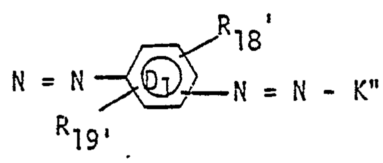

$c_2$') 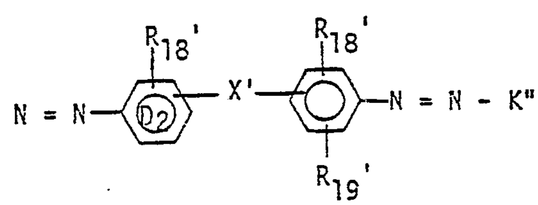

bedeutet,

oder S'' falls r für 1 steht,

$d_1$') einen Rest der Formel

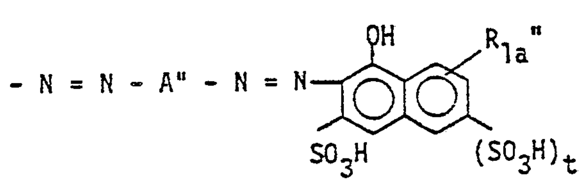

d2')
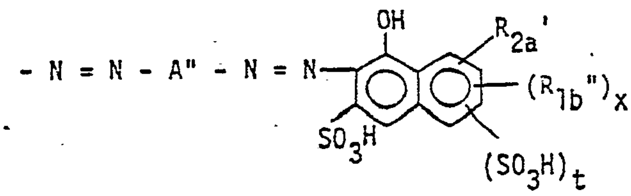

d3')
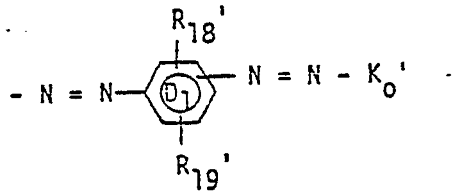

d4')
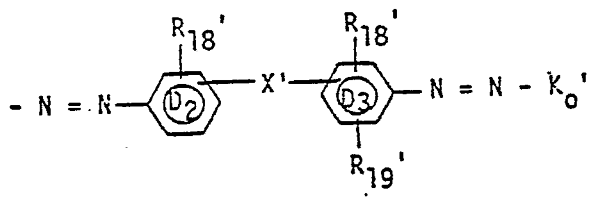

bedeutet, worin

$K_0'$ einen Rest der Formel

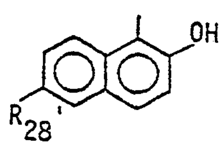

aa')

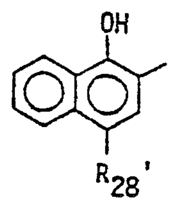

(aa1')

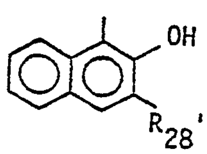

aa2')

A" unabhängig voneinander einen Rest der Formel

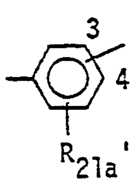

oder

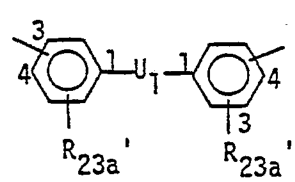

(mm) (nn)

wobei der Rest (mm) über die Positionen 1,3 oder 1,4 verknüpft ist und der Rest (nm) über die Positionen 3,3 ; 3,4 ; oder 4,4 gebunden ist,

$R_{1b}''$ falls x für 1 steht, einen Rest der Formel

e") $-N=N-K_1''$

f") $-N=N-A''-N=N-K_1''$

g")
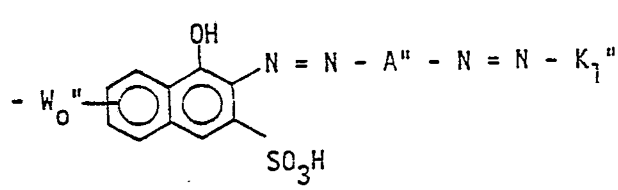

K″ einen Rest der Formel

aa$_4$′) $B_2$, worin r 1 oder 2 ist,

aa$_5$) ein Phenol,

aa$_6$′) 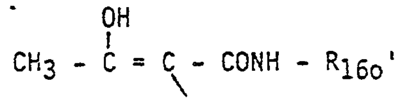

aa$_7$′) 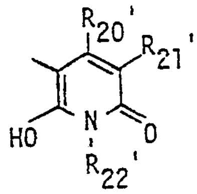

aa$_8$′) 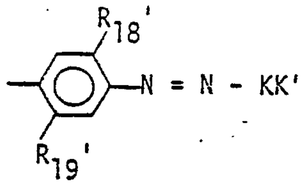

aa$_9$′) 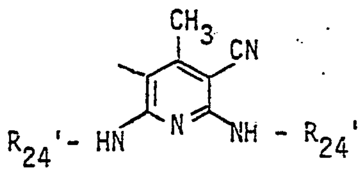

aa$_{10}$′) 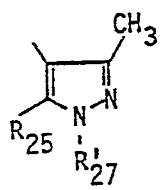

aa$_{11}$′) 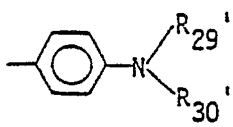

aa$_{12}$′) 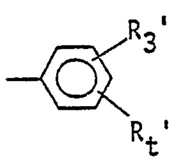

$R_1$″ 1,2-Aethylen, 1,3-Propylen oder meta- oder para-Phenylen,

$R_0$″ einen Rest der Formel

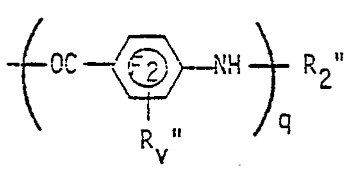 (Ic)

$R_v$″ Wasserstoff, Chlor oder Methyl bedeuten,

$R_2$″ unabhängig voneinander steht für einen Rest der Formel

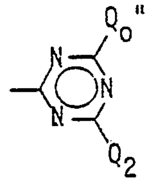

h₁") oder

$- CO - CH_2 - Z_2$  $h_4'')$

oder auch für Wasserstoff, worin

$R_3''$ Wasserstoff oder Methyl,

$Q_2$ Chlor, OH, $—NH_2$, Mono(1-2C)-alkylamino, $OCH_3$, Monohydroxy(2-4C)-alkylamino, Bis [hydroxy(2-4C)-alkyl] amino oder $Q_0''$ und

$$Q_0'' \; - \underset{R_{50}}{\underset{|}{N}} - R_x'$$

bedeuten, worin

$R_x'$ einen der Reste der Formeln

$- (CH_2)_{2-3} - NR_{50} - (CH_2)_{2-3} - NR_{50} - C_2H_5,$

$- (CH_2)_{2-3} - \overset{\oplus}{N}(R_{13}'')_2 - (CH_2)_{2-3} - \overset{\oplus}{N}(R_{13}'')_2 - C_2H_5 \quad 2A^{\ominus}$

$- (CH_2)_{2-3} - NR_{50} - C_2H_5,$

$- (CH_2)_{2-3} - \overset{\oplus}{N}(R_{13}'')_2 - C_2H_5 \quad A^{\ominus}, \; - NHCOCH_2 - Z_2,$

$- CH_2 - CO - NH - V' - Z_2,$

$- V' - Z_2,$ 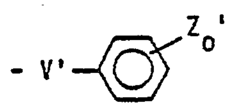 , 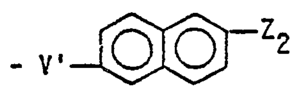 ,

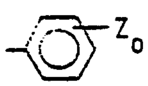 , 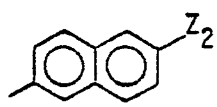 , 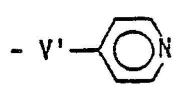 , 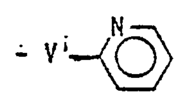

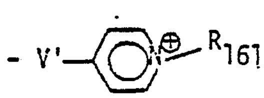 $A^{\ominus}$ 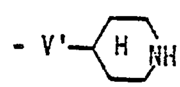 , 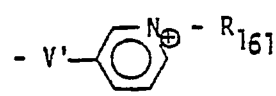 $A^{\ominus}$,

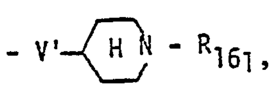 , 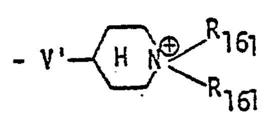 $A^{\ominus}$,

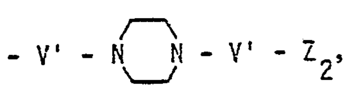 , 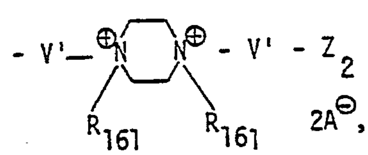 $2A^{\ominus}$,

oder $R_x'$ und $R_{50}$ zusammen mit dem an sie gebundenen N-Atom einen Rest

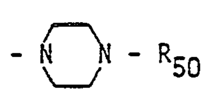 oder 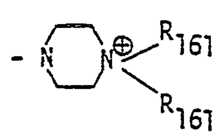 $A^{\ominus}$

bilden können,

$R_{161}$ Methyl oder Aethyl,

V' unabhängig voneinander einen (1-4C)-Alkylenrest,

$Z_0'$ $—N(CH_3)_2$,

$$- \overset{\oplus}{N}(CH_3)_3 \; A^{\ominus}$$

oder einen Rest —CONH—$V_0'$—$Z_2$, —NH—CO—$V_0'$—$Z_2$, —CO—$V_0'$—$Z_2$, —$SO_2$NH—$V_0'$—$Z_2$, —$V_0'$—$Z_2$ oder —NHNHCO$CH_2$—$Z_2$ und

$V_0'$ für einen (1-4C)-Alkylenrest steht,

$R_{50}$ Wasserstoff oder Methyl,

$Z_2$ unabhängig voneinander einen Rest —$NR_{11}''R_{12}''$ oder

$$-\overset{\oplus}{N}R_{13}''R_{14}''R_{15}''\ A^{\ominus},$$

$R_{11}''$ und $R_{12}''$ unabhängig voneinander Wasserstoff, Methyl oder Aethyl,

$R_{13}''$ und $R_{14}''$ unabhängig voneinander Methyl oder Aethyl,

$R_{15}''$ Methyl, Aethyl oder Benzyl bedeuten,

die Reste $R_{11}''$ und $R_{12}''$ zusammen mit dem an sie gebundenen N-Atom einen Morpholin-, Piperazin- oder N-Methylpiperazinring, die Reste $R_{13}''$, $R_{14}''$ und $R_{15}''$ zusammen mit dem an sie gebundenen N-Atom einen Pyridin- oder α- oder β-Picolinring oder einen N-Methylmorpholin-, N-Methylpiperidin-, N-Methylpiperazin oder N,N'-Dimethylpiperazinring bilden können,

worin

$F_0''$ unabhängig voneinander Wasserstoff, $NO_2$, Chlor, Methyl, Methoxy oder Chlorphenoxy,

X' falls die Ringe $D_2$ und $D_3$ unsubstituiert sind, für $X_1$, $X_5$, $X_6$, $X_7$, $X_{10}$, $X_{11}$, $X_{12}$, $X_{16}$, $X_{17}$, $X_{22}$, $X_{25}$, $X_{26}$, $X_{27}$, $X_{30}$, $X_{31}$, $X_{49}$, $X_{50}$, $X_{51}$, $X_{52}$, $X_{53}$, $X_{54}$, $X_{53}$, $X_{59}$, $X_{52}$, $X_{53}$, $X_{64}$, $X_{101}$ ($R_{43}$ = —$(CH_2)_{1-2}$), $X_{103}$ ($R_{43}$ = —$(CH_2)_{1-2}$ und $R_{45}$ = —NH—$(CH_2)_{2-3}$—$N(C_2H_5)_2$, $X_{104}$ ($R_{44}$ = H, g = 2 oder 3), $X_{108}$ ($R_{43}$ = —$(CH_2)_{1-3}$),

$X_{70}$ -NH-CO-NH-, $X_{71}$ 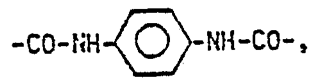

$X_{72}$ 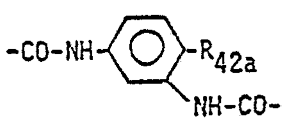

$X_{73}$ -NH-CO-$CH_2$-$CH_2$-CO-NH-,

$X_{74}$ -NH-CO-CH=CH-CO-NH-,

$X_{75}$ -NH-CO-$(CH_2)_4$-CO-NH-,

$X_{76}$ 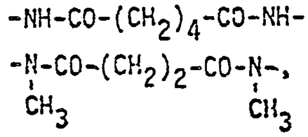

$X_{77}$ 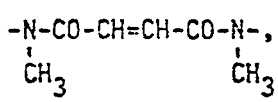

$X_{78}$ 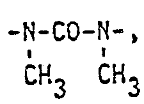

$X_{79}$ 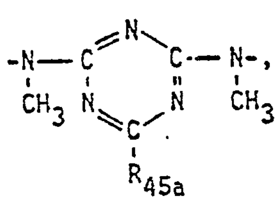 $X_{80}$ 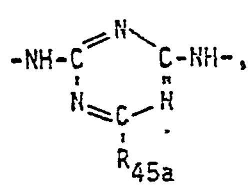

$X_{81}$ $-CH_2-$, $X_{82}$ $-(CH_2)_2-$, $X_{83}$ $-(CH_2)_3-$,
$X_{84}$ $-(CH_2)_4-$, $X_{85}$ $-CO-NH-(CH_2)_2-NH-CO-$,
$X_{86}$ $-CO-NH-(CH_2)_3-NH-CO-$, $X_{87}$ $-CO-NH-(CH_2)_4-NH-CO-$,
$X_{88}$ 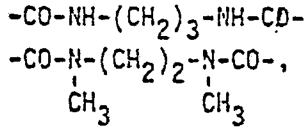, $X_{89}$ 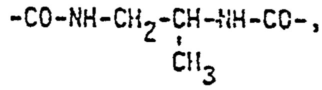,

$X_{90}$ 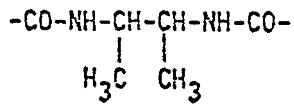

steht,

$R_{42a}$ unabhängig voneinander Wasserstoff, —Cl, —$CH_3$ oder —$OCH_3$ und
$R_{45a}$ unabhängig voneinander

$-Cl$, $-NH-CH_2-CH_2-OH$, $OCH_3$,
OH, $NH_2$, $-N(CH_2-CH_2OH)_2$, 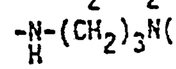, $OC_2H_5$,

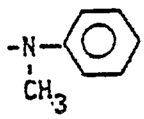, 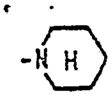 oder 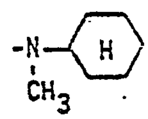

bedeuten, oder

X, falls die Ringe $D_2$ und $D_3$ substituiert sind, insbesondere in ortho-Stellung zu den beiden Azobrücken durch Chlor, Methyl oder Methoxy X für die direkte Bindung $X_1$, $X_{40}$, $X_{70}$, $X_{71}$, $X_{80}$, $X_{81}$, X 82 oder X 85 steht,

$R_s'$ und $R_t'$ unabhängig voneinander Wasserstoff, Methyl, Methoxy, Aethyl, Aethoxy oder Butyl,

$R_{160}'$ einen Rest $—(CH_2)_{v'}—Z_2$,

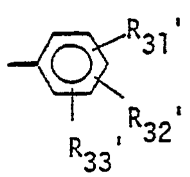, 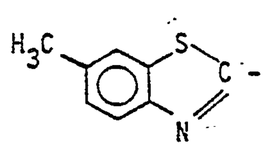,

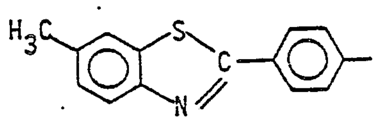 oder 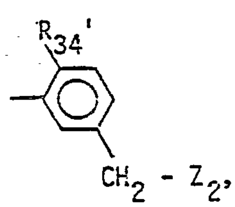

v' 2, 3 oder 4,

$R_{18}'$ und $R_{19}'$ unabhängig voneinander Wasserstoff, Chlor, $CH_3$, $C_2H_5$, $OCH_3$ oder $OC_2H_5$,

$R_{20}'$ Methyl oder Phenyl,

$R_{21}'$ einen der Reste von $T_2$,

$R_{22}'$ einen Rest $—R_1''—NH—R_0''$, Wasserstoff, Methyl, Aethyl, Benzyl, Phenyläthyl,

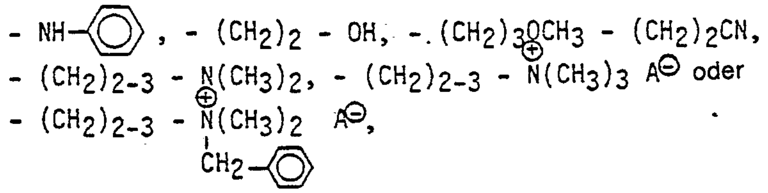

$R_{24}'$ unabhängig voneinander Wasserstoff,

$- (CH_2)_2OH,$
$-(CH_2)_3 - OCH_3, - (CH_2)_{2-3} - N(CH_3)_2$ oder
$- (CH_2)_{2-3} - \overset{\oplus}{N}(CH_3)_3 \; A^{\ominus},$

$R_{27}'$ Wasserstoff, 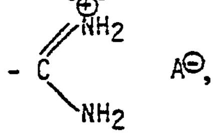

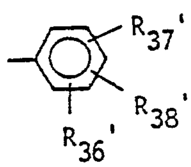

$R_{28}'$ unabhängig voneinander

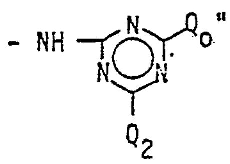 oder $- NHCOCH_2 - Z_2$

$- CONH(CH_2)_{2-3} - Z_2$ oder 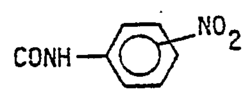

$R_{29}'$ $CH_3$, $C_2H_5$, $C_2H_4CN$ oder Benzyl,
$R_{30}'$ $CH_3$, $C_2H_5$ oder $-(CH_2)_v'-Z_2$,
$R_{31}'$ und $R_{32}'$ unabhängig voneinander Wasserstoff, Chlor, Brom, Methyl, Aethyl, Methoxy, Aethoxy, $NO_2$ oder CN,
$R_{33}'$ Wasserstoff,

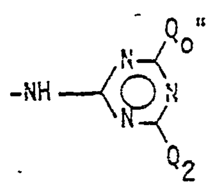

$-NH-CO(CH_2)_{2-3}-Z_2$ oder $-SO_2-NH(CH_2)_{2-3}-Z_2$,
$R_{34}'$ Methoxy oder Aethoxy,
$R_{36}'$ und $R_{37}'$ unabhängig voneinander Wasserstoff, Chlor, Methyl oder Methoxy,
$R_{38}'$ Wasserstoff,

$-CO-NH-(CH_2)_{v}'-Z_2$,

$-NHCO(CH_2)_{2-3}-Z_2$ oder $-NH-$ 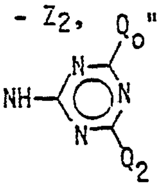

$R_{21a}'$ H, Cl, $CH_3$, OH, $OCH_3$, $C_2H_5$ oder $OC_2H_5$,
$R_{23a}'$ unabhängig voneinander H, Cl, $CH_3$, OH oder $OCH_3$,
$U_1$ die direkte Bindung, —NHCO— oder —NHCONH—,
$R_{1a}''$ Wasserstoff,

$-NR_{50}-(CH_2)_{2-3}-NR_{11}''R_{12}''$
$-NR_{50}-(CH_2)_{2-3}-NR_{13}''R_{14}''R_{15}''\ A^{\ominus}$ oder
$-NR_{50}$ 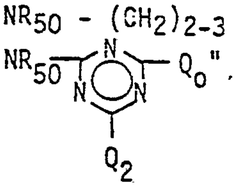

$W_0''$ —NH—, —NH—CO—CH = CH—CONH— oder —NHCO$(CH_2)_2$CONH—,
$K_1''$ einen Rest der Formel aa'), $aa_1'$), $aa_2'$), $aa_3'$), $aa_4'$), $aa_5$), $aa_6'$), $aa_7'$), $aa_8'$), $aa_9'$), $aa_{10}'$), $aa_{11}'$), $aa_{12}'$) oder einen Rest der Formel

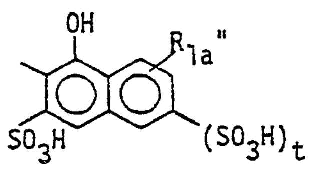

$aa_{13}'$) oder

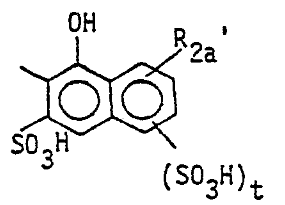

$aa_{14}'$)

und KK' einen Rest der Formel aa'), $aa_1'$), $aa_2'$), $aa_4'$), $aa_5'$), $aa_6'$) $aa_7'$), $aa_8$), $aa_9'$), $aa_{10}'$), $aa_{11}'$), $aa_{12}'$), $aa_{13}'$), $aa_{14}'$) bedeuten,
Gemische der Verbindungen der Formel III, deren Säureadditionssalze oder Salze von Säuren sowie 1 : 1- oder 1 : 2-Metallkomplexe davon, mit den zuvor genannten Bedingungen und der Massgabe, dass

$\beta_2$) falls r für 2, S'' für Wasserstoff,

$R_0''$ für Wasserstoff stehen, $R_1''$ verschieden ist von 1,2-Aethylen, $T_1$ verschieden von CN und der Rest M'' von Sulfonsäuregruppen frei.

Ganz besonders bevorzugte basische Verbindungen entsprechen in einer der möglichen tautomeren Formen der Formel

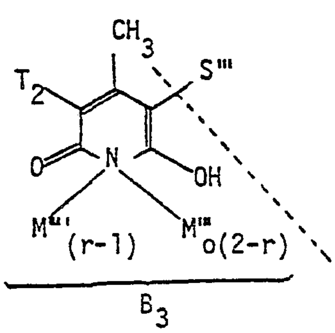

worin

M''' unabhängig voneinander $-R_1''-NH-R_0'''$,

$M_0'''$ unabhängig voneinander $-R''_1-NH-R_0'''$, H, $CH_3$, $C_2H_5$, Benzyl,

$-(CH_2)_{2-3}-NR''_{13}\ R''_{14}$ oder

$-(CH_2)_{2-3}-\overset{\oplus}{N}R''_{13}\ R''_{14}\ R''_{15}\ A^{\ominus}$

S''' falls r für 2 steht ;

a) Wasserstoff,

b''') einen Rest der Formel

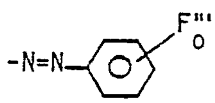 oder 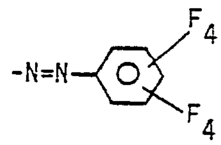

$c''_1$) 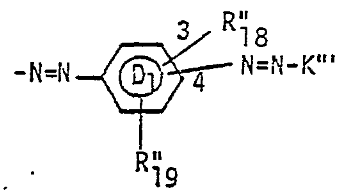

mit der Bindung von —N = N— in 3- oder 4-Stellung,

$c''_2$) 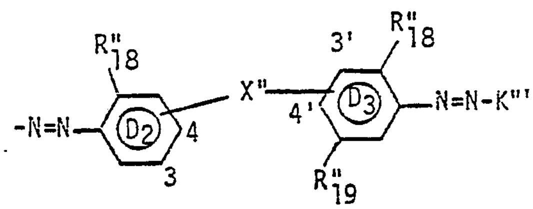

wobei der Rest X'' in 4,4'-, 3,4'- oder 3',4-Stellung gebunden ist, bedeutet,

oder S''' falls r für 1 steht,

$d_1''$) einen Rest der Formel

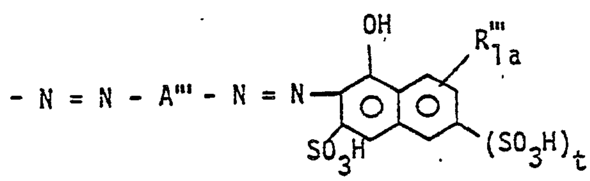

worin vorteilhaft t = 0 und $R_{1a}'''$ in 6-Stellung steht,

$d''_2$) 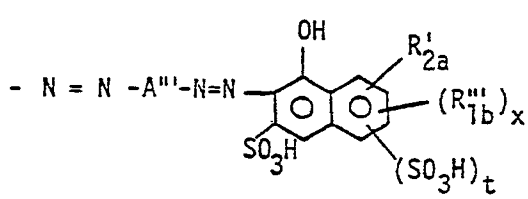

$d''_3$) 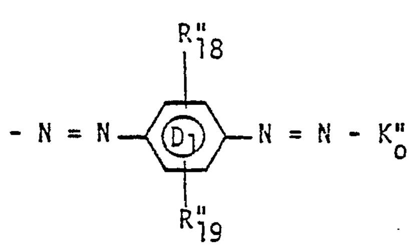

d''₄) 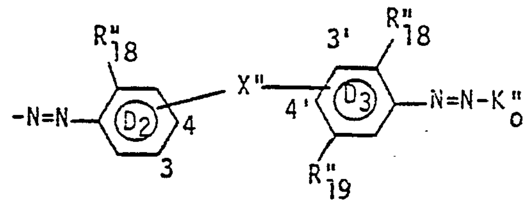

wobei der Rest X'' in 4,4'-, 3,4'- oder 3',4-Stellung gebunden ist, worin
$K_0''$ einen Rest der Formel

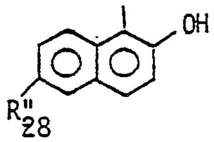 , 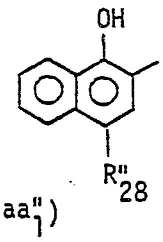 , 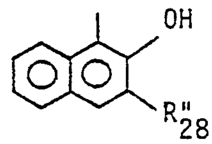

aa'')   $aa_1'')$   $aa_2'')$

A''' unabhängig voneinander

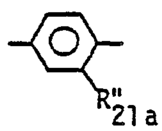 oder 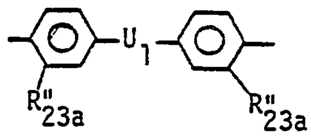

$R_{1b}'''$ falls x für 1 steht, einen Rest der Formel

e''') $-N=N-K_1'''$
f''') $-N=N-A'''-N=N-K_1'''$
g''') 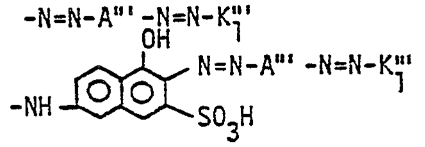

K''' einen Rest der Formel
$aa_4'')$ $B_3$, worin r sowohl für 1 oder 2 stehen kann,
$aa_5)$ ein Phenol,

$aa_6'')$ 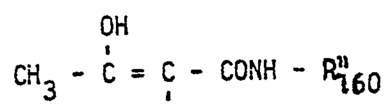

$aa_7'')$ 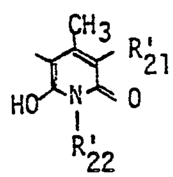

$aa_8'')$ 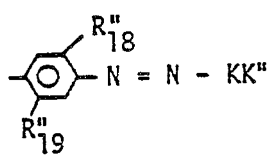

$aa_9'')$ 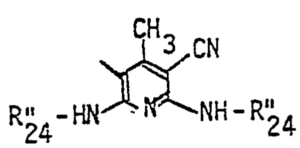

(Fortsetzung)

aa''10) 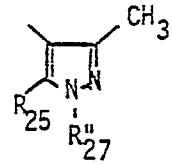

aa''11) 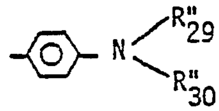

aa''12) 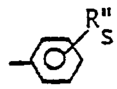 oder 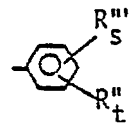

$R_0'''$ einen Rest der Formel

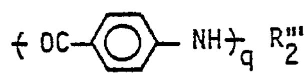 (Id)

bedeutet,

$R_2'''$ steht für einen Rest der Formel

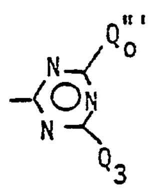 h'''1)

oder für $-CO-CH_2-Z_3$
oder für Wasserstoff, worin

$Q_3$ Chlor, OH, $-NH_2$, $-NHC_2H_4OH$, $-N(C_2H_4OH)_2$, $OCH_3$ oder $Q_0'''$ und

$Q_0'''$ 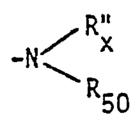

bedeuten, worin

$R_x''$ $\overset{*}{-}NHCOCH_2.-Z_3$, $-(CH_2)_{2-3}-Z_3$,

$-(CH_2)_{2-3}$ 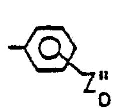 oder 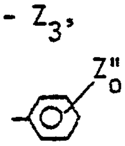

$R_x''$ und $R_{50}$ zusammen mit dem an sie gebundenen N-Atom einen Rest

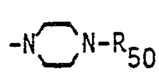 oder 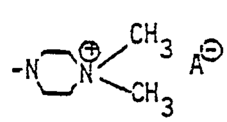

bilden können,

$Z_0''$ $-N(CH_3)_2$, $-\overset{\oplus}{N}(CH_3)_3\ A^{\ominus}$ , $-CONH(CH_2)_{2-3}-Z_3$,

$-NHCO(CH_2)_{2-3}-Z_3$, $-CO(CH_2)_{2-3}-Z_3$ oder $-SO_2NH(CH_2)_{2-3}-Z_3$,

$Z_3$ unabhängig voneinander

$-NH_2$, $-N(CH_3)_2$, $-N(C_2H_5)_2$

$-\overset{\oplus}{N}(CH_3)_3\ A^{\ominus}$, $-\overset{\oplus}{N}(C_2H_5)_3\ A^{\ominus}$, 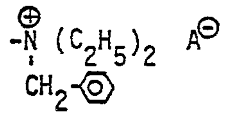

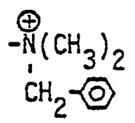 , 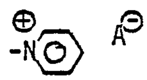 oder 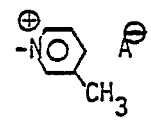

$F_0'''$ Wasserstoff, Chlor, Nitro, Methyl, Methoxy oder

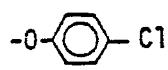

ein $F_4$ Nitro, Chlor oder Methoxy und das andere $F_4$ Nitro, Chlor oder Methyl oder beide $F_4$ Methyl oder Methoxy bedeuten,

X'', falls die Ringe $D_2$ und $D_3$ unsubstituiert sind, für $X_1$, $X_{11}$, $X_{17}$, $X_{27}$, $X_{51}$, $X_{52}$, $X_{54}$, $X_{64}$, $X_{70}$, $X_{71}$, $X_{72}$, $X_{73}$-$X_{77}$, $X_{79}$, $X_{80}$, $X_{82}$, $X_{85}$-$X_{80}$,

X'' falls die Ringe $D_2$ und $D_3$ substituiert sind oder falls $D_2$ unsubstituiert und $D_3$ substituiert sind durch Chlor, Methyl oder Methoxy, für die direkte Bindung $X_1$ oder $X_{40}$ steht, worin

$R_s''$ Methyl, Butyl oder Methoxy,

$R_t''$ Methyl,

$R_s'''$ Methyl oder Methoxy,

$R_{160}''$ einen Rest der Formel

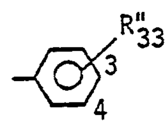

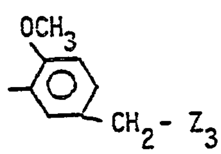 oder 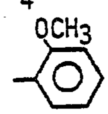

in 3- oder 4-Stellung,

$R_{18}''$ Wasserstoff, Chlor, Methyl oder Methoxy,

$R_{19}''$ Wasserstoff, Methyl oder Methoxy,

$R_{24}''$ unabhängig voneinander Wasserstoff oder $-(CH_2)_{2-3}-N(CH_3)_2$,

$R_{27}''$ Wasserstoff,

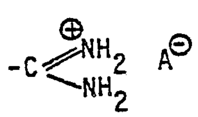 oder 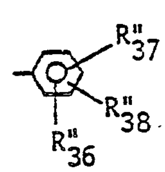

$R_{28}''$ 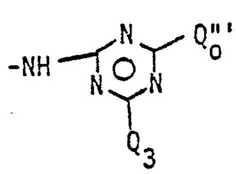

oder $-CONH(CH_2)_{2-3}-Z_3$,

$R_{29}''$ $CH_3$ oder $C_2H_5$,
$R_{30}''$ $—C_2H_4—Z_3$,
$R_{33}''$ Wasserstoff,

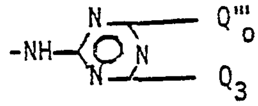

$—NHCO(CH_2)_{2\text{-}3}—Z_3$ oder $—SO_2NH(CH_2)_{2\text{-}3}—Z_3$ jeweils in 3- oder 4-Stellung,
$R_{36}''$ und $R_{37}''$ Wasserstoff oder Chlor,
$R_{38}''$ Wasserstoff,

$CONH(CH_2)_{2\text{-}3}-Z_3$,

$-NHCO(CH_2)_{2\text{-}3}-Z_3$ oder 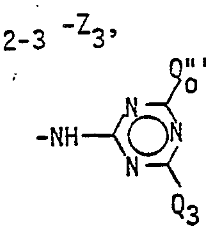

$R_{21a}''$ und $R_{23a}''$ unabhängig voneinander H, Cl, OH, Methyl oder Methoxy,
$R_{1a}'''$ Wasserstoff,

oder $-NH(CH_2)_3-NR''_{11}R''_{12}$ oder $-NH(CH_2)_3-\overset{\oplus}{N}R''_{13}R''_{14}R''_{15}\ A^{\ominus}$

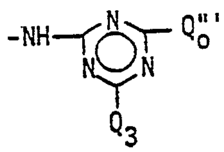

bedeuten,
$K_1'''$ einen Rest der Formel aa''), $aa_1''$), $aa_2''$), $aa_4''$), $aa_5''$), $aa_6''$), $aa_7''$), $aa_8''$), $aa_9''$), $aa_{10}''$), $aa_{11}''$), oder einen Rest der Formel

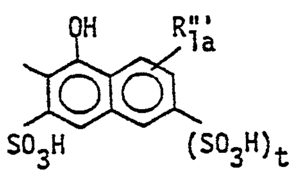

$aa''_{13}$)

worin vorteilhaft t = 0 und $R_{1a}'''$ in 6-Stellung steht,

oder 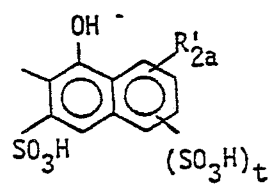

$aa''_{14}$)

KK'' einen Rest der Formel aa''), $aa_1''$), $aa_2''$), $aa_4''$), $aa_5$), $aa_6''$), $aa_7''$), $aa_9''$), $aa_{10}''$), $aa_{11}''$), $aa_{12}''$), $aa_{13}''$), $aa_{14}''$)
und $K_0'''$ einen Rest der Formel $aa_4''$), $aa_5''$), $aa_6''$), $aa_7''$), $aa_8''$), $aa_9''$), $aa_{10}''$), $aa_{11}''$) bedeuten,
Gemische der Verbindungen der Formel IV, deren Säureadditionssalze oder Salze von Säuren, sowie 1 : 1- und 1 : 2-Metallkomplexe davon, mit den zuvor genannten Massgaben unter α) und β) falls r für 2, S''' und $R_0'''$ für Wasserstoff stehen, ist $T_2$ verschieden von CN, $R_1''$ verschieden von 1,2-Aethylen und der Rest M''' von Sulfonsäuregruppen frei.

Besonders bevorzugte 1 : 1- und 1 : 2-Metallkomplexverbindungen entsprechen den Formeln

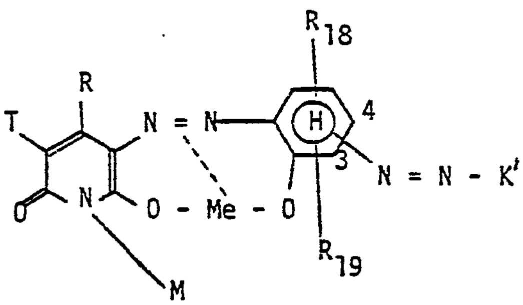

(V)

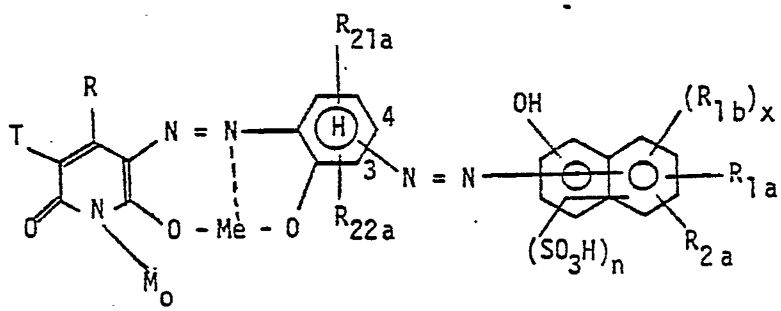

(VI)

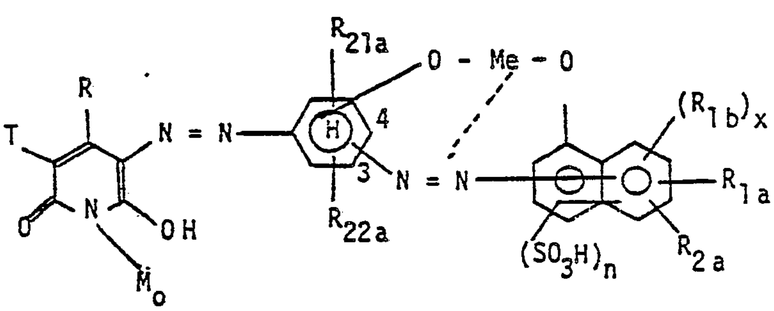

(VII)

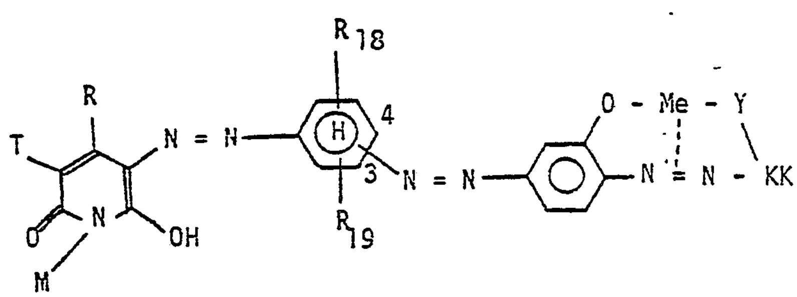

(VIII)

worin Y —O— oder —NH— bedeutet,

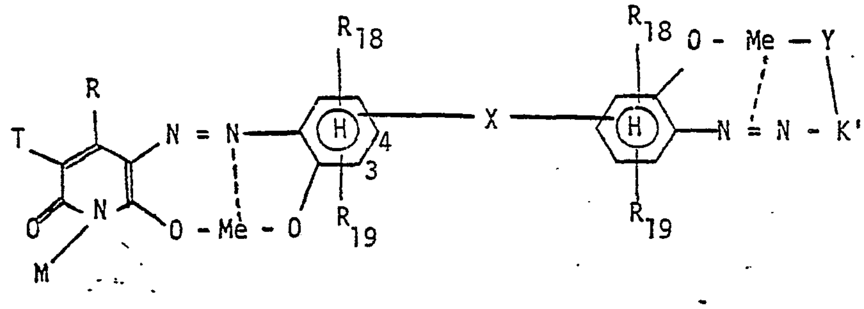

(IX)

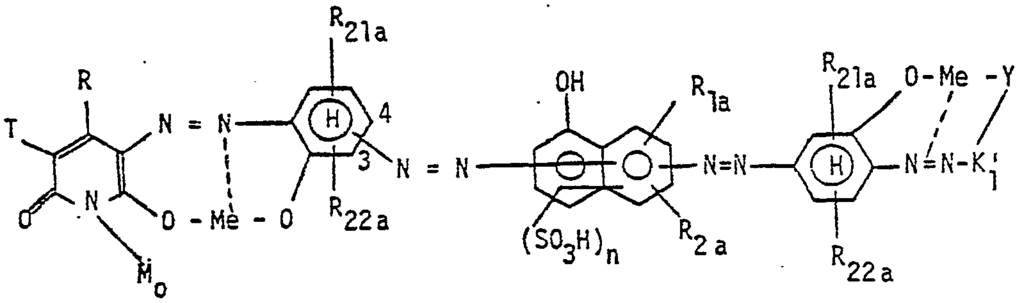

(X)

wobei die Azoreste in den Ringen H in 3- oder 4-Stellung gebunden sind, und

Me ein Kupfer-, Chrom-, Kobalt-, Nickel- oder Manganatom für die 1 : 1-Komplexe, vorteilhaft ein Kupferatom und

Me ein Chrom-, Kobalt- oder Eisenatom, vorteilhaft Eisenatom für die 1 : 2-Metallkomplexe bedeutet.

In den obigen Formeln steht B in zunehmender Bedeutung für $B_1$, besonders für $B_2$ und ganz besonders für $B_3$ ; S für S', bzw. für S'', bzw. für S''' ; R für R', bzw. für R'', bzw. für $CH_3$ ; T für $T_1$, bzw. für $T_2$ ; M für M', bzw. für M'', bzw. für M''' ; $M_0$ für $M_0'$, bzw. für $M_0''$, bzw. für $M_0'''$ ; $R_1$ für $R_1'$ bzw. für $R_1''$ ; $R_0$ für $R_0'$ ; bzw. für $R_0''$, bzw. für $R_0'''$ ; $V_1$ für $-(CH_2)-_{1\text{-}3}$, bzw. für $-(CH_2)-_{2\text{-}3}$ ; $V_2$ für $-(CH_2)-_{2\text{-}3}$, $R_3$ für $R_3'$ bzw. $R_3''$ ; $R_5$ und $R_6$ für $R_5'$ und $R_6'$ bzw. für $R_{50}$ ; $F_0$ für $F_0'$ bzw. für $F_0''$ bzw. für $F_0'''$ oder $F_4$ ; K für K', bzw. für K'' bzw. K''' oder $K_0'''$ ; $K_1$ für $K_1'$ bzw. für $K_1''$ bzw. für $K_1'''$ ; $K_0$ für $K_0'$ bzw. für $K_0''$ ; KK für KK' bzw. für KK'' ; A für A' bzw. A'' bzw. A''' ; $A_1$ für A', bzw. A'' bzw. A''' ; $W_0$ für $W_0'$ bzw. $W_0''$ bzw. —NH—, $R_{11}$ und $R_{12}$ für $R_{11}'$ und $R_{12}'$ bzw. $R_{11}''$ und $R_{12}''$ ; $R_{13}$, $R_{14}$ und $R_{15}$ für $R_{13}'$, $R_{14}'$ und $R_{15}'$ bzw. für $R_{13}''$, $R_{14}''$ und $R_{15}''$ ; $R_{1b}$ für $R_{1b}'$ bzw. $R_{1b}''$ bzw. $R_{1b}'''$ ; $R_{1a}$ für $R_{1a}'$, bzw. $R_{1a}''$ bzw. $R_{1a}'''$ ; $R_{2a}$ für $R_{2a}'$ ; Z für $Z_1$ bzw. $Z_2$ bzw. $Z_3$ ; Q für $Q_1$ bzw. $Q_2$ bzw. $Q_3$ ; $Q_0$ für $Q_0'$ bzw. $Q_0''$ bzw. $Q_0'''$ ; $R_v$ für $R_v'$ bzw. $R_v''$ bzw. Wasserstoff, $Q_{10}$ für $Q_{10}'$ bzw. $-(CH_2)-_{2\text{-}3}$, $Z_0$ für $Z_0'$ bzw. $Z_0''$ ; $R_{18}$ und $R_{19}$ für $R_{18}'$ und $R_{19}'$ bzw. $R_{18}''$ und $R_{19}''$ ; $R_{21a}$ für $R_{21a}'$ bzw. $R_{21a}''$ ; $R_{23a}$ für $R_{23}'$ bzw. $R_{23a}''$, U für $U_1$ bzw. $U_2$, X für X' bzw. X'' ; $R_{160}$ für $R_{160}'$ bzw. $R_{160}''$ ; $R_{17}$ für $CH_3$ ; $R_{20}$ für $R_{20}'$ bzw. $CH_3$ ; $R_{21}$ für $R_{21}'$ ; $R_{22}$ für $R_{22}'$ ; $R_{23}$ für $CH_3$ ; $R_{24}$ für $R_{24}'$ bzw. $R_{24}''$ ; $R_{26}$ für $CH_3$ ; $R_{27}$ für $R_{27}'$ bzw. $R_{27}''$ ; $R_{28}$ für $R_{28}'$ bzw. $R_{28}''$ ; $R_{29}$ und $R_{30}$ für $R_{29}'$ und $R_{30}'$ bzw. für $R_{29}''$ und $R_{30}''$ ; $R_s$ und $R_t$ für $R_s'$ und $R_t'$ bzw. $R_s''$ und $R_t''$ bzw. $R_s'''$ ; $R_x$ für $R_x'$ bzw. $R_x''$ ; V für V' bzw. $-(CH_2)-_{2\text{-}3}$, $V_0$ für $V_0'$ bzw. $-(CH_2)-_{2\text{-}3}$, Arylen für Phenylen oder Naphthylen, $R_{16}$ für $R_{161}$ bzw. für $CH_3$ ; $R_{42}$ für $R_{42a}$ ; $R_{43}$ für $-(CH_2)-_{1\text{-}4}$, $R_{44}$ für Wasserstoff oder $CH_3$, $R_{45}$ für $R_{45a}$ ; $R_{31}$ für $R_{31}'$ bzw. H, $R_{32}$ für $R_{32}'$ bzw. H, $R_{33}$ für $R_{33}'$ bzw. $R_{33}''$ ; $R_{34}$ für $R_{34}'$ bzw. für $OCH_3$, v für v' ; $R_{36}$ und $R_{37}$ für $R_{36}'$ und $R_{37}'$ bzw. $R_{36}''$ und $R_{37}''$ ; $R_{38}$ für $R_{38}'$ bzw. $R_{38}''$ ;

Alkyl steht, wo nicht spezielles erwähnt wird, hauptsächlich für (1-12C)-Alkyl, besonders für (1-6C)-Alkyl, ganz besonders für (1-4C)-Alkyl und insbesondere für Methyl oder Aethyl.

Alkoxy steht, wo nicht spezielles erwähnt wird, hauptsächlich für (1-4C)-Alkoxy, besonders für Methoxy oder Aethoxy. Halogen steht besonders für Chlor, Brom oder Fluor, besonders für Chlor.

Alkylen steht, wo nicht anders vermerkt, für (1-12C)- vorteilhaft (1-6C)- und hauptsächlich für (1-4C)-Alkylen.

Alkenylen steht hauptsächlich für 3-8C-, besonders für 3-6C-Alkenylen. Arylen steht besonders für Phenylen und Aryl für Phenyl.

Das Verfahren zur Herstellung einer Verbindung der Formel I, worin S verschieden von Wasserstoff ist, ist dadurch gekennzeichnet, dass man die Diazoverbindung aus einem Amin der Formel

DD—$NH_2$ (XI)

worin DD einen Rest

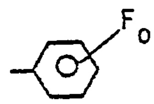

(XIa)

—A—N = N—K (XIb)

oder

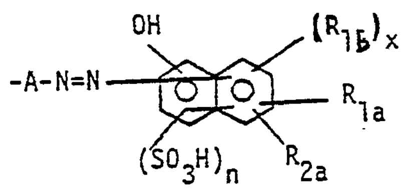

(XIc)

bedeutet, mit einer Verbindung der Formel

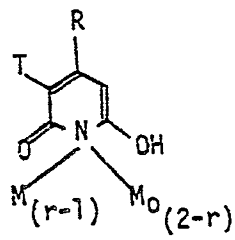

(XII)

kuppelt, und gewünschtenfalls die erhaltenen Verbindungen in die Metallkomplexe überführt.

Man kann aber auch die Diazoverbindung aus einer Aminoazoverbindung der Formel

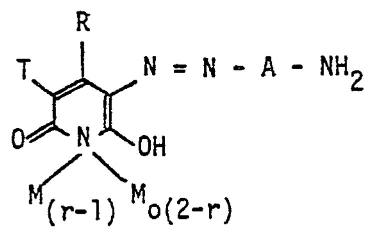

(XIII)

mit einer Verbindung der Formel

H—K (XIV)

oder

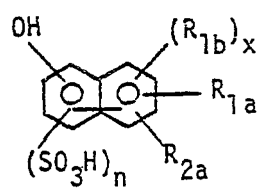

(XV)

kuppeln und gewünschtenfalls die erhaltenen Verbindungen in die Metallkomplexe überführen.

Ein weiteres Verfahren zur Herstellung von Verbindungen der Formel I, worin $R_2$ für einen Rest $h_3$) steht, ist dadurch gekennzeichnet, dass man eine Verbindung der Formel

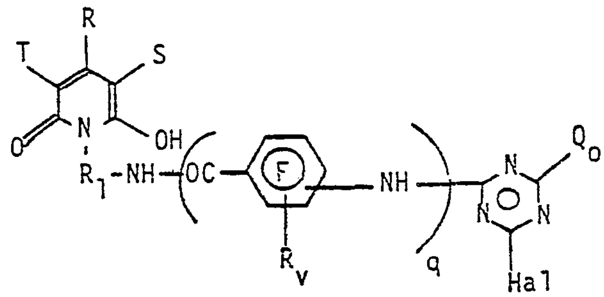

(XVIII)

worin Hal ein Halogenatom, vorzugsweise ein Chlor-, Brom- oder Fluoratom bedeutet, mit einer Verbindung der Formel

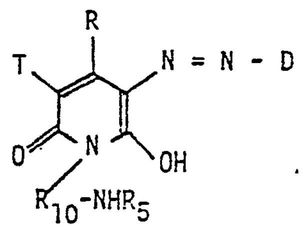

(XIX)

kondensiert und gewünschtenfalls die erhaltenen Verbindungen in die Metallkompexe überführt.

Ein weiteres Verfahren zur Herstellung von Verbindungen der Formel I, worin $R_2$ für einen Rest der Formel $h_1$) und $h_2$) steht, ist dadurch gekennzeichnet, dass man eine Verbindung der Formel

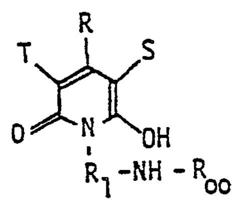

(XX)

worin $R_{00}$ Wasserstoff oder einen Rest

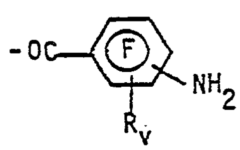

(XXa)

bedeutet, mit einer Verbindung der Formel

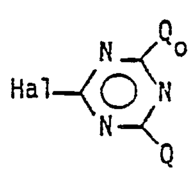

XXI

oder

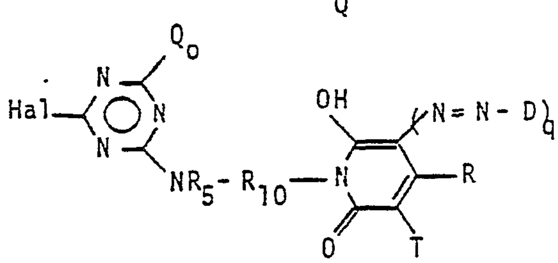

XXII

umsetzt, oder für den Fall, dass $R_2$ für den Rest $h_4$) steht, eine Verbindung der Formel

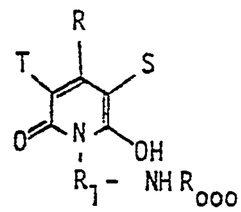

XXIII

worin $R_{000}$ eine Verbindung der Formel

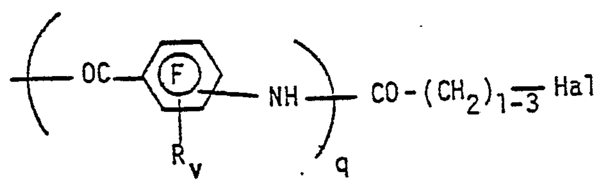

bedeutet, mit einer Verbindung der Formel

$HNR_{11}R_{12}$ XXIV

oder

$NR_{13}R_{14}R_{15}$ XXV

umsetzt, und gewünschtenfalls die erhaltenen Verbindungen in die Metallkomplexe überführt.

Das Verfahren zur Herstellung von Verbindungen der Formel I, worin S für Wasserstoff steht, ist dadurch gekennzeichnet, dass man eine Verbindung der Formel

$T—CH_2—CO—NH—R_1—NH—M_{00}$ XXVI,

worin $M_{00}$ eine beliebige Schutzgruppe bedeutet, vorteilhaft, dass man eine Verbindung der Formel

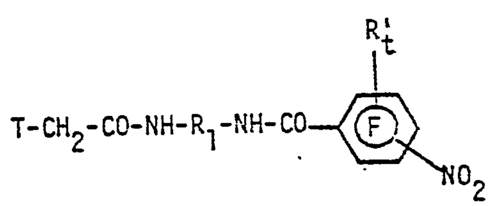

XXVII

worin $R_t'$ Wasserstoff, Halogen, (1-4C)-Alkyl oder (1-4C)-Alkoxy bedeutet, mit einem Essigsäurealkylester der Formel

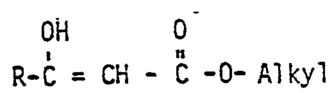

XXVIII

nach an sich bekannten Methoden zu einer Verbindung der Formel

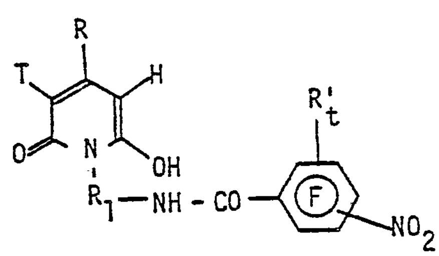

XXIX

cyclisiert, und für Verbindungen, worin $R_0$ Wasserstoff bedeutet, diese Verbindung verseift, oder für Verbindungen, worin $R_{00}$ einen Rest der Formel

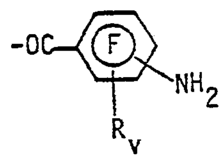

XXa

bedeutet, die Nitrogruppe zur Aminogruppe reduziert.

Das Verfahren zur Herstellung von Verbindungen der Formel I, worin S von Wasserstoff verschieden ist, ist dadurch gekennzeichnet, dass man die Diazoverbindung aus einem Amin der Formel XIII

α) auf eine Verbindung der Formel

$$H—K_x \qquad XXXI$$

worin $K_x$ einen Rest der Formel

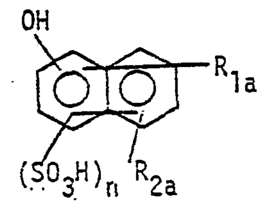

oder

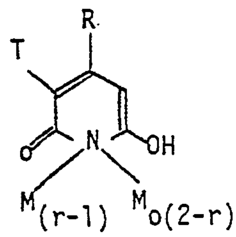

XXXII XXXIII

bedeutet, kuppelt, gegebenenfalls in der so erhaltenen Verbindung der Formel I, worin $R_{1a}$ für Wasserstoff steht, und die im Rest der Verbindung der Formel XXXII eine freie Aminogruppe enthalten, diese Aminogruppe diazotiert und die Diazoniumverbindung auf eine Verbindung der Formel

$$H—K_1 \; XXXIV \quad oder \quad H—A_1—N = N—K_1 \qquad XXXV$$

kuppelt, oder gegebenenfalls die so erhaltene Verbindung der Formel I, worin $R_{1a}$ für Wasserstoff steht, mit der Diazoniumverbindung der Aminoverbindung

$$K_1—NH_2 \qquad XXXVI$$

oder

$$K_1—N = N—A—NH_2 \qquad XXXVII$$

kuppelt, oder

β) gegebenenfalls im Gemisch mit der Diazoverbindung des Amins der Formel

$$K_1—N = N—A_1—NH_2 \qquad XXXVIII$$

auf die Verbindung der Formel

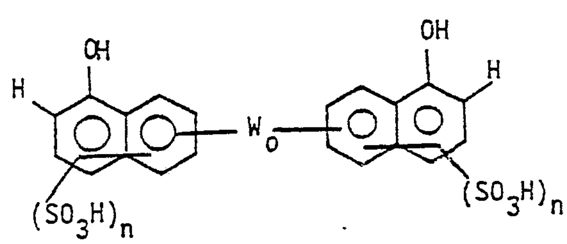

XXXIX

kuppelt und gewünschtenfalls die erhaltenen Verbindungen der Formel I in die 1 : 1- oder 1 : 2-Metallkomplexe überführt.

Das Verfahren zur Herstellung von Verbindungen der Formel XXII, worin q = 1 ist, ist dadurch gekennzeichnet, dass man die Diazoverbindung aus einem Amin der Formel

$$D—NH_2 \qquad XL$$

worin D eine beliebige Diazoverbindung bedeutet, mit einer Verbindung der Formel

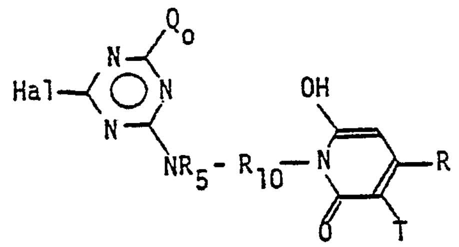

XLI

kuppelt.

Es können solche Azoverbindungen in die Metallkomplexform übergeführt werden, die in ortho, ortho'-Stellung zu je einer —N = N-Gruppe eine zur Metallkomplexbildung befähigende Gruppe tragen, beispielsweise eine OH, $NH_2$ oder Methoxygruppe, d. h. die OH-Gruppe des Pyridonrestes B und eine OH- oder Methoxygruppe in einem der Ringe $D_1$, $D_2$ oder $D_3$, oder eine OH- oder $NH_2$-Gruppe in der Kupplungskomponente XII, XIV, XV, XVII, XXXI, XXXIV, XXXV, XXXIX, XLI und eine OH- oder Methoxygruppe in einem der Ringe $D_1$, $D_2$ oder $D_3$ bilden zusammen eine Gruppe —NH—Me—O— oder —O—Me—O—, worin Me für ein Metallatom steht, das entweder einen 1 : 1-Metallkomplex oder einen 1 : 2-Metallkomplex oder einen 1 : 1 und einen 1 : 2-Metallkomplex bilden kann. 1 : 1-Metallkomplexe bilden hauptsächlich Kupfer-, Chrom-, Kobalt-, Eisen-, Nickel- oder Mangansalze, hauptsächlich Kupfer-, Chrom- oder Kobalt- und insbesondere Kupfersalze. 1 : 2-Metallkomplexe bilden Chrom-, Kobalt-, Eisen- oder Nickelsalze, hauptsächlich, Chrom-, Kobalt- oder Eisen- und insbesondere Eisensalze. 1 : 1-Metallkomplexe werden durch Umsetzen von 1 Mol einer Verbindung der Formel I mit einer ein Aequivalent metallabgebender Verbindung nach bekannten Methoden erhalten. 1 : 2-Metallkomplexe werden durch Umsetzen von 2 Mol einer Verbindung der Formel I mit einer ein Aequivalent metallabgebender Verbindung nach bekannten Methoden erhalten oder durch Umsetzen von 1 Mol einer 1 : 1-Metallkomplexverbindung der Formel I mit 1 Mol einer metallfreien Verbindung der Formel I oder mit 1 Mol einer beliebigen anderen metallisierbaren Azoverbindung.

Man kann 1 : 2-Metallkomplexe auch durch Umsetzen von 1 Mol einer ein Aequivalent metallabgebender Verbindung mit 1 Mol einer metallfreien Verbindung der Formel I und 1 Mol einer beliebigen anderen metallisierbaren Azoverbindung erhalten.

Anstelle des Amins der Formel XIII (Erstkupplung auf Pyridon) kann auch das entsprechende Amin der Formel XIIIb eingesetzt werden, welches hergestellt wird durch Erstkupplung auf das Naphtholderivat XV ; die Zweitkupplung erfolgt dann auf die Pyridonkomponente XII.

Diazotierungs- und Kupplungsreaktionen werden analog zu an sich bekannten Methoden durchgeführt. Auch Kondensations- und Cyclisierungsreaktionen werden nach an sich bekannten Methoden durchgeführt, z. B. kann eine Verbindung aus XXVII und XXVIII bei einer Temperatur von etwa 20-40° unter Verwendung einer Base (wie NaOH) und eines Acylessigsäurealkylester, zu einer neuen Verbindung XXIX cyclisiert werden. Auch die Verseifung kann nach an für sich bekannten Methoden durchgeführt werden, z. B. bei erhöhten Temperaturen im sauren oder alkalischen Medium.

Verbindungen der Formel I, welche orthoständig zu einer Azogruppe metallisierbare Gruppen enthalten, können in die Metallkomplexe übergeführt werden, indem man die mindestens einem Aequivalent Metall entsprechende Menge einer metallabgebenden Verbindung (bezogen auf ein Aequivalent Azoverbindung) einwirken lässt.

Die dabei bevorzugte 1 : 1-Metallisierung wird nach an sich bekannten Methoden durchgeführt ; für die bevorzugte Herstellung von Kupferkomplexen wendet man zweckmässig entweder die oxidative Kupferung vorzugsweise in einem Temperaturbereich von 40-70 °C und bei pH 4-7 in Gegenwart von Cu(II)-Salzen oder mit Kupferpulver in Gegenwart von Wasserstoffperoxid oder einem anderen üblichen Oxidationsmittel an oder bevorzugt die entmethylierende Kupferung vorzugsweise in einem pH-Bereich von 3-4 bei erhöhter bis Kochtemperatur.

Die erhaltenen Verbindungen der Formel I werden auf an sich bekannte Weise aus dem Reaktionsgemisch isoliert.

Die basische Gruppen tragenden Verbindungen der Formel I können in Wasserlösliche Salze übergeführt werden, indem man sie mit mindestens stöchiometrischen Mengen einer anorganischen Mineralsäure, beispielsweise Salzsäure, Schwefelsäure oder Phosphorsäure, oder vorzugsweise einer

organischen Säure, beispielsweise Ameisensäure, Essigsäure, Milchsäure, Citronensäure, Glykolsäure oder Methansulfonsäure, behandelt.

Die als Ausgangsmaterial verwendeten Verbindungen der Formel XIII oder XIIIb können, soweit sie bekannt bzw. nicht bekannt sind, hergestellt werden, indem man eine den Rest A enthaltende Tetrazokomponente einseitig kuppelt, wobei Voraussetzung ist, dass A so beschaffen ist, dass eine asymmetrische Kupplung erfolgen kann.

Andernfalls muss anstelle der Tetrazokomponente die entsprechende Nitro-amino- oder auch Acetylamino-Verbindung verwendet werden, in welcher nach erfolgter erster Kupplung die Nitrogruppe reduziert oder die Acetylaminogruppe verseift wird.

Verbindungen der Formel XXXII sind aus der DE-OS-2 915 323 bekannt oder können analog zu dem dort beschriebenen Verfahren hergestellt werden. Verbindungen der Formel XXXIX sind bekannt oder sie können analog zu an sich bekannten Kondensationsreaktionen hergestellt werden.

Die Verbindungen der Formeln XXXIV und XXXVI sind bekannt.

Die Verbindungen der Formel XXXV und XXXVII sind bekannt oder sie können analog zu an sich üblicher Diazotierungs- und Kupplungsreaktionen hergestellt werden.

In den Verbindungen lässt sich das Anion $A^{\ominus}$ durch andere Anionen austauschen, z. B. mit Hilfe eines Ionenaustauschers oder durch Umsetzen mit Salzen oder Säuren, gegebenenfalls in mehreren Stufen, z. B. über das Hydroxid oder über das Bicarbonat oder gemäss den deutschen Offenlegungsschriften 2 001 748 oder 2 001 816.

Als Anion $A^{\ominus}$ kommen die in der basischen Farbstoffchemie üblichen in Frage, hauptsächlich eignen sich farblose Anionen.

Unter Anion $A^{\ominus}$ sind sowohl organische wie anorganische Ionen zu verstehen, wie z. B. Halogen-, wie Chlorid-, oder Bromid-, ferner Sulfat-, Bisulfat-, Methylsulfat-, Aminosulfonat-, Perchlorat-, Benzolsulfonat-, Oxalat-, Maleinat-, Acetat-, Propionat-, Lactat-, Succinat-, Tartrat-, Malat-, Methansulfonat- oder Benzoationen oder komplexe Anionen, wie das von Chlorzinkdoppelsalzen, ferner die Anionen der folgenden Säuren : Borsäure, Citronensäure, Glykolsäure, Diglykolsäure oder Adipinsäure oder Additionsprodukte von ortho-Borsäure mit Polyalkoholen wie z. B. cis-Polyolen.

Die Verbindungen der Formel I, worin S für Wasserstoff steht, sind zum Teil neue Zwischenprodukte und können zur Herstellung von Farbstoffen, insbesondere Azofarbstoffen verwendet werden.

Die Verbindungen der Formel I in Form ihrer wasserlöslichen Säureadditionssalze oder als quaternäre Ammoniumsalze stellen Farbstoffe dar ; sie dienen zum Färben oder Bedrucken von Fasern, Fäden oder daraus hergestellten Textilien, die aus Cellulosematerial, z. B. Baumwolle, bestehen oder diese enthalten, nach an sich bekannten Methoden ; Baumwolle wird dabei vorzugsweise nach dem Ausziehverfahren gefärbt, beispielsweise aus langer oder kurzer Flotte und bei Raum- bis Kochtemperatur.

Das Bedrucken erfolgt durch Imprägnieren mit einer Druckpaste, welche nach an sich bekannter Methode zusammengestellt wird.

Insbesondere eignen sich die Verbindungen der Formel I (in Form ihrer wasserlöslichen Säureadditionssalze oder als quaternäre Ammoniumsalze) jedoch zum Färben oder Bedrucken von Papier, z. B. für die Herstellung von geleimtem oder ungeleimtem, holzfreiem oder halzhaltigem Papier in der Masse wie in der Leimpresse. Sie können aber auch zum Färben von Papier nach dem Tauchverfahren verwendet werden. Das Färben und Bedrucken von Papier erfolgt nach bekannten Methoden.

Die neuen Farbstoffe können weiter auch zum Färben oder Bedrucken von Leder, insbesondere von niederaffinen Lederarten, die vegetabil nachgegerbt wurden, nach an sich bekannten Methoden verwendet werden. Die Verbindungen dienen auch zum Färben oder Bedrucken von Bastfasern, wie Hanf, Flachs, Sissal, Jute, Kokos oder Stroh.

Die dabei erhaltenen Färbungen und Drucke (insbesondere die auf Papier) zeigen gute Gebrauchsechtheiten.

Die Verbindungen der Formel I können auch in Form von Färbepräparaten eingesetzt werden. Die Verarbeitung in stabile flüssige, vorzugsweise wässrige Färbepräparate kann auf allgemein bekannte Weise erfolgen, vorteilhaft durch Lösen in geeigneten Lösungsmitteln, gegebenenfalls unter Zugabe eines Hilfsmittels, z. B. eines Stabilisators ; beispielsweise können solche Präparationen wie in der französischen Patentschrift Nr. 1 572 030 beschrieben hergestellt werden.

Eine günstige Zusammensetzung solcher flüssigen Präparate ist beispielsweise die folgende (Teile bedeuten Gewichtsteile) :

100 Teile einer Verbindung der Formel I als Säureadditionssalz oder als quaternäres Ammoniumsalz,
1-100, vorzugsweise 1-10 Teile eines anorganischen Salzes,
1-100 Teile einer organischen Säure wie Ameisen-, Essig-, Milch-, Citronensäure,
100-800 Teile Wasser,
0-500 Teile eines Lösungsvermittlers (z. B. Glykole wie Diäthylenglykol, Triäthylenglykol, Hexylenglykol ; Glykoläther wie Methylcellosolve, Methylcarbitol, Butylpolyglykol ; Harnstoff, Formamid, Dimethylformamid).

Ebenso können die Verbindungen der Formel I auf an sich bekannte Weise zu festen, bevorzugt granulierten Färbepräparaten verarbeitet werden, vorteilhaft durch Granulieren wie in der französischen Patentschrift Nr. 1 581 900 beschrieben.

Eine günstige Zusammensetzung für feste Präparate ist beispielsweise die folgende (Teile bedeuten Gewichtsteile) :

100 Teile einer Verbindung der Formel I als Säureadditionssalz oder als quaternäres Ammoniumsalz,
1-100, vorzugsweise 1-10 Teile eines anorganischen Salzes,
0-800 Teile eines Stellmittels (vorzugsweise nichtionogen wie Dextrin, Zucker, Traubenzucker oder Harnstoff).

In der festen Präparation kann noch bis zu 10 % an Restfeuchtigkeit vorhanden sein.

Die Farbstoffe der Formel I (als Säureadditionssalz oder quaternäres Ammoniumsalz) besitzen gute Löslichkeitseigenschaften, insbesondere zeichnen sie sich durch gute Kaltwasserlöslichkeit aus. Da die Farbstoffe hochsubstantiv sind, färben sie die Abwässer bei der Herstellung von geleimtem wie auch ungeleimtem Papier praktisch gar nicht oder nur geringfügig an. Sie melieren auf Papier gefärbt nicht und sind weitgehend unempfindlich gegen Füllstoff und pH-Schwankungen. Die Färbungen auf Papier sind brillant und zeichnen sich durch bemerkenswerte Lichtechtheit aus ; nach längerem Belichten ändert sich die Nuance Ton-in-Ton. Die gefärbten Papiere sind sehr gut nassecht nicht nur gegen Wasser, sondern auch gegen Milch, Fruchtsäfte, gesüsste Mineralwasser, Seifen- und Kochsalzlösung ; zudem besitzen sie gute Alkoholechtheit.

Die Farbstoffe haben hohe Substantivität, d. h. sie ziehen praktisch quantitativ auf und zeigen dabei ein gutes Aufbauvermögen ; sie können in der Papiermasse direkt, d. h. ohne bisheriges Auflösen, als Trockenpulver oder Granulat zugesetzt werden, ohne dass eine Minderung in der Brillanz oder Verminderung in der Farbstoffausbeute eintritt. Die geleimte Papierfärbung zeigt gegenüber der ungeleimten keinen Stärkeabfall. Mit den erfindungsgemässen Farbstoffen kann auch in Weichwasser mit voller Farbausbeute gefärbt werden.

Faserstoffe, die Holzschliff enthalten, werden mit den Farbstoffen der vorliegenden Erfindung in guter und egaler Qualität gefärbt.

Weiterhin können die Farbstoffe für die Herstellung von gestrichenem Papier Verwendung finden. Aufgrund ihrer Neigung zur Pigmentierung wird ein verwendbares Füllmittel, z. B. Kaolin, mit den erfindungsgemässen Verbindungen angefärbt und mit dieser Masse dem Papier ein einseitiger Oberflächenanstrich gegeben. Die gefärbten Papiere sind sowohl oxidativ als auch reduktiv bleichbar, was für die Wiederverwendung von Ausschuss- und Altpapier von Wichtigkeit ist.

Die europäische Auslegeschrift 41040 beschreibt sulfonsäuregruppenfreie Azokomponenten in metallfreier, 1 : 1 und 1 : 2-Metallkomplexform, welche im Mittel mindestens 1,3 und vorteilhaft 2 wasserlöslichmachende basische Gruppen enthalten und der Formel

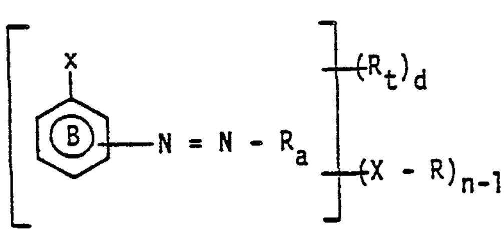

(I)

entsprechen, worin

$R_a$ einen Rest der Formel Ia-Ic

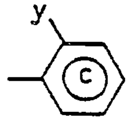

I,

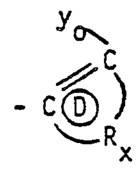

(Ib) oder

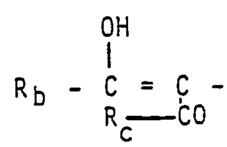

(Ic)

die Gruppe Ib) den Rest eines substituierten Pyrazolons-5, eines substituierten 5-Aminopyrazols, eines substituierten 4-Alkyl-2-pyridons oder eines substituierten β-Hydroxynaphthalins,

$y_0$ OH oder $NH_2$,

$R_b$ eine (1-4C)-Alkylgruppe oder eine substituierte (1-3C)-Alkylgruppe,

$R_c$ —$NH_2$, eine substituierte Alkylgruppe, eine substituierte Alkylaminogruppe, eine substituierte Phenylaminogruppe, eine unsubstituierte oder substituierte Naphthylaminogruppe, eine unsubstituierte oder substituierte Benzthiazolaminogruppe, eine unsubstituierte oder eine substituierte Benzoxazolaminogruppe, eine unsubstituierte oder eine substituierte Benzimidazolaminogruppe,

x und y unabhängig voneinander Wasserstoff OH, (1-4C)-Alkyl, —$NH_2$ oder—COOH oder

a) x und y bilden zusammen eine Gruppe —NH—Me—O—, —NH—Me—OOC—, —O—Me—O—, —O—Me—OOC— oder —NH—Me—NH— oder

b) x und $y_0$ bilden zusammen eine Gruppe —NH—Me—O—, —NH—Me—NH— oder —O—Me—O—,

Me ein Metallatom, das entweder einen 1 : 1- oder 1 : 2-Metallkomplex oder einen 1 : 1 und einen 1 : 2-Metallkomplex bilden kann,

n 1 oder 2,

d 0, 1 oder 2,

$R_t$ eine oder mehrere der Gruppen Id bis Ig

$$-N=N-D-[W-(Z)_a]_b \quad Id$$

$$-N=N-B_1-N=N-K \quad Ie$$

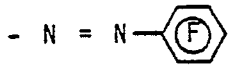 If

$$-Y-Z \quad Ig$$

D den Rest einer Diazokomponente,

W die direkte Bindung oder ein Brückenglied,

$B_1$ Phenylen, Naphthylen, Tetrahydronaphthylen oder eine Gruppe der Formel

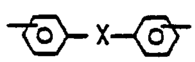

Z eine basische Amino- oder eine quaternäre Ammoniumgruppe,

a 1 oder 2,

b eine Zahl zwischen 1 und 2,

K eine Kupplungskomponente der 4-Alkyl-2-pyridon-, β-Hydroxynaphthylin-, Anilin-, Pyrazolon-5- oder Acetoacylreihe,

wobei die Kupplungskomponente durch wenigstens eine wasserlöslich machende basische Gruppe substituiert ist,

Y die direkte Bindung oder ein Brückenglied,

X die direkte Bindung oder ein Brückenglied,

R eine Gruppe der Formel

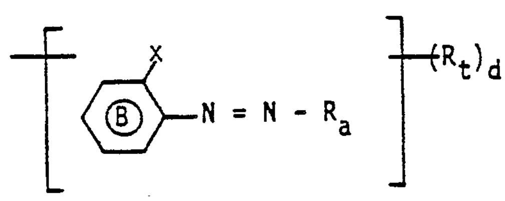 Ih

jeder der Ringe, B, C und D unabhängig voneinander durch bis zu zwei weitere Substituenten bis zu einem Total von 3 Substituenten substituiert sein kann, und der Ring F bis zu drei Substituenten substituiert sein kann, mit der Massgabe, dass

1) wenn $R_a$ die Gruppe Ic bedeutet, $R_t$ die Gruppen $I_g$ ist,

2) wenn $R_t$ die Gruppen $I_g$, $R_a$ für die Gruppen Ia steht und wenn n = 2 ist, die Gruppe —X—R an die Gruppe $R_a$ oder $R_t$ gebunden ist,

3) wenn n = 2 und $R_a$ die Gruppe der Formel Ia ist, x und z nicht für Wasserstoff oder (1-4C)-Alkoxy stehen

und $A^\ominus$ ein nicht-chromophores Anion bedeuten.

Die französische Patentschrift 2 383 997, äquivalent mit der deutschen Offenlegungsschrift 2 809 601 beschreibt.

Salze von sulfonsäuregruppenfreien asymmetrischen Dis- und Tris-azoverbindungen, die eine, zwei oder drei Ammonium Gruppen, als eine Endkupplungskomponente T den Rest eines in 5-Stellung kuppelnden 4-Methyl-6-hydroxy-3-N-pyridinium- oder 4-Methyl-6-hydroxy-3-N-hydroxy-(1-4C)-alkylpyridinium- oder 4-Methyl-6-hydroxy-3-N-(1-4C)-alkoxy-pyridinium-pyridons-2, worin das Pyridon-N-atom Wasserstoff trägt oder durch einen gegebenenfalls durch eine OH-Gruppe substituierten (1-4C)-Alkylrest oder durch eine tertiäre Aminogruppe substituiert ist, oder über einen (1-4C)-Alkylenrest an die Dimethylamino- oder an die Trimethylammonium- oder an die Dimethyl-2-hydroxyäthylammonium- oder an die Pyridiniumgruppe gebunden ist, und als andere Endkupplungskomponente $T_1$ den Rest eines Pyrazolon-5, eines 5-Amino-pyrazols, eines β-Naphthols, eines α-Naphthols-, eines Anilins, eines Phenols, eines Acetoacetylphenylamids oder den Rest von Barbitursäure oder von Dimedon oder eines Dimedoncarbonsäureesters enthalten und als Mittelkomponente den Rest einer tetrazotierbaren Diaminoverbindung der Diphenylreihe aufweist, worin die Diphenylreste direkt oder über ein in der Bis- oder Trisazochemie, d. h. Direktfarbstoffchemie, übliches zweiwertiges Brückenglied Z miteinander verbunden sind und eine weitere Dimethylamino-, Trimethylammonium-, Dimethyl-2-hydroxyäthylammonium- oder Pyridiniumgruppe über ein zweiwertiges Brückenglied B an die Reste der Kupplungskomponenten $T_1$ gebunden sein kann, oder eine quaternäre Ammoniumgruppe über ein C-Atom allein an das 1-N-Atom von 3-Methylpyrazolon-5 als einen Rest der Kupplungskomponente $T_1$ gebunden ist, und das quaternäre N-Atom an ein C-Atom des zweiwertigen Brückengliedes B gebunden ist.

Die deutsche Offenlegungsschrift 2 752 282 betrifft Verbindungen der 3-Pyridinium- oder im wesentlichen 3-Cyan-Pyridonreihe, die eine mindestens 5C-Atome aufweisende Alkylkette tragen. Ein solcher Alkylrest kann in der Diazokomponente als Esterrest einer Carbonsäure oder als Alkylrest einer Alkyl-Sulfonsäureamidgruppe oder als Aetherrest oder Alkyl-Ketonrest, oder als Alkylrest eines Alkyl-Phenylätherrestes, Alkyl-Diphenylmethanrestes, Carbonylalkylphenylrestes, Alkylphenylcarbonsäurerestes, Alkylcarbonamidrestes, Alkylphenylsulfonsäureamidrestes, eines Alkylcarbonylaminorestes, Alkylacylaminorestes, eines Alkylharnstoffrestes, Alkylsulfonylaminorestes sein oder auch in der Kupplungskomponente als Alkylrest am N-Pyridinium-Pyridon oder als Alkylrest einer quaternären Ammoniumgruppe, welche einerseits über ein Brückenglied am 3-Cyan-N-Pyridon gebunden ist oder über ein Brückenglied an einer Anilin-Diazokomponente gebunden ist.

In den folgenden Beispielen bedeuten die Teile Gewichts- bzw. Volumenteile ; die Temperaturen sind in Celsiusgraden angegeben.

Beispiel 1

a) 390 Teile (2 Mol) para-Nitrobenzoesäureäthylester werden in 3 200 Teilen Aethanol mit 360 Teilen (6 Mol) Aethylendiamin auf 60-65° erwärmt. Es entsteht nach mehrstündigem Rühren ein feiner, kristalliner Niederschlag, der abfiltriert und verworfen wird. Das Filtrat wird unter Vakuum eingedampft. Man erhält etwa 360 Teile der Verbindung der Formel

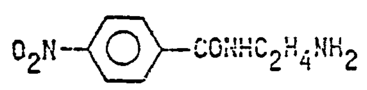

ab)

mit einem Rohschmelzpunkt von 132-134 °C.

b) Nach bekannten Methoden wird ein geringer Ueberschuss von Pyridin mit 1 Mol Chloressigsäuremethylester quaterniert und in Aethanol bei einer Temperatur von 55° zusammen mit der Verbindung ab) in die Verbindung der Formel

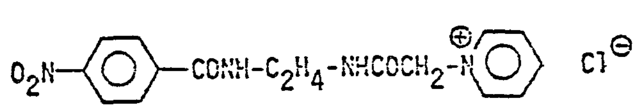

ac)

umgesetzt,

c) Ohne die Verbindung ac) zu isolieren wird Acetessigsäuremethylester zum Reaktionsgemisch gegeben und nach bekannten Methoden bei etwa 25° unter Zugabe von Lauge zum Pyridon der Formel

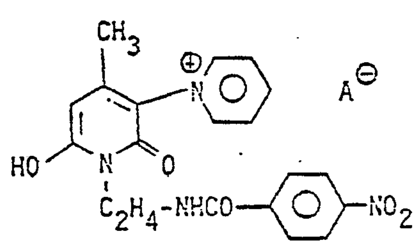

ad)

zyklisiert. Nach Zugabe von Wasser zum Reaktionsgemisch fällt die Verbindung ad) aus und wird abfiltriert.

d) Die Verbindung ad) wird bei 95-98° in etwa 5-7 %iger Salzsäurelösung zum Amin der Formel

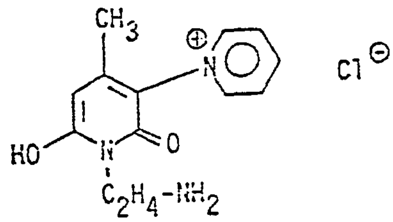

ae)

verseift.

e) Die Verbindung ae) wird nach bekannten Methoden in Wasser bei etwa 90° und einem pH-Wert von 9 mit einer Verbindung der Formel

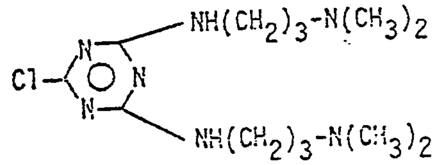

af)

zu einer Verbindung der Formel

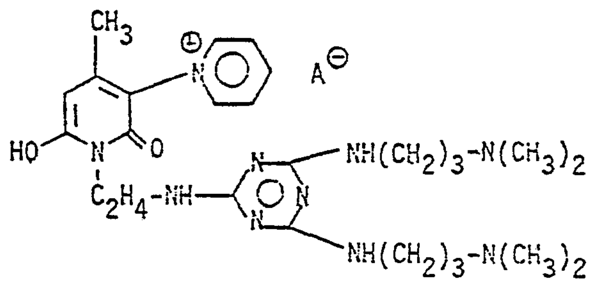

ag)

umgesetzt. Diese Verbindung ag) ist sehr gut wasserlöslich, insbesondere in saurem Medium und kann ohne Isolierung als Kupplungskomponente für die Herstellung von Azofarbstoffen eingesetzt werden.

Beispiel 2

a) Verwendet man anstelle des im Beispiels 1a) genannten Aethylendiamins eine äquivalente Menge (6 Mol = 444 Teile) 1,3-Diaminopropan, so erhält man als Zwischenverbindung

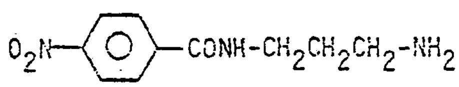

ah)

mit einem Rohschmelzpunkt von 130-132°. Nach den unter 1b) bis 1d) genannten Reaktionsverfahren unter Verwendung der dort genannten Verbindungen und ah) erhält man das Pyridon der Formel

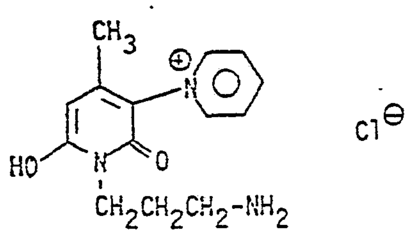

ai)

b) Nach bekannten Methoden setzt man die Verbindung ai) in Wasser bei 35-40° und einem pH-Wert von 9-10 mit Chloressigsäurechlorid zum Pyridon der Formel

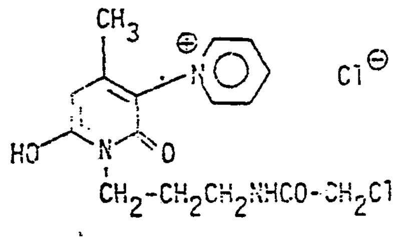

ak)

um.

c) Nach bekannten Methoden setzt man die Verbindung ak) in Wasser bei 35° und einem pH-Wert von 9 mit dem Trimethylamin $N(CH_3)_3$ um und erhält das Pyridon

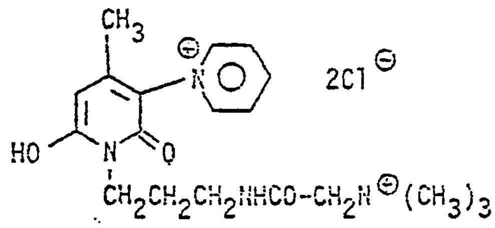

al)

welche ohne Isolierung als Kupplungskomponente zur Herstellung von Azofarbstoffen verwendet werden kann.

Beispiel 3

a) In einer mit Natriumacetat versetzten Mischung aus gleichen Volumenteilen Eisessig und Wasser werden 225 Teile meta-Amino-acetanilid in Form des salzsauren Salzes (1,5 Mol) vorgelegt und unter Aussenkühlung bei 0-5° in 2 Stunden mit 254 Teilen Chloracetylchlorid (2,25 Mol) versetzt.

Das ausgeschiedene Produkt der Formel

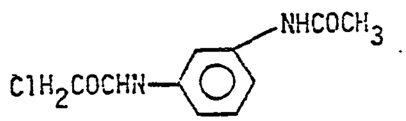

am)

wird abfiltriert und weiter verarbeitet.

b) Die Verbindung am) wird nach bekannten Methoden mit Pyridin zur Verbindung der Formel

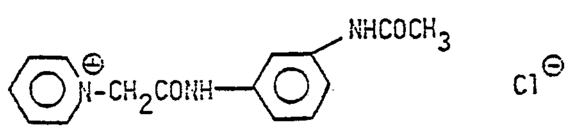

an)

quaterniert.

c) Ohne Isolierung der Verbindung an) wird dem Reaktionsgemisch Acetessigsäuremethylester zugegeben und nach bekannten Methode bei Raumtemperatur mit Lauge zum Pyridon der Formel

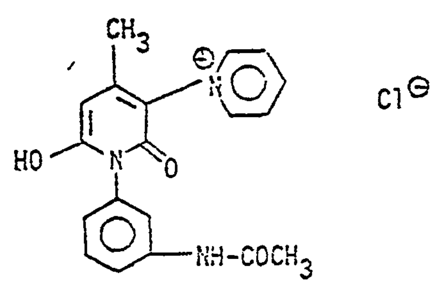

ao)

zyklisiert.

d) Bei 95-98° wird die Verbindung ao) mit ca. 5 %iger Salzsäure zur Verbindung

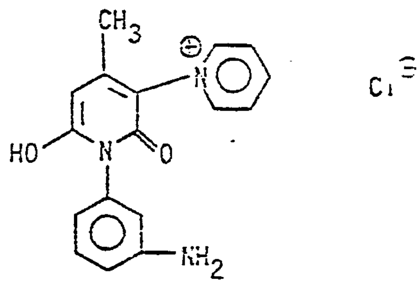

aq)

verseift.

e) Die Verbindung aq) wird nach bekannten Methoden in Wasser bei etwa 90° und einem pH-Wert von 9 mit einer Verbindung af) zu einer Verbindung der Formel

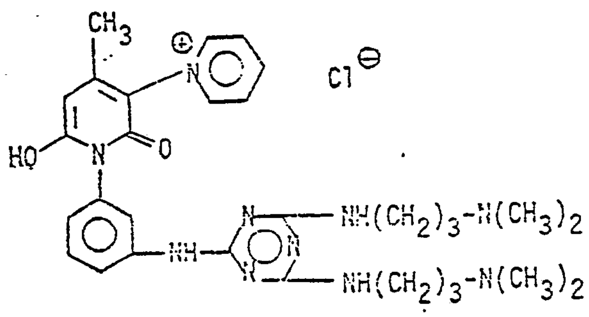

ar)

kondensiert.

Beispiel 4

Reduziert man nach Béchamp die Nitroverbindung ad) zum Amin der Formel

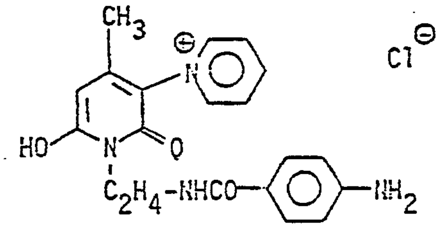

as)

und kondensiert as) nach bekannten Methoden in Wasser bei 90° und einem pH-Wert von 9 mit einer Verbindung af) so erhält man die Verbindung der Formel

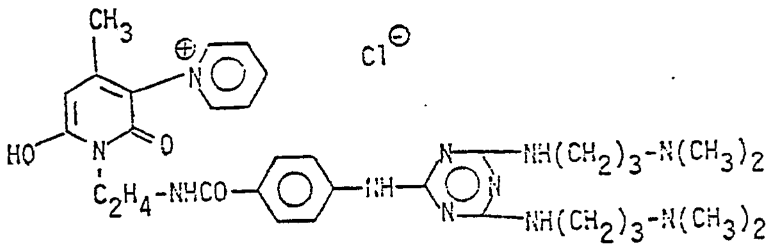

at)

Beispiel 5

Verwendet man gemäss Beispiel 1c) und 4) die entsprechende quaternäre Verbindung der Formel (erhalten aus af) durch quaternieren mit Dimethylsulfat)

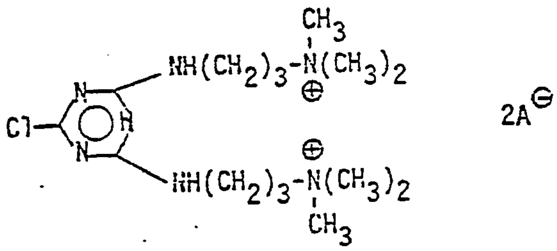

au)

so erhält man die entsprechenden quaternären Verbindungen.

Beispiel 6

Setzt man das Amin gemäss Beispiel 3d) mit Chloressigsäurechlorid gemäss Beispiel 2d) um, so erhält man eine Verbindung der Formel

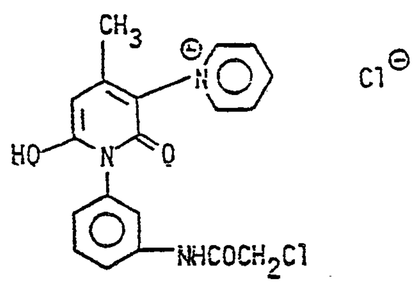

av)

die gemäss Beispiel 2c) mit Trimethylamin zur Verbindung

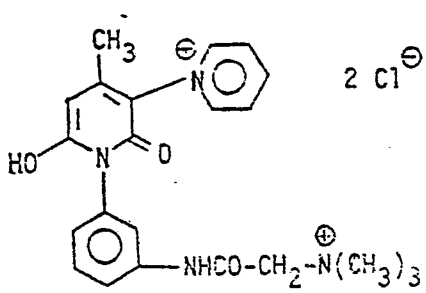

aw)

quaterniert werden kann.

Beispiel 7

Verwendet man anstelle von Beispiel 3a) eine bekannte Verbindung der Formel

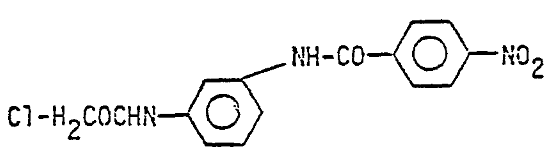

ax)

quaterniert, zyklisiert und reduziert ax) gemäss Beispiel 3b) und 3c) und 4) so erhält man das Amin der Formel

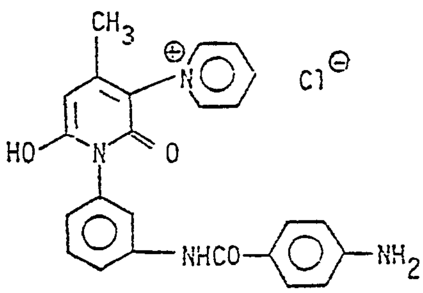

ay),

welches durch Umsetzen mit af) oder au) zu den entsprechenden Triazinylgruppenhaltigen Verbindungen umgesetzt werden kann, d. h. mit den Halogen-, vorzugsweise Cyanurchloridverbindungen bzw. Benzoyl- oder Chloracetylchloridverbindungen der Reste aus der Tabelle A.

Beispiel 8.

Setzt man gemäss Beispiel Ia an Stelle von Aethylendiamin eine äquivalente Menge von Cyclohexylendiamin oder eine äquivalente Menge von 1,4-Diaminobuten-2 ein, so erhält man die entsprechende Cyclohexylen- bzw. Buten-2-verbindung von ab), welche nach an sich bekannten Methoden gemäss den übrigen Beispielen 1b)-6) in die entsprechenden Pyridone übergeführt werden kann.

In der folgenden Tabelle I ist die Zusammensetzung von weiteren Kupplungskomponenten der Formel I angegeben. Sie können nach den Angaben in den Beispielen 1-8 hergestellt werden und entsprechen der Formel

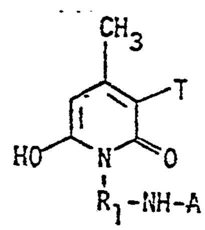

Als Anion $A^{\ominus}$ kommen die in der Beschreibung aufgeführten in Frage.

T Tabelle

| | | | |
|---|---|---|---|
| $T_1$ | bedeutet | H | |
| $T_2$ | do. | CN | |
| $T_3$ | do. | $-CONH_2$ | |
| $T_4$ | do. | 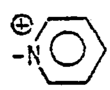 | $A^{\ominus}$ |
| $T_5$ | do. | 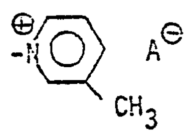 | $A^{\ominus}$ |
| $T_6$ | do. | 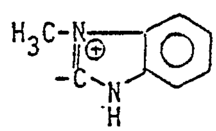 | $A^{\ominus}$ |
| $T_7$ | do. | 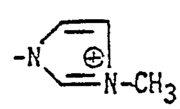 | $A^{\ominus}$ |

Tabelle für den Rest A

$A_1$ bedeutet H

$A_2$ do. 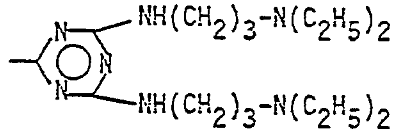

$A_3$ do. 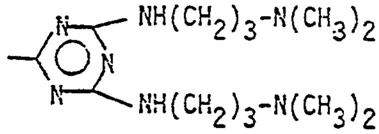

$A_4$ do. $-COCH_2-\overset{\oplus}{N}(CH_3)_3 \quad A^{\ominus}$

$A_5$ do. 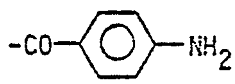

$A_6$ do. 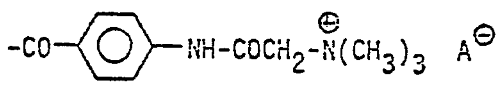

$A_7$ do. 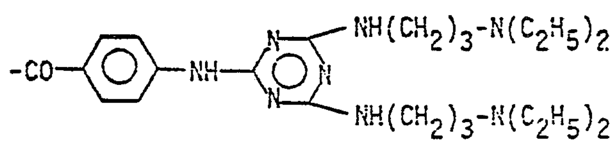

$A_8$ do. 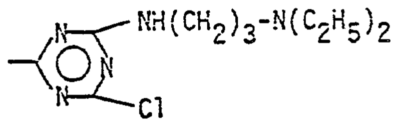

$A_9$ do. 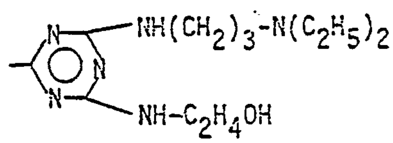

$A_{10}$ bedeutet 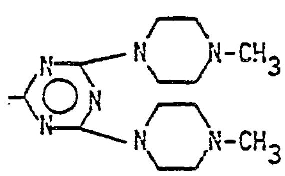

$A_{11}$ do. 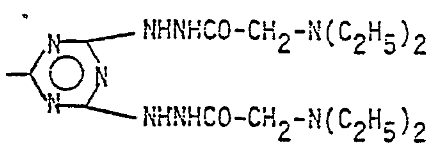

(Fortsetzung)

$A_{12}$ do. 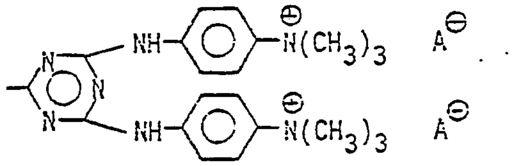

$A_{13}$ do. 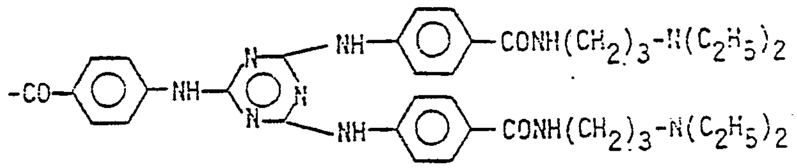

$A_{14}$ do. 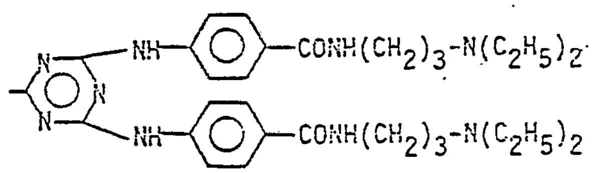

$A_{15}$ do. 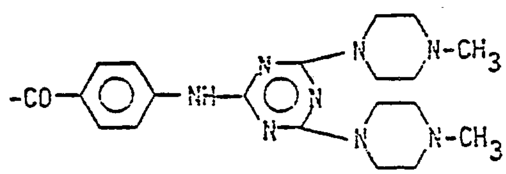

$A_{16}$ do. 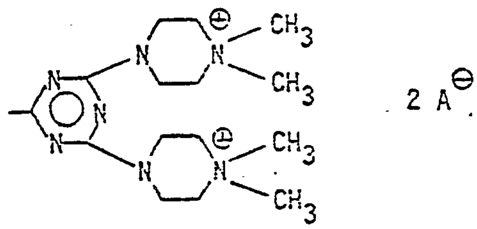

$A_{17}$ bedeutet 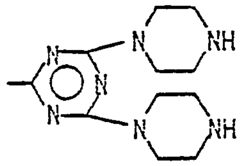

$A_{18}$ do. 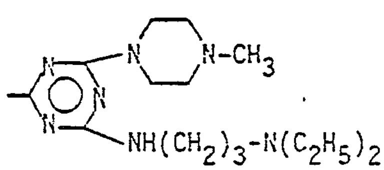

(Fortsetzung)

A19 do. 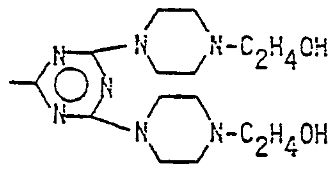

A20 do. 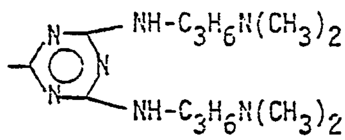

A21 do. 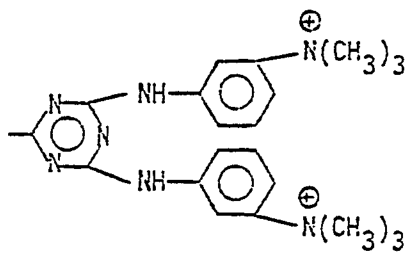 $2\ A^{\ominus}$

A22 do. 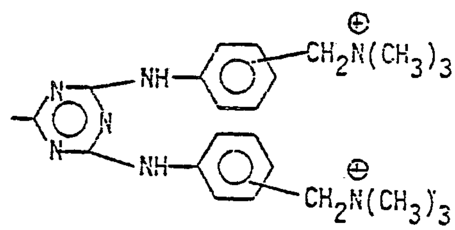 $2\ A^{\ominus}$

A23 do. 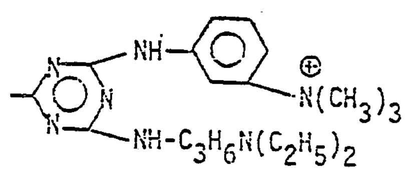 $A^{\ominus}$

A24 bedeutet 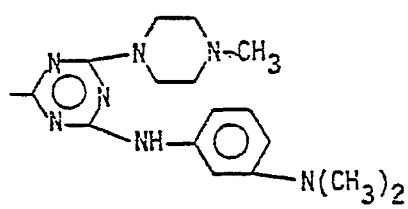

A25 do. 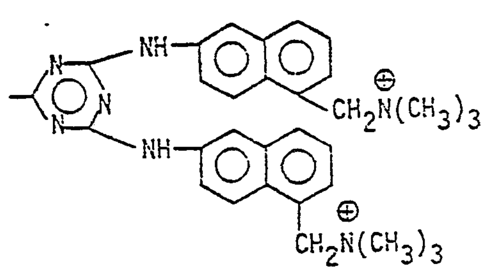 $2A^{\ominus}$

(Fortsetzung)

$A_{26}$ do. 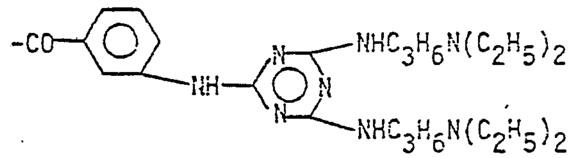

$A_{27}$ do. 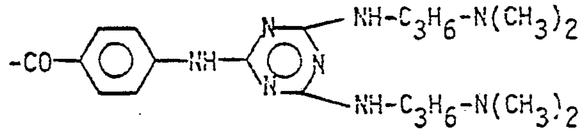

$A_{28}$ do. 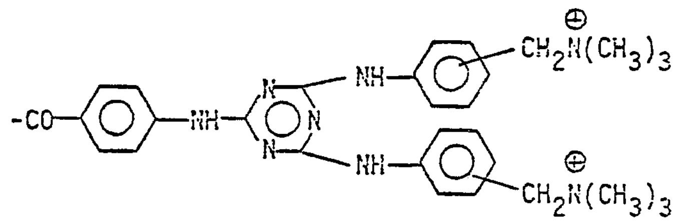 2 $A^{\ominus}$

$A_{29}$ do. 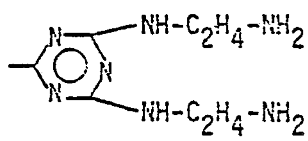

$A_{30}$ bedeutet 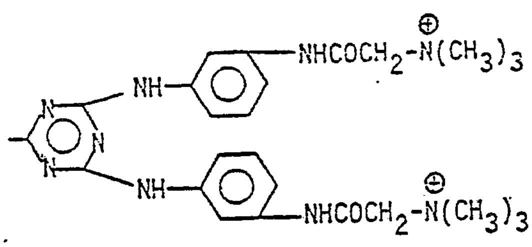 2 $A^{\ominus}$

$A_{31}$ do. 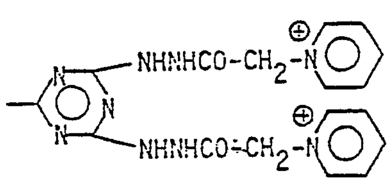 2 $A^{\ominus}$

$A_{32}$ do. 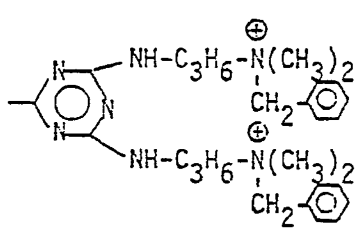 2 $A^{\ominus}$

Tabelle I

| Kupplungs-Komponente No. | T | $R_1$ | A |
|---|---|---|---|
| 1 | H | $-C_2H_4-$ | $A_1$ |
| 2 | $T_2$ | do. | $A_2$ |
| 3 | $T_3$ | do. | $A_3$ |
| 4 | $T_4$ | do. | $A_4$ |
| 5 | $T_6$ | do. | $A_5$ |
| 6 | $T_7$ | do. | $A_6$ |
| 7 | $T_4$ | 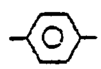 | $A_7$ |
| 8 | $T_4$ | do. | $A_8$ |
| 9 | $T_5$ | do. | $A_9$ |
| 10 | $T_4$ | 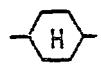 | $A_{10}$ |
| 11 | $T_4$ | $-CH_2-CH=CH-CH_2-$ | $A_{11}$ |
| 12 | $T_2$ | do. | $A_{12}$ |
| 13 | $T_4$ | $-C_2H_4-$ | $A_{13}$ |
| 14 | $T_4$ | do. | $A_{14}$ |
| 15 | $T_4$ | do. | $A_{15}$ |
| 16 | $T_4$ | do. | $A_{16}$ |
| 17 | $T_4$ | do. | $A_{17}$ |
| 18 | $T_4$ | do. | $A_{18-32}$ |

Beispiel 9

a) 15 Teile (1/10 Mol) 4-Aminoacetanilid werden nach bekannter Methode in salzsäurem wässerigem Medium bei 0-5° mit 6,9 Teilen (1/10 Mol) Natriumnitrit diazotiert. Mit Natriumacetat wird ein pH Wert von 2,0 bis 3,0 eingestellt. Hierzu tropft man 1/10 Mol der in Wasser gelösten Kupplungskomponente gemäss Beispiel 1e). Man erhält einen gelben Farbstoff der Formel

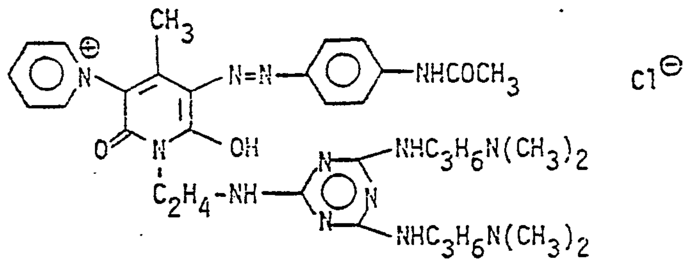

b) Durch Zugabe von 30 %-iger salzsäure zur wässerigen Farbstofflösung wird eine 7 %-ige salzsäure Reaktionslösung eingestellt und 10 Stunden unter Rückfluss gebracht. Man erhält den Acetylgruppenfreier Farbstoff der Formel

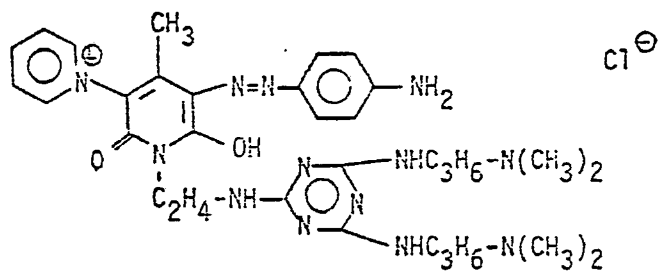

c) Zur salzsauren Verseifungslösung von 0-5° wird eine 4n Natriumnitritlösung getropft bis eine Probe auf Kaliumjodidpapier violett anzeigt. Zur erhaltenen Diazolösung gibt man eine äquivalente Menge einer Verbindung der Formel

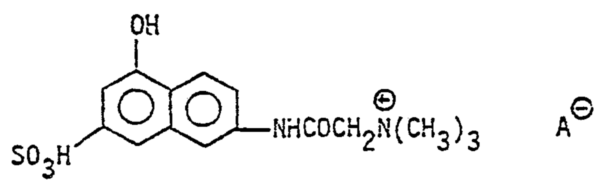

und stellt durch Zutropfen von Natriumhydroxyd auf einen pH-Wert von 5. Man erhält einen Farbstoff der Formel

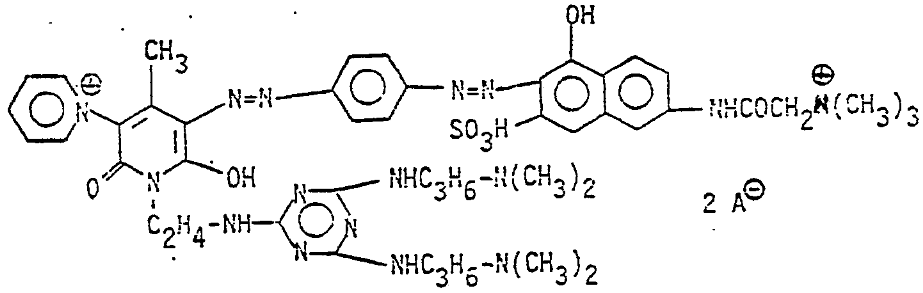

der Papier in blauviolетten Tönen anfärbt.

d) Ersetzt man die unter 9c) genannte Naphtholkupplungskomponente durch eine äquivalente Naphtholverbindung der Formel

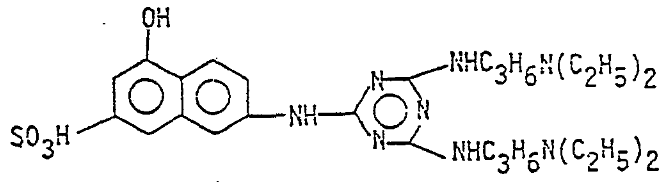

so erhält man einen ähnlichen Farbstoff von blauvioletter Nuance auf Papier.

Beispiel 10

Nach bekannten Methoden werden 6,9 Teile (1/20 Mol) 1-Amino-4-nitrobenzol in salzsaurer Lösung bei 0-5° mit 3,5 Teilen (1/20 Mol) Natriumnitrit diazotiert und zuerst 1/40 Mol Diazoniumlösung bei einen pH-Wert von 1 auf 8 Teile (1/40 Mol) 1-Amino-8-hydroxynaphthalin-3,6-disulfonsäure gekuppelt. Ein weiteres 1/40 Mol Diazoniumlösung wird bei einem pH-Wert von 9,5 auf den Monoazofarbstoff zum Disazofarbstoff der Formel

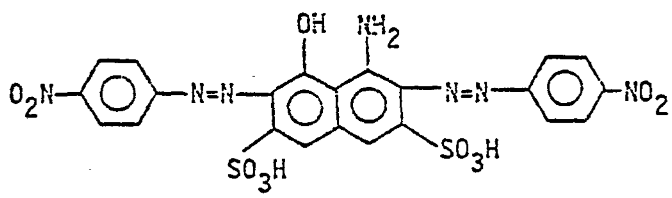

gekuppelt.

Die Nitrogruppen des Disazofarbstoffes werden nach bekannten Methoden mit Natriumsulfid bei 40-50° und einem pH-Wert von 11-12 zu Aminogruppen reduziert.

13,8 Teile (1/40 Mol) des Diaminodisazofarbstoffes werden salzsauer bei 0-5° mit 3,5 Teilen (1/20 Mol) Natriumnitrit tetrazotiert und bei einem pH-Wert von 1,5-3 auf 30,7 Teile (1/20 Mol) der Verbindung von Beispiel 1e) gekuppelt. Man erhält den Farbstoff der Formel

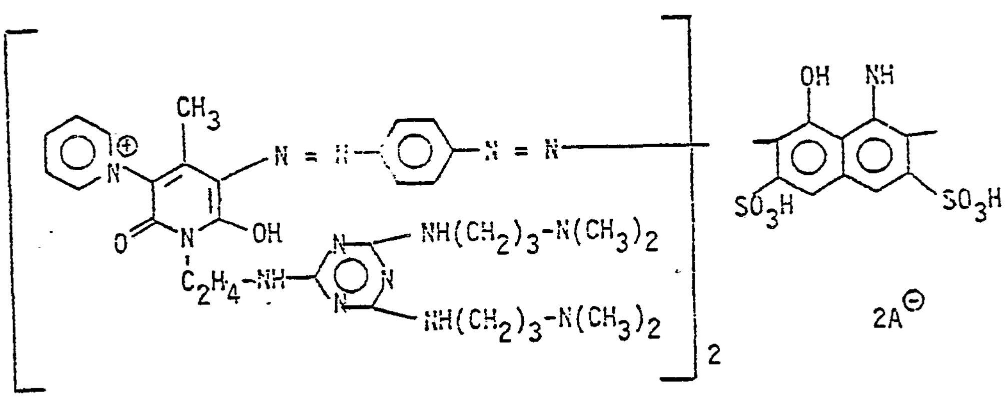

der Papier in schwarzen Tönen echt anfärbt.

In der folgenden Tabelle II ist der strukturelle Aufbau weiterer Farbstoffe angegeben, wie sie gemäss Beispiel 10 erhalten werden können. Sie entsprechen der Formel

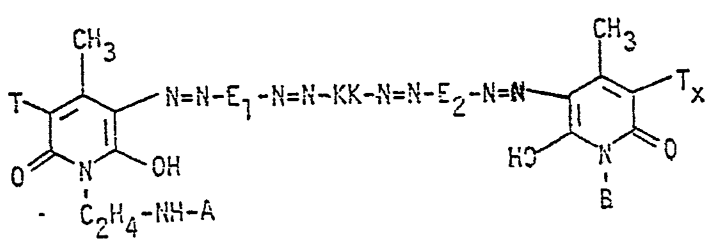

worin die Symbole die in den einzelnen Kolonnen angegebenen Bedeutungen besitzen, die Farbstoffe färben Papier in schwarzer Nuance. Der Rest B ist in der nachstehenden B-Tabelle aufgeführt.

B-Tabelle

| | | |
|---|---|---|
| $B_1$ | bedeutet | H |
| $B_2$ | do. | $-C_2H_5$ |
| $B_3$ | do. | $-C_2H_4OH$ |
| $B_4$ | do. | $-(CH_2)_3N(CH_3)_2$ |
| $B_5$ | do. | $-(CH_2)_3\overset{\oplus}{N}(CH_3)_3\ CH_3SO_4^{\ominus}$ |
| $B_6$ | do. | $-(CH_2)_2\ NH_2$ |
| $B_7$ | do. | $-(CH_2)_3\ -NH_2$ |
| $B_8$ | do. | 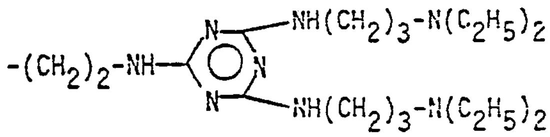 |
| $B_9$ | do. | $-(CH_2)_2-NHCOCH_2-\overset{\oplus}{N}(CH_3)_3\ \ Cl^{\ominus}$ |
| $B_{10}$ | do. | 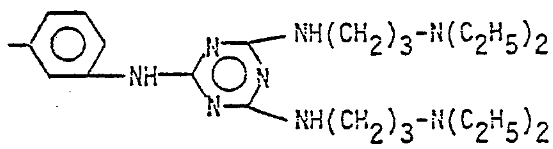 |
| $B_{11}$ | do. | 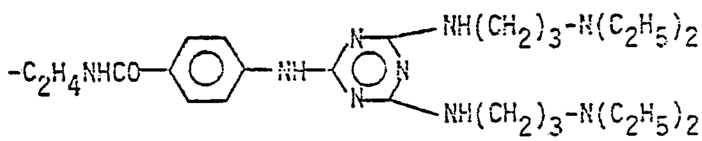 |

Tabelle II

| Bsp. Nr. | T | A | $T_x$ | B | $E_1$ | $E_2$ | KK |
|---|---|---|---|---|---|---|---|
| 11 | $T_5$ | $A_2$ | $T_5$ | $B_8$ | 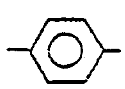 | 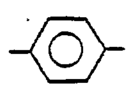 | 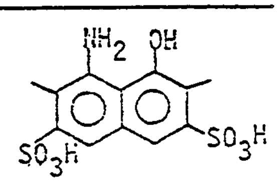 |
| 12 | $T_4$ | $A_2$ | $T_4$ | $B_4$ | do. | do. | do. |
| 13 | $T_4$ | $A_1$ | $T_5$ | $B_4$ | do. | 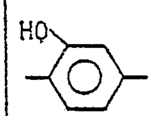 | do. |
| 14 | $T_4$ | $A_2$ | $T_5$ | $B_4$ | 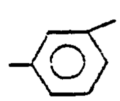 | 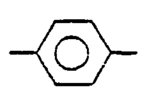 | do. |
| 15 | $T_5$ | $A_2$ | $T_5$ | $B_8$ | 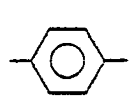 | 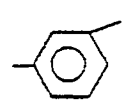 | do. |

Tabelle II (Fortsetzung)

| Bsp. Nr. | T | A | $T_x$ | B | $E_1$ | $E_2$ | KK |
|---|---|---|---|---|---|---|---|
| 16 | $T_4$ | $A_2$ | $T_4$ | $B_4$ | do. | 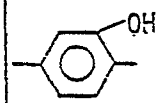 | do. |
| 17 | $T_5$ | $A_2$ | $T_5$ | $B_3$ | do. | do. | do. |
| 18 | $T_5$ | $A_2$ | $T_6$ | $B_8$ | do. | 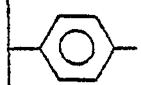 | 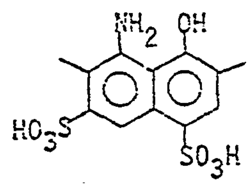 |
| 19 | $T_4$ | $A_2$ | $T_4$ | $B_4$ | do. | do. | do. |
| 20 | $T_7$ | $A_4$ | $T_5$ | $B_8$ | 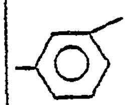 | do. | do. |
| 21 | $T_4$ | $A_2$ | $T_4$ | $B_4$ | 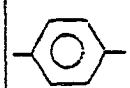 | 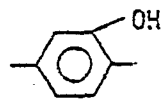 | do. |
| 22 | $T_4$ | $A_2$ | $T_4$ | $B_8$ | do. | do. | do. |
| 23 | $T_4$ | $A_2$ | $T_4$ | $B_4$ | 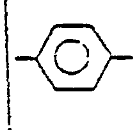 | 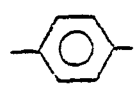 | 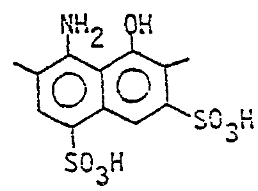 |
| 24 | $T_5$ | $A_2$ | $T_5$ | $B_8$ | do. | do. | do. |
| 25 | $T_4$ | $A_2$ | $T_7$ | $B_2$ | do. | 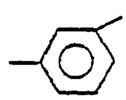 | do. |
| 26 | $T_5$ | $A_2$ | $T_4$ | $B_1$ | do. | do. | do. |
| 27 | $T_4$ | $A_2$ | $T_4$ | $B_4$ | do. | 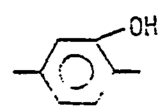 | do. |
| 28 | $T_5$ | $A_2$ | $T_5$ | $B_8$ | do. | do. | do. |

Beispiel 29

6,9 Teile (1/20 Mol) para-Nitroanilin werden salzsauer bei 0-5° mit 3,5 Teilen (1/20 Mol) Natriumnitrit diazotiert und bei einem pH-Wert von 1-2 auf 16 Teile (1/20 Mol) 1-Amino-8-hydroxy-naphthalin-3,6-disulfonsäure gekuppelt. Hierauf werden 7,7 Teile (1/20 Mol) 1-Amino-2-hydroxy-4-nitrobenzol wie oben

diazotiert und bei einem pH-Wert von 9-9,5 auf den Monoazofarbstoff gekuppelt. Man erhält den Disazofarbstoff der Formel

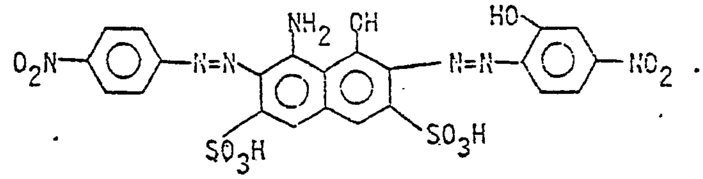

(α)

Die Nitrogruppen der Verbindung (α) werden nach bekannten Methoden mit Natriumsulfid bei 40-50° und einem pH-Wert von 11-12 zu Aminogruppen reduziert. Der Diaminodisazofarbstoff wird wie im Beispiel 10 tetrazotiert und auf 1/10 Mol der Verbindung der Formel (1e) gekuppelt.

Man erhält den Farbstoff der Formel

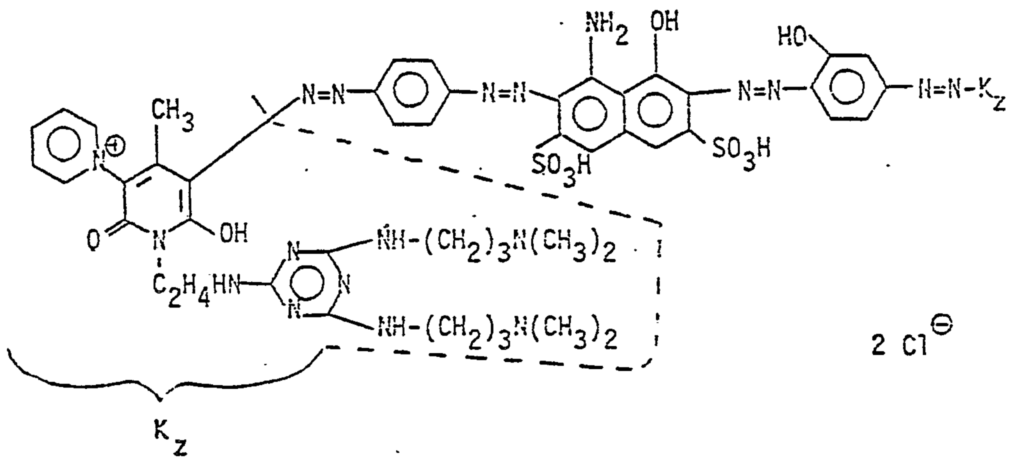

der Papier in schwarzen Tönen färbt.

Dieser Farbstoff kann nach bekannten Methoden in die entsprechenden Kupfer-, Chrom-, Kobalt- oder Eisenkomplexeverbindung übergeführt werden, z. B. in wässeriger Lösung durch Zugabe von Kupfersulfat-Pentahydrat bei Temperaturen von 60-75°.

Beispiel 30

Einen ebenso guten schwarzen Farbstoff erhält man, wenn man die Tetrazoverbindung aus dem reduzierten Dinitrofarbstoff (α) von Beispiel 29 auf die Kupplungskomponente der Formel

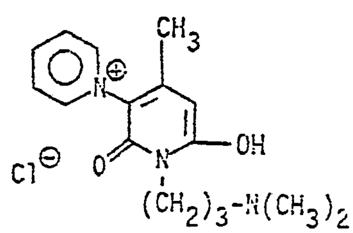

($α_1$)

kuppelt. Man erhält den Farbstoff der Formel

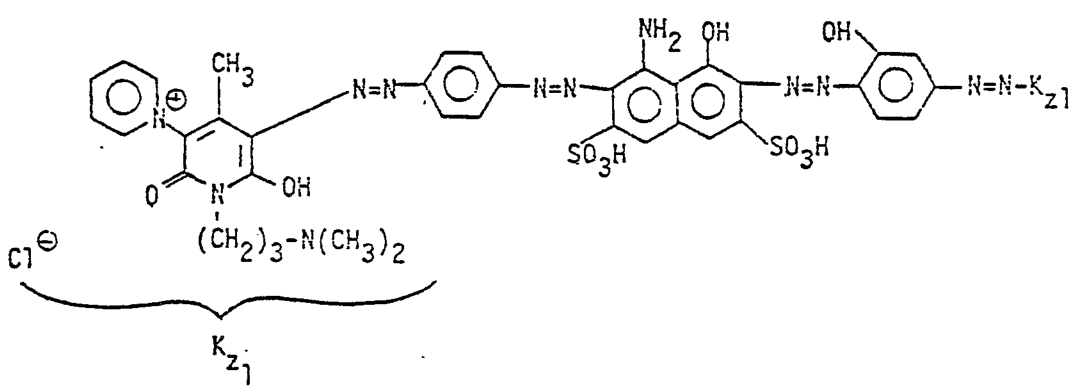

der sich gemäss Beispiel 29 in die entsprechende 1 : 1-Kupfer-, Chrom-, Kobalt oder Eisenkomplexform überführen lässt. Er färbt Papier in schwarzen Tönen mit guten Echtheiten.

Beispiel 31

a) Kuppelt man nach bekannten Methoden die Diazoverbindung aus dem Aminoazofarbstoff von Beispiel 9b) auf eine Verbindung der Formel

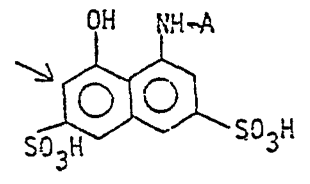

β) oder

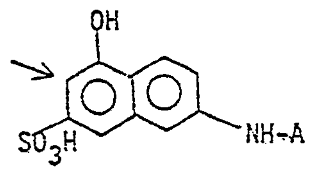

γ)

worin A aus der Tabelle für den Rest A jeweils einen Rest $A_1$ bis $A_{32}$ bedeutet, so erhält man ähnlich gute Farbstoffe, die Papier in blauen Tönen färben.

b) Kuppelt man nach bekannten Methoden die Diazoverbindung aus einen Amin der Formel

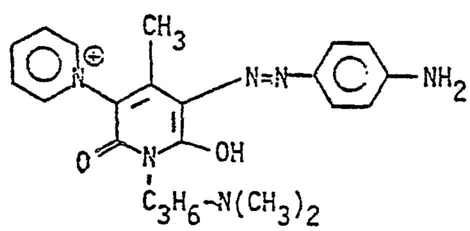

$A^{\ominus}$

auf eine der Kupplungskomponenten β) oder γ) gemäss Beispiel 31 so erhält man ähnlich gute Farbstoffe, die Papier ebenfalls in blauen Tönen färben.

Die Verbindungen der Formeln β) und γ) sind an sich bekannt oder können nach an sich bekannten Methoden, z. B. gemäss der englischen Patentschrift 2 082 015 hergestellt werden oder gemäss der europäischen Offenlegungsschrift 62 824, 62 825, 56 574 oder der DOS 3 114 088 und 2 915 323.

Sie können wie folgt erhalten werden: 1-Hydroxy-8-aminonaphthalin-3,6-disulfonsäure oder 1-Hydroxy-6-aminonaphthalin-3-sulfonsäure wird nach an sich bekannte Methoden bei etwa 0 bis 5° mit Cyanurchlorid kondensiert.

Das erhaltene Produkt wird dann stufenweise mit einem Diamin, z. B. Piperazin, Girardreagens, Aethylen- oder Propylendiamin bei 40-60° und dem gleichen oder einem anderen Diamin bei 80-95° weiterkondensiert. Man erhält Kupplungskomponenten die gleiche oder voneinander verschiedene Amine tragen.

An stelle von Cyanurchlorid kann man die Aminoreste aus β) und γ) mit einer Benzoylchloridverbindung wie sie als Rest $A_5$-$A_7$, $A_{13}$, $A_{26}$-$A_{28}$ offenbart sind oder mit Chloracetylchlorid und anschliessender Umsetzung mit einem Amin, beispielsweise einem sekundären oder tertiären Amin umsetzen, z. B. gemäss dem Verfahren aus Beispiel 6).

Beispiel 32

22,1 Teile (1/20 Mol) der Aminoazoverbindung aus Beispiel 31b) werden in salzsauer wässeriger Lösung bei 0-5° mit 3,5 Teilen Natriumnitrit diazotiert. Ein Ueberschuss an salpetriger Säure wird mit Aminosulfonsäure zerstört. Mit Natriumhydrogencarbonat wird der pH-Wert der Diazolösung auf 5 eingestellt.

11,9 Teile (1/20 Mol) 2-Amino-5-hydroxynaphthalin-7-sulfonsäure werden in 250 Teilen Wasser von 0-5° gegeben und mit 10 %-iger Natriumcarbonatlösung ein pH-Wert von 8 eingestellt.

Man erhält so die Kupplungslösung.

In etwa 30 Minuten wird die Diazoniumlösung bei 0-5° zur Kupplerlösung getropft und der pH-Wert

durch Zugabe von 10 %-iger Natriumcarbonatlösung bei 8 gehalten. Man erhält den violetten Farbstoff der Formel

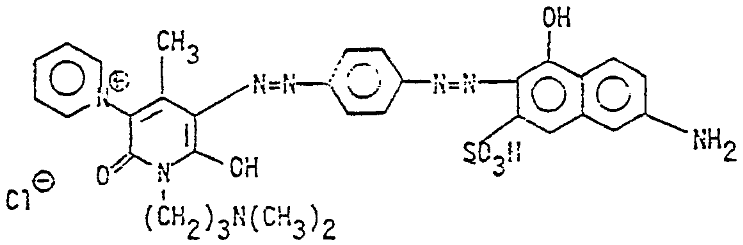

(ε)

Dieser Farbstoff (ε) wird salzsauer bei 0-5° diazotiert. Zur Diazolösung gibt man 14,3 Teile (1/20 Mol) einer Verbindung ($\alpha_1$) gemäss Beispiel 30 und stellt den pH-Wert durch Zugabe von 30 %-iger Natronlauge auf 5. Der entstandene Farbstoff der Formel

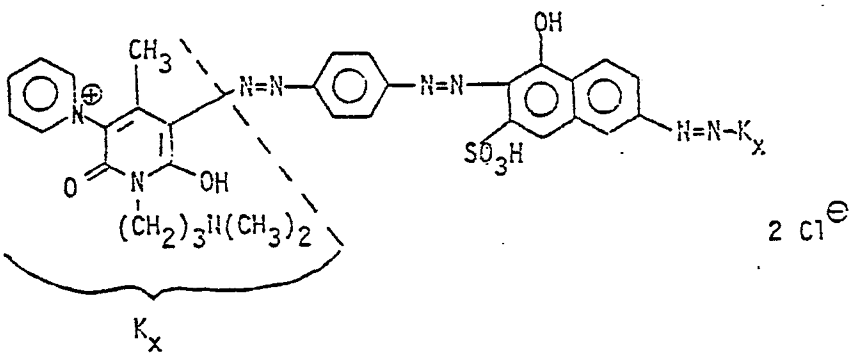

wird durch Zugabe von Aceton ausgefällt. Er färbt Papier in schwarzer Nuance mit guten Echtheiten.

In der folgenden Tabelle III ist der strukturelle Aufbau weiterer Farbstoffe angegeben. Sie können nach den Angaben im Beispiel 32 hergestellt werden und entsprechen der Formel

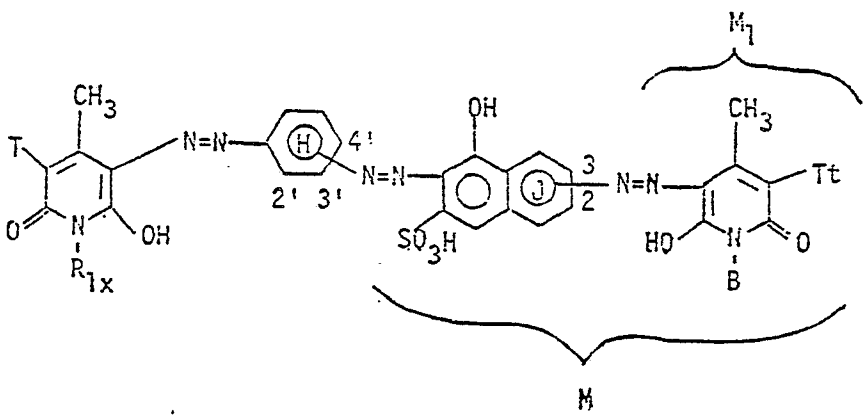

S)

worin die Symbole die in den Klamern angegebenen Bedeutungen besitzen.

(Siehe Tabelle III Seite 58 ff.)

Tabelle III

| Bsp. | T | Tt | Bindung im Ring H | Bindung im Ring J | $R_{1x}$ | B |
|---|---|---|---|---|---|---|
| 33 | $T_4$ | $T_4$ | 4' | 3 | $-(CH_2)_3-N(CH_3)_2$ | $B_4$ |
| 34 | $T_4$ | $T_4$ | 3' | 2 | do. | $B_4$ |
| 35 | $T_4$ | $T_4$ | 3' | 3 | do. | $B_4$ |
| 35a | $T_4$ | $T_4$ | 4' | 3 | do. | $B_1$ |
| 36 | $T_4$ | $T_2$ | 4' | 3 | do. | $B_4$ |
| 37 | $T_4$ | $T_2$ | 4' | 2 | do. | $B_4$ |
| 38 | $T_4$ | $T_2$ | 4' | 2 | do. | $B_4$ |
| 39 | $T_4$ | $T_2$ | 3' | 2 | do. | $B_4$ |
| 40 | $T_4$ | $T_4$ | 4' | 2 | $B_8$ | $B_8$ |
| 41 | $T_4$ | $T_4$ | 3' | 2 | $B_8$ | $B_8$ |
| 42 | $T_4$ | $T_4$ | 3' | 3 | $B_8$ | $B_8$ |
| 43 | $T_4$ | $T_4$ | 4' | 3 | $B_8$ | $B_8$ |
| 44 | $T_4$ | $T_4$ | 4' | 3 | $B_8$ | $B_1$ |
| 45 | $T_4$ | $T_4$ | 4' | 2 | $B_8$ | $B_1$ |
| 46 | $T_4$ | $T_4$ | 3' | 3 | $B_8$ | $B_1$ |
| 47 | $T_4$ | $T_4$ | 3' | 2 | $B_8$ | $B_1$ |
| 48 | $T_4$ | $T_4$ | 4' | 3 | $B_8$ | $B_4$ |
| 49 | $T_4$ | $T_4$ | 4' | 2 | $B_8$ | B[illegible] |
| 50 | $T_4$ | $T_4$ | 3' | 3 | $B_8$ | $B_4$ |
| 51 | $T_4$ | $T_4$ | 3' | 2 | $B_8$ | $B_4$ |
| 52 | $T_4$ | $T_2$ | 4' | 3 | $B_8$ | $B_4$ |
| 53 | $T_4$ | $T_2$ | 4' | 2 | $B_8$ | $B_4$ |
| 54 | $T_4$ | $T_2$ | 3' | 3 | $B_8$ | $B_4$ |
| 55 | $T_4$ | $T_2$ | 3' | 2 | $B_8$ | $B_4$ |
| 56 | $T_2$ | $T_4$ | 4' | 3 | $B_4$ | $B_4$ |
| 57 | $T_2$ | $T_4$ | 4' | 2 | $B_4$ | $B_8$ |

Tabelle III (Fortsetzung)

| Bsp. | T | Tt | Bindung im Ring H | Bindung im Ring J | $R_{1x}$ | B |
|---|---|---|---|---|---|---|
| 56 | $T_4$ | $T_4$ | 4' | 2 | $B_4$ | $B_8$ |
| 59 | $T_4$ | $T_4$ | 4' | 3 | $B_1$ | $B_8$ |
| 60 | $T_4$ | $T_4$ | 4' | 2 | $B_1$ | $B_8$ |
| 61 | $T_4$ | $T_4$ | 4' | 2 | 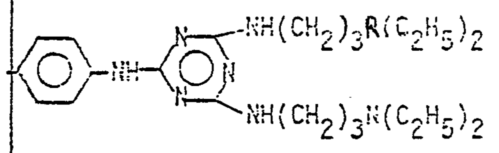 | $B_8$ |
| 62 | $T_4$ | $T_4$ | 4' | 3 | do. | $B_1$ |
| 63 | $T_4$ | $T_4$ | 4' | 2 | do. | $B_4$ |
| 64 | $T_4$ | $T_4$ | 4' | 3 | do. | wie $R_{1x}$ (Bsp. 61 |
| 65 | $T_4$ | $T_4$ | 4' | 2 | $B_8$ | wie $R_{1x}$ (Bsp. 61) |

Die Verbindungen der Formel (β) können in den Stellungen 2', 3' und 4' durch QH oder $OCH_3$ substituiert sein. Tragen die Verbindungen einen dieser Reste in einer der genannten Stellungen, lassen sie sich mit Kupfersalzen in die entsprechenden 1 : 1-Kupferkomplexe und mit OH mit Chrom-, Kobalt- oder Eisensalzen in die entsprechenden 1 : 1 und/oder 1 : 2-Chrom-, Kobalt- oder Eisenkomplexe überführen.

Sie entsprechen den Formeln

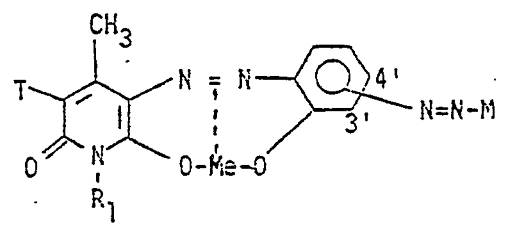

oder ($\rho_1$)

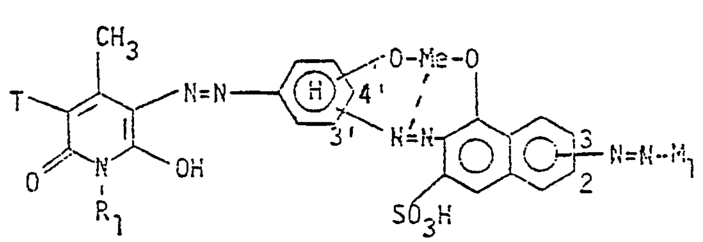

($\rho_2$)

worin Me ein Kupferatom für die 1 : 1-Kupferkomplexe und

Me ein Chrom-, Kobalt- oder Eisenatom für die 1 : 2-Metallkomplexe,

wobei in der Formel-($\rho_1$) im Ring (H) der —O—Me—O— und die —N = N— Reste in ortho-Stellung zueinander stehen.

Beispiel 66

12,3 Teile (1/10 Mol) ortho-Anisidin werden salzsauer bei 0 bis 5° mit 6,9 Teilen (1/10 Mol) Natriumnitrit diazotiert und mit Natriumacetat ein pH-Wert von 2,0 bis 3,0 eingestellt. Hierzu tropft man 61,5 Teile (1/10 Mol) die in Wasser gelöste Verbindung der Formel

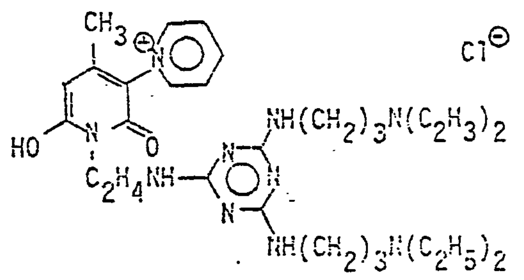

(α)

Man erhält den Farbstoff der Formel

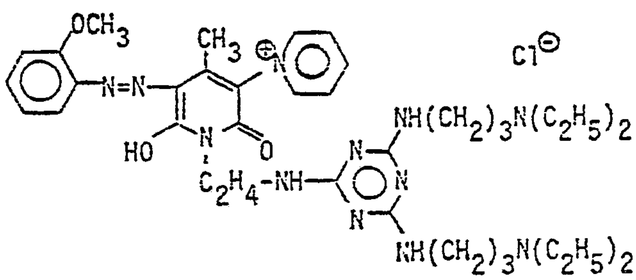

der Papier in gelben Tönen färbt. Er dient auch zum Gelfärben von Polyacrylnitril.

In der folgenden Tabelle IV ist der strukturelle Aufbau weiterer Farbstoffe angegeben. Sie können nach den Angaben im Beispiel 66 hergestellt werden und entsprechen der Formel

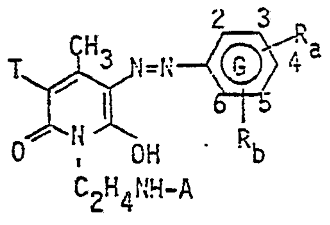

worin die Symbole die in den Kolonnen angegebenen Bedeutungen besitzen.

Als Anion A$^{\ominus}$ kommen die in der Beschreibung aufgeführten in Frage.

(Siehe Tabelle IV Seite 61 f.)

Die Farbstoffe der Beispiele 67 bis 91 dienen, sofern sie eine basische Gruppe tragen zum Färben von Polyacrylnitril ; tragen sie zwei und mehr basische Gruppen dienen sie zum Färben von Papier und zum Gelfärben von Polyacrylnitril. Die Nuancen sind für alle Farbstoffe gelb.

Beispiel 92

9,9 Teile (1/20 Mol) 4-Amino-1,1'-azobenzol werden nach bekannter Methode in wässriger salzsaurer Lösung bei 0-5° mit 3.5 Teilen (1/20 Mol) Natriumnitrit diazotiert und bei einem pH-Wert von 2-3 mit einer Verbindung der Formel (α) gemäss Beispiel 66, gekuppelt. Man erhält den Farbstoff der Formel

Tabelle IV

| Bsp. No. | T | A | Bedeutung von $R_a$ und Stellung in Ring G | Bedeutung von $R_b$ und Stellung im Ring G |
|---|---|---|---|---|
| 67 | $T_1$ | $A_2$ | H | H |
| 68 | $T_2$ | $A_3$ | 3-Cl | H |
| 69 | $T_3$ | $A_2$ | 4-Cl | H |
| 70 | $T_4$ | $A_1$ | H | H |
| 71 | $T_4$ | $A_1$ | $2-NO_2$ | H |
| 72 | $T_4$ | $A_1$ | $2-NO_2$ | 4-Cl |
| 73 | $T_4$ | $A_2$ | H | H |
| 74 | $T_4$ | $A_2$ | $2-NO_2$ | -H |
| 75 | $T_4$ | $A_2$ | 2-Cl | $4-NO_2$ |
| 76 | $T_4$ | $A_2$ | 4-O-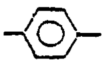-Cl | H |
| 77 | $T_4$ | $A_3$ | H | H |
| 78 | $T_4$ | $A_4$ | $2-NO_2$ | 4-Cl |
| 79 | $T_4$ | $A_4$ | $4-OCH_3$ | -H |
| 80 | $T_5$ | $A_1$ | H | H |
| 81 | $T_5$ | $A_2$ | $4-NO_2$ | -H |
| 82 | $T_5$ | $A_2$ | 2-Cl | $4-NO_2$ |
| 83 | $T_5$ | $A_4$ | 4-O-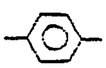-Cl | -H |
| 84 | $T_6$ | $A_1$ | -H | H |
| 85 | $T_6$ | $A_2$ | $4-NO_2$ | H |
| 86 | $T_6$ | $A_4$ | 2-Cl | $4-NO_2$ |
| 87 | $T_7$ | $A_2$ | $2-NO_2$ | 4-Cl |
| 88 | $T_7$ | $A_4$ | 4-O-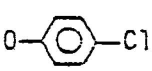-Cl | H |
| 89 | $T_4$ | $A_2$ | $2-OCH_3$ | $5-CH_3$ |
| 90 | $T_5$ | $A_4$ | $2-OCH_3$ | $5-OCH_3$ |
| 91 | $T_4$ | $A_4$ | $2-CH_3$ | $3-CH_3$ |

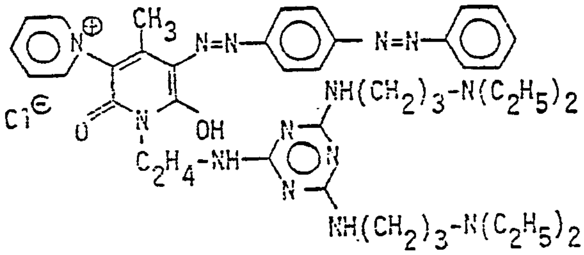

der Papier in goldgelben Tönen anfärbt.

In der folgenden Tabelle V ist der strukturelle Aufbau weiterer Farbstoffe angegeben, wie sie gemäss Beispiel 92 hergestellt werden können. Sie entsprechen der allgemeinen Formel

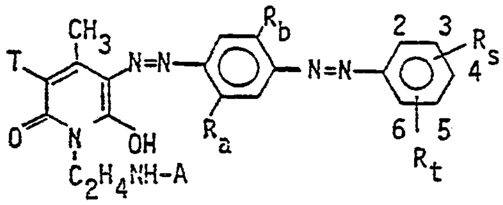

worin die Symbole die in den Kolonnen angegebenen Bedeutungen besitzen.

Tabelle V

| Bsp. No. | T | A | $R_a$ | $R_b$ | $R_s$ | $R_t$ |
|---|---|---|---|---|---|---|
| 93 | $T_5$ | $A_2$ | $-OCH_3$ | $-CH_3$ | $4-CH_3$ | H |
| 94 | $T_7$ | $A_2$ | do | $-OCH_3$ | $2-OCH_3$ | H |
| 95 | $T_4$ | $A_4$ | do | do | $2-CH_3$ | $5-CH_3$ |
| 96 | $T_6$ | $A_2$ | do | $-CH_3$ | $4-C_4H_9$ | H |
| 97 | $T_5$ | $A_4$ | do | $-OCH_3$ | $2-OCH_3$ | $5-CH_3$ |
| 98 | $T_4$ | $A_2$ | $-CH_3$ | $-OCH_3$ | $2-OCH_3$ | H |

Die Farbstoffe der Beispiele 93-98 färben Papier und Polyacrylnitril im Gelzustand in goldgelben bis orangen Tönen mit guten Echtheiten.

Beispiel 99

a) 15 Teile (1/10 Mol) 4-Aminoacetanilid werden nach bekannter Methode in wässriger salzsaurer Lösung bei 0-5° mit 6,9 Teilen (1/10 Mol) Natriumnitrit diazotiert. Durch Zufügen von Natriumacetat wird ein pH-Wert von 2,0-3,0 eingestellt. Zu dieser Lösung tropft man 61,5 Teile (1/10 Mol) die in Wasser gelöste Verbindung (α) von Beispiel 65. Man erhält den gelben Farbstoff der Formel

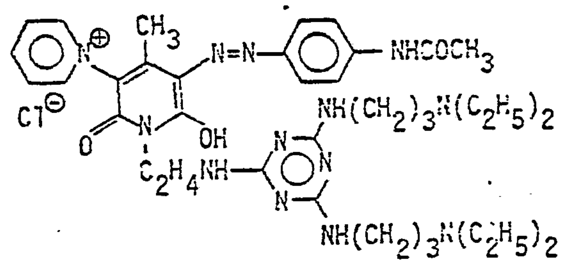

(β).

welcher direkt weiter umgesetzt wird.

b) Durch Zugabe von 30 %iger Salzsäure zur wässrigen Farbstofflösung wird eine 7 %ige salzsaure Reaktionslösung eingestellt und 10 Stunden lang unter Rückfluss gekocht. Man erhält den Acetylgruppenfreien Farbstoff der Formel

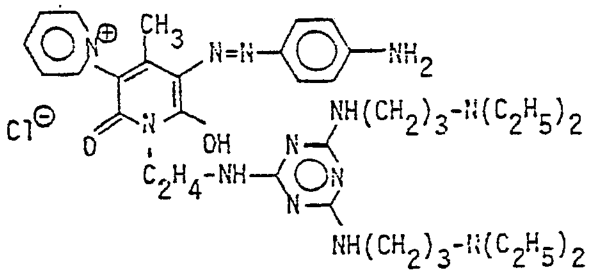

(γ)

c) Zur salzsauren Verseifungslösung mit der Verbindung (γ) von 0-5° wird eine 4n Natriumnitritlösung getropft bis eine Probe auf Kaliumjodidstärkepapier violett anzeigt. Dies ist nach etwa 85-90 % der Theorie der Fall. Zur Diazolösung gibt man eine entsprechende Menge der Verbindung der Formel

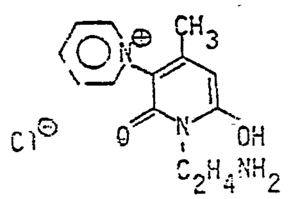

und stellt durch Zutropfen einer 30 %igen wässrigen Natriumhydroxydlösung den pH-Wert auf 5.

Man erhält den asymmetrischen Farbstoff der Formel

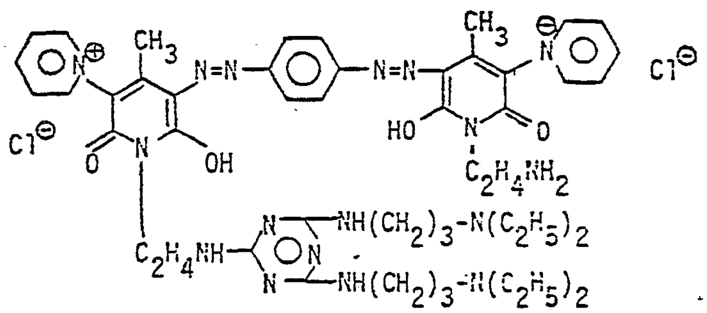

der Papier in violetten Tönen mit guten Echtheiten anfärbt.

In der folgenden Tabelle VI ist der strukturelle Aufbau weiterer Farbstoffe angegeben, wie sie gemäss Beispiel 99 erhalten werden können.

Sie entsprechen der Formel

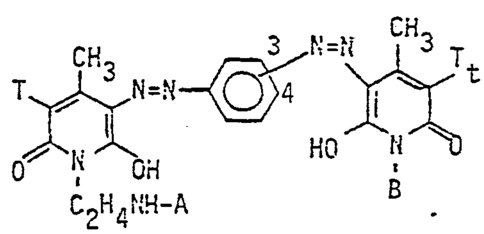

worin die Symbole die in den Kolonnen angegebenen Bedeutungen besitzen.

(Siehe Tabelle VI Seite 64 f.)

Die Farbstoffe der Beispiele 100-102, 113, 118 und 121, d. h. die Azobrücke ist in 4-Stellung gebunden, färben Papier in violetten Tönen und die Farbstoffe der Beispiele 111, 112, 114, 117, 119 und

Tabelle VI

| Bsp. No. | T | A | 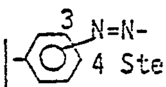 4 Stellung von -N=N | $T_t$ | B |
|---|---|---|---|---|---|
| 100 | $T_1$ | $A_2$ | 4 | $T_1$ | $B_1$ |
| 101 | $T_2$ | $A_2$ | 4 | $T_3$ | $B_2$ |
| 102 | $T_3$ | $A_4$ | 4 | $T_4$ | $B_3$ |
| 103 | $T_4$ | $A_1$ | 4 | $T_1$ | $B_4$ |
| 104 | $T_5$ | $A_1$ | 4 | $T_1$ | $B_5$ |
| 105 | $T_1$ | $A_2$ | 4 | $T_4$ | $B_6$ |
| 106 | $T_6$ | $A_2$ | 4 | $T_2$ | $B_7$ |
| 107 | $T_7$ | $A_1$ | 4 | $T_1$ | $B_8$ |
| 108 | $T_1$ | $A_1$ | 4 | $T_4$ | $B_9$ |
| 109 | $T_1$ | $A_2$ | 4 | $T_1$ | $B_8$ |
| 110 | $T_1$ | $A_4$ | 4 | $T_1$ | $B_9$ |
| 111 | $T_4$ | $A_1$ | 3 | $T_4$ | $B_6$ |
| 112 | $T_5$ | $A_1$ | 3 | $T_5$ | $B_6$ |
| 113 | $T_4$ | $A_1$ | 4 | $T_4$ | $B_6$ |
| 114 | $T_5$ | $A_1$ | 3 | $T_5$ | $B_6$ |
| 115 | $T_4$ | $A_2$ | 3 | $T_5$ | $B_1$ |
| 116 | $T_5$ | $A_4$ | 3 | $T_6$ | $B_1$ |
| 117 | $T_3$ | $A_2$ | 3 | $T_5$ | $B_2$ |
| 118 | $T_4$ | $A_1$ | 4 | $T_4$ | $B_{10}$ |
| 119 | $T_5$ | $A_2$ | 3 | $T_4$ | $B_{10}$ |
| 120 | $T_4$ | $A_1$ | 3 | $T_4$ | $B_{11}$ |
| 121 | $T_5$ | $A_2$ | 4 | $T_4$ | $B_{11}$ |

120, d. h. die Azobrücke ist in 3-Stellung gebunden färben Papier in rotstichig gelben bis orangen Tönen echt an.

In der folgenden Tabelle VII ist der strukturelle Aufbau weiterer Farbstoffe angegeben wie sie gemäss Beispiel 99 erhalten werden können. Sie entsprechen der Formel

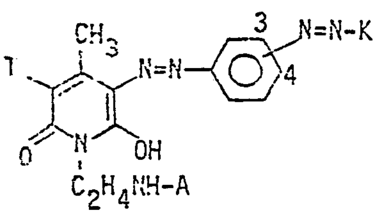

worin die Symbole die in den Kolonnen angegebenen Bedeutungen besitzen.

K-Tabelle

$K_1$ bedeutet 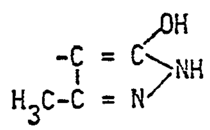

$K_2$ do 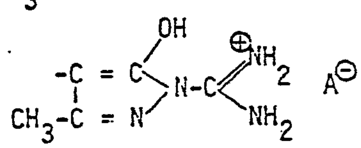

$K_3$ do 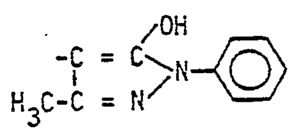

$K_4$ do 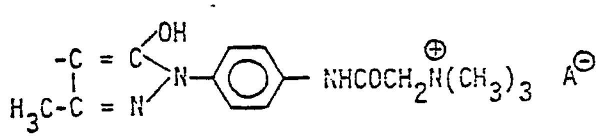

$K_5$ do 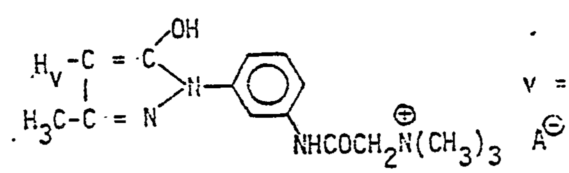 v = 0 oder 1

$K_6$ do 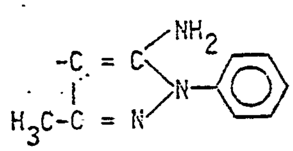

$K_7$ do 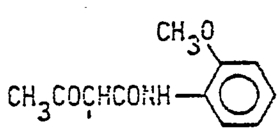

$K_8$ do 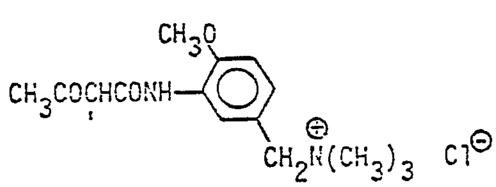

(Fortsetzung)

$K_9$ do 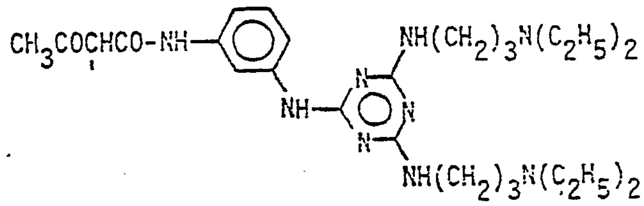

$K_{10}$ do 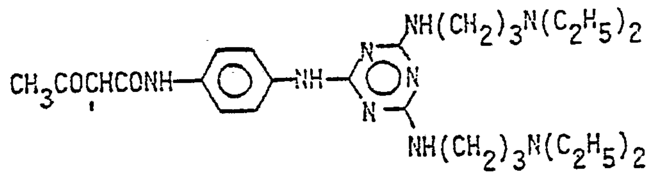

$K_{11}$ bedeutet 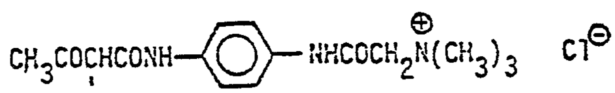

$K_{12}$ do 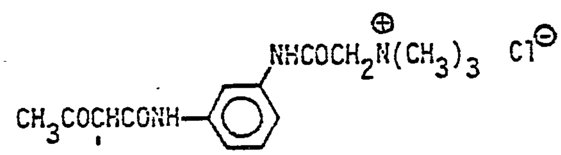

$K_{13}$ do 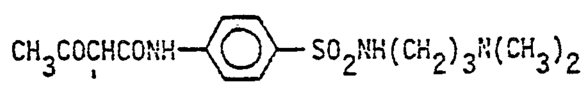

$K_{14}$ do 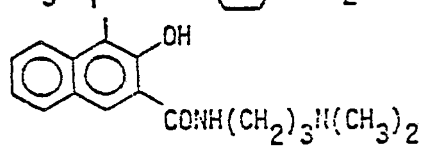

$K_{15}$ do 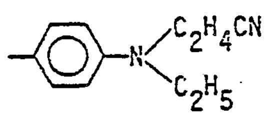

$K_{16}$ do 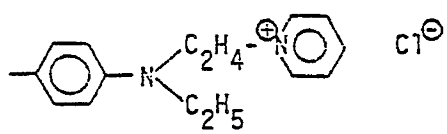

$K_{17}$ do 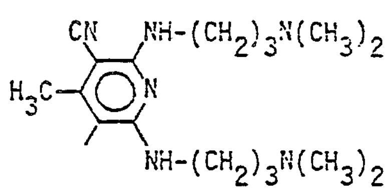

$K_{18}$ do 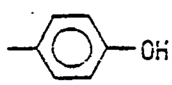

Tabelle VII

| Bsp. No. | T | A | -N = N-K (Stellung) |
|---|---|---|---|
| 122 | $T_4$ | $A_2$ | 4 - $K_1$ |
| 123 | $T_5$ | $A_2$ | 4 - $K_2$ |
| 124 | $T_6$ | $A_2$ | 4 - $K_3$ |
| 125 | $T_4$ | $A_4$ | 4 - $K_4$ |
| 126 | $T_5$ | $A_4$ | 4 - $K_5$ v = 0 |
| 127 | $T_2$ | $A_2$ | 4 - $K_6$ |
| 128 | $T_1$ | $A_2$ | 4 - $K_7$ |
| 129 | $T_4$ | $A_4$ | 4 - $K_8$ |
| 130 | $T_1$ | $A_4$ | 4 - $K_9$ |
| 131 | $T_5$ | $A_1$ | 4 - $K_{10}$ |
| 132 | $T_5$ | $A_4$ | 4 - $K_{11}$ |
| 133 | $T_6$ | $A_4$ | 4 - $K_{12}$ |
| 134 | $T_7$ | $A_2$ | 4 - $K_{13}$ |
| 135 | $T_4$ | $A_2$ | 4 - $K_{14}$ |
| 136 | $T_4$ | $A_2$ | 4 - $K_{15}$ |
| 137 | $T_5$ | $A_4$ | 4 - $K_{16}$ |
| 138 | $T_6$ | $A_2$ | 4 - $K_{17}$ |
| 139 | $T_5$ | $A_2$ | 4 - $K_{18}$ |
| 140 | $T_4$ | $A_2$ | 3 - $K_1$ |
| 141 | $T_6$ | $A_1$ | 3 - $K_5$ v = 0 |
| 142 | $T_7$ | $A_1$ | 3 - $K_8$ |
| 143 | $T_3$ | $A_4$ | 3 - $K_{12}$ |
| 144 | $T_4$ | $A_2$ | 3 - $K_{14}$ |
| 145 | $T_5$ | $A_1$ | 3 - $K_{17}$ |

Die Farbstoffe der Beispiele 122-127, 138 und 144 färben Papier in roten Tönen, die Farbstoffe der Beispiele 128-134, 142 und 143 in gelben Tönen, die Farbstoffe der Beispiele 135, 136, 137 in blauen Tönen und die Farbstoffe der Beispiele 135, 137 in blauen Tönen und die Farbstoffe der Beispiele 139-141 und 145 in orangen Tönen echt an.

Beispiel 146

8,6 Teile (1/40 Mol des Diamins der Formel

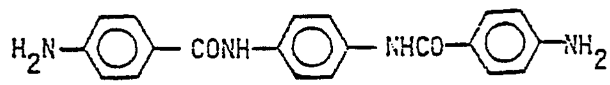

werden in salzsaurer wässriger Lösung bei 0-5° mit 3,5 Teilen (1/20 Mol) Natriumnitrit diazotiert und zu einer wässrigen Lösung von 30,8 Teilen (1/20 Mol) der Verbindung (α) gemäss Beispiel 66 gegeben. Mit Natronlauge wird die Lösung auf einen pH-Wert von 4-5 gestellt.

Man erhält den Farbstoff der Formel

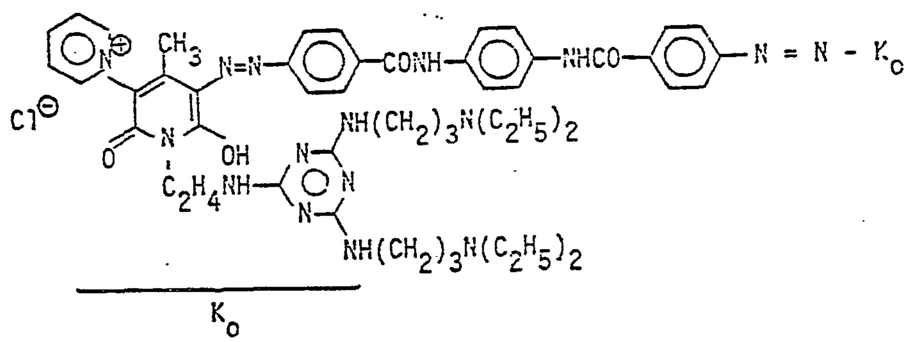

der Papier in gelben Tönen mit guten Echtheiten färbt.

In der folgenden Tabelle VIII ist der strukturelle Aufbau weiterer Farbstoffe angegeben, wie sie gemäss Beispiel 146 erhalten werden können. Sie entsprechen der Formel

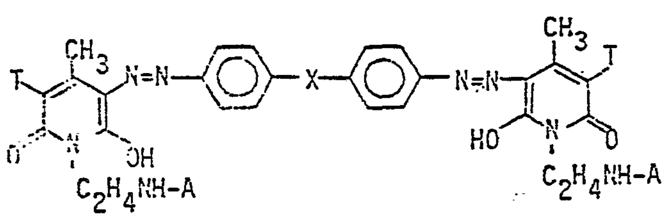

worin die Symbole die in den Kolonnen eingegebenen Bedeutungen besitzen. Der Rest X ist in der Beschreibung offenbart.

(Siehe Tabelle VIII Seite 69 ff.)

Tabelle VIII

| Bsp. No. | T | A | X |
|---|---|---|---|
| 147 | $T_1$ | $A_2$ | $X_1$ |
| 148 | $T_2$ | $A_2$ | $X_5$ |
| 149 | $T_3$ | $A_2$ | $X_6$ |
| 150 | $T_4$ | $A_4$ | $X_7$ |
| 151 | $T_5$ | $A_4$ | $X_{10}$ |
| 152 | $T_6$ | $A_2$ | $X_{11}$ |
| 153 | $T_7$ | $A_2$ | $X_{12}$ |
| 154 | $T_4$ | $A_2$ | $X_{16}$ |
| 155 | $T_1$ | $A_2$ | $X_{17}$ |
| 156 | $T_4$ | $A_4$ | $X_{22}$ |
| 157 | $T_1$ | $A_2$ | $X_{26}$ |
| 158 | $T_4$ | $A_4$ | $X_{27}$ |
| 159 | $T_5$ | $A_1$ | $X_{30}$ |
| 160 | $T_6$ | $A_2$ | $X_{45}$ |
| 161 | $T_7$ | $A_2$ | $X_{49}$ |
| 162 | $T_4$ | $A_4$ | $X_{50}$ |
| 163 | $T_5$ | $A_2$ | $X_{51}$ |
| 164 | $T_6$ | $A_2$ | $X_{52}$ |
| 165 | $T_7$ | $A_2$ | $X_{53}$ |
| 166 | $T_1$ | $A_2$ | $X_{54}$ |
| 167 | $T_2$ | $A_2$ | $X_{58}$ |
| 168 | $T_4$ | $A_4$ | $X_{59}$ |
| 169 | $T_5$ | $A_1$ | $X_{60}$ |
| 170 | $T_6$ | $A_2$ | $X_{61}$ |

| Bsp. No. | T | A | X |
|---|---|---|---|
| 171 | $T_7$ | $A_4$ | $X_{62}$ |
| 172 | $T_4$ | $A_2$ | $X_{70}$ |
| 173 | $T_1$ | $A_2$ | $X_{71}$ |
| 174 | $T_2$ | $A_2$ | $X_{73}$ |
| 175 | $T_4$ | $A_2$ | $X_{94}$ |
| 176 | $T_5$ | $A_4$ | $X_{75}$ |
| 177 | $T_6$ | $A_2$ | $X_{79}$ |
| 178 | $T_4$ | $A_4$ | $X_{80}$ |
| 179 | $T_5$ | $A_2$ | $X_{81}$ |
| 180 | $T_4$ | $A_2$ | $X_{82}$ |
| 181 | $T_4$ | $A_2$ | $X_{85}$ |
| 182 | $T_5$ | $A_2$ | $X_{86}$ |
| 183 | $T_6$ | $A_1$ | $X_{87}$ |
| 184 | $T_7$ | $A_3$ | $X_{88}$ |
| 185 | $T_2$ | $A_2$ | $X_{89}$ |
| 186 | $T_3$ | $A_4$ | $X_{90}$ |

Tabelle VIII (Fortsetzung)

| Bsp. No. | T | A | X |
|---|---|---|---|
| 187 | $T_4$ | $A_2$ | $X_{103}$ ($R_{43}$-$C_2H_4$-, $R_{45}$=$NH(CH_2)_{2-3}$-$N(C_2H_5)_2$ |
| 188 | $T_5$ | $A_2$ | $X_{107}$ |
| 189 | $T_1$ | $A_2$ | $-CONH(CH_2)_2CONH(CH_2)_2NHCO-$ |
| 190 | $T_2$ | $A_2$ | do |
| 191 | $T_3$ | $A_2$ | $-CONHC_2H_4NHCOCH=CHCONHC_2H_4NHCO-$ |
| 192 | $T_4$ | $A_3$ | $-CONHC_2H_4NHCOC_2H_4CONHC_2H_4NHCO-$ |
| 193 | $T_5$ | $A_4$ | $X_{107}$ |
| 194 | $T_5$ | $A_2$ | 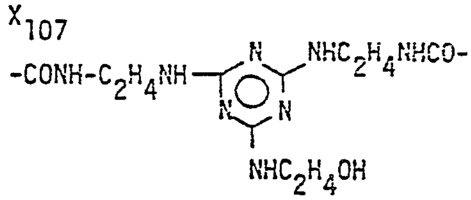 |
| 195 | $T_4$ | $A_2$ | 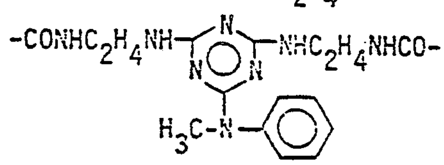 |
| 196 | $T_4$ | $A_1$ | $X_{11}$ |
| 197 | $T_4$ | $A_1$ | $X_{85}$ |
| 198 | $T_4$ | $A_1$ | $X_{82}$ |
| 199 | $T_4$ | $A_1$ | $X_{71}$ |
| 200 | $T_4$ | $A_2$ | $X_{11}$ |
| 201 | $T_4$ | $A_2$ | $X_{85}$ |
| 202 | $T_4$ | $A_2$ | $X_{82}$ |
| 203 | $T_4$ | $A_2$ | $X_{71}$ |

Die Farbstoffe der Beispiele 147 und 172 färben Papier in roten Tönen, die Farbstoffe der Beispiele 148-150, 154-157, 159-161, 163-171, 173-177, 179-195, 197-199, 201-203 in gelben Tönen, der Farbstoff des Beispiels 151 in blauen Tönen, die Farbstoffe der Beispiele 152, 153, 158, 162, 173, 177, 178, 196, 200 in orange Tönen echt an.

Beispiel 204

5,6 Teile (1/40 Mol) 4,4'-Diaminobenzanilid werden bei 0-5° in salzsaurer wässriger Lösung mit 3,5 Teilen (1/20 Mol) Natriumnitrit diazotiert. Zur stark sauren Tetrazoniumlösung tropft man langsam 7 Teile (1/40 Mol) der Verbindung der Formel

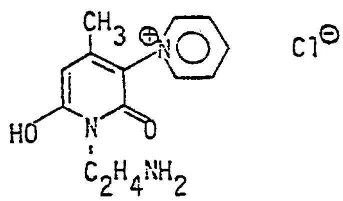

Dabei wird durch Zugabe von Natriumacetat der pH-Wert bei 1,5 gehälten. Man erhält einen gelben Monoazofarbstoff. Hierzu gibt man 8,1 Teile (1/40 Mol) einer zweiten Kupplungskomponente der Formel

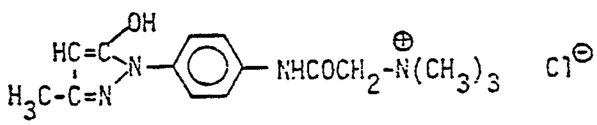

Mit Lauge wird der pH-Wert der Lösung auf 5 eingestellt und man erhält einen rotgelben Farbstoff der Formel

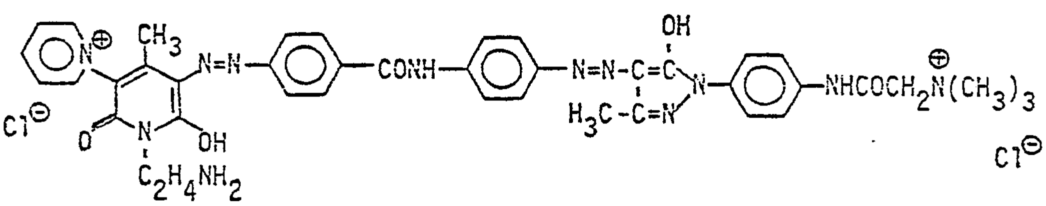

der Papier in rotgelben Tönen färbt.

In der folgenden Tabelle IX ist der strukturelle Aufbau weiterer Farbstoffe angegeben, wie sie gemäss Beispiel 204 hergestellt werden können. Sie entsprechen der Formel

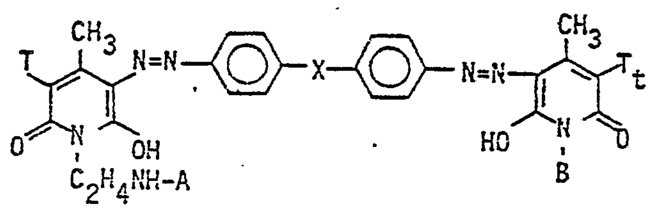

worin die Symbole die in den Kolonnen angegebenen Bedeutungen besitzen.

Tabelle IX

| Bsp. No. | T | A | $T_t$ | B | X |
|---|---|---|---|---|---|
| 205 | $T_1$ | $A_2$ | $T_4$ | $B_1$ | $X_1$ |
| 206 | $T_2$ | $A_2$ | $T_5$ | $B_2$ | $X_2$ |
| 207 | $T_3$ | $A_3$ | $T_6$ | $B_3$ | $X_{10}$ |

Tabelle IX (Fortsetzung)

| Bsp. No. | T | A | $T_t$ | B | X |
|---|---|---|---|---|---|
| 208 | $T_4$ | $A_1$ | $T_1$ | $B_4$ | $X_{11}$ |
| 209 | $T_5$ | $A_2$ | $T_4$ | $B_6$ | $X_{51}$ |
| 210 | $T_6$ | $A_2$ | $T_5$ | $B_7$ | $X_{53}$ |
| 211 | $T_7$ | $A_2$ | $T_1$ | $B_9$ | $X_{59}$ |
| 212 | $T_4$ | $A_4$ | $T_5$ | $B_1$ | $X_{70}$ |
| 213 | $T_5$ | $A_2$ | $T_1$ | $B_1$ | $X_{81}$ |
| 214 | $T_4$ | $A_4$ | $T_2$ | $B_4$ | $X_{82}$ |
| 215 | $T_5$ | $A_2$ | $T_4$ | $B_1$ | $X_{25}$ |
| 216 | $T_4$ | $A_4$ | $T_4$ | $B_1$ | $X_{86}$ |
| 217 | $T_4$ | $A_2$ | $T_4$ | $B_{10}$ | $X_{11}$ |
| 218 | $T_4$ | $A_2$ | $T_4$ | $B_{11}$ | $X_{70}$ |

In der folgenden Tabelle X ist der strukturelle Aufbau weiterer Farbstoffe angegeben, wie sie gemäss Beispiel 204 hergestellt werden können. Sie entsprechen der Formel

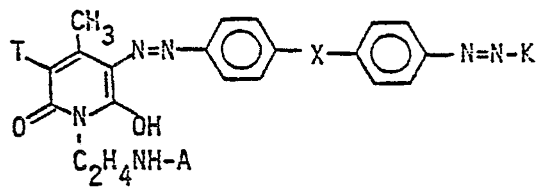

worin die Symbole die in der Tabelle angegebenen Bedeutungen besitzen.

Tabelle X

| Bsp. No. | T | A | X | K |
|---|---|---|---|---|
| 219 | $T_4$ | $A_2$ | $X_1$ | $K_1$ |
| 220 | $T_5$ | $A_2$ | $X_6$ | $K_4$ |
| 221 | $T_1$ | $A_4$ | $X_{11}$ | $K_5$ v = 0 |
| 222 | $T_4$ | $A_1$ | $X_{18}$ | $K_7$ |
| 223 | $T_5$ | $A_4$ | $X_{55}$ | $K_8$ |
| 224 | $T_4$ | $A_2$ | $X_{59}$ | $K_{10}$ |
| 225 | $T_6$ | $A_4$ | $X_{70}$ | $K_{11}$ |
| 226 | $T_7$ | $A_2$ | $X_{82}$ | $K_{16}$ |
| 227 | $T_4$ | $A_2$ | $X_{85}$ | $K_{17}$ |
| 228 | $T_5$ | $A_2$ | $X_{11}$ | $K_{14}$ |

Die Farbstoffe der Beispiele 205, 212, 218 und 228 färben Papier in roten Tönen, diejenigen der Beispiele 206, 208, 217-221 in orangen Tönen, derjenige des Beispiels 207 in blauen Tönen, diejenigen der Beispiele 209-211, 213-216 und 222-227 in gelben Tönen echt an.

Beispiel 229

18,4 Teile (1/10 Mol) Cyanurchlorid werden in Wasser zuerst mit 37,2 Teilen (1/10 Mol) des Monoazofarbstoffes der Formel

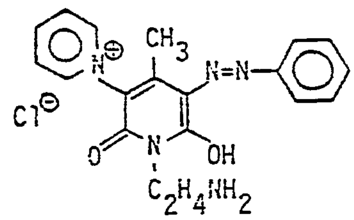

bei 5-10° und einem pH-Wert von 7, dann mit 13 Teilen (1/10 Mol) N,N-Diäthylaminopropylamin bei 20-25° und einem pH-Wert von 9 umgesetzt. Man erhält den Monoazofarbstoff der Formel

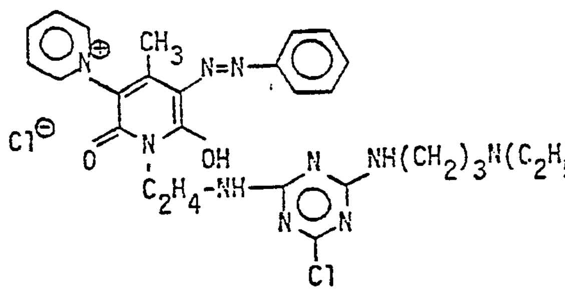

δ)

23,9 Teile (1/20 Mol) des erhaltenen Farbstoffs δ) werden zusammen mit 19,3 Teilen (1/20 Mol) des Monoazofarbstoffes der Formel

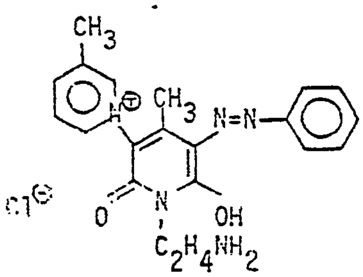

in Wasser bei 90° und einem pH-Wert von 9 zu einem Farbstoff der Formel

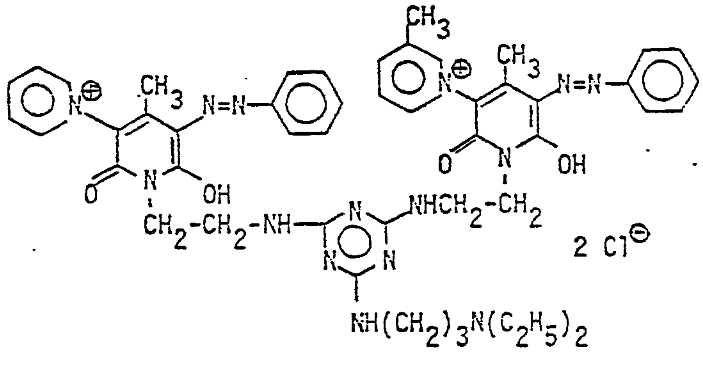

umgesetzt. Er färbt Papier in gelben echten Tönen.

Beispiel 230

a) 18,8 Teile des Aminomonoazofarbstoffes (γ) gemäss Beispiel 99b) werden bei 0-5° in salzsaurer Lösung mit 1,75 Teilen (1/40 Mol) Natriumnitrit diazotiert und bei einem pH-Wert von 1-1,5 auf 3 Teile (1/40 Mol) 2-Amino-1,4-dimethylbenzol gekuppelt. Der entstandene Disazofarbstoff wird wiederum mit 1,75 Teilen (1/40 Mol) Natriumnitrit bei 0-5° in Salzsaurer wässriger Lösung diazotiert und bei einem pH-Wert von 4-5 auf 8,1 Teile (1/40 Mol) der Kupplungskomponente $K_5$ (v = 1) gekuppelt. Man erhält den Trisazofarbstoff der Formel

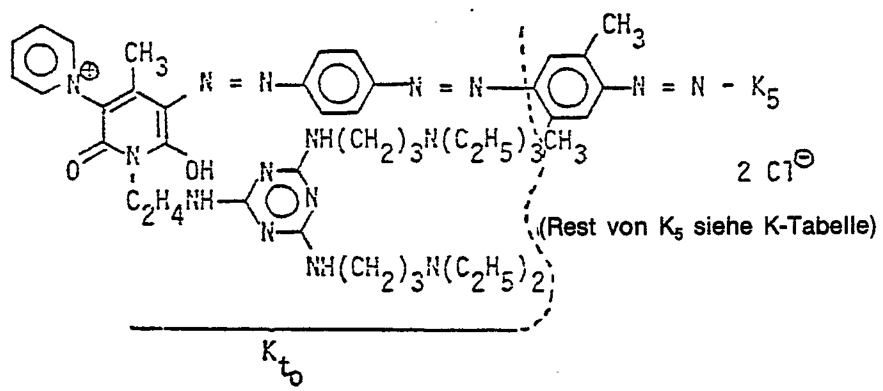
(Rest von $K_5$ siehe K-Tabelle)

der Papier in braunen Tönen färbt.

b) Verwendet man anstelle von 2-Amino-1,4-dimethylbenzol die äquivalente Menge 2-Methoxy-5-methylanilin, so erhält man nach den obigen Angaben den Farbstoff der Formel

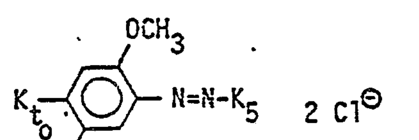
(ε)

(Rest von $K_5$ siehe K-Tabelle)

der Papier in braunen Tönen färbt.

Dieser Farbstoff (ε) kann nach bekannten Methoden in die Metallkomplexform, wie Kupferkomplexform übergeführt werden, er entspricht der Formel

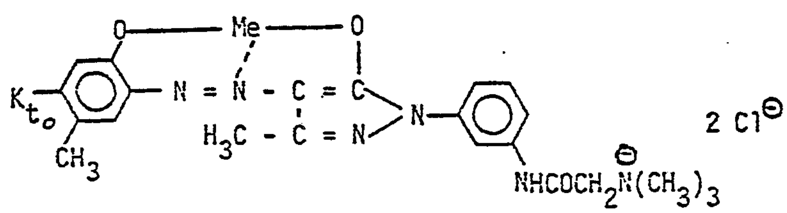

worin Me ein Kupfer-, atom bedeutet.

In der folgenden Tabelle XI ist der strukturelle Aufbau weiterer Farbstoffe angegeben, wie sie gemäss den Angaben im Beispiel 230 hergestellt werden können. Sie entsprechen der Formel

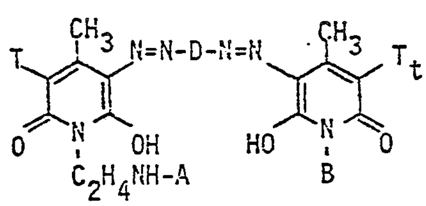

worin die Symbole die in den Kolonnen angegebenen Bedeutungen besitzen.

Tabelle XI

| Bsp. No. | T | A | $T_t$ | B | D |
|---|---|---|---|---|---|
| 231 | $T_5$ | $A_2$ | $T_5$ | $B_8$ | 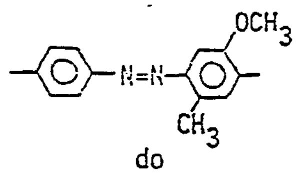 |
| 232 | $T_4$ | $A_4$ | $T_3$ | $B_4$ | do |
| 233 | $T_2$ | $A_2$ | $T_6$ | $B_6$ | do |
| 234 | $T_4$ | $A_2$ | $T_4$ | $B_8$ | 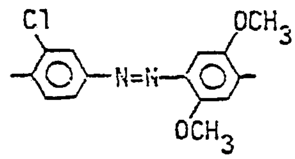 |
| 235 | $T_1$ | $A_2$ | $T_5$ | $B_1$ | do |
| 236 | $T_4$ | $A_2$ | $T_7$ | $B_2$ | do |
| 237 | $T_4$ | $A_1$ | $T_4$ | $B_6$ | 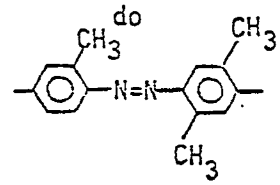 |
| 238 | $T_5$ | $A_2$ | $T_4$ | $B_3$ | do |
| 239 | $T_4$ | $A_2$ | $T_4$ | $B_8$ | do |
| 240 | $T_4$ | $A_2$ | $T_4$ | $B_8$ | 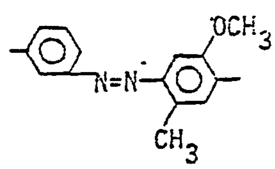 |
| 241 | $T_5$ | $A_4$ | $T_7$ | $B_1$ | do |
| 242 | $T_4$ | $A_2$ | $T_4$ | $B_8$ | 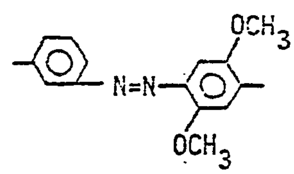 |
| 243 | $T_5$ | $A_4$ | $T_7$ | $B_1$ | do |

In der folgenden Tabelle XII ist der strukturelle Aufbau weiterer Farbstoffe angegeben. Sie können nach den Angaben im Beispiel 230 hergestellt werden und entsprechen der Formel

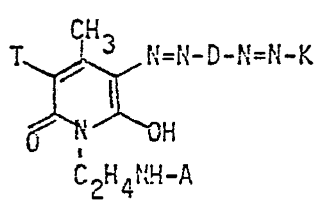

K = K-Tabelle
worin die Symbole die in den Kolonnen angegebenen Bedeutungen besitzen.

Tabelle XII

| Bsp. No. | T | A | K | D |
|---|---|---|---|---|
| 244 | $T_5$ | $A_2$ | $K_5$ v = 0 | 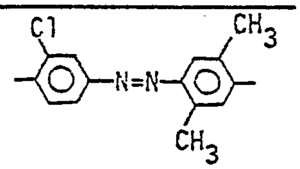 |
| 245 | $T_6$ | $A_4$ | $K_9$ | do |
| 246 | $T_1$ | $A_1$ | $K_{17}$ | do |
| 247 | $T_4$ | $A_2$ | $K_3$ | 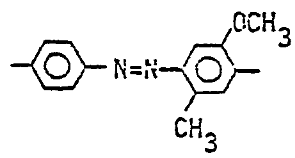 |
| 248 | $T_5$ | $A_2$ | $K_{10}$ | do |
| 249 | $T_6$ | $A_2$ | $K_8$ | do |
| 250 | $T_4$ | $A_2$ | $K_4$ | 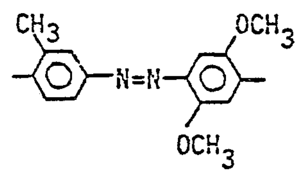 |
| 251 | $T_7$ | $A_4$ | $K_{16}$ | do |
| 252 | $T_4$ | $A_2$ | $K_{11}$ | 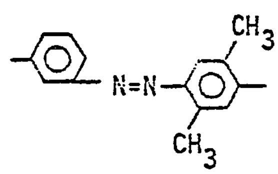 |
| 253 | $T_5$ | $A_4$ | $K_{14}$ | do |
| 254 | $T_4$ | $A_2$ | $K_2$ | 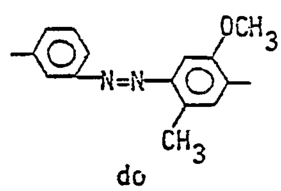 |
| 255 | $T_4$ | $A_2$ | $K_{13}$ | do |
| 256 | $T_4$ | $A_2$ | $K_{10}$ | 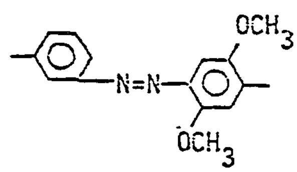 |
| 257 | $T_5$ | $A_2$ | $K_5$ v = 0 | do |

Die Farbstoffe der Beispiele 231-233, 237-241, 244-249, 252-255 färben Papier in violetten Tönen, diejenigen der Beispiele 234-236, 242, 243, 250, 251, 256 und 257 in blauen Tönen echt an.

Beispiel 258

a) 1/10 Mol der Aminoazoverbindung (γ) gemäss Beispiel 99 b) werden nach bekannter Methode mit 4n Natriumnitrit diazotiert und in einer wässrigen Lösung, die einen pH-Wert von 5 aufweist, mit 1 Mol einer Kupplungskomponente der Formel

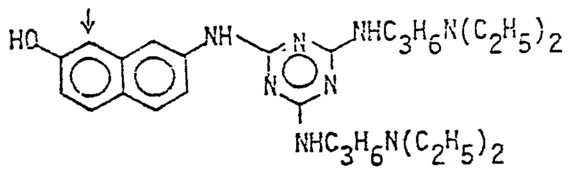

(γ1)

gekuppelt. Man erhält einen wasserlöslichen Farbstoff, der Papier in violetten Tönen echt färbt.

b) Die Verbindung (γ1) lässt sich wie folgt herstellen :

91 Teile Cyanurchlorid werden in 125 Teilen Eis und Wasser zu einer feinen Suspension verrührt. Bei 5 bis 10° werden 133 Teile N,N-Diäthylaminopropylamin innerhalb 3 Stunden zugetropft. Nach einer Stunde rühren werden 75 Teile 2-Amino-7-hydroxynaphthalin zugefügt. Man erwärmt das Gemisch auf 90° und lässt 3 Stunden bei dieser Temperatur rühren. Nach dieser Zeit ist die Kondensation beendet. Während der ganzen Kondensationszeit wird der pH-Wert der Lösung durch Zugabe von Natriumacetat bei 3-4 gehalten.

Die Verbindung (γ1) lässt sich aber auch herstellen, wenn man in einer 1. Stufe 2-Amino-7-hydroxynaphthalin mit Cyanurchlorid kondensiert und die so erhaltene Zwischenverbindung der Formel

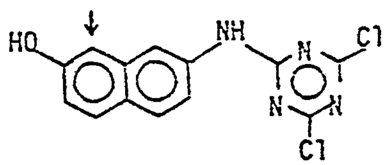

stufenweise mit einem Diamin oder mit verschiedenen Diaminen weiter kondensiert, d. h. man kann mit einem aliphatischen, aromatischen oder heterocyclischen Amin umsetzen, welches keine externe protonierbare Gruppe enthält. Man kann beispielsweise in einer ersten Stufe mit Monoäthanolamin und in einer zweiten Stufe mit N,N-Diäthylaminopropylamin zu einer Verbindung der Formel

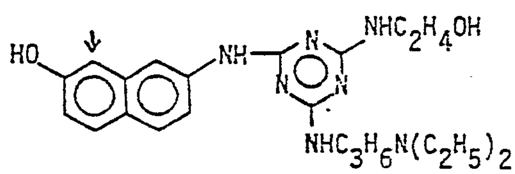

kondensieren.

Anstelle des Diamins bei der Herstellung zur Verbindung (γ1) können auch äquivalente Menge anderer Diamine eingesetzt werden ; wie Aethylendiamin, N,N-Dimethyläthylen- oder propylendiamin, Propylendiamin, Dipropylentriamin, Diäthylentriamin, Piperazin, N-Methylpiperazin, N-hydroxyäthylpiperazin oder die entsprechenden Salze oder auch aromatische Amine, wie Anilin, das im Phenylkern, z. B. in meta- oder para-Stellung durch eine Trimethylammoniumgruppe oder durch eine Methylendimethyl-, diäthylaminogruppe oder durch eine Methylentrimethylammoniumgruppe substituiert sein kann. Anstelle des gemäss Beispiel 258 b) genannten 2-Amino-7-hydroxynaphthalin kann man auch 3-, 4-, 5-, 6- oder 7-Amino-1-naphthol oder 3-, 4-, 5- oder 6-Amino-2-naphthol oder 8-Amino-2-naphthol einsetzen. Farbstoffe mit einer Aminonaphtholkomponente ergeben auf Papier gefärbte blauviolette Töne.

Beispiel 259

Kuppelt man nach den Angaben im Beispiel 258 die Diazoverbindung der Verbindung (γ) auf a) 2-Aminonaphthalin-5-methyltrimethylammoniumchlorid Verbindung, so erhält man einen Farbstoff mit blaustichig roter Nuance oder auf eine Verbindung der Formel b)

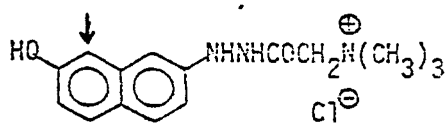

so erhält man einen ähnlich guten Farbstoff, der Papier in blauvioletten Tönen echt anfärbt.

Beispiel 260

Kuppelt man die Diazoverbindung gemäss Beispiel 259 auf eine Verbindung der Formel

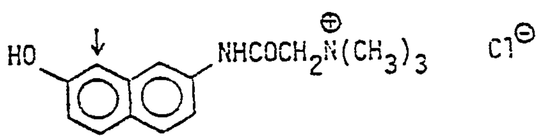

(γ2)

so erhält man einen ähnlich guten Farbstoff, der Papier in blauvioletten Tönen echt färbt.

Die Verbindung (γ2) kann wie folgt hergestellt werden : 16 Teile 2-Amino-7-hydroxynaphthalin werden in 300 Teilen Wasser suspendiert und auf 60° erwärmt. Zur Lösung tropft man 15 Teile Chloracetylchlorid zu und hält den pH-Wert der Lösung durch Zugabe von Natriumacetat bei 3-4. Das erhaltene chloracetylierte Produkt wird in Wasser bei Kochtemperatur in einem Ueberschuss mit wässrigem Trimethylamin umgesetzt. Nach 2 Stunden ist die Quaternierung zur Verbindung (γ2) beendet. Anstelle von Trimethylamin kann man auch α- oder β-Picolin, Pyridin, Piperazin, Piperidin, Morpholin, N,N-Dimethylhydrazin, N,N-Dimethyläthanolamin, N,N-Dimethylaminopropylamin, oder N,N,N',N'-Tetramethyldiaminopropan verwenden.

Als Ausgangsnaphthole kann man die gemäss Beispiel 258 genannten Aminonaphthole einsetzen.

Die Herstellung dieser Naphthole ist aus « The chemistry and Technology of naphthalene compounds by Norman Donaldson (1958) bekannt und beschrieben.

Beispiel 261

Herstellung der Ausgangsverbindungen (261a und 261b)

261a) Kupplungskomponente der Formel

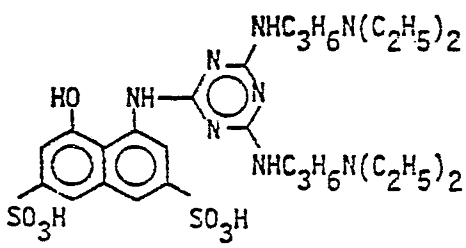

91 Teile Cyanurchlorid werden in 240 Teilen Eiswasser suspendiert. Danach tropft man bei 5-10° 133 Teile N,N-Diäthylaminopropylamin zu, lässt 3 Stunden nachrühren und setzt dann 127 Teile 1-Hydroxy-8-aminonaphthalin-3,6-disulfonsäure zu. Die Temperatur wird auf 90° erhöht, der pH durch portionenweises Eintragen von 54 Teilen Natriumacetat bei 2,5-3 gehalten. Nach 2 Stunden ist die Kondensation beendet. Man lässt unter Rühren auf 20° abkühlen, worauf das gewünschte Produkt kristallin ausfällt und abgesaugt wird. Nach dem Trocknen erhält man 265 Teile eines grauen Pulvers, das 217 Teile der oben angeführten Kupplungskomponente enthält.

261b) Aminoazofarbstoff der Formel

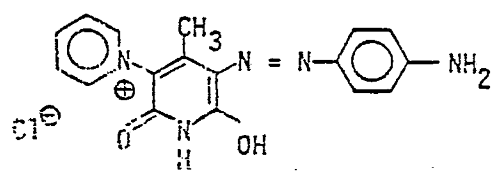

30 Teile 4-Aminoacetanilid werden in 500 Teilen Eiswasser bei 0° mit 50 Teilen konzentrierter Salzsäure versetzt ; anschliessend werden 55 Teile einer 4N Natriumnitritlösung zugetropft. Man trägt dann 42 Teile 6-Hydroxy-4-methyl-pyridonyl-(3)-pyridinium-betainbase ein und stellt den pH mit Natriumcarbonat auf 6 ein. Die Kupplung zu einem orangen Farbstoff, der vollständig ausfällt, setzt sofort ein. Nach beendeter Kupplung wird der Farbstoff abgesaugt, der erhaltene Presskuchen wird in 300 Teilen Wasser suspendiert, mit 100 Teilen konzentrierter Salzsäure versetzt und auf 95° erhitzt. Die Abspaltung der Acetylgruppe ist nach 2 Stunden beendet, es entsteht eine Lösung. Man lässt anschliessend auf Raumtemperatur kaltrühren, worauf der Aminoazofarbstoff in Form gelber Kristallplättchen ausfällt. Er wird abgesaugt, mit Sole gewaschen und getrocknet. Man erhält 170 Teile eines gelben Pulvers, das 68 Teile des Farbstoffs obiger Formel enthält.

261c) Endfarbstoff der Formel

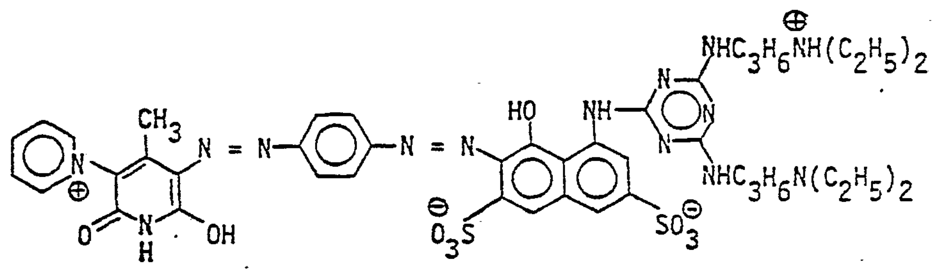

20,5 Teile der gemäss Beispiel 261a) hergestellten Kupplungskomponente werden in 200 Teilen Wasser suspendiert und durch Zugabe von Natronlauge gelöst.

22,4 Teile des gemäss Beispiel 261b) hergestellten Aminoazofarbstoffes werden auf übliche Weise mit Natriumnitrit diazotiert. Die entstandene Diazolösung wird zu obiger Vorlage der Kupplungskomponente getropft, wobei der pH durch Zusatz von Natronlauge bei 8-9 gehalten wird. Die Kupplung zu einer blauen Farbstofflösung setzt sofort ein. Nach beendeter Kupplung wird der pH durch Zutropfen von Salzsäure auf 7,5 gesenkt, der Farbstoff fällt dabei vollständig aus, er wird abfiltriert und getrocknet. Man erhält den Farbstoff obiger Formel als blaues Pulver ; der Farbstoff färbt in Form eines Säureadditionssalzes Papier in blauen Tönen an. Das Abwasser ist farblos, die Nassechtheiten der Färbung sind perfekt.

## Beispiel 262

Wird die entsprechende Menge 4-Aminoacetanilid zunächst mit der Kupplungskomponente gemäss Beispiel 261a) gekuppelt, sauer verseift, diazotiert und dann auf 6-Hydroxy-4-methyl-pyridonyl-(3)-pyridinium-betainbase gekuppelt, so erhält man den identischen Farbstoff wie in Beispiel 261c) beschrieben.

## Beispiel 263

Wird anstelle von 4-Aminoacetanilid die entsprechende Menge p-Nitroanilin eingesetzt, dessen Nitrogruppe in alkalischem Medium mit einem Salz des Schwefelwasserstoffs reduziert wird, und ansonsten analog verfahren wie in den Beispielen 261 und 262, so erhält man den identischen Farbstoff wie in Beispiel 261c) angegeben.

Analog der in den Beispielen 261-263 beschriebenen Methode können weitere Verbindungen hergestellt werden, für welche entsprechend der Formel

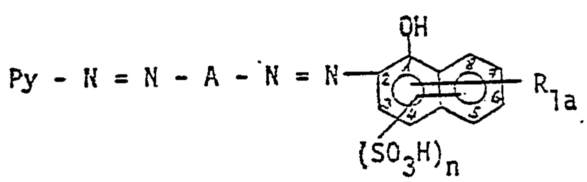

in der nachfolgenden Tabelle XIII die Variablen und für die n Sulfogruppen wie für den Rest $R_{1a}$ auch die Stellungen im Naphtholrest angegeben sind. Es bedeuten im Rest $R_{1a}$ dabei $Y_1$ bis $Y_3$ den Rest

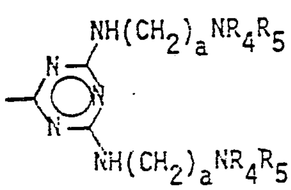

mit für $Y_1$ $R_4 = R_5 = C_2H_5$ und a = 3 ; für $Y_2$ $R_4 = R_5 = CH_3$ und a = 3 ; und für $Y_3$ $R_4 = R_5 = C_2H_5$ und a = 2 ;

und $Y_4$ den Rest 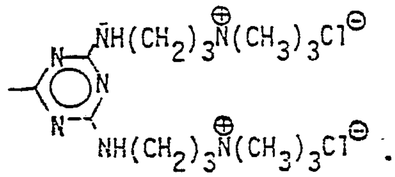

Im Rest A ist das mit *bezeichnete C-Atom an die Azogruppe zum Pyridonrest gebunden.

Die mit diesen Farbstoffen (als Säureadditionssalz oder quaternäres Ammoniumsalz) erhältenen Färbungen auf Papier, deren Farbton in der Kolonne I angeführt ist, zeigen gute Nassechtheiten. Die in Kolonne I verwendeten Zahlen bedeuten folgenden Farbton :

1 = orange ; 2 = blaustichig rot ; 3 = rotstichig blau ; 4 = blau ;
5 = graublau ; 6 = rotviolett ; 7 = blauviolett ; 8 = violett ; 9 = braun.

Tabelle XIII

| Bsp. Nr. | Py | A | n (Stellung der $SO_3H$) | $R_{12}$ (Stellung) | I |
|---|---|---|---|---|---|
| 264 | 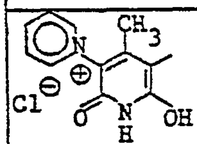 | 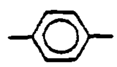 | 1 (3) | $-NHC_3H_6N(CH_3)_2$ (6) | 8 |
| 265 | do. | do. | do. | $-NH-Y_1$ (6) | 7 |
| 266 | do. | do. | do. | $-NH-Y_2$ (6) | 7 |
| 267 | do. | do. | do. | $-NH-Y_4$ (6) | 7 |
| 268 | do. | do. | do. | $-NH-Y_1$ (7) | 5 |
| 269 | do. | do. | do. | $-NH-Y_3$ (7) | 5 |
| 270 | do. | do. | do. | $-NHCOCH_2$ 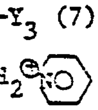 $Cl^{\ominus}$ (6) | 6 |
| 271 | do. | do. | 2 (3;6) | $-NH-Y_2$ (8) | 4 |
| 272 | do. | do. | do. | $-NH-Y_3$ (8) | 4 |
| 273 | do. | do. | do. | $-NH-Y_4$ (8) | 4 |
| 274 | do. | 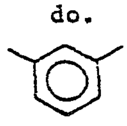 | 1 (3) | $-NH-Y_1$ (6) | 1 |
| 275 | do. | do. | do. | $-NH-Y_2$ (6) | 1 |

(Siehe Fortsetzung Seite 81 ff.)

(Fortsetzung)

| Bsp. Nr. | Py | A | n (Stellung der $SO_3H$) | $R_{1a}$ (Stellung) | I |
|---|---|---|---|---|---|
| 276 | 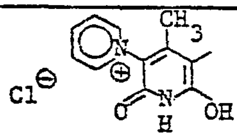 | 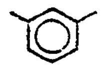 | 2 (3;6) | $-NH-Y_1$ (8) | 6 |
| 277 | do. | do. | do. | $-NH-Y_3$ (8) | 6 |
| 278 | do. | 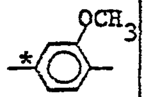 | do. | $-NH-Y_1$ (8) | 4 |
| 279 | do. | do. | do. | $-NH-Y_2$ (8) | 4 |
| 280 | do. | 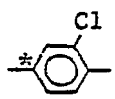 | do. | $-NH-Y_1$ (8) | 4 |
| 281 | do. | 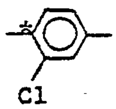 | do. | do. | 4 |
| 282 | do. | 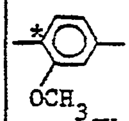 | do. | do. | 4 |
| 283 | do. | 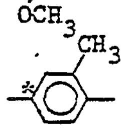 | do. | do. | 4 |
| 284 | do. | 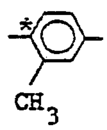 | do. | do. | 4 |
| 285 | do. | do. | do. | $-NH-Y_2$ (8) | 4 |
| 286 | do. | 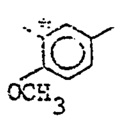 | do. | $-NH-Y_1$ (8) | 3 |
| 287 | 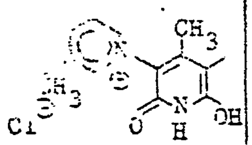 | 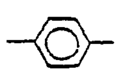 | do. | do. | 4 |
| 288 | do. | do. | do. | $-NH-Y_2$ (8) | 4 |

(Fortsetzung)

| Bsp. Nr. | Py | A | n (Stellung der $SO_3H$) | $R_{1a}$ (Stellung) | z |
|---|---|---|---|---|---|
| 289 | 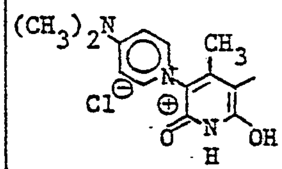 | 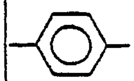 | 2 (3;6) | $-NH-Y_1$ (8) | 4 |
| 290 | 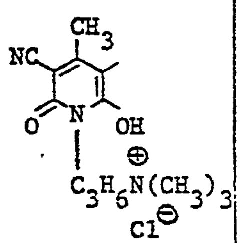 | do. | do. | do. | 3 |
| 291 | 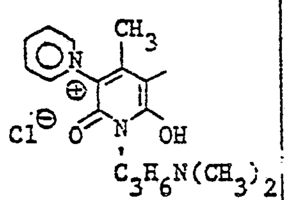 | do. | do. | do. | 4 |
| 292 | do. | do. | do. | $-NH-Y_2$ (8) | 4 |
| 292 | 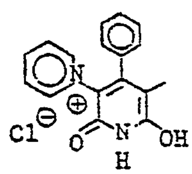 | do. | do. | $-NH-Y_1$ (8) | 4 |
| 293 | 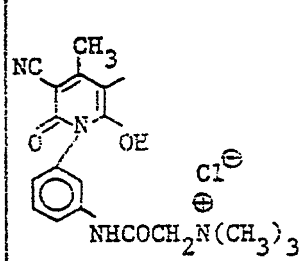 | do. | do. | do. | 4 |
| 294 | 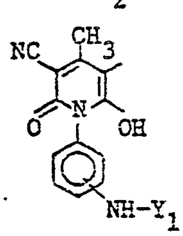 | do. | do. | do. | 4 |

(Fortsetzung)

| Bsp. Nr. | Py | A | n (Stellung der $SO_3H$ | $R_{1a}$ (Stellung) | I |
|---|---|---|---|---|---|
| 296 | 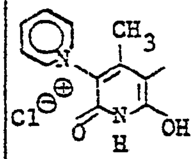 | 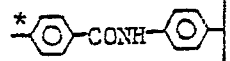 | 2 (3;6) | $-NH-Y_1$ (8) | 9 |
| 297 | do. | do. | do. | $-NH-Y_3$ (8) | 9 |
| 298 | do. | 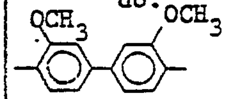 | do. | $-NH-Y_1$ (8) | 4 |
| 299 | 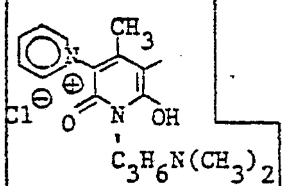 | 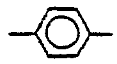 | 1 (4) | H | 2 |
| 300 | 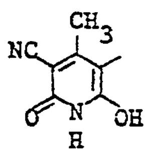 | do. | 0 | $-NH-Y_1$ (4) | 2 |

Die in der vorstehenden wie in der folgenden Tabelle angeführten Beispiele können dadurch, dass eine basische oder kationische Gruppe in Wechselwirkung tritt mit einer Sulfogruppe, als innere Salze vorliegen. Ueberzählige basische Gruppen sind durch Behandlung mit Säure in wasserlösliche Säureadditionssalze überführbar.

Die der Einfachheit halber als Chloride angeführten kationischen Gruppen können auch als innere Salze mit einer Sulfogruppe formuliert werden oder in Abhängigkeit von Herstellungs- und Isolierungsbedingungen oder durch entsprechende zusätzliche Massnahmen durch ein anderes übliches Anion neutralisiert sein.

Eine exakte Zuordnung von Kation zu Anion ist nicht möglich, da bei Auflösung des Farbstoffs in Wasser zugeordnete Ionen nicht mehr lokalisiert, sondern verschiebbar sind und die Zuordnung dabei auch von Fremdionen beeinflusst wird. Die jeweils gewählte ionisierte Form des Farbstoffmoleküls stellt deshalb nur eine von mehreren Möglichkeiten dar.

## Beispiel 301

a) Der als Ausgangsmaterial eingesetzte Aminoazofarbstoff der Formel

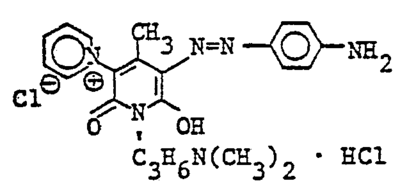

wird folgendermassen hergestellt :

11,7 Teile 4-Aminoacetanilid werden in 400 Teilen Eiswasser mit 13 Teilen konzentrierter Salzsäure versetzt und mit 13 Teilen einer 4N Natriumnitritlösung diazotiert. Anschliessend trägt man 20,5 Teile 3-Pyridinium-1-(3'-dimethylamino)-propyl-6-hydroxy-4-methylpyridon(2)-chlorid ein und stellt mit

Natronlauge auf pH 6 ein. Nach kurzer Zeit ist die Kupplung beendet und eine orange Farbstofflösung entstanden. Man gibt dann 100 Teile konzentrierte Salzsäure zu und erhitzt die Lösung auf 95°. Nach 2 Stunden ist die Abspaltung der Acetylgruppe vollendet. Es resultieren 600 Teile einer Lösung, die 35 Teile des Farbstoffs obiger Formel enthält.

75 Teile der unter a) hergestellten Lösung werden mit 3 Teilen einer 4N Natriumnitritlösung diazotiert und anschliessend in eine sodaalkalische Lösung enthaltend 2,5 Teile 5,5'-Dihydroxy-7,7'-disulfo-2,2'-dinapthylharnstoff getropft. Die Kupplung wird durch Zugabe von 10 Teilen Natronlauge vervollständigt. Es bildet sich ein blauer Farbstoff, der vollständig ausfällt, abgesaugt und getrocknet wird. Man erhält den Farbstoff entsprechend der Formel

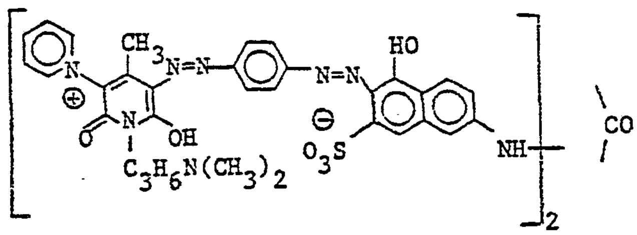

in Form eines blauen Pulvers. Als Säureadditionssalz färbt der Farbstoff Papier in graublauen Tönen.

Analog der in Beispiel 301 angeführten Methode können weitere Verbindungen durch Umsetzung mit anderen zweimal kuppelbaren Naphtholderivaten hergestellt werden, die in Tabelle XIV aufgezählt sind. Sie entsprechen der Formel

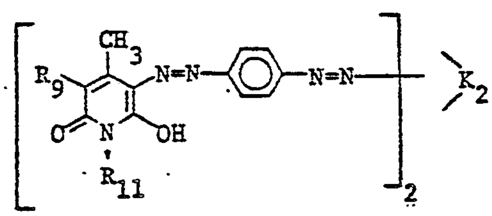

die Bedeutung der Reste $Y_1$ und $Y_2$ ist dieselbe wie für Tabelle XII. Aus der letzten Kolonne I ist der Farbton der mit dem Säureadditionssalz des jeweiligen Farbstoffes erreichten Papierfärbung ersichtlich.

Tabelle XIII

| Bsp.Nr. | $R_9$ | $R_{11}$ | $-K_2-$ | I |
|---|---|---|---|---|
| 302 | 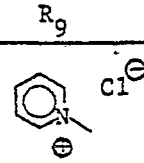 | $-(CH_2)_3N(C_2H_5)_2$ | 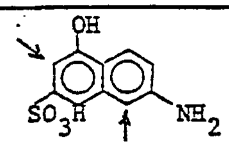 | braun-rot |
| 303 | do. | $-(CH_2)_3N(CH_3)_2$ | do. | do. |
| 304 | do. | do. | 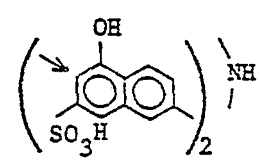 | violett |
| 305 | do. | do. | 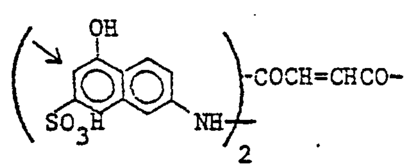 | blau |

Tabelle XIII (Fortsetzung)

| Bsp.Nr. | $R_9$ | $R_{11}$ | $-K_2-$ | T |
|---|---|---|---|---|
| 306 | 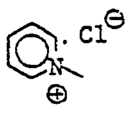 | $-(CH_2)_3N(CH_3)_2$ | 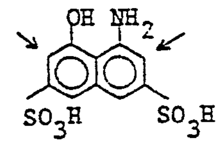 | schwarz |
| 307 | CN | 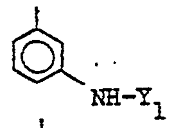 | do. | do. |
| 308 | do. | 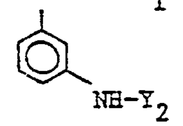 | do. | do. |

## Beispiel 309

22,4 Teile des gemäss Beispiel 261c) hergestellten Aminoazofarbstoffs werden mit 8 Teilen einer 4N Natriumnitritlösung diazotiert und bei pH 9-10 in eine Lösung enthaltend 7,6 Teile 2-Amino-5-hydroxy-naphthalin-7-sulfonsäure getropft. Nach erfolgter Kupplung wird sauer gestellt und nochmals mit 8 Teilen einer 4N Natriumnitritlösung diazotiert. Man kuppelt anschliessend mit 6,5 Teilen 6-Hydroxy-4-methyl-pyridonyl-(3)pyridinium-betainbase und erhält den Farbstoff der Formel

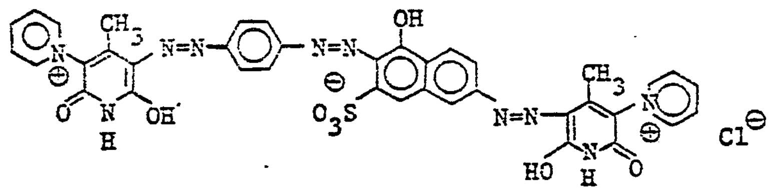

der Papier in grauvioletten Tönen färbt.

## Beispiel 310

10 Teile des Farbstoffs aus Beispiel 278 werden in 200 Teilen Wasser verrührt und auf 80° erwärmt. Dann giesst man eine Lösung bestehend aus 3 Teilen Kupfersulfat · Pentahydrat in 20 Teilen Wasser und 15 Teilen konzentrierter Ammoniaklösung zu und erhöht die Temperatur auf 90-95°. Nach 4 Stunden ist die entmethylierende Kupferung beendet. Es werden 30 Teile Kochsalz zugefügt, auf Raumtemperatur abgekühlt und anschliessend 5 Teile Natronlauge zugetropft. Der ausgefallene Farbstoff wird abfiltriert und getrocknet. Man erhält den Farbstoff der Formel

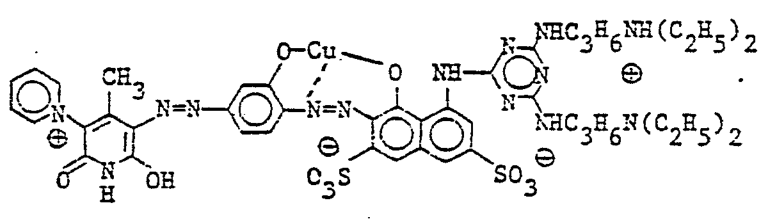

als Pulver; er färbt (als Säureadditionssalz) Papier in graublauen Tönen. Das Abwasser ist farblos. Die Nassechtheiten der Papierfärbung sind perfekt, die Lichtechtheit ausgezeichnet.

## Beispiel 311

28 Teile des unter Beispiel 261c) erhaltenen Farbstoffpulvers werden in 90 Teilen Wasser und 10 Teilen Milchsäure verrührt und bei 60° gelöst. Anschliessend wird die blaue Farbstofflösung klärfiltriert. Man erhält 125 Teile eines Filtrats, das bei Raumtemperatur mehrere Monate lagerungsstabil ist und weder in der Kälte noch in der Wärme irgendwelche Ausscheidungen aufweist. Die Lösung wird direkt oder nach Verdünnen mit Wasser zum Färben von Papier eingesetzt.

Analog können die Farbstoffsalze der übrigen Beispiele zu stabilen flüssig-wässrigen Farbstoffzubereitungen verarbeitet werden.

Beispiel 312

24 Teile des unter Beispiel 261c) erhaltenen Pulvers werden in 500 Teilen Wasser verrührt und mit 5 Teilen Eisessig sauer gestellt. Der Farbstoff geht dabei in Lösung. Hierauf wird die Lösung zur Trockne eingedampft. Man erhält das Farbstoffsalz entsprechend der Formel

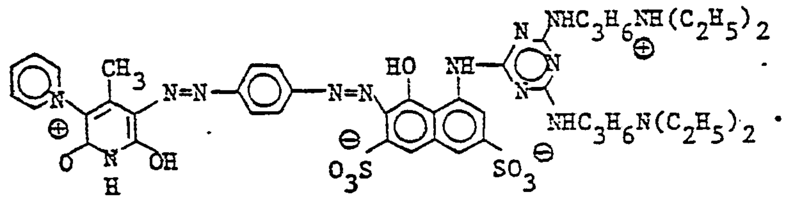 · $CH_3COOH$

in Pulverform, das eine hohe Kaltwasserlöslichkeit besitzt.

Anstelle der in den Beispielen 311 und 312 eingesetzten Säuren können zur Salzbildung auch Salzsäure, Schwefelsäure, Phosphorsäure, Ameisensäure oder andere organische Säuren eingesetzt werden.

Auf analoge Weise können die Farbstoffe der übrigen Beispiele in ihre Säureadditionssalze übergeführt werden.

Beispiel 313

80 Teile des unter Beispiel 261c) erhaltenen Pulvers werden bei 40° in eine Lösung aus 20 Teilen Dextrin, 20 Teilen Eisessig und 300 Teilen Wasser eingetragen und zu einer homogenen Suspension verrührt. Durch Zerstäubungstrocknung erhält man ein blaues Granulat, das gut benetzbar ist und nicht stäubt. Papier lässt sich damit in blauen Tönen färben, indem man das Granulat der Papiermasse trocken zugibt oder vorher in Wasser auflöst.

Analog können die Farbstoffsalze der übrigen Beispiele zu Granulaten verarbeitet werden.

Anwendungsmöglichkeiten der vorstehend angeführten Farbstoffbeispiele werden in den folgenden Applikationsvorschriften illustriert.

Anwendungsbeispiele

Färbevorschrift A

In einem Holländer werden 70 Teile chemisch gebleichter Sulfitcellulose aus Nadelholz und 30 Teile chemisch gebleichter Sulfitcellulose aus Birkenholz in 2 000 Teilen Wasser gemahlen. Zu dieser Masse streut man 0,5 Teile des Farbstoffs aus Beispiel 261 (als Säureadditionssalz, z. B. gemäss Beispiel 312). Nach 20 Minuten Mischzeit wird daraus Papier hergestellt. Das auf diese Weise erhaltene saugfähige Papier ist blau gefärbt. Das Abwasser ist farblos.

Färbevorschrift B

2 Teile der Farbstofflösung gemäss Beispiel 311 werden zu 100 Teilen gebleichter Sulfitcellulose, die mit 2 000 Teilen Wasser in einem Holländer gemahlen wurde, gegossen. Nach 15 Minuten Durchmischung erfolgt die Leimung. Papier, das aus diesem Material hergestellt wird, zeigt eine blaue Nuance und besitzt hervorragende Nassechtheiten.

Färbevorschrift C

Eine saugfähige Papierbahn aus ungeleimtem Papier wird bei 40-50° durch eine Farbstofflösung folgender Zusammensetzung gezogen :

0,5 Teile des Farbstoffs aus Beispiel 274 (als Säureadditionssalz, z. B. gemäss Beispiel 312)
0,5 Teile Stärke und
99,0 Teile Wasser.

Die überschüssige Farbstofflösung wird durch zwei Walzen abgepresst. Die getrocknete Papierbahn ist orange gefärbt. Analog den Färbevorschriften A bis C kann auch mit den Farbstoffen der übrigen Beispiele in Form ihrer wasserlöslichen Salze oder als Präparation gemäss den Beispielen 312 oder 313 gefärbt werden.

Färbevorschrift D

100 Teile frisch gegerbtes und neutralisiertes Chromnarbenleder werden in einer Flotte aus 250 Teilen Wasser von 55° und 1 Teil des nach Beispiel 306 hergestellten Farbstoffs (als Säureadditionssalz) während 30 Minuten im Fass gewalkt und im gleichen Bad mit 2 Teilen eines anionischen Fettlickers auf sulfonierter Tranbasis während weiterer 30 Minuten behandelt. Die Leder werden in der üblichen Art getrocknet und zugerichtet. Man erhält egal gefärbtes Leder in schwarzen Tönen.

Weitere niederaffine, vegetabil nachgegerbte Leder können ebenfalls nach bekannten Methoden gefärbt werden. Auf analoge Weise kann auch mit den Farbstoffen der übrigen Beispiele gefärbt werden.

Färbevorschrift E

2 Teile des Farbstoffs aus Beispiel 310 (als Säureadditionssalz) werden in 4 000 Teilen enthärtetem Wasser gelöst und auf 40° erwärmt. Man bringt 100 Teile vorgenetztes Baumwollgewebe in das Bad ein und erhitzt in 30 Minuten auf Siedetemperatur. Das Bad wird während einer Stunde bei Siedetemperatur gehalten. Hierauf nimmt man das Gewebe aus der Flotte heraus, spült mit Wasser und trocknet. Der Farbstoff zieht praktisch quantitativ auf die Faser auf, das Färbebad ist nahezu farblos. Man erhält eine graublaue Färbung von guter Lichtechtheit und guten Nassechtheiten. Analog kann auch mit den Farbstoffen der übrigen Beispiele gefärbt werden.

**Patentansprüche** (für die Vertragsstaaten : BE, CH, DE, FR, GB, IT, LI, NL, SE)

1. Basische Verbindungen bzw. Azoverbindungen, die in einer der möglichen tautomeren Form der Formel

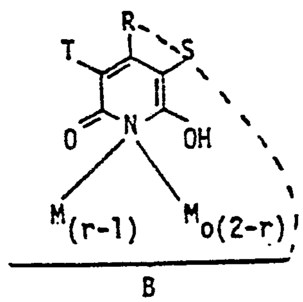

(I)

entsprechen, worin

R unabhängig voneinander Wasserstoff, einen (1-4C)-Alkyl-, (5-6C) Cycloalkyl- oder Phenylrest, Benzyl oder Phenyläthyl,

T unabhängig voneinander Wasserstoff,

i) -CN

$i_1$) 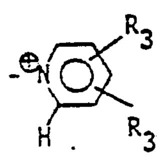 $A^{\ominus}$

$i_2$) $-COOR_4$

$i_3$) $-CONR_5R_6$

$i_4$) $-SO_2NR_5R_6$

$i_5$) 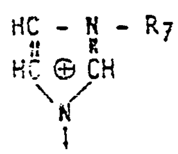 $A^{\ominus}$ oder

i6) 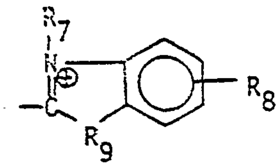 $A^{\ominus}$

r 1 oder 2,
M unabhängig voneinander einen Rest $—R_1—NH—R_0$,
$M_0$ unabhängig voneinander einen Rest $—R_1—NH—R_0$ Wasserstoff, $—NR_{11}R_{12}$, (1-4C)-Alkyl, Hydroxy(2-4C)-alkyl, (1-4C)-Alkoxy-(1-4C)-alkyl, gegebenenfalls durch 1 bis 3 (1-4C)-Alkyl substituiertes (5-6C)-Cycloalkyl, Phenyl(1-3C)-alkyl, $—V_1—NR_{13}R_{14}$ oder $—V_2—$

$$\overset{\oplus}{N}R_{13}R_{14}R_{15}A^{\ominus}$$

$A^{\ominus}$ ein Anion, bedeuten,
S falls r für 2 steht,
a) Wasserstoff,
b) einen Rest der Formel

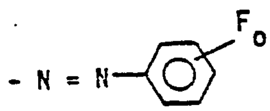 oder

c) $—N = N—A—N = N—K$

und $F_0$ einen in der Azochemie üblichen Substituenten, vorzugsweise Halogen, $NO_2$, $NH_2$, Alkyl, Alkoxy, CN, Trifluoralkyl, Phenyl, Anilin, Benzoyl, Carbamoyl, Phenoxy, Halogenphenoxy, Dihalogenphenoxy, Alkylsulfonyl, Phenylsulfonyl, Alkylsulfonylamine, Di-N-alkylaminosulfonyl oder Alkyl-sulfonyl, bedeuten oder, falls r für 1 steht,
d) einen Rest der Formel

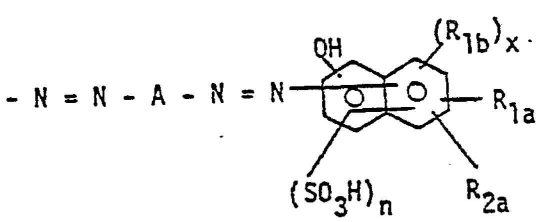

bedeutet, worin
n 0,1 oder 2,
x 0 oder 1,
A unabhängig voneinander den Rest einer Tetrazokomponente,
K den Rest einer beliebigen, vorzugsweise sulfonsäuregruppenfreien Diazo- oder Kupplungskomponente aus der Reihe Pyrazolon-5, 5-Aminopyrazol, Anilin, Phenol, Acetoacetylalkyl- oder -arylamid, Barbitursäure, Dimedon, Dimedoncarbonsäureester, Pyridon (z. B. des Restes B, worin r 1 oder 2 ist) oder 2,6-Diaminopyridin,
$R_{1b}$, falls x für 1 steht, einen Rest der Formel
e) $—N = N—K_1$
f) $—N = N—A_1—N = N—K_1$

g) 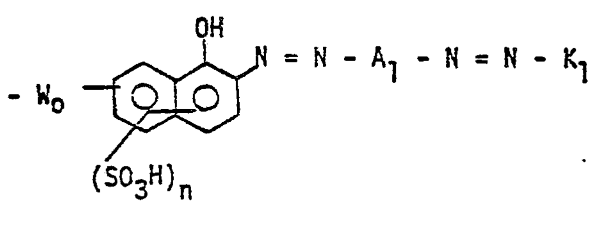

$K_1$ einen Rest K oder ein α- oder β-Naphthol, ein α- oder β-Aminonaphthalin oder ein Aminonaphthol,
$R_1$ unabhängig voneinander einen gegebenenfalls durch ein oder 2 Heteroatome unterbrochenen Alkylen- oder Alkenylenrest, einen Phenylen- oder Cyclohexylenrest,
$R_0$ oder einen Rest der Formel

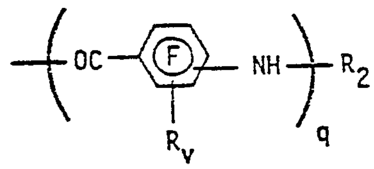

(Ia)

q 0 oder 1 und
$R_v$ Wasserstoff, Halogen, Nitro, (1-4C)-Alkyl oder (1-4C)-Alkoxy, bedeuten,
$R_2$ steht für einen Rest der Formel

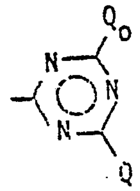

$h_1$)

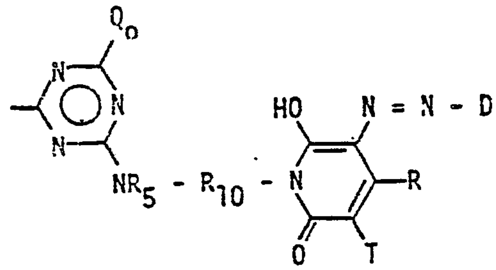

$h_3$) oder

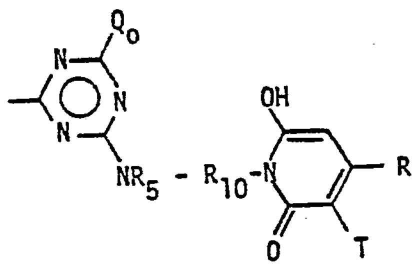

$h_2$),

$$- CO - (CH_2)_{1-3} - Z$$

$h_4$)

oder auch für Wasserstoff, worin

D unabhängig voneinander den Rest einer Diazokomponente, die durch in der Azochemie übliche Substituenten substituiert sein kann, vorzugsweise einen Rest

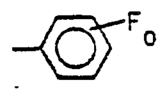

$- A - N = N - K$ oder $- A - N = N$

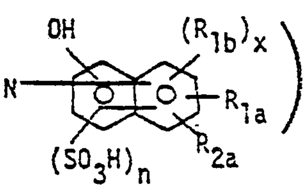

$R_3$ unabhängig voneinander Wasserstoff, einen (1-4C)-Alkylrest, $—NR_5R_6$ oder $—CONR_5R_6$,
$R_4$ unabhängig voneinander (1-6C)-Alkyl oder Phenyl-(1-3C)-alkyl,
$R_5$ und $R_6$ unabhängig voneinander Wasserstoff oder (1-4C)-Alkyl,
$R_7$ unabhängig voneinander (1-4C)-Alkyl,
$R_8$ unabhängig voneinander Wasserstoff oder (1-4C)-Alkyl,
$R_9$ unabhängig voneinander —S—, —O— oder

$$-\overset{|}{N}-R_5$$

$R_{10}$ unabhängig voneinander eine (2-6C) Alkylenrest oder

$$\overset{*}{-NHCOCH_2-}$$

wobei das mit *bezeichnete Atom am —$NR_5$— Rest gebunden ist,

$Q_0$ einen aliphatischen, cycloaliphatischen, aromatischen oder heterocyclischen Aminrest, in welchen das N-Atom an das C-Atom des Triazinylrestes gebunden ist,

Q Halogen, OH, $NH_2$, (1-4C)-Alkoxy, Phenyl oder $Q_0$,

Z unabhängig voneinander einen Rest der Formel —$NR_{11}R_{12}$ oder

$$-\overset{\oplus}{N}R_{13}R_{14}R_{15}\ A^{\ominus}$$

$R_{11}$ und $R_{12}$ unabhängig voneinander Wasserstoff, unsubstituiertes (1-6C)-Alkyl, durch OH, CN oder Halogen substituiertes (2-6C)-Alkyl, Phenyl-(1-3C)-Alkyl, dessen Phenylrest durch 1 bis 3 Substituenten aus der Reihe Chlor, (1-4C)-Alkyl und (1-4C)-Alkoxy substituiert sein kann, unsubstituiertes oder durch 1 bis 3 (1-4C)-Alkylgruppen substituiertes (5-6C)-Cycloalkyl,

$R_{13}$ und $R_{14}$ unabhängig voneinander eine der Bedeutungen von $R_{11}$ und $R_{12}$, wobei einer der Reste $R_{13}$ und $R_{14}$ und vorzugsweise beide Reste von Wasserstoff verschieden sind,

$R_{15}$ unabhängig voneinander einen gegebenenfalls durch Phenyl substituierten (1-4C)-Alkylrest,

$V_1$ unabhängig voneinander (1-6C)-Alkylen oder (2-6C)-Alkenylen,

$V_2$ unabhängig voneinander (2-6C)-Alkylen oder (2-6C)-Alkenylen bedeuten,

die Reste $R_5$ und $R_6$ bzw. $R_{11}$ und $R_{12}$, bzw. $R_{13}$ und $R_{14}$ zusammen mit dem an sie gebundenen N-Atom einen gesättigten heterocyclischen Ring und $R_{13}$, $R_{14}$ und $R_{15}$ zusammen mit dem an sie gebundenen N-Atom einen Pyridinring oder einen gesättigten heterocyclischen Ring bilden können und der Pyridin- und der heterocyclische Ring durch eine, zwei oder drei (1-4C)-Alkylgruppen substituiert sein können,

$R_{1a}$ Wasserstoff,

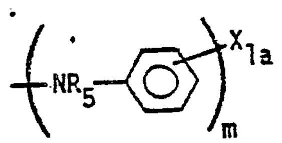

oder

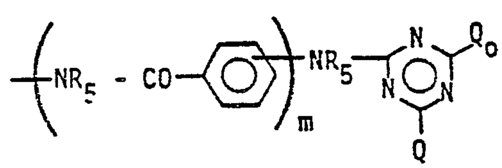

wobei für x = 1, $R_{1a}$ Wasserstoff bedeutet,

$$X_{1a}\ -NR_5-Q_{10}-NR_{11}R_{12},\ -COCH_2-\overset{\oplus}{N}R_{13}R_{14}R_{15}\ A^{\ominus},$$
$$-NR_5-COCH_2-NR_{11}R_{12},\ -CO-Q_{10}-\overset{\oplus}{N}R_{13}R_{14}R_{15}\ A^{\ominus},$$
$$-NR_5-Q_{10}-\overset{\oplus}{N}R_{13}R_{14}R_{15}\ A^{\ominus},\ -NR_5-COCH_2\overset{\oplus}{N}R_{13}R_{14}R_{15}A^{\ominus}$$
$$\text{oder } -CO-Q_{10}-\overset{\oplus}{N}(CH_3)_2-(CH_2)_4-Z\ A^{\ominus},$$

m 0 oder 1,

$Q_{10}$ $\overset{*}{-NHCOCH_2-}$

oder lineares oder verzweigtes (2-6C)-Alkylen,

$R_{2a}$ Wasserstoff oder, wenn $R_{1a}$ für Wasserstoff und $R_{1b}$ für einen Rest der Formel e) oder f) steht, auch OH, $NH_2$, (1-4C)-Alkylcarbonylamino, Benzoylamino oder Phenylamino, deren Phenylrest durch 1 oder 2 Substituenten aus der Reihe Halogen, $NO_2$, $NH_2$ (1-4C)-Alkyl und (1-4C)-Alkoxy substituiert sein kann,

$A_1$ unabhängig voneinander den Rest einer Tetrazokomponente oder den Rest einer Diazo-/-Kupplungskomponente und

$W_0$ ein zweiwertiges Brückenglied bedeuten,
Gemische der Verbindungen der Formel I, deren Säureadditionssalze oder Salze von Säuren sowie Metallkomplexe, wie 1 : 1- oder 1 : 2-Metallkomplexe davon, mit der Massgabe, dass sie,

α) falls die Monoazoverbindungen b) der Formel I sulfogruppenfrei sind, eine und vorzugsweise zwei und, falls die Dis- und Polyazoverbindungen der Formel I sulfogruppenfrei sind, mindestens zwei wasserlöslich machende kationische oder mindestens zwei wasserlöslichmachende protonierbare basische Gruppen oder mindestens eine kationische und mindestens eine protonierbare basische Gruppen enthalten und, falls die Verbindungen der Formel I Sulfogruppen enthalten, die Anzahl der kationischen und/oder protonierbaren basischen Gruppen die Anzahl der Sulfogruppen und der eventuell zusätzlich vorhandenen anionischen Gruppen um mindestens 1 übersteigt, und

β) falls r für 2, S für Wasserstoff und $R_0$ für Wasserstoff stehen, $R_1$ verschieden ist von Alkylen, T verschieden von CN und der Rest M von Sulfonsäuregruppen frei.

2. Basische Verbindungen gemäss Anspruch 1 in einer der möglichen tautomeren Form der Formel

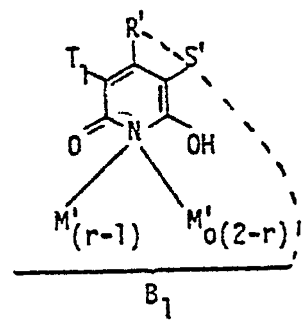

(II)

worin

R' unabhängig voneinander Methyl, Aethyl, Phenyl, Benzyl oder Cyclohexyl,

$T_1$ unabhängig voneinander Wasserstoff,

i) - CN

i$_1'$) 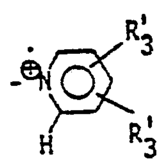 $A^{\ominus}$ oder

i$_3'$) - $CONR_5'R_6'$,

M' unabhängig voneinander einen Rest $—R_1'—NH—R_0'$,

$M_0'$ unabhängig voneinander einen Rest $—R_1'$ $—NHR_0'$, Wasserstoff, $—CH_3$, $—C_2H_5$, $—C_2H_4OH$, Cyclohexyl, Benzyl, $—(CH_2)_{1\text{-}3}—NR_{13}'R_{14}'$ oder

$$-(CH_2)_{2\text{-}3} - \overset{\oplus}{N}R_{13}'R_{14}'R_{15}'A^{\ominus}$$

S' falls r für 2 steht,

a) Wasserstoff,

b') einen Rest der Formel

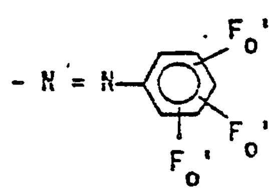

c$_1$) 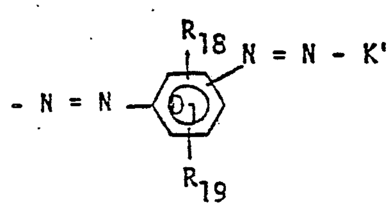 oder

c2) 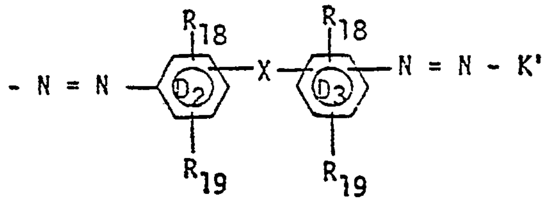

bedeutet, oder S' falls r für 1 steht,

$d_1$) einen Rest der Formel

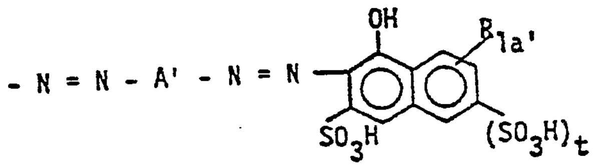

d2) 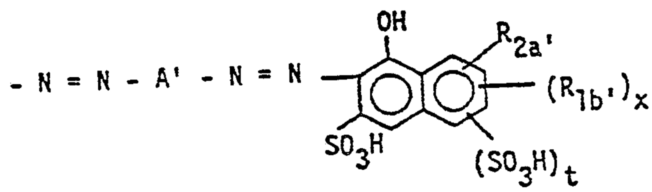

d3) 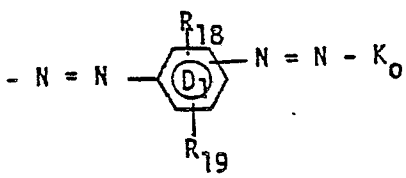 oder

d4) 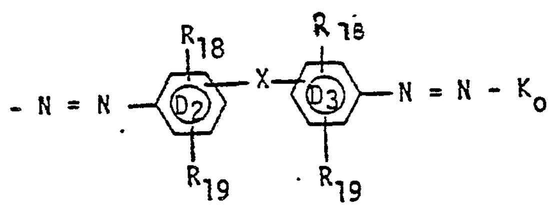

bedeutet, worin

$K_0$ einen Rest der Formel

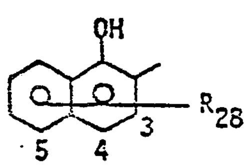

aa),

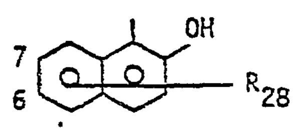

$aa_1$),

A' unabhängig voneinander einen Rest der Formel

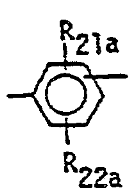

(m)

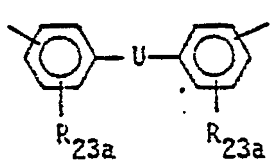

(n)

oder 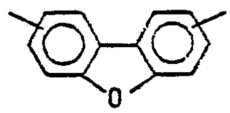 (o),

$R_{1b}'$ falls x für 1 steht, einen Rest der Formel

e') $—N = N—K_1'$

f') $—N = N—A'—N = N—K_1'$

g') 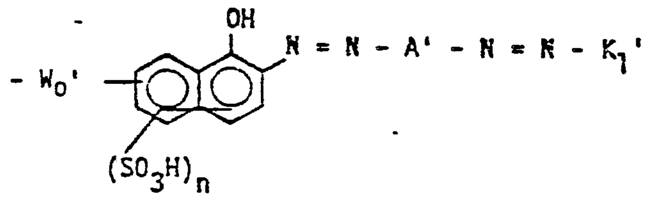

K' einen Rest der Formel

$aa_4$) $B_1$, worin r sowohl 1 als auch 2 sein kann,

$aa_5$) ein Phenol,

$aa_6$) 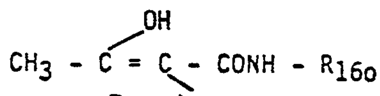

$aa_7$) 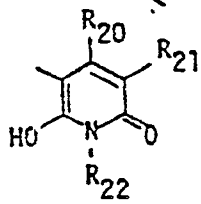

$aa_8$) 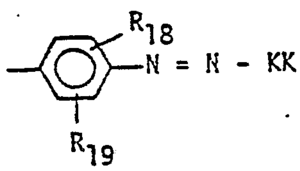

$aa_9$) 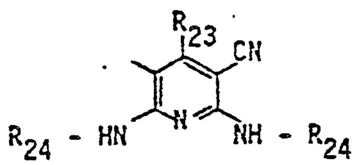

$aa_{10}$) 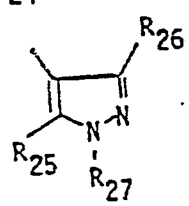

$aa_{11}$) 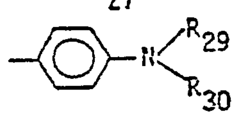

$aa_{12}$) 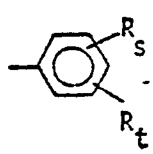

$R_1'$ unabhängig voneinander einen geradkettigen oder verzweigten (2-8C)-Alkylen oder meta- oder para-Phenylenrest,

$R_0'$ einen Rest der Formel

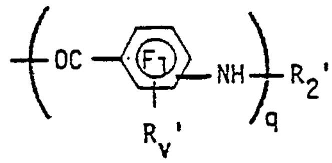

(Ib)

$R_v'$ unabhängig voneinander Wasserstoff, Chlor, Nitro, Methyl oder Methoxy bedeuten,
$R_2'$ steht für einen Rest der Formel

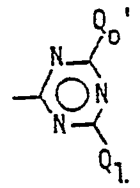

$h_1'$ oder

$- CO -(CH_2)_{1-2} - Z_1$ $h_4'$)

oder auch für Wasserstoff,
$R_3'$ unabhängig voneinander Wasserstoff, Methyl, Aethyl, $-NH_2$ oder $-N(CH_3)_2$,
$R_5'$ und $R_6'$ unabhängig voneinander Wasserstoff, Methyl oder Aethyl,
$Q_1$ Chlor, OH, $NH_2$,

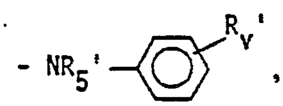 , 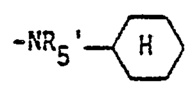 , $OCH_3$,

Mono(1-4C)-alkylamino, Di-(1-2C)-alkylamino, Monohydroxy(2-4C)-alkylamino, Bis [hydroxy(2-4C)alkyl]-amino oder $Q_0'$ und

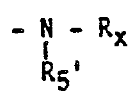

bedeuten, worin
$R_x$ einen durch eine und/oder mehrere Gruppen der Formel $-NR_5'-$ oder

$$-\overset{\oplus}{N}R_7R_7 - A^{\ominus}$$

unterbrochenen, gegebenenfalls durch OH substituierten (1-12C)-Alkylrest,

$- NHCOCH_2 - Z_1$,
$- CH_2CONH - V - Z_1$, $- V - Z_1$, $- V -$ Arylen $- Z_0$,

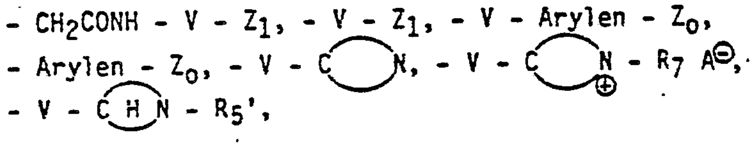

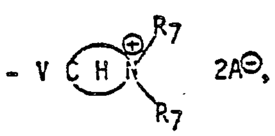

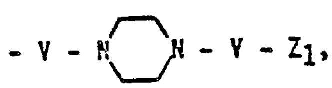

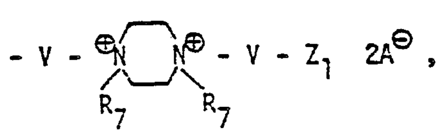

oder $R_x$ und $R_5'$ zusammen mit dem an sie gebundenen N-Atom einen Rest

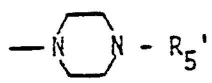 oder 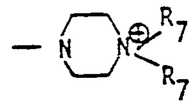 $2A^{\ominus}$

bilden können,

V unabhängig voneinander einen (1-8C)-Alkylenrest oder einen (3-8)-Alkenylenrest,

$Z_0$ $—N(CH_3)_2$, $—N(C_2H_5)_2$,

$$-\overset{\oplus}{N}(CH_3)_3\ A^{\ominus}$$

$$-\overset{\oplus}{N}(C_2H_5)_3\ A^{\ominus}$$

oder einen Rest $—CONH—V_0—Z_1$, $—NHCO—V_0—Z_1$, $—CO—V_0—Z_1$, $—SO_2—NH—V_0—Z_1$, $—V_0—Z_1$ oder $—NHNHCOCH_2—Z_1$,

und der Arylenrest durch Halogen, OH, $NO_2$, (1-4C)-Alkyl oder (1-4C)-Alkoxy substituiert sein kann,

$V_0$ für einen (1-8C)-Alkylenrest steht und die Gruppe der Formel

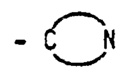 oder 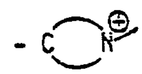

für einen ungesättigten Heterocyclus und die Gruppe der Formel

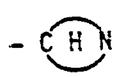 oder 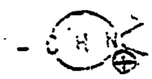

für einen gesättigten Heterocyclus stehen,

$Z_1$ unabhängig voneinander einen Rest der Formel

$$NR_{11}'R_{12}' \text{ oder } -\overset{\oplus}{N}R_{13}'R_{14}'R_{15}'\ A^{\ominus}$$

$R_{11}'$ und $R_{12}'$ unabhängig voneinander Wasserstoff, lineares oder verzweigtes (1-6C)-Alkyl, unverzweigtes Hydroxy(2-3C)-alkyl, Benzyl, 2-Cyanäthyl oder 2-Chloräthyl,

$R_{13}'$ und $R_{14}'$ unabhängig voneinander lineares oder verzweigtes (1-6C)-Alkyl, unverzweigtes Hydroxy(2-3C)-alkyl, Benzyl, 2-Cyanoäthyl oder 2-Chloräthyl,

$R_{15}'$ Methyl, Aethyl, Propyl oder Benzyl

bedeuten,

die Reste $R_5'$ und $R_6'$ bzw. und $R_{11}'$ und $R_{12}'$ zusammen mit dem an sie gebundenen N-Atom einen Morpholin-, Piperidin-, Pyrrolidin-, Piperazin- oder N-Methylpiperazinring, oder die Reste $R_{13}'$, $R_{14}'$ und $R_{15}'$ zusammen mit dem an sie gebundenen N-Atom einen Pyridin-, Picolin- oder Lutidinring oder einen Ring der Formel

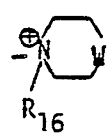 $A^{\ominus}$

worin $R_{16}$ Methyl oder Aethyl und W $—CH_2$, —O—, —S—, $—SO_2—$, —SO—,

$$-\overset{|}{N}H,\ -\overset{|}{N}-R_{16} \text{ oder } -\overset{|}{\underset{\oplus}{N}}(R_{16})_2\ A^{\ominus}$$

bedeuten, bilden können,

worin

$F_0'$ unabhängig voneinander Wasserstoff, Halogen, $—NO_2$, $—NH_2$, (1-4C)-Alkyl, (1-4C)-Alkoxy, CN, Trifluormethyl, Phenyl, Anilino, Benzoyl, Carbamoyl, Phenoxy, Halogenphenoxy, Dihalogenphenoxy, (1-4C)-Alkylsulfonyl, Phenylsulfonyl, (1-4C)-Alkylsulfonylamino, Di-N(1-4C)-alkylaminosulfonyl oder (1-4C)-Alkylsulfonyl bedeuten,

X, falls die Ringe $D_2$ und $D_3$ unsubstituiert sind, für $X_1$ die direkte Bindung,

$X_2$ einen geradkettigen oder verzweigten Alkylenrest mit 1 bis 4 Kohlenstoffatomen,

(Siehe Formeln Seite 96 ff.)

$X_3$ $-CO-$, $X_4$ 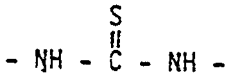, $X_5$ $-S-$, $X_6$ $-O-$

$X_7$ $-CH=CH-$, $X_8$ $-S-S-$, $X_9$ $-SO_2-$,

$X_{10}$ $-NH-$, $X_{11}$ $-NH-CO-$, $X_{12}$ 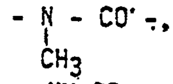,

$X_{13}$ 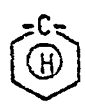, $X_{14}$ 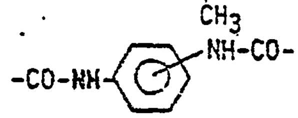,

$X_{15}$ 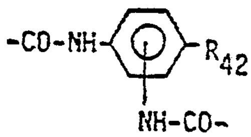, $X_{16}$ 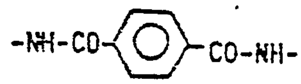

$X_{17}$ $-SO_2-NH-$, $X_{18}$ 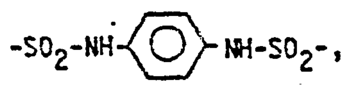,

$X_{19}$ 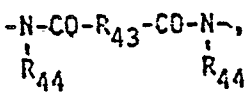, $X_{20}$ 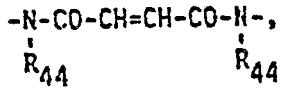,

$X_{21}$ 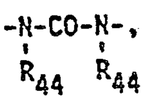, $X_{22}$ $-CO-NH-NH-CO-$,

$X_{23}$ $-CH_2CO-NH-NH-CO-CH_2-$, $X_{24}$ $-CH=CH-CO-NH-NH-CO-CH=CH-$,

$X_{25}$ 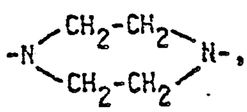, $X_{26}$ 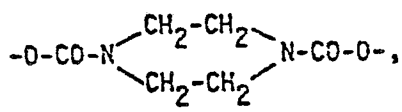,

$X_{27}$ 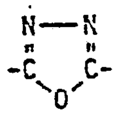, $X_{28}$ 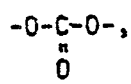, $X_{29}$ 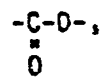, $X_{30}$ 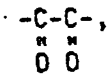,

$X_{31}$ $-O-(CH_2)_g-O-$,

$X_{32}$ 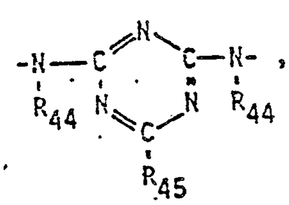, $X_{33}$ 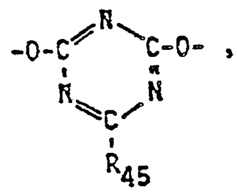,

$X_{34}$ 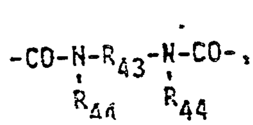, $X_{35}$ 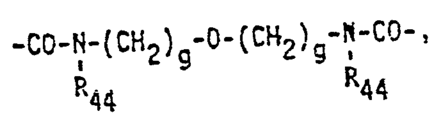,

(Fortsetzung)

$X_{36}$ 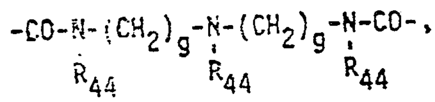

$X_{37}$ 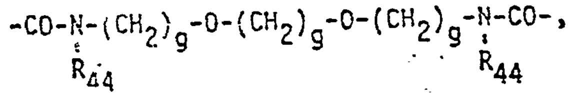

$R_{38}$ 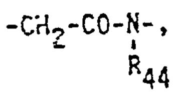 $R_{39}$ 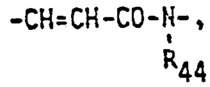

$X_{40}$ $-N=N-$, $X_{41}$ $-CH_2-S-CH_2-$, $X_{42}$ $-SO-$,

$X_{43}$ $-CH_2-SO-CH_2-$,

$X_{44}$ $-CH_2-SO_2-CH_2-$, $X_{45}$ $-CH_2-NH-CO-NH-CH_2-$,

$X_{46}$ $-CH_2-NH-CS-NH-CH_2-$,

$X_{47}$ 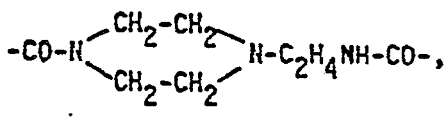

$X_{48}$ 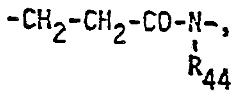 $X_{49}$ $-CH_2-CO-CH_2-$,

$X_{50}$ $-CH=CH-CH=CH-$, $X_{51}$ 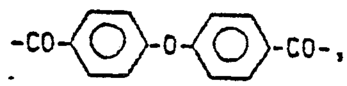

$X_{52}$ 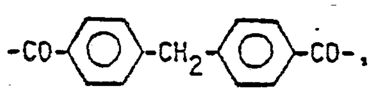 $X_{53}$ 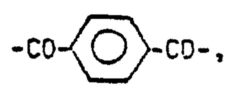

$X_{54}$ 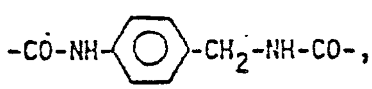

$X_{55}$ 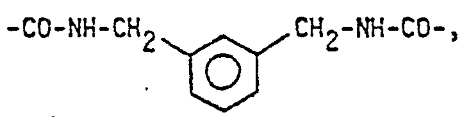

$X_{56}$ 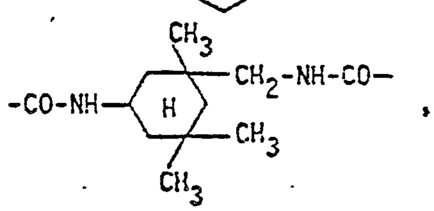

(Fortsetzung)

$X_{57}$ 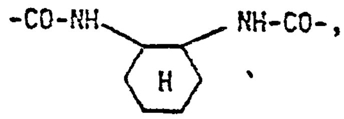 $X_{58}$ $-CH_2-CO-$,

$X_{59}$ $-CH=CH-CO-CH=CH-$, $X_{60}$ 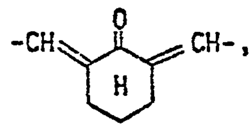

$X_{61}$ 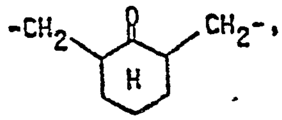 $X_{62}$ $-O-CH_2-CH_2-O-$,

$X_{63}$ $-O-CH_2-O-$, $X_{64}$ $-CO-NH-R_{43}-CO-NH-$,

$X_{100}$ $-CO-NH-R_{43}-CO-NH-R_{43}-NH-CO-$,

$X_{101}$ $-CO-NH-R_{43}-NH-CO-CH_2-CH_2-CO-NH-R_{43}-NH-CO-$,

$X_{102}$ $-CO-NH-R_{43}-NH-CO-CH=CH-CO-NH-R_{43}-NH-CO-$,

$X_{103}$ 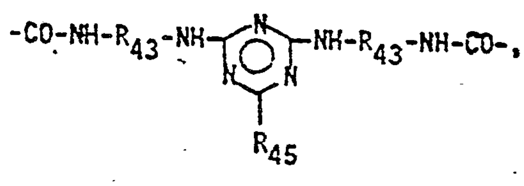

$X_{104}$ $-SO_2-NR_{44}-(CH_2)_g-NR_{44}-SO_2-$,

$X_{105}$ $-CO-NR_{44}-R_{43}-O-CO-$,

$X_{106}$ 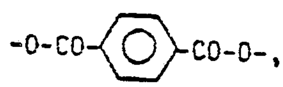

$X_{107}$ 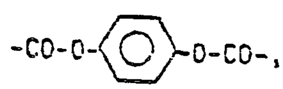

$X_{108}$ $-CONH-R_{43}-NH-CO-NH-R_{43}-NH-CO-$,

$R_{42}$ unabhängig voneinander Halogen, (1-4C)-Alkyl oder (1-4C)-Alkoxy,
$R_{43}$ unabhängig voneinander einen geradkettigen oder verzweigten (1-4C)-Alkylenrest,
$R_{44}$ unabhängig voneinander Wasserstoff oder (1-4C)-Alkyl,
$R_{45}$ unabhängig voneinander Halogen,

$-NH-CH_2-CH_2-OH$,

$-N(CH_2-CH_2-OH)_2$, $NH_2$, OH, $NH-(CH_2)_{2-3}N(C_2H_5)_2$,

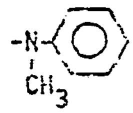, 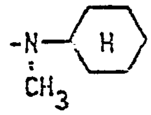, 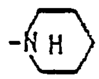, $-OCH_3$, $OC_2H_5$

g unabhängig voneinander 1, 2, 3 oder 4,
X falls die Ringe $D_2$ und/oder $D_3$ substituiert sind, insbesondere in ortho-Stellung zu den beiden Azobrücken, für die direkte Bindung $X_1$ oder für $X_2$, $X_{14}$, $X_{21}$, $X_{32}$, $X_{34}$ oder $X_{40}$ steht,
$R_s$ und $R_t$ unabhängig voneinander Wasserstoff, (1-4C)-Alkyl oder (1-4C)-Alkoxy,
$R_{160}$ einen Rest $-(CH_2)_v-Z_1$,

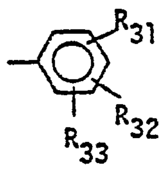, 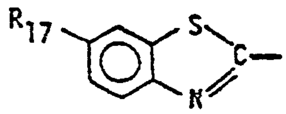,

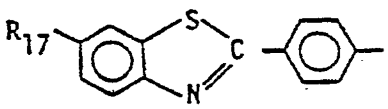, 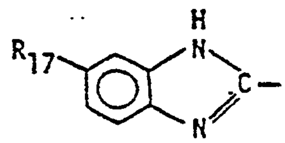,

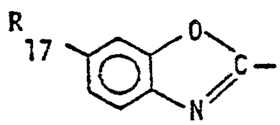, 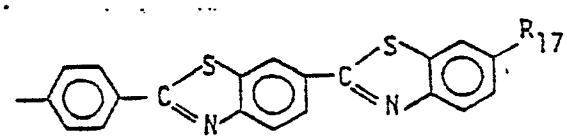

oder 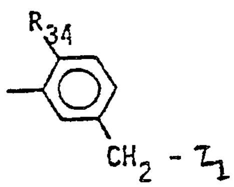

$R_{17}$ unabhängig voneinander H oder Methyl,
$R_{18}$ und $R_{19}$ unabhängig voneinander Wasserstoff, Halogen, (1-4C)-Alkyl oder (1-4C)-Alkoxy,
$R_{20}$ H, Methyl, Aethyl, Phenyl, Benzyl oder Cyclohexyl,
$R_{21}$ einen der Reste $T_1$ oder

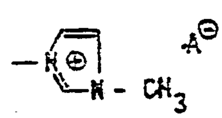

$R_{22}$ einen Rest der Formel $-R_1'-NH-R_0'$, Wasserstoff, einen gegebenenfalls durch Phenyl substituierten (1-4C)-Alkylrest, $-(CH_2)-CN$, $-(CH_2)_v-Z_1$, $-(CH_2)_2-OH$, $-(CH_2)_2OCH_3$ oder

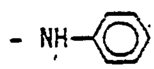

$R_{23}$ (1-4C)-Alkyl oder Phenyl,
$R_{24}$ unabhängig voneinander Wasserstoff, (1-4C)-Alkyl, $-(CH_2)_{2-3}-OCH_3$, $-(CH_2)_2-OH$, $-(CH_2)_{2-3}-N(CH_3)_2$ oder

$- (CH_2)_{2-3}-\overset{\oplus}{N}(CH_3)_3\ A^{\ominus}$,

$R_{25}$ OH oder $NH_2$,
$R_{26}$ (1-4C)-Alkyl oder $-COR_{35}$,
$R_{27}$ Wasserstoff,

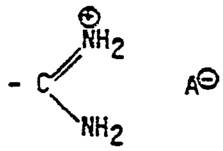

oder 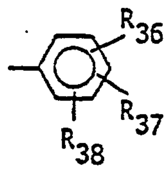 ,

$R_{28}$ in einer der Stellungen 3, 4, 5, 6 oder 7 unabhängig voneinander Wasserstoff,

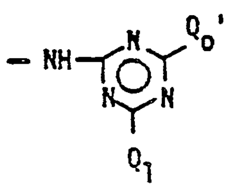 , $-NHCO(CH_2)_{1-3}-Z_1$,

$-SO_2-NH-V_0-Z_1$, $-CONH-V_0-Z_1$, $-CONHNH_2$, $-NH-V_0-Z_1$, $-CH_2-Z_1$, $-NHNHCOCH_2-Z_1$ oder

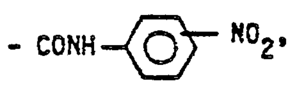,

$R_{29}$ (1-4C)-Alkyl,

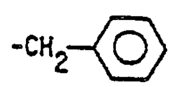

oder $-(CH_2)_2-CN$,
$R_{30}$ (1-4C)-Alkyl oder $-(CH_2)_v-Z_1$,
v unabhängig voneinander 1, 2, 3, 4, 5 oder 6,
$R_{31}$ und $R_{32}$ unabhängig voneinander Wasserstoff, Halogen, (1-4C)-Alkyl, (1-4C)-Alkoxy, $NO_2$ oder CN,
$R_{33}$ Wasserstoff,

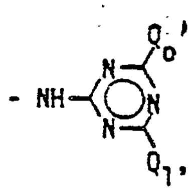,

$-NHCO-(CH_2)_{2-3}-Z_1$, $-SO_2-NH-(CH_2)_{2-3}-Z_1$ oder $-CONH-V_0-Z_1$,
$R_{34}$ (1-4C)-Alkoxy,
$R_{35}$ $-O-R_{39}$, $-NH_{40}R_{41}$ oder $-NH-V_0-Z_1$,
$R_{36}$ und $R_{37}$ unabhängig voneinander Wasserstoff, Halogen, (1-4C)-Alkyl, (1-4C)-Alkoxy, $-NO_2$ oder $-NH_2$,
$R_{38}$ Wasserstoff, $-CONH-V_0-Z_1$, $-SO_2NH-V_0-Z_1$, $-NHCO(CH_2)_{2-3}-Z_1$ oder

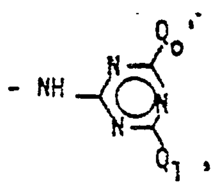 ,

$R_{39}$ (1-4C)-Alkyl,

$R_{40}$ und $R_{41}$ unabhängig voneinander Wasserstoff oder (1-4C)-Alkyl,

$R_{21a}$ H, OH, Halogen, (1-4C)-Alkyl, (1-4C)-Alkoxy, CN, $-CONH_2$, —NHCO(1-4C)-Alkyl, $-NHCONH_2$, COOH oder $-SO_3H$,

$R_{22a}$ H, Halogen, (1-4C)-Alkyl oder (1-4C)-Alkoxy und die Reste $R_{21a}$ und $R_{22a}$ paraständig zueinander stehen,

$R_{23a}$ unabhängig voneinander H, OH, Halogen, CN, (1-4C)-Alkyl, (1-4C)-Alkoxy, —COOH oder $-SO_3H$,

U die direkte Bindung, $-(CH_2)_{1\text{-}3}$, —O—, —S—, $-SO_2-$, —NHCO—, —NHCONH—, $-NHCO(CH_2)_{2\text{-}3}-CONH-$, $-CONH(CH_2)_{2\text{-}3}-NHCO-$, $-O(CH_2)_{2\text{-}3}-O-$, —N = N—, —CH = CH—CO—CH = CH—,

t 0 oder 1,

$R_{2a}'$ H, OH oder $NH_2$,

$R_{1a}'$ Wasserstoff,

$$-\overset{\oplus}{N}R_5' - Q_{10}' - NR_{11}'R_{12}',$$

$$-NR_5' - Q_{10}' - \overset{\oplus}{N}R_{13}'R_{14}'R_{15}'\ A^{\ominus},$$

$-NR_5'COCH_2NR_{11}'R_{12}'$, $-NR_5'$ 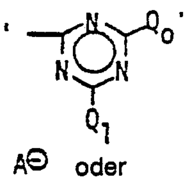 ,

$$-NR_5' - COCH_2\overset{\oplus}{N}R_{13}'R_{14}'R_{15}'\ A^{\ominus}\ \text{oder}$$

$$-CO - Q_{10}' - \overset{\oplus}{N}(CH_3)_2 - (CH_2)_{1\text{-}4} - Z_1\ A^{\ominus},$$

$Q_{10}'$ $-(CH_2)_{2\text{-}6}-$, $-CH_2-CH(CH_3)-$ oder $-CH_2-CH(CH_3)-CH_2-$,

$W_0'$ $-NH-$, $-NHCONH-$, $-NHCO-CH=CH-CO-NH-$,

$-NHCO(CH_2)_{2\text{-}3}-CONH-$, $-NHCO$ 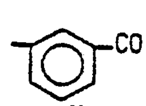 $CO-NH-$,

$-NH-CO$ 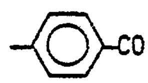 $CO-NH-$ oder 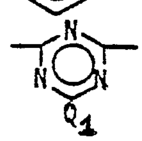

$K_1'$ einen Rest der Formel aa), $aa_1$), $aa_2$), $aa_3$), $aa_4$), $aa_5$), $aa_6$), $aa_7$), $aa_8$), $aa_9$), $aa_{10}$), $aa_{11}$), $aa_{12}$), oder

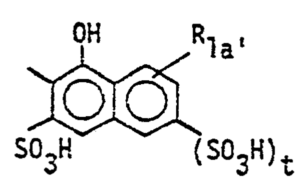

$aa_{13}$) oder

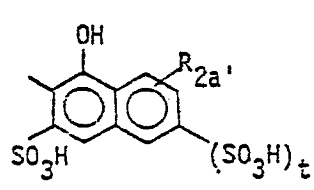

$aa_{14}$) ,

und KK einen Rest der Formel $aa_1$), $aa_2$), $aa_3$), $aa_4$), $aa_5$), $aa_6$), $aa_7$), $aa_8$), $aa_9$), $aa_{10}$), $aa_{11}$), $aa_{12}$), $aa_{13}$), oder $aa_{14}$) bedeuten,

Gemische der Verbindungen der Formel II, deren Säureadditionssalze oder Salze von Saüren sowie 1 : 1- oder 1 : 2-Metallkomplexe davon, mit der Massgabe, dass

$\alpha_1$) die Monoazoverbindungen b' der Formel II eine und vorzugsweise zwei und, falls die Dis- und Polyazoverbindungen der Formel II von sulfonsäuregruppenfrei sind [Formeln $c_1$), $c_2$), $d_3$), $d_4$)], sie mindestens zwei wasserlöslich machende kationische oder mindestens zwei protonierbare basische Gruppen oder mindestens eine kationische und mindestens eine protonierbare Gruppe enthalten und, falls die Verbindungen der Formel II Sulfogruppen enthalten, die Anzahl der kationischen und/oder protonierbaren basischen Gruppen die Anzahl der Sulfogruppen und die eventuell zusätzlich vorhandenen —COOH-Gruppen um mindestens 1 übersteigt und

$\beta_1$) falls r für 2, S' für Wasserstoff, $R_0$' für Wasserstoff stehen, $R_1$' verschieden ist von Alkylen, $T_1$ verschieden von CN und der Rest M' von Sulfonsäuregruppen frei.

3. Basische Verbindungen gemäss Anspruch 1 in einer der möglichen tautomeren Form der Formel

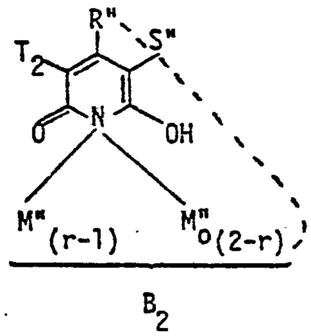

(III)

worin

R'' Methyl oder Phenyl,

$T_2$ unabhängig voneinander

- CN oder 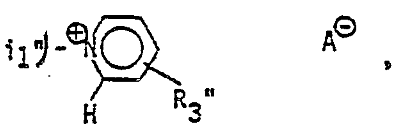

M'' unabhängig voneinander $—R_1''—NH—R_0''$,

$M_0''$ unabhängig voneinander $—R_1''—NH—R_0''$, Wasserstoff, $CH_3$, $—C_2H_5$, Benzyl, $—(CH_2)_{2\text{-}3}—NR_{13}''R_{14}''$ oder $—(CH_2)_{2\text{-}3}—NR_{13}''R_{14}''R_{15}''\ A^{\ominus}$,

S'' falls r für 2 steht,

a) Wasserstoff,

b'') einen Rest der Formel

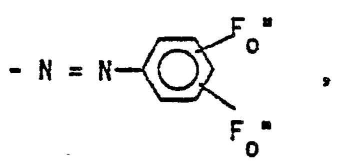

$c_1$') 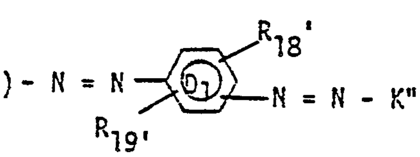

$c_2$') 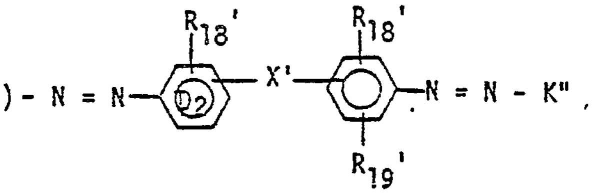

bedeudet,

oder S'' falls r für 1 steht,

$d_1')$ einen Rest der Formel

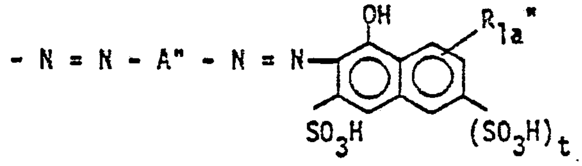

d2')

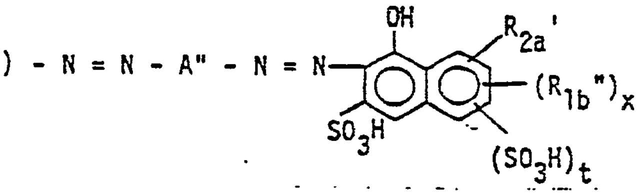

d3')

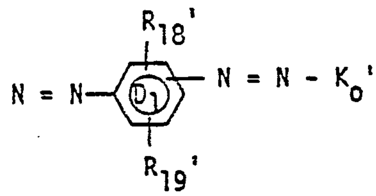

d4')

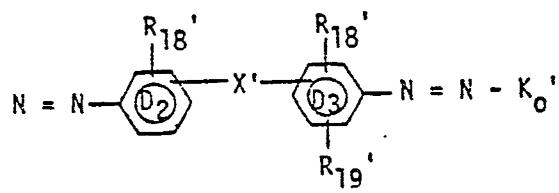

bedeutet, worin

$K_0'$ einen Rest der Formel

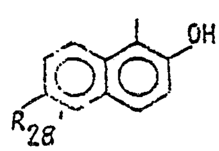

aa')

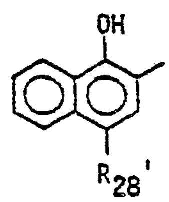

(aa1')

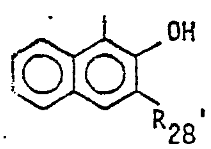

aa2')

A" unabhängig voneinander einen Rest der Formel

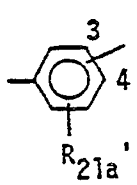

(mm)

oder

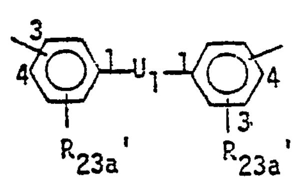

(nm)

wobei der Rest (mm) über die Positionen 1, 3 oder 1,4 verknüpft ist und der Rest (nm) über die Positionen 3,3 ; 3,4 ; oder 4,4 gebunden ist,

$R_{1b}''$ falls x für 1 steht, einen Rest der Formel

e") $-N = N-K_1''$

f") $-N = N-A''-N = N-K_1''$

g")
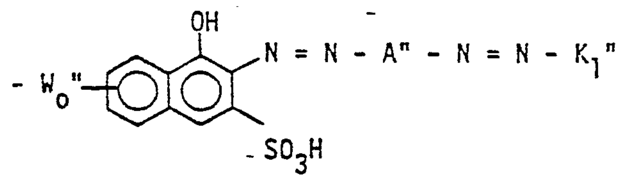

K" einen Rest der Formel

$aa_4'$) $B_2$, worin r 1 oder 2 ist,

$aa_5$) ein Phenol,

$aa_6'$)
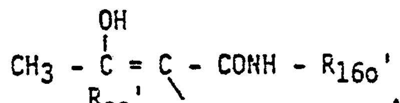

$aa_7'$)
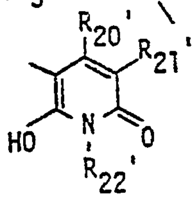

$aa_8'$)
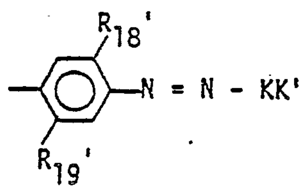

$aa_9'$)
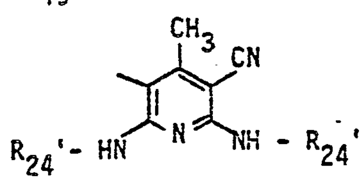

$aa_{10}'$)
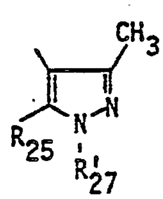

$aa_{11}'$)
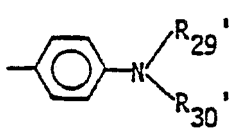

$aa_{12}'$)
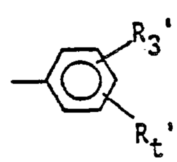

$R_1''$ 1,2-Aethylen, 1,3-Propylen oder meta- oder para-Phenylen,

$R_0''$ einen Rest der Formel

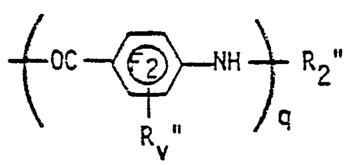
(Ic)

$R_v''$ Wasserstoff, Chlor oder Methyl bedeuten,
$R_2''$ unabhängig voneinander steht für einen Rest der Formel

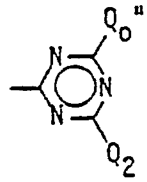

$h_1'')$ oder

$- CO - CH_2 - Z_2$ $h_4'')$

oder auch für Wasserstoff, worin $R_3''$ Wasserstoff oder Methyl,

$Q_2$ Chlor, OH, $—NH_2$, Mono (1-2C)-alkylamino, $OCH_3$, Monohydroxy (2-4C)-alkylamino, Bis [hydroxy(2-4C)-alkyl] amino oder $Q_0''$ und

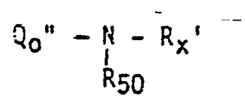

bedeuten, worin

$R_x'$ einen der Reste der Formeln

$- (CH_2)_{2-3} - NR_{50} - (CH_2)_{2-3} - NR_{50} - C_2H_5$,

$- (CH_2)_{2-3} - \overset{\oplus}{N}(R_{13}'')_2 - (CH_2)_{2-3} - \overset{\oplus}{N}(R_{13}'')_2 - C_2H_5 \quad 2A^{\ominus}$

$- (CH_2)_{2-3} - NR_{50} - C_2H_5$,

$- (CH_2)_{2-3} - \overset{\oplus}{N}(R_{13}'')_2 - C_2H_5 \quad A^{\ominus}$, $- NHCOCH_2 - Z_2$,

$- CH_2 - CO - NH - V' - Z_2$,

$- V' - Z_2$, 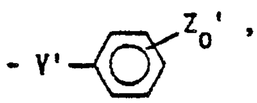 , 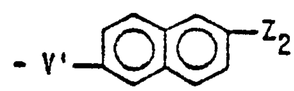 ,

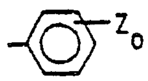 , 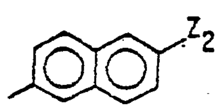 , 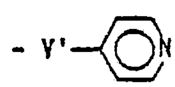 , 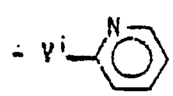

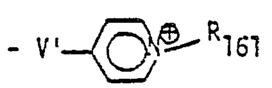 $A^{\ominus}$ 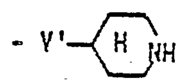 , 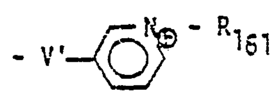 $A^{\ominus}$,

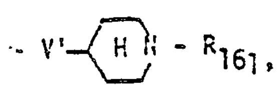 , 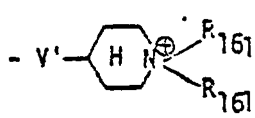 $A^{\ominus}$,

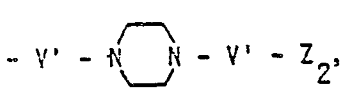 , 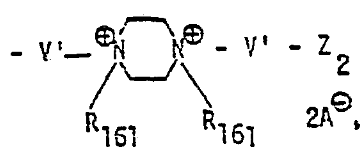 $2A^{\ominus}$,

oder $R_x'$ und $R_{50}$ zusammen mit dem an sie gebundenen N-Atom einen Rest

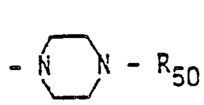 oder 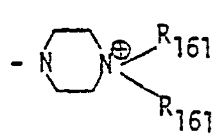 $A^{\ominus}$

bilden können,

$R_{161}$ Methyl oder Aethyl,

V' unabhängig voneinander einen (1-4C)-Alkylenrest,

$Z_0'$—$N(CH_3)_2$,

$-\overset{\oplus}{N}(CH_3)_3\ A^{\ominus}$,

oder einen Rest $—CONH—V_0'—Z_2$, $—NH—CO—V_0'—Z_2$, $—CO—V_0'—Z_2$, $—SO_2NH—V_0'—Z_2$, $—V_0'—Z_2$ oder $—NHNHCOCH_2—Z_2$ und

$V_0'$ für einen (1-4C)-Alkylenrest steht,

$R_{50}$ Wasserstoff oder Methyl,

$Z_2$ unabhängig voneinander einen Rest $—NR_{11}''R_{12}''$ oder

$-\overset{\oplus}{N}R_{13}''R_{14}''R_{15}''\ A^{\ominus}$,

$R_{11}''$ und $R_{12}''$ unabhängig voneinander Wasserstoff, Methyl oder Aethyl,

$R_{13}''$ und $R_{14}''$ unabhängig voneinander Methyl oder Aethyl,

$R_{15}''$ Methyl, Aethyl oder Benzyl bedeuten,

die Reste $R_{11}''$ und $R_{12}''$ zusammen mit dem an sie gebundenen N-Atom einen Morpholin-, Piperazin- oder N-Methylpiperazinring, die Reste $R_{13}''$, $R_{14}''$ und $R_{15}''$ zusammen mit dem an sie gebundenen N-Atom einen Pyridin- oder α- oder β-Picolinring oder einen N-Methylmorpholin-, N-Methylpiperidin-, N-Methylpiperazin oder N,N'-Dimethylpiperazinring bilden können,

worin

$F_0''$ unabhängig voneinander Wasserstoff, $NO_2$, Chlor, Methyl, Methoxy oder Chlorphenoxy,

X' falls die Ringe $D_2$ und $D_3$ unsubstituiert sind, für $X_1$, $X_5$, $X_6$, $X_7$, $X_{10}$, $X_{11}$, $X_{12}$, $X_{16}$, $X_{17}$, $X_{22}$, $X_{25}$, $X_{26}$, $X_{27}$, $X_{30}$, $X_{31}$, $X_{49}$, $X_{50}$, $X_{51}$, $X_{52}$, $X_{53}$, $X_{54}$, $X_{58}$, $X_{59}$, $X_{62}$, $X_{63}$, $X_{54}$, $X_{101}$ ($R_{43} = —(CH_2)—_{1\text{-}2}$), $X_{103}$($R_{43} = —(CH_2)—_{1\text{-}2}$) und $R_{45} = —NH—(CH_2)_{2\text{-}3}—N(C_2H_5)_2$, $X_{104}$($R_{44}$ = H, g = 2 oder 3), $X_{108}$($R_{43} = —(CH_2)—_{1\text{-}3}$),

$X_{70}$ $-NH-CO-NH-$, $X_{71}$ 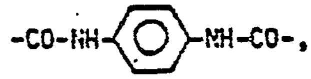

$X_{72}$ 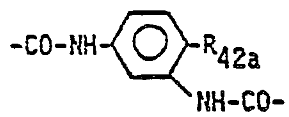

$X_{73}$ $-NH-CO-CH_2-CH_2-CO-NH-$,

$X_{74}$ $-NH-CO-CH{=}CH-CO-NH-$,

$X_{75}$ $-NH-CO-(CH_2)_4-CO-NH-$,

$X_{76}$ 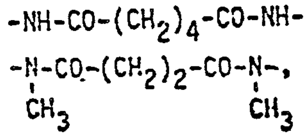

$X_{77}$ 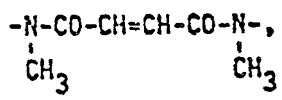

$X_{78}$ 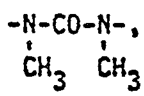

$X_{79}$ 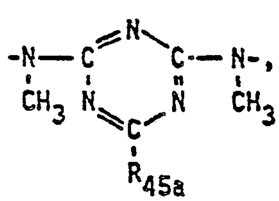 $X_{80}$ 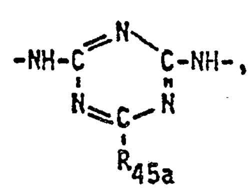

$X_{81}$ $-CH_2-$, $X_{82}$ $-(CH_2)_2-$, $X_{83}$ $-(CH_2)_3-$,

$X_{84}$ $-(CH_2)_4-$, $X_{85}$ $-CO-NH-(CH_2)_2-NH-CO-$,

$X_{86}$ $-CO-NH-(CH_2)_3-NH-CO-$, $X_{87}$ $-CO-NH-(CH_2)_4-NH-CO-$,

$X_{88}$ 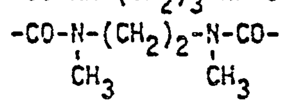 $X_{89}$ 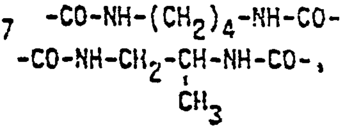

$X_{90}$ 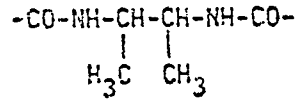

steht,

$R_{42a}$ unabhängig voneinander Wasserstoff, $-Cl$, $-CH_3$ oder $-OCH_3$ und

$R_{45a}$ unabhängig voneinander

$-Cl$, $-NH-CH_2-CH_2-OH$, $OCH_3$, $OH$, $NH_2$, $-N(CH_2-CH_2OH)_2$, $-NH-(CH_2)_3N(C_2H_5)_2$, $OC_2H_5$,

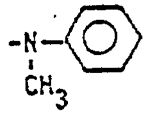 , 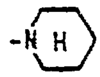 oder 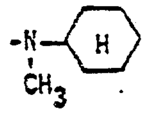

bedeuten,

oder

X, falls die Ringe $D_2$ und $D_3$ substituiert sind, insbesondere in ortho-Stellung zu den beiden Azobrücken durch Chlor, Methyl oder Methoxy X für die direkte Bindung $X_1$, $X_{40}$, $X_{70}$, $X_{71}$, $X_{80}$, $X_{81}$, X 82 oder X 85 steht,

$R_s'$ und $R_t'$ unabhängig voneinander Wasserstoff, Methyl, Methoxy, Aethyl, Aethoxy oder Butyl,

$R_{160}'$ einen Rest $-(CH_2)_{v'}-Z_2$,

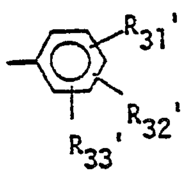 , 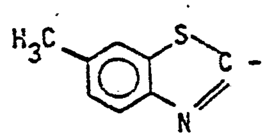 ,

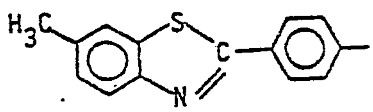 oder 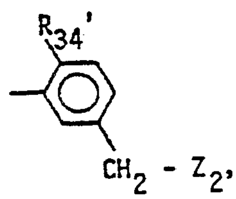

v' 2, 3 oder 4

$R_{18}'$ und $R_{19}'$ unabhängig voneinander Wasserstoff, Chlor, $CH_3$, $C_2H_5$, $OCH_3$ oder $OC_2H_5$,

$R_{20}'$ Methyl oder Phenyl,

$R_{21}'$ einen der Reste von $T_2$,

$R_{22}'$ einen Rest $-R_1''-NH-R_0''$, Wasserstoff, Methyl, Aethyl, Benzyl, Phenyläthyl,

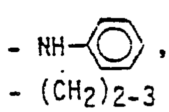 , $-(CH_2)_2-OH$, $-(CH_2)_3OCH_3$ $-(CH_2)_2CN$,

$-(CH_2)_{2-3}-N(CH_3)_2$, $-(CH_2)_{2-3}-\overset{\oplus}{N}(CH_3)_3\ A^{\ominus}$ oder

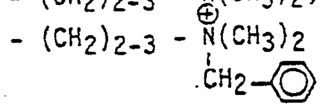 $A^{\ominus}$,

$R_{24}'$ unabhängig voneinander Wasserstoff, $-(CH_2)_2OH$,

$-(CH_2)_3-OCH_3$, $-(CH_2)_{2-3}-N(CH_3)_2$

$-(CH_2)_{2-3}-\overset{\oplus}{N}(CH_3)_3\ A^{\ominus}$.

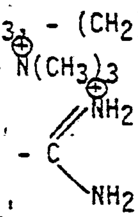 $A^{\ominus}$,

$R_{27}'$ Wasserstoff, 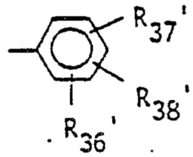

$R_{28}'$ unabhängig voneinander

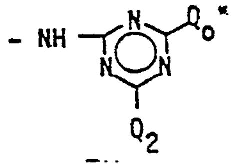 oder - NHCOCH$_2$ - Z$_2$.

- CONH(CH$_2$)$_{2-3}$ - Z$_2$ oder 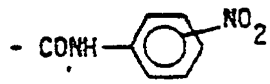

$R_{29}'$ $CH_3$, $C_2H_5$, $C_2H_4CN$ oder Benzyl,

$R_{30}'$ $CH_3$, $C_2H_5$ oder $-(CH_2)_{v'}-Z_2$,

$R_{31}'$ und $R_{32}'$ unabhängig voneinander Wasserstoff, Chlor, Brom, Methyl, Aethyl, Methoxy, Aethoxy, $NO_2$ oder CN,

$R_{33}'$ Wasserstoff,

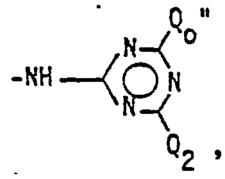 ,

$-NH-CO(CH_2)_{2-3}-Z_2$ oder $-SO_2-NH(CH_2)_{2-3}-Z_2$,

$R_{34}'$ Methoxy oder Aethoxy,

$R_{36}'$ und $R_{37}'$ unabhängig voneinander Wasserstoff, Chlor, Methyl oder Methoxy,

$R_{38}'$ Wasserstoff,

-CO-NH - (CH$_2$)$_{v'}$ - Z$_2$,

- NHCO(CH$_2$)$_{2-3}$ - Z$_2$ oder 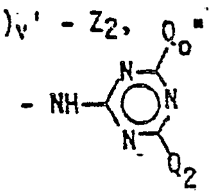

$R_{21a}'$ H, Cl, $CH_3$, OH, $OCH_3$, $C_2H_5$ oder $OC_2H_5$,

$R_{23a}'$ unabhängig voneinander H, Cl, $CH_3$, OH oder $OCH_3$,

$U_1$ die direkte Bindung, —NHCO— oder —NHCONH—,

$R_{1a}''$ Wasserstoff,

- NR$_{50}$ - (CH$_2$)$_{2-3}$ - NR$_{11}$"R$_{12}$",

- NR$_{50}$ - (CH$_2$)$_{2-3}$ - NR$_{13}$"R$_{14}$"R$_{15}$" A$^{\ominus}$ oder

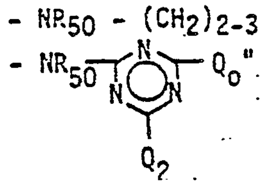

$W_0''$ —NH—, —NH—CO—CH = CH—CONH— oder —NHCO$(CH_2)_2$CONH—,

$K_1''$ einen Rest der Formel aa'), $aa_1'$), $aa_2'$), $aa_3'$), $aa_4'$), $aa_5'$), $aa_6'$), $aa_7'$), $aa_8'$), $aa_9'$), $aa_{10}'$), $aa_{11}'$); $aa_{12}'$) oder einen Rest der Formel

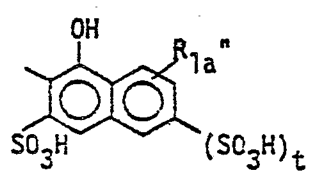 $aa_{13}'$) oder

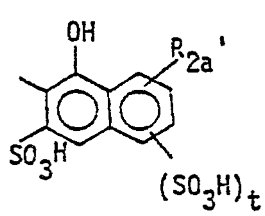 $aa_{14}'$)

und KK' einen Rest der Formel aa'), $aa_1'$), $aa_2'$), $aa_4'$), $aa_5'$), $aa_6'$), $aa_7'$), $aa_8'$), $aa_9'$), $aa_{10}'$), $aa_{11}'$), $aa_{12}'$), $aa_{13}'$), $aa_{14}'$) bedeuten,
Gemische der Verbindungen der Formel III, deren Säureadditionssalze oder Salze von Säuren sowie 1 : 1- oder 1 : 2-Metallkomplexe davon, mit den zuvor genannten Bedingungen und der Massgabe, dass

$\beta_2$) falls r für 2, S'' für Wasserstoff, $R_0''$ für Wasserstoff stehen, $R_1''$ verschieden ist von 1,2-Aethylen, $T_1$ verschieden von CN und der Rest M'' von Sulfonsäuregruppen frei.

4. Metallkomplexverbindungen gemäss Anspruch 1 entsprechend den Formeln

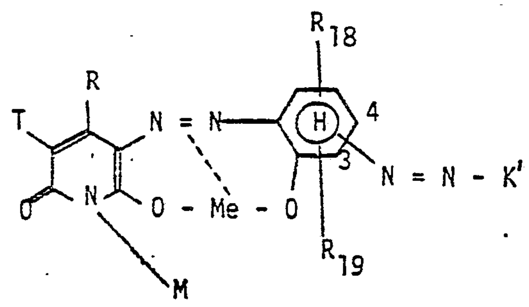

(V)

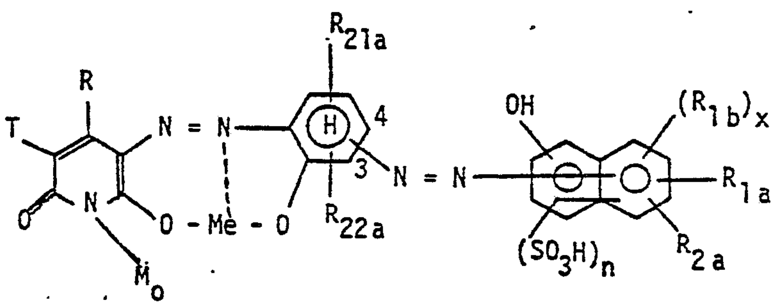

(VI)

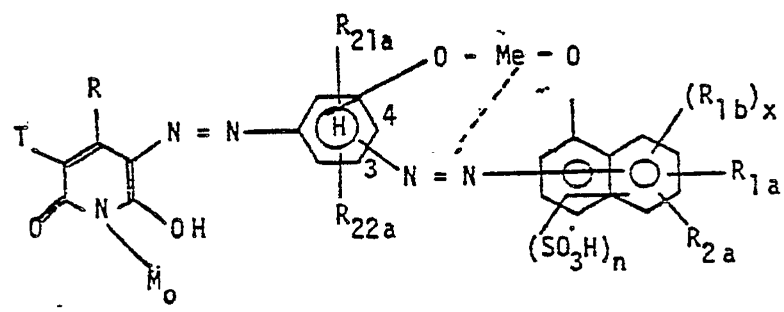

(VII)

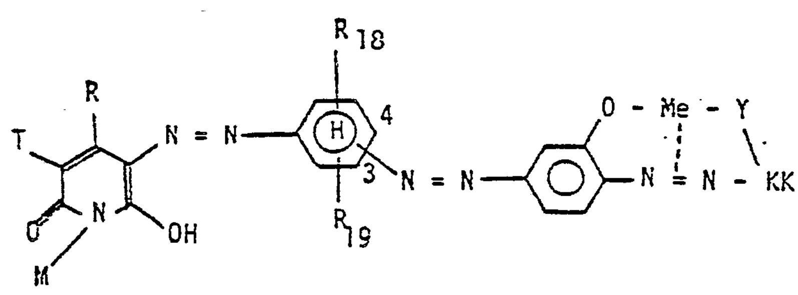

(VIII)

worin Y—O— oder —NH— bedeutet,

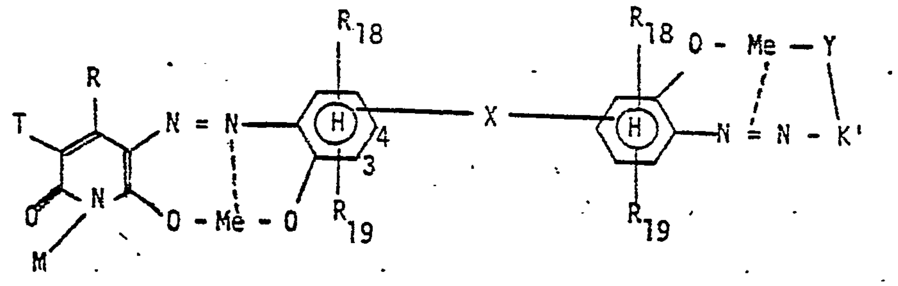

(IX)

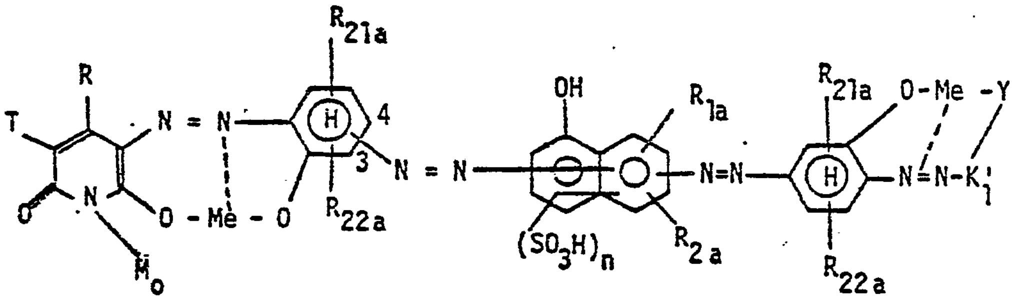

(X)

wobei die Azoreste in den Ringen H in 3- oder 4-Stellung gebunden sind, und

Me ein Kupfer-, Chrom-, Kobalt-, Nickel- oder Manganatom für die 1 : 1-Komplexe, vorteilhaft ein Kupferatom und

Me ein Chrom-, Kobalt- oder Eisenatom, vorteilhaft Eisenatom für die 1 : 2-Metallkomplexe bedeutet.

5. Verfahren zur Herstellung von Verbindungen der Formel I gemäss Anspruch 1, worin S verschieden von Wasserstoff ist, dadurch gekennzeichnet, dass man die Diazoverbindung aus einem Amin der Formel

$$DD—NH_2, \qquad (XI)$$

worin DD einen Rest

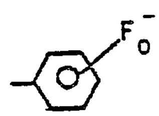

(XIa)

$$—A—N = N—K \qquad (XI\ b)$$

oder

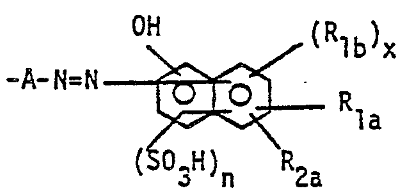

XI c)

bedeutet, mit einer Verbindung der Formel

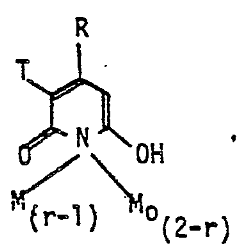

(XII)

kuppelt, und gewünschtenfalls die erhaltenen Verbindungen in die Metallkomplexe überführt.

6. Verfahren zur Herstellung von Verbindungen der Formel I gemäss Anspruch 1, worin S für Wasserstoff steht, dadurch gekennzeichnet, dass man eine Verbindung der Formel

$$T—CH_2—CO—NH—R_1—NH—M_{00} \qquad XXVI,$$

worin $M_{00}$ eine beliebige Schutzgruppe bedeutet, vorteilhaft, dass man eine Verbindung der Formel

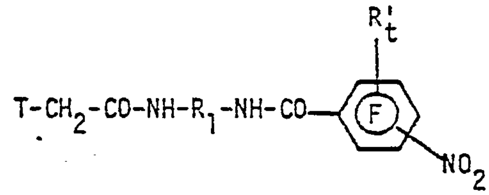

XXVII

worin $R_t'$ Wasserstoff, Halogen, (1-4C)-Alkyl oder (1-4C)-Alkoxy bedeutet, mit einem Essigsäurealkylester der Formel

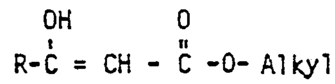

XXVIII

nach an sich bekannten Methoden zu einer Verbindung der Formel

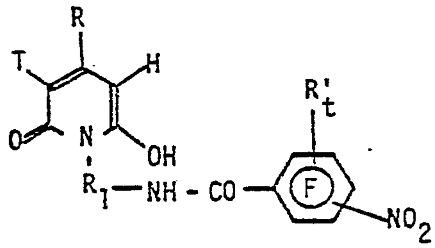

XXIX

cyclisiert, und für Verbindungen, worin $R_0$ Wasserstoff bedeutet, diese Verbindung verseift, oder für Verbindungen, worin $R_{00}$ einen Rest der Formel

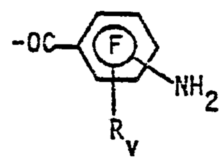

XXa

bedeutet, die Nitrogruppe zur Aminogruppe reduziert.

7. Verwendung der Verbindungen der Formel I gemäss Anspruch 1, worin S von Wasserstoff verschieden ist, zum Färben oder Bedrucken von Papier und Leder.

8. Verwendung der Verbindungen der Formel I gemäss Anspruch 1, worin S von Wasserstoff verschieden ist, zum Färben, Foulardieren oder Bedrucken von natürlicher oder regenerierter Cellulose sowie von Bastfasern, wie Hanf, Flachs, Sissal, Jute, Kokos oder Stroh.

9. Das gemäss Anspruch 7 gefärbte oder bedruckte Material.

10. Das gemäss Anspruch 8 gefärbte, foulardierte oder bedruckte Material.

**Patentansprüche** (für den Vertragsstaat AT)

1. Verfahren zur Herstellung von Verbindungen bzw. Azoverbindungen, die in einer der möglichen tautomeren Form der Formel

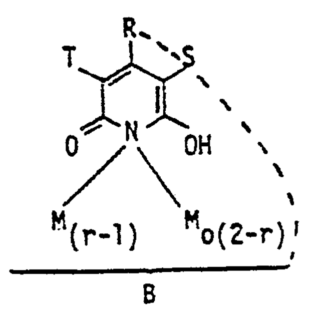

(I)

entsprechen, worin

R unabhängig voneinander Wasserstoff, einen (1-4C)-Alkyl-, (5-6C)-Cycloalkyl- oder Phenylrest, Benzyl oder Phenyläthyl,

T unabhängig voneinander Wasserstoff,

i) -CN

$i_1)$ 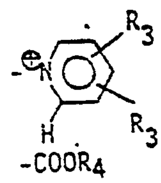 $A^{\ominus}$

$i_2)$ $-COOR_4$

$i_3)$ $-CONR_5R_6$

$i_4)$ $-SO_2NR_5R_6$

$i_5)$ 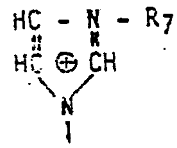 $A^{\ominus}$ oder

$i_6)$ 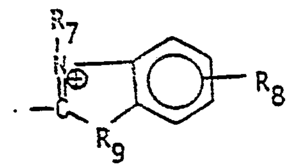 $A^{\ominus}$

r 1 oder 2,

M unabhängig voneinander einen Rest $—R_1—NH—R_0$,

$M_0$ unabhängig voneinander einen Rest $—R_1—NH—R_0$, Wasserstoff, $—NR_{11}R_{12}$, (1-4C)-Alkyl, Hydroxy (2-4C)-alkyl, (1-4C)-Alkoxy-(1-4C)-alkyl, gegebenenfalls durch 1 bis 3 (1-4C)-Alkyl substituiertes (5-6C)-Cycloalkyl, Phenyl (1-3C)-alkyl, $—V_1—NR_{13}R_{14}$ oder $—V_2—$

$$\overset{\oplus}{N}R_{13}R_{14}R_{15}A^{\ominus}$$

$A^{\ominus}$ ein Anion, bedeuten,

S falls r für 2 steht,

a) Wasserstoff,

b) einen Rest der Formel

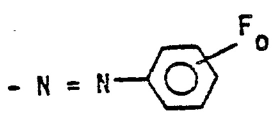 oder

c) $—N=N—A—N=N—K$

und $F_0$ einen in der Azochemie üblichen Substituenten, vorzugsweise Halogen, $NO_2$, $NH_2$, Alkyl, Alkoxy, CN, Trifluoralkyl, Phenyl, Anilin, Benzoyl, Carbamoyl, Phenoxy, Halogenphenoxy, Dihalogenphenoxy, Alkylsulfonyl, Phenylsulfonyl, Alkylsulfonylamine, Di-N-alkylaminosulfonyl oder Alkylsulfonyl, bedeuten oder, falls r für 1 steht,

d) einen Rest der Formel

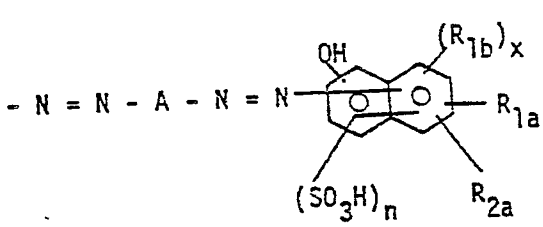

bedeutet, worin

n 0, 1 oder 2,
x 0 oder 1,
A unabhängig voneinander den Rest einer Tetrazokomponente,
K den Rest einer beliebigen, vorzugsweise sulfonsäuregruppenfreien Diazo- oder Kupplungskomponente aus der Reihe Pyrazolon-5, 5-Aminopyrazol, Anilin, Phenol, Acetoacetylalkyl- oder -arylamid, Barbitursäure, Dimedon, Dimedoncarbonsäureester, Pyridon (z. B. des Restes B, worin r 1 oder 2 ist) oder 2,6-Diaminopyridin,
$R_{1b}$, falls x für 1 steht, einen Rest der Formel
e) $-N=N-K_1$
f) $-N=N-A_1-N=N-K_1$

g) 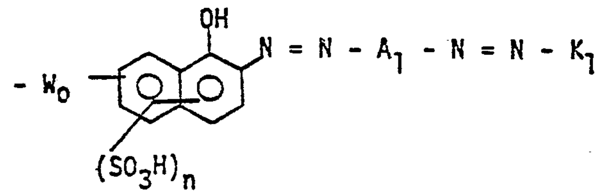

$K_1$ einen Rest K oder ein α- oder β-Naphthol, ein α- oder β-Aminonaphthalin oder ein Aminonaphthol,
$R_1$ unabhängig voneinander einen gegebenenfalls durch ein oder 2 Heteroatome unterbrochenen Alkylen- oder Alkenylenrest, einen Phenylen- oder Cyclohexylenrest,
$R_0$ oder einen Rest der Formel

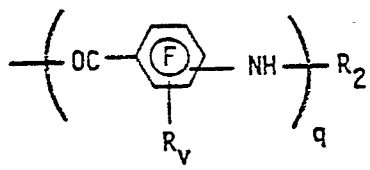
(Ia)

q 0 oder 1 und
$R_v$ Wasserstoff, Halogen, Nitro, (1-4C)-Alkyl oder (1-4C)-Alkoxy, bedeuten,
$R_2$ steht für einen Rest der Formel

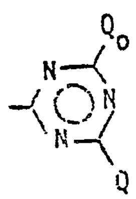
$h_1$)

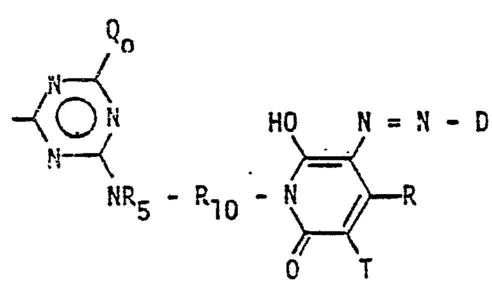
$h_3$) oder

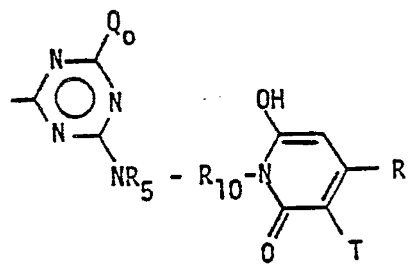
$h_2$)

$- CO - (CH_2)_{1-3} - Z$ $h_4$)

oder auch für Wasserstoff, worin

D unabhängig voneinander den Rest einer Diazokomponente, die durch in der Azochemie übliche Substituenten substituiert sein kann, vorzugsweise einen Rest

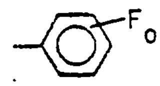

- A - N = N - K oder - A - N = N 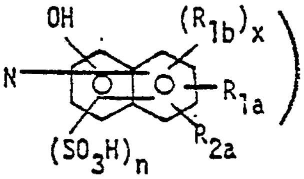

$R_3$ unabhängig voneinander Wasserstoff, einen (1-4C)-Alkylrest, $—NR_5R_6$ oder $—CONR_5R_6$,

$R_4$ unabhängig voneinander (1-6C)-Alkyl oder Phenyl-(1-3C)-alkyl,

$R_5$ und $R_6$ unabhängig voneinander Wasserstoff oder (1-4C)-Alkyl,

$R_7$ unabhängig voneinander (1-4C)-Alkyl,

$R_8$ unabhängig voneinander Wasserstoff oder (1-4C)-Alkyl,

$R_9$ unabhängig voneinander —S—, —O— oder

$$-\overset{|}{N}-R_5$$

$R_{10}$ unabhängig voneinander einen (2-6C)-Alkylenrest oder

$$-\overset{*}{N}HCOCH_2-$$

wobei das mit * bezeichnete Atom am $—NR_5—$ Rest gebunden ist,

$Q_0$ einen aliphatischen, cycloaliphatischen, aromatischen oder heterocyclischen Aminrest, in welchen das N-Atom an das C-Atom des Triazinylrestes gebunden ist,

Q Halogen, OH, $NH_2$, (1-4C)-Alkoxy, Phenyl oder $Q_0$,

Z unabhängig voneinander einen Rest der Formel $—NR_{11}R_{12}$ oder

$$-\overset{\oplus}{N}R_{13}R_{14}R_{15} \quad A^{\ominus}$$

$R_{11}$ und $R_{12}$ unabhängig voneinander Wasserstoff, unsubstituiertes (1-6C)-Alkyl, durch OH, CN oder Halogen substituiertes (2-6C)-Alkyl, Phenyl-(1-3C)-Alkyl, dessen Phenylrest durch 1 bis 3 Substituenten aus der Reihe Chlor, (1-4C)-Alkyl und (1-4C)-Alkoxy substituiert sein kann, unsubstituiertes oder durch 1 bis 3 (1-4C)-Alkylgruppen substituiertes (5-6C)-Cycloalkyl,

$R_{13}$ und $R_{14}$ unabhängig voneinander eine der Bedeutungen von $R_{11}$ und $R_{12}$, wobei einer der Reste $R_{13}$ und $R_{14}$ und vorzugsweise beide Reste von Wasserstoff verschieden sind,

$R_{15}$ unabhängig voneinander einen gegebenenfalls durch Phenyl substituierten (1-4C)-Alkylrest,

$V_1$ unabhängig voneinander (1-6C)-Alkylen oder (2-6C)-Alkenylen, unabhängig voneinander (2-6C)-Alkylen oder

$V_2$ (2-6C)-Alkenylen bedeuten,

die Reste $R_5$ und $R_6$ bzw. $R_{11}$ und $R_{12}$, bzw. $R_{13}$ und $R_{14}$ zusammen mit dem an sie gebundenen N-Atom einen gesättigten heterocyclischen Ring und $R_{13}$, $R_{14}$ und $R_{15}$ zusammen mit dem an sie gebundenen N-Atom einen Pyridinring oder einen gesättigten heterocyclischen Ring bilden können und der Pyridin- und der heterocyclische Ring durch eine, zwei oder drei (1-4C)-Alkylgruppen substituiert sein können,

$R_{1a}$ Wasserstoff,

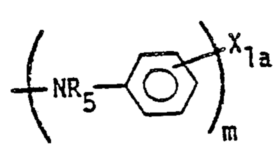 oder

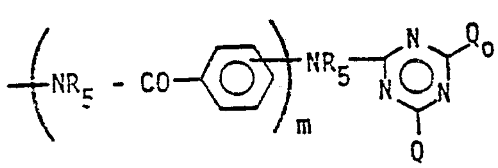

wobei für x = 1, $R_{1a}$ Wasserstoff bedeutet,

$$X_{1a}\ -NR_5-Q_{10}-NR_{11}R_{12},\ -COCH_2-\overset{\oplus}{N}R_{13}R_{14}R_{15}\ A^{\ominus},$$
$$-NR_5-COCH_2-\overset{\oplus}{N}R_{11}R_{12},\ -CO-Q_{10}-\overset{\oplus}{N}R_{13}R_{14}R_{15}\ A^{\ominus}$$
$$-NR_5-Q_{10}-\overset{\oplus}{N}R_{13}R_{14}R_{15}\ A^{\ominus},\ -NR_5-COCH_2\overset{\oplus}{N}R_{13}R_{14}R_{15}A^{\ominus}$$
$$\text{oder}\ -CO-Q_{10}-\overset{\oplus}{N}(CH_3)_2-(CH_2)_4-Z\ A^{\ominus},$$

m 0 oder 1,
$Q_{10}$

$$-\overset{*}{N}HCOCH_2-$$

oder lineares oder verzweigtes (2-6C)-Alkylen,

$R_{2a}$ Wasserstoff oder, wenn $R_{1a}$ für Wasserstoff und $R_{1b}$ für einen Rest der Formel e) oder f) steht, auch OH, $NH_2$, (1-4C)-Alkylcarbonylamino, Benzoylamino oder Phenylamino, deren Phenylrest durch 1 oder 2 Substituenten aus der Reihe Halogen, $NO_2$, $NH_2$ (1-4C)-Alkyl und (1-4C)-Alkoxy substituiert sein kann,

$A_1$ unabhängig voneinander den Rest einer Tetrazokomponente oder den Rest einer Diazo-/-Kupplungskomponente und

$W_0$ ein zweiwertiges Brückenglied bedeuten,

Gemische der Verbindungen der Formel I, deren Säureadditionssalze oder Salze von Säuren sowie Metallkomplexe, wie 1 : 1- oder 1 : 2-Metallkomplexe davon, mit der Massgabe, dass sie,

α) falls die Monoazoverbindungen b) der Formel I sulfogruppenfrei sind, eine und und vorzugsweise zwei und, falls die Dis- und Polyazoverbindungen der Formel I sulfogruppenfrei sind, mindestens zwei wasserlöslich machende kationische oder mindestens zwei wasserlöslichmachende protonierbare basische Gruppen oder mindestens eine kationische und mindestens eine protonierbare basische Gruppen enthalten und, falls die Verbindungen der Formel I Sulfogruppen enthalten, die Anzahl der kationischen und/oder protonierbaren basischen Gruppen die Anzahl der Sulfogruppen und der eventuell zusätzlich vorhandenen anionischen Gruppen um mindestens 1 übersteigt, und

β) falls r für 2, S für Wasserstoff und $R_0$ für Wasserstoff stehen, $R_1$ verschieden ist von Alkylen, T verschieden von CN und der Rest M von Sulfonsäuregruppen frei,

dadurch gekennzeichnet, dass man falls S verschieden von Wasserstoff ist, die Diazoverbindung aus einem Amin der Formel

$$DD—NH_2, \qquad XI,$$

worin DD einen Rest

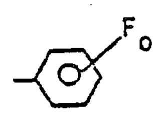

XI a)

$$—A—N=N—K \qquad XI\ b)$$

oder

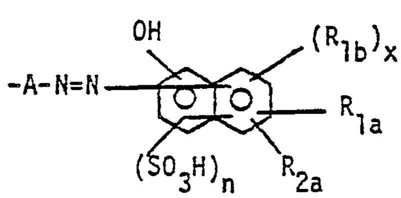

XI c)

bedeutet, mit einer Verbindung der Formel

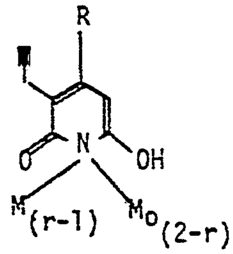

XII

kuppelt, und gewünschtenfalls die erhaltenen Verbindungen in die Metallkomplexe überführt, oder, falls S' Wasserstoff bedeutet, eine Verbindung der Formel

$$T—CH_2—CO—NH—R_1—NH—M_{00} \qquad XXVI,$$

worin $M_{00}$ eine beliebige Schutzgruppe bedeutet, vorteilhaft, dass man eine Verbindung der Formel

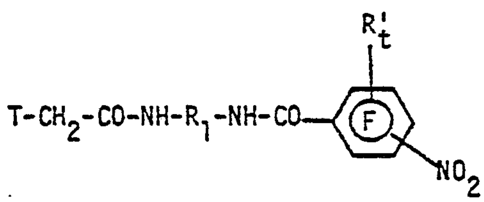

XXVII

worin $R_t'$ Wasserstoff, Halogen (1-4C)-Alkyl oder (1-4C)-Alkoxy bedeutet, mit einem Essigsäurealkylester der Formel

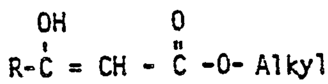

XXVIII

nach an sich bekannten Methoden zu einer Verbindung der Formel

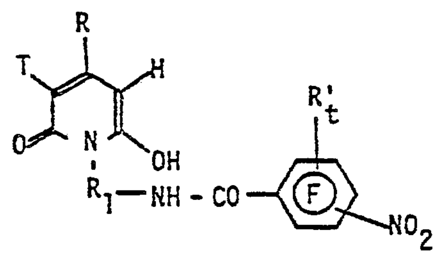

XXIX

cyclisiert, und für Verbindungen, worin $R_0$ Wasserstoff bedeutet, diese Verbindung verseift, oder für Verbindungen, worin $R_{00}$ einen Rest der Formel

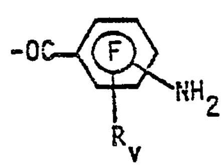

XXa

bedeutet, die Nitrogruppe zur Aminogruppe reduziert.

2. Verfahren zur Herstellung von Metallkomplexverbindungen der Formel I gemäss Anspruch 1, worin S von Wasserstoff verschieden ist, dadurch gekennzeichnet, dass man eine Verbindung der Formel I mit solchen metallabgebenden Verbindungen umsetzt, dass entweder 1 : 1 oder 1 : 2-Metallkomplexe erhalten werden.

3. Lagerstabile, flüssige Farbstoffpräparationen enthaltend eine Verbindung der Formel I gemäss Anspruch 1 in Form eines wasserlöslichen Säureadditionssalzes oder als quaternäres Ammoniumsalz.

4. Wasserlösliche, feste granulierte Farbstoffpräparate enthaltend eine Verbindung der Formel I gemäss Anspruch 1 in Form eines wasserlöslichen Säureadditionssalzes oder als quaternäres Ammmoniumsalz.

5. Verwendung der Verbindungen der Formel I gemäss Anspruch 1 zum Färben oder Bedrucken von Papier und Leder.

6. Verwendung der Verbindungen der Formel I gemäss Anspruch 1 zum Färben, Foulardieren oder Bedrucken von natürlicher oder regenerierter Cellulose oder von Bastfasern, wie Hanf, Flachs, Sissal, Jute, Kokos oder Stroh.

**Claims** (for the Contracting States : BE, CH, DE, FR, GB, IT, LI, NL, SE)

1. Basic and azo compounds, corresponding to one of the possible tautomeric forms of the formula

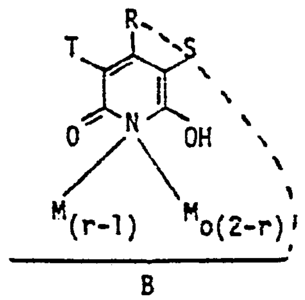

(I)

wherein

each R independently is hydrogen a (1-4C)-alkyl, (5-6C)-cycloalkyl or phenyl group, benzyl or phenylethyl,

each T independently is hydrogen,

i) -CN

$i_1$)

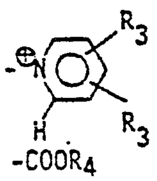

$A^{\ominus}$.

$i_2$) $-COOR_4$

$i_3$) $-CONR_5R_6$

$i_4$) $-SO_2NR_5R_6$

$i_5$)

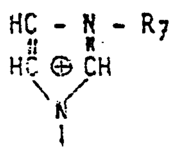

$A^{\ominus}$ oder

$i_6$)

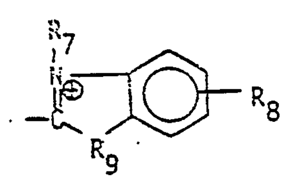

$A^{\ominus}$

r is 1 oder 2,

each M independently is a group $—R_1—NH—R_0$,

each $M_0$ independently is a group $R_1—NH—R_0$, hydrogen, $—NR_{11}R_{12}$, (1-4C)-alkyl, hydroxy-(2-4C)-alkyl, (1-4C)-alkoxy-(1-4C)-alkyl, (5-6C)-cycloalkyl optionally substituted by 1 to 3 (1-4C)-alkyl groups, phenyl-(1-3C)-alkyl, phenyl-(1-3C)-alkyl, $—V_1— NR_{13}R_{14}$ or $—V_2—$

$$\overset{\oplus}{N}R_{13}R_{14}R_{15}A^{\ominus}$$

$A^{\ominus}$ is an anion,

S, when r stands for the number 2, is
a) hydrogen,
b) a group of formula

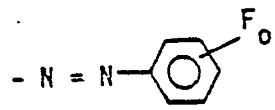

or

c) $—N=N—A—N=N—K$,
and $F_0$ is a substituent conventionally used in azo chemistry, preferably $F_0$ is halogen, $NO_2$, $NH_2$, alkyl, alkoxy, CN, trifluoroalkyl, phenyl, anilino, benzoyl, carbamoyl, phenoxy, halogenphenoxy, dihalogenphenoxy, alkylsulfonyl, phenylsulfonyl, alkylsulfonylamino, di-N-alkylaminosulfonyl or alkylsulfonyl, or S, when r stands for the number 1, is
d) a group of the formula

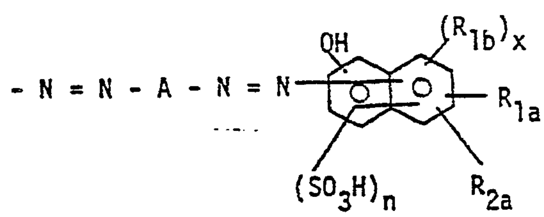

wherein
n is 0, 1 or 2,
x is 0 or 1,
each A independently is the residue of a tetrazo component,
K is the residue of a diazo or coupling component, preferably free from sulfonic acid groups, of the pyrazolon-5, 5-aminopyrazole, aniline, phenol, acetoacetylalkyl- or arylamide, barbituric acid, dimedone, dimedonecarbonic acid ester or pyridone series e. g. the residue B, wherein r is 1 or 2, or 2,6-diaminopyridine,
$R_{1b}$, when x stands for 1, is a group of the formula
e) $—N=N—K_1$,
f) $—N=N—A_1—N=N—K_1$,

g) 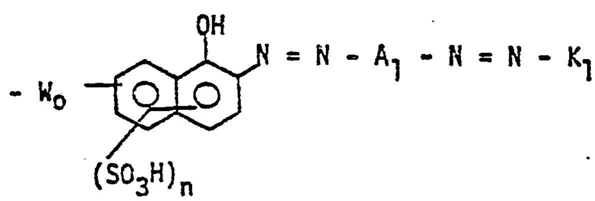

$K_1$ has one of the significances of the group K or is an α- or β-naphthol, an α- or β-aminonaphthalene or an aminonaphthol,
each $R_1$ independently is an alkylene- or alkenylene group optionally interrupted by 1 or 2 heteroatoms or a phenylene- or cyclohexylene group,
$R_0$ is a group of the formula

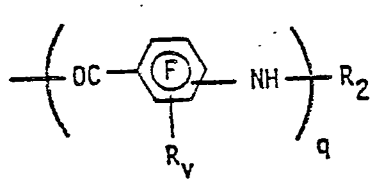

(Ia)

q is 0 or 1 and
$R_v$ is hydrogen, halogen, nitro, (1-4C)-alkyl or (1-4C)-alkoxy,
$R_2$ is a group of the formula

(See formula page 119)

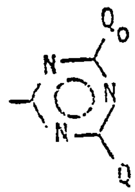

h1)

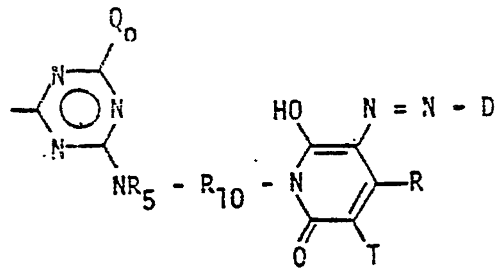

h3) or

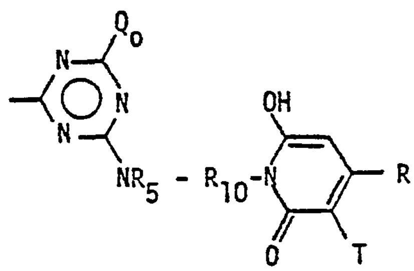

h2)

$- CO - (CH_2)_{1-3} - Z$ h4)

or hydrogen, wherein

each D independently is the residue of a diazo compound, which may be substituted by substituents conventionally used in the azo chemistry, preferably a group

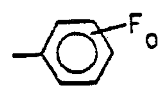

- A - N = N - K or - A - N = N 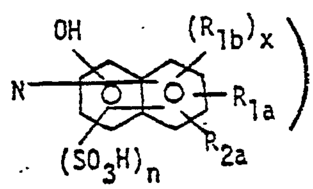

each $R_3$ independently is hydrogen, (1-4C)-alkyl, $—NR_5R_6$ or $—CONR_5R_6$,
each $R_4$ independently is (1-6C)-alkyl or phenyl-(1-3C)-alkyl,
each $R_5$ and $R_6$ independently is hydrogen or (1-4C)-alkyl,
each $R_7$ independently is (1-4C)-alkyl,
each $R_8$ independently is hydrogen or (1-4C)-alkyl,
each $R_9$ independently is —S—, —O— or

$$-\overset{|}{N}-R_5$$

each $R_{10}$ independently is an (2-6C)-alkylene group or

$$-\overset{*}{N}HCOCH_2-$$

where the atom marked * is bound to the $—NR_5—$,

$Q_0$ is an aliphatic, cycloaliphatic, aromatic or heterocyclic amino group, wherein the N-atom is bound at the C-atom of the triazinyl group,

Q is halogen, OH, $—NH_2$, (1-4C)-alkoxy, phenyl or $Q_0$,

each Z independently is a group of formula $—NR_{11}R_{12}$ or

$$-\overset{\oplus}{N}R_{13}R_{14}R_{15}\ A^{\ominus}$$

each $R_{11}$ and $R_{12}$ independently is hydrogen, unsubstituted (1-6C)-alkyl, (2-6C) alkyl substituted by OH, CN or halogen, phenyl-(1-3C)-alkyl which may be substituted in the phenyl group by 1 to 3 substituents selected from chloro, (1-4C)-alkyl or (1-4C)-alkoxy, unsubstituted (5-6C)-cycloalkyl or (5-6C)-cycloalkyl substituted by 1 to 3 (1-4C)-alkyl groups,

each $R_{13}$ and $R_{14}$ independently have one of the meanings of $R_{11}$ and $R_{12}$, whereby one of the group $R_{13}$ and $R_{14}$ and preferably both groups are different from hydrogen,

each $R_{15}$ independently is a (1-4C)-alkyl group optionally substituted by phenyl,

each $V_1$ independently is (1-6C)-alkylene or (2-6C)-alkenylene,

$V_2$ independently is (2-6C)-alkylene or (2-6C)-alkenylene, the groups $R_5$ and $R_4$, $R_{11}$ and $R_{12}$, $R_{13}$ and $R_{14}$ respectively may form together with the N-atom, to which they are attached a saturated heterocyclic ring and $R_{13}$, $R_{14}$ and $R_{15}$ may form together with the N-atom, to which they are attached a pyridine ring or a saturated heterocyclic ring and the pyridine and the heterocyclic ring may be substituted by one, two or three (1-4C)-alkyl groups,

$R_{1a}$ is hydrogen,

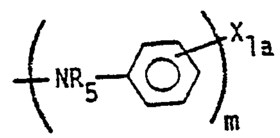

or 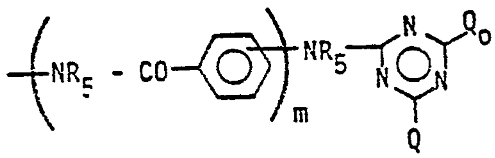

wherein when x is the number 1, $R_{1a}$ is hydrogen,

$X_{1a}$ is

$$-NR_5-Q_{10}-NR_{11}R_{12},\ -COCH_2-\overset{\oplus}{N}R_{13}R_{14}R_{15}\ A^{\ominus},$$
$$-NR_5-COCH_2-NR_{11}R_{12},\ -CO-Q_{10}-\overset{\oplus}{N}R_{13}R_{14}R_{15}\ A^{\ominus},$$
$$-NR_5-Q_{10}-\overset{\oplus}{N}R_{13}R_{14}R_{15}\ A^{\ominus},\ -NR_5-COCH_2\overset{\oplus}{N}R_{13}R_{14}R_{15}A^{\ominus}$$
$$\text{or}\quad -CO-Q_{10}-\overset{\oplus}{N}(CH_3)_2-(CH_2)_4-Z\ A^{\ominus},$$

m is 0 or 1,

$Q_{10}$ is

$$-\overset{*}{N}HCOCH_2-$$

or linear or branched (2-6C)-alkylene,

$R_{2a}$ is hydrogen or, when $R_{1a}$ is hydrogen and $R_{1b}$ stands for a group of formulae e) or f) is also OH, $NH_2$, (1-4C)-alkylcarbonylamino, benzoylamino or phenylamino, whose phenyl group may be substituted by 1 or 2 substituents selected from halogen, $NO_2$, $NH_2$, (1-4C)-alkyl and (1-4C)-alkoxy,

each $A_1$ independently is the residue of a tetrazo compound or the residue of a diazo- and/or coupling component and

$W_0$ is a divalent bridging group,

mixtures of compounds of formula I, their acid addition salts or salts of acids and 1 : 1- or 1 : 2-metal complexes thereof, with the proviso that

α) when the monoazo compounds b) of formula I are free from sulfonic acid groups, the compounds of formula b) have one and preferably two water solubilizing cationic groups or two water solubilizing protonatable basic groups or one cationic and one protonatable basic groups, and when the dis- and polyazo compounds of formula I are free from sulfonic acid groups, the dis- and polyazo compounds have at least two water-solubilizing cationic or at least two water-solubilizing protonatable basic groups or at least one cationic and at least one protonatable basic groups, and when the compounds of formula I contain sulfonic acid groups, the number of the cationic and/or protonatable basic groups is at least one greater than the sum of the sulfonic acid groups or possible additional anionic groups and

β) when r stands for the number 2, S is hydrogen and $R_0$ is hydrogen, $R_1$ is different from alkylene, T is different from CN and the group M is free from sulfonic acid groups.

2. Basic compounds according to Claim 1 corresponding to one of the possible tautomeric form of the formula

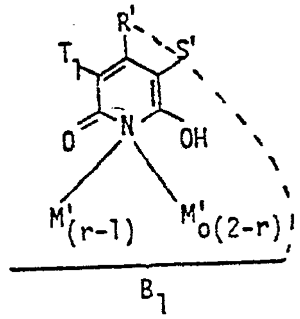

(II)

wherein

each R′ independently is methyl, ethyl, phenyl, benzyl or cyclohexyl,

each $T_1$ independently is hydrogen,

i) - CN

i$_1'$) 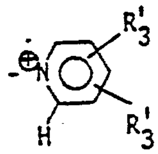 $A^{\ominus}$ or

i$_3'$) - $CONR_5'R_6'$,

each M′ independently is a group $-R_1'-NH-R_0'$,

each $M_0'$ independently is a group $-R_1'-NH-R_0'$, hydrogen, $CH_3$, $-C_2H_5$, $C_2H_4OH$, cyclohexyl, benzyl, $-(CH_2)_{1-3}-NR_{13}'R_{14}'$ or

$$-(CH_2)_{2-3} - \overset{\oplus}{N}R_{13}'R_{14}'R_{15}'A^{\ominus}$$

S′, when r stands for the number 2, is

a) hydrogen,

b) is a group of formula

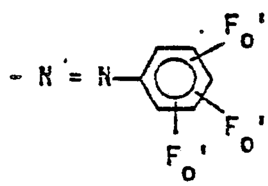

c$_1$) 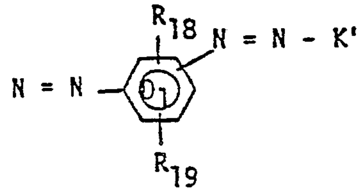 or

c$_2$) 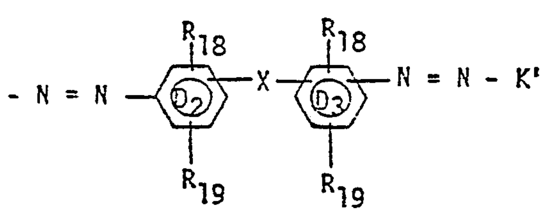

or S', when r stands for the number 1, is
d$_1$) a group of formula

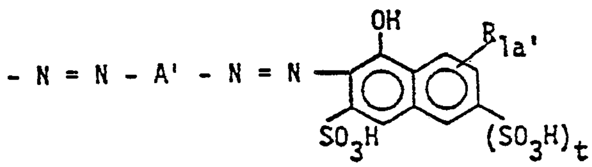

d$_2$) 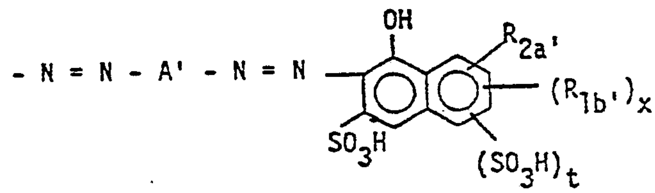

d$_3$) 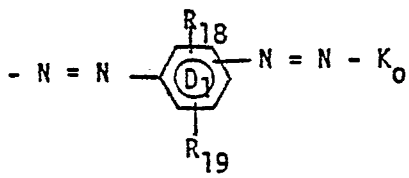 or

d$_4$) 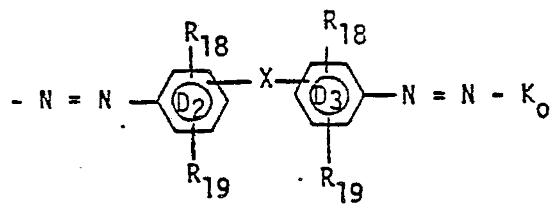

wherein
$K_0$ is a group of formula

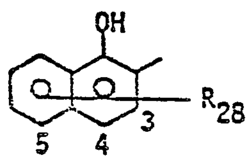

aa),

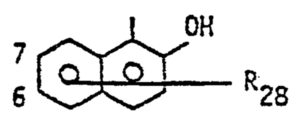

aa$_1$),

each A' independently is a group of formula

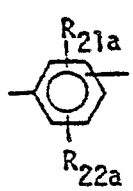

(m)

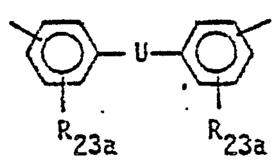

(n) or

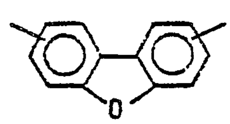

(o),

$R_{1b}'$, when x stands for the number 1, is a group of formula
e') —N = N—$K_1$'
f') —N = N—A'—N = N—$K_1$'

g') 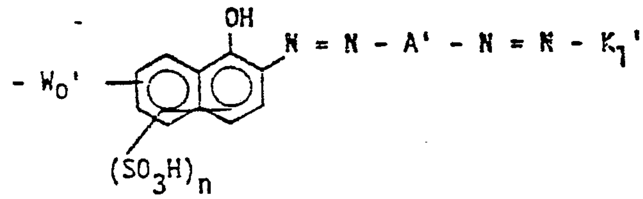

K' is a group of formula

$aa_4$) $B_1$, wherein r is 1 or 2,

$aa_5$) a phenol,

$aa_6$) 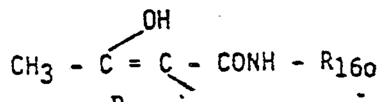

$aa_7$) 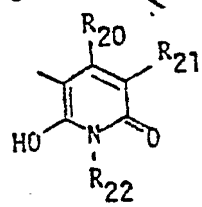

$aa_8$) 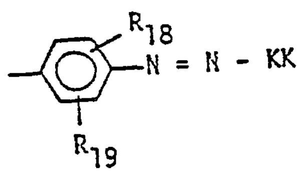

$aa_9$) 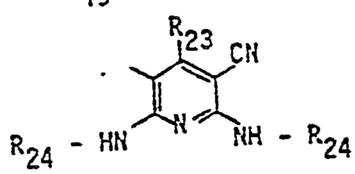

$aa_{10}$) 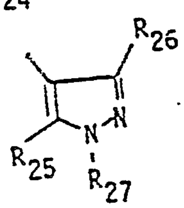

$aa_{11}$) 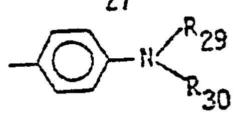

$aa_{12}$) 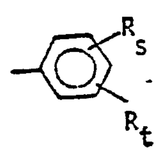

each $R_1'$ independently is a straight or branched (2-8C)-alkylene group or metaphenylene group, $R_0'$ is a group of formula

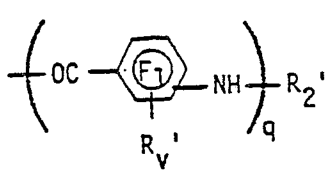 (Ib)

each $R_v'$ independently is hydrogen, chloro, nitro, methyl or ethyl $R_2'$ is a group of formula

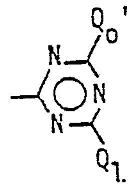 $h_1'$ or

$- CO -(CH_2)_{1-2} - Z_1$ $h_4'$) or

hydrogen,

each $R_3'$ independently is hydrogen, methyl, ethyl, $-NH_2$ or $-N(CH_3)_2$,
each $R_5'$ and $R_6'$ independently is hydrogen, methyl or ethyl,
$Q_1$ is chloro, —OH, $-NH_2$,

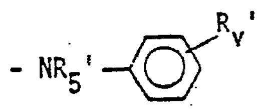 , 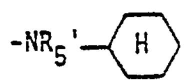 , $-OCH_3$

mono (1-4C)-alkylamino, di-(1-2C)-alkylamino, monohydroxy (2-4C)-alkylamino, bis [hydroxy (2-4C)-alkyl]-amino or $Q_0'$ and

$Q_0'$ is

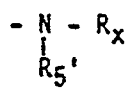

wherein

$R_x$ is an (1-12C)-alkyl radical, optionally substituted by OH, which may be interrupted by one and/or several groups of formula

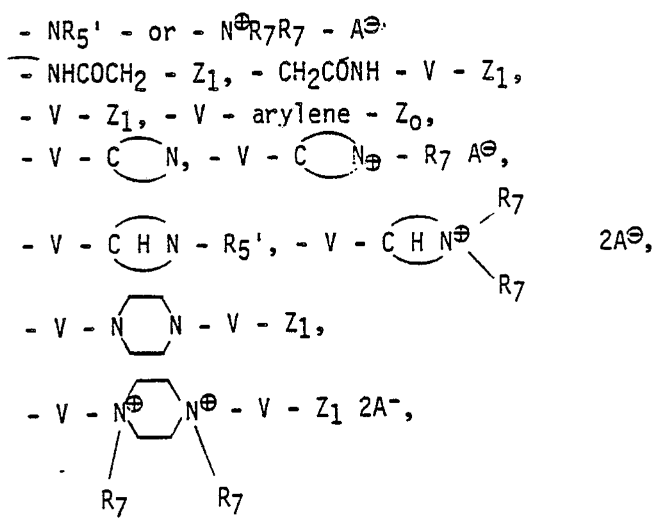

or $R_x$ and $R_5'$ together with the N-atom to which they are attached may form a group of formula

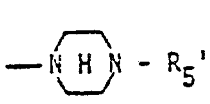 or 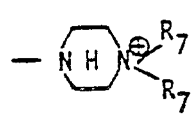 $2A^{\ominus}$

each V independently is an (1-8C)-alkylene radical or an (3-8C)-alkenylene radical,
$Z_0$ is $-N(CH_3)_2$,

$$-\overset{\oplus}{N}(C_2H_5)_3\ A^{\ominus}$$

or a group of formula $-CONH-V_0-Z_1$, $-NHCO-V_0-Z_1$, $-CO-V_0-Z_1$, $-SO_2-NH-V_0-Z_1$, $-V_0-Z_1$ or $-NHNHCOCH_2-Z_1$,
and the arylene group may be substituted by halogen, —OH, $-NO_2$, (1-4C)-alkyl or (1-4C)-alkoxy,
$V_0$ is an (1-8C) alkylene group, and the groups of formula

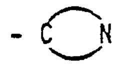 or 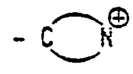

is an unsaturated heterocyclic ring and the group of formula

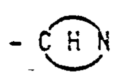 or 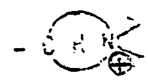

is a saturated heterocyclic ring,

each $Z_1$ independently is a group of formula

$$NR_{11}'R_{12}' \quad \text{or} \quad -\overset{\oplus}{N}R_{13}'R_{14}'R_{15}'\ A^{\ominus}$$

each $R_{11}'$ and $R_{12}'$ independently is hydrogen, linear or branched (1-6C)-alkyl, unbranched hydroxy (2-3C)-alkyl, benzyl, 2-cyanoethyl or 2-chloroethyl,

each $R_{13}'$ and $R_{14}'$ independently is linear or branched (1-6C)-alkyl, unbranched hydroxy (2-3C)-alkyl, benzyl, 2-cyanoethyl or 2-chloroethyl,

$R_{15}'$ is methyl, ethyl, propyl or benzyl,

the group $R_5'$ and $R_6'$ or $R_{11}'$ and $R_{12}'$ respectively may form together with the N-atom to which they are attached a morpholine-, piperidine-, pyrrolidine-, piperazine- or N-methylpiperazine ring or the groups $R_{13}'$, $R_{14}'$ and $R_{15}'$ form together with the N-atom to which they are attached a pyridine-, picoline- or lutidinering or a ring of formula

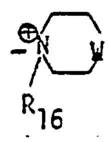 $A^{\ominus}$

wherein $R_{16}$ is methyl or ethyl and W is $-CH_2-$, $-O-$, $-S-$, $-SO_2-$, $-SO-$,

$$-NH, \quad -N-R_{16} \quad \text{or} \quad -\overset{\oplus}{N}(R_{16})_2\ A^{\ominus}$$

wherein

each $F_0'$ independently is hydrogen, halogen, $-NO_2$, $-NH_2$, (1-4C)-alkyl, (1-4C)-alkoxy, $-CN$, trifluoromethyl, phenyl, anilino, benzoyl, carbamoyl, phenoxy, halogenphenoxy, dihalogenphenoxy, (1-4C)-alkylsulfonyl, phenylsulfonyl, (1-4C)-alkylsulfonylamino, di-N(1-4C)-alkylaminosulfonyl or (1-4C)-alkylsulfonyl,

X, when the rings $D_2$ and $D_3$ are unsubstitutued, is $X_1$ the direct bound,

$X_2$ a straight or branched alkylene group with 1 to 4 carbon atoms,

$$X_3\ -CO-,\ X_4\ -NH-\overset{S}{\overset{\|}{C}}-NH-,\ X_5\ -S-,\ X_6\ -O-,$$

$$X_7\ -CH=CH-,\ X_8\ -S-S-,\ X_9\ -SO_2-,$$

$$X_{10}\ -NH-,\ X_{11}\ -NH-CO-,\ X_{12}\ -\underset{CH_3}{\underset{|}{N}}-CO-,$$

$X_{13}$ 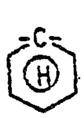 , $X_{14}$ 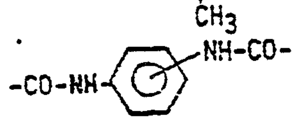 ,

$X_{15}$ 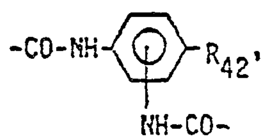 , $X_{16}$ 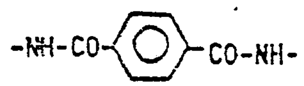 ,

(Continued)

$X_{17}$ $-SO_2-NH-$, $X_{18}$ 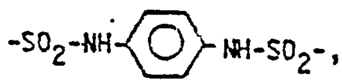

$X_{19}$ $-\underset{R_{44}}{N}-CO-R_{43}-CO-\underset{R_{44}}{N}-$, $X_{20}$ $-\underset{R_{44}}{N}-CO-CH=CH-CO-\underset{R_{44}}{N}-$,

$X_{21}$ $-\underset{R_{44}}{N}-CO-\underset{R_{44}}{N}-$, $X_{22}$ $-CO-NH-NH-CO-$,

$X_{23}$ $-CH_2CO-NH-NH-CO-CH_2-$, $X_{24}$ $-CH=CH-CO-NH-NH-CO-CH=CH-$,

$X_{25}$ 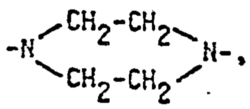 , $X_{26}$ 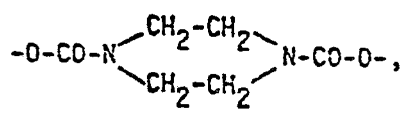 ,

$X_{27}$ 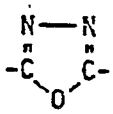 , $X_{28}$ $-O-\underset{O}{C}-O-$, $X_{29}$ $-\underset{O}{C}-O-$, $X_{30}$ $-\underset{O}{C}-\underset{O}{C}-$,

$X_{31}$ $-O-(CH_2)_g-O-$,

$X_{32}$ 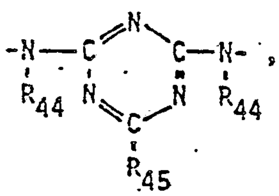 , $X_{33}$ 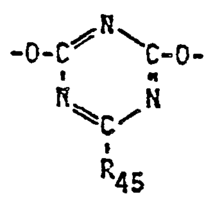 ,

$X_{34}$ $-CO-\underset{R_{44}}{N}-R_{43}-\underset{R_{44}}{N}-CO-$, $X_{35}$ $-CO-\underset{R_{44}}{N}-(CH_2)_g-O-(CH_2)_g-\underset{R_{44}}{N}-CO-$,

$X_{36}$ $-CO-\underset{R_{44}}{N}-(CH_2)_g-\underset{R_{44}}{N}-(CH_2)_g-\underset{R_{44}}{N}-CO-$,

$X_{37}$ $-CO-\underset{R_{44}}{N}-(CH_2)_g-O-(CH_2)_g-O-(CH_2)_g-\underset{R_{44}}{N}-CO-$,

$R_{38}$ $-CH_2-CO-\underset{R_{44}}{N}-$, $R_{39}$ $-CH=CH-CO-\underset{R_{44}}{N}-$,

(Continued)

$X_{40}$ $-N{=}N-$, $X_{41}$ $-CH_2-S-CH_2-$, $X_{42}$ $-SO-$,
$X_{43}$ $-CH_2-SO-CH_2-$,
$X_{44}$ $-CH_2-SO_2-CH_2-$, $X_{45}$ $-CH_2-NH-CO-NH-CH_2-$,
$X_{46}$ $-CH_2-NH-CS-NH-CH_2-$,

$X_{47}$ 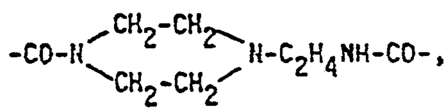

$X_{48}$ 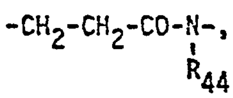 $X_{49}$ $-CH_2-CO-CH_2-$,

$X_{50}$ $-CH{=}CH-CH{=}CH-$, $X_{51}$ 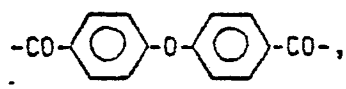

$X_{52}$ 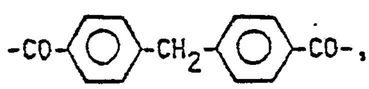 $X_{53}$ 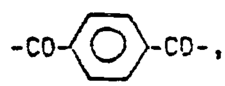

$X_{54}$ 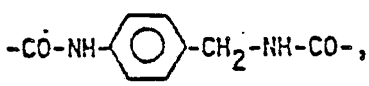

$X_{55}$ 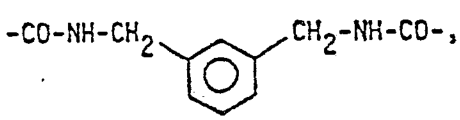

$X_{56}$ 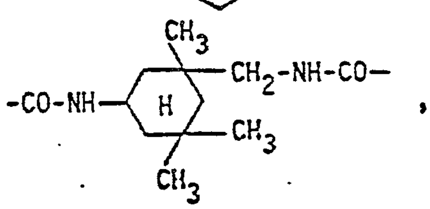

$X_{57}$ 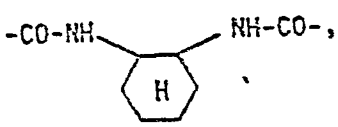 $X_{58}$ $-CH_2-CO-$,

$X_{59}$ $-CH{=}CH-CO-CH{=}CH-$, $X_{60}$ 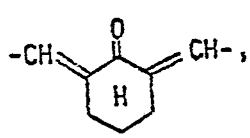

$X_{61}$ 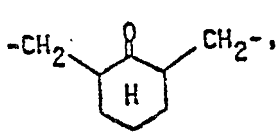 $X_{62}$ $-O-CH_2-CH_2-O-$,

(Continued)

$X_{63}$ $-O-CH_2-O-$, $X_{64}$ $-CO-NH-R_{43}-CO-NH-$,
$X_{100}$ $-CO-NH-R_{43}-CO-NH-R_{43}-NH-CO-$,
$X_{101}$ $-CO-NH-R_{43}-NH-CO-CH_2-CH_2-CO-NH-R_{43}-NH-CO-$,
$X_{102}$ $-CO-NH-R_{43}-NH-CO-CH=CH-CO-NH-R_{43}-NH-CO-$,

$X_{103}$ 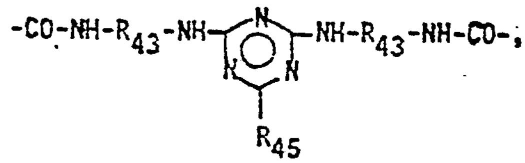

$X_{104}$ $-SO_2-NR_{44}-(CH_2)_g-NR_{44}-SO_2-$,
$X_{105}$ $-CO-NR_{44}-R_{43}-O-CO-$,

$X_{106}$ 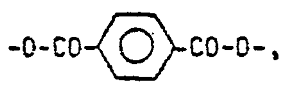

$X_{107}$ 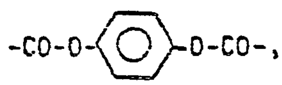

$X_{108}$ $-CONH-R_{43}-NH-CO-NH-R_{43}-NH-CO-$,

each $R_{42}$ independently is halogen, (1-4C)-alkyl or (1-4C)-alkoxy,
each $R_{43}$ independently is a straight or branched (1-4C)-alkylene group,
each $R_{44}$ independently is hydrogen or (1-4C)-alkyl,
each $R_{45}$ independently is halogen,

$-NH-CH_2-CH_2-OH$,
$-N(CH_2-CH_2-OH)_2$, $NH_2$, OH, $NH-(CH_2)_{2-3}N(C_2H_5)_2$,

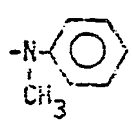 , 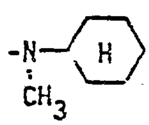 , 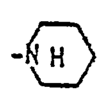 , $-OCH_3$, $OC_2H_5$,

each g independently is 1, 2, 3 or 4,
X when the rings $D_2$ and/or $D_3$ are substituted, especially in ortho-position to both azo-bridges, stands for the direct bond or is $X_2$, $X_{14}$, $X_{21}$, $X_{32}$, $X_{34}$ or $X_{40}$,
each $R_s$ and $R_t$ independently is hydrogen, (1-4C)-alkyl or (1-4C)-alkoxy,
$R_{160}$ is a group of formula $-(CH_2)_v-Z_1$,

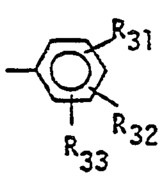 , 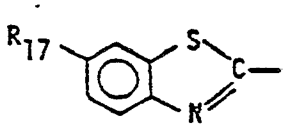 ,

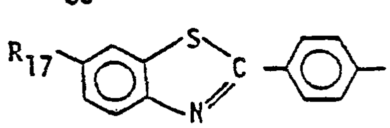 , 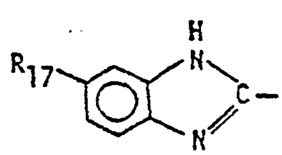 ,

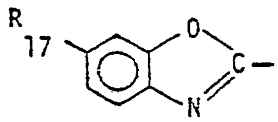,
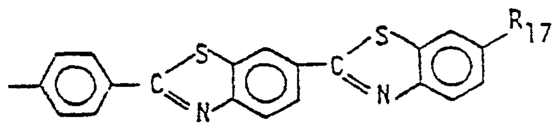

or 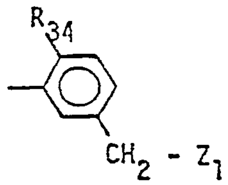

each $R_{17}$ independently is hydrogen or methyl,
each $R_{18}$ and $R_{19}$ independently is hydrogen, halogen, (1-4C)-alkyl or (1-4C)-alkoxy,
$R_{20}$ is hydrogen, methyl, ethyl, phenyl, benzyl or cyclohexyl,
$R_{21}$ is one of a group of $T_1$ or

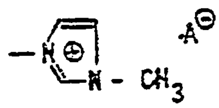

$R_{22}$ is a group of formula $-R_1'-NH-R_0'$, hydrogen, an (1-4C)-alkyl group, optionally substituted by phenyl, $-(CH_2)-CN$, $-(CH_2)_v-Z_1$, $-(CH_2)_2\ OH$, $-(CH_2)_2OCH_3$ or

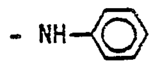

$R_{23}$ is (1-4C)-alkyl or phenyl,
each $R_{24}$ independently is hydrogen, (1-4C)-alkyl, $-(CH_2)_{2\text{-}3}-OCH_3$, $-(CH_2)_2-OH$, $-(CH_2)_{2\text{-}3}-N(CH_3)_2$ or

$$-(CH_2)_{2\text{-}3}-\overset{\oplus}{N}(CH_3)_3\ A^{\ominus}$$

$R_{25}$ is OH or $NH_2$,
$R_{26}$ is (1-4C)-alkyl or $-COR_{35}$,
$R_{27}$ is hydrogen,

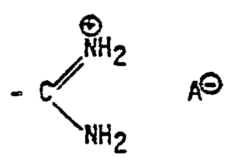

or 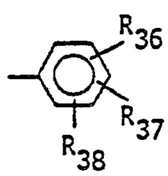,

each $R_{28}$ in one of the positions 3, 4, 5, 6 or 7 independently is hydrogen,

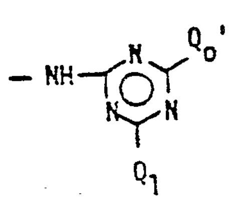, $-NHCO(CH_2)_{1\text{-}3}-Z_1$,

$-SO_2-NH-V_0-Z_1$, $-CONH-V_0-Z_1$, $-CONHNH_2$, $-NH-V_0-Z_1$, $-CH_2-Z_1$, $-NHNHCOCH_2-Z_1$ or

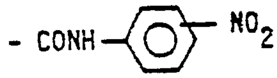

$R_{29}$ (1-4C)-Alkyl,

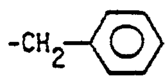

or $-(CH_2)_2-CN$,

$R_{30}$ is (1-4C)-alkyl or $-(CH_2)_v-Z_1$,

each v independently is 1, 2, 3, 4, 5 or 6,

each $R_{31}$ and $R_{32}$ independently is hydrogen, halogen, (1-4C)-alkyl, (1-4C)-alkoxy, $NO_2$ or CN,

$R_{33}$ is hydrogen,

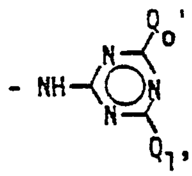

$-NHCO-(CH_2)_{2\text{-}3}-Z_1$, $-SO_2-NH-(CH_2)_{2\text{-}3}-Z_1$ or $-CONH-V_0-Z_1$,

$R_{34}$ is (1-4C)-alkoxy,

$R_{35}$ is $-O-R_{39}$, $-NR_{40}R_{41}$ or $-NH-V_0-Z_1$,

each $R_{36}$ and $R_{37}$ independently is hydrogen, halogen, (1-4C) alkyl, (1-4C)-alkoxy, $-NO_2$ or $-NH_2$,

$R_{38}$ is hydrogen, $-CONH-V_0-Z_1$, $-SO_2NH-V_0-Z_1$, $-NHCO(CH_2)_{2\text{-}3}-Z_1$ or

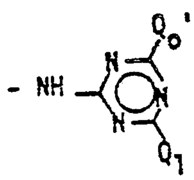

$R_{39}$ is (1-4C)-alkyl,

each $R_{40}$ and $R_{41}$ independently is hydrogen or (1-4C)-alkyl,

$R_{21a}$ is hydrogen, OH, halogen, (1-4C)-alkyl, (1-4C)-alkoxy, $-CN$, $-CONH_2$, $-NHCO$(1-4C)-alkyl, $-NHCONH_2$, $-COOH$ or $-SO_3H$,

$R_{22a}$ is hydrogen, halogen, (1-4C)-alkyl or (1-4C)-alkoxy whereby the groups $R_{21a}$ and $R_{22a}$ are standing para to one another,

each $R_{23a}$ independently is hydrogen, OH, halogen, $-CN$, (1-4C)-alkyl, (1-4C)-alkoxy, $-COOH$ or $-SO_3H$,

U is a direct bond, $-(CH_2)_{1\text{-}3}$, $-C-$, $-S-$, $-SO_2-$, $-NHCO-$, $NHCONH-$, $NHCO(CH_2)_{2\text{-}3}$ $-CONH(CH_2)_{2\text{-}3}-NHCO-$, $-O(CH_2)_2)_{2\text{-}3}-O-$, $-N=N-$, $-CH=CH-CO-CH=CH-$,

t is 0 or 1,

$R_{2a}'$ is hydrogen, OH or $-NH_2$,

$R_{1a}$ is hydrogen,

$$- \overset{\oplus}{N}R_5' - Q_{10}' - NR_{11}'R_{12}',$$

$$- NR_5' - Q_{10}' - \overset{\oplus}{N}R_{13}'R_{14}'R_{15}' \; A^{\ominus},$$

$- NR_5'COCH_2NR_{11}'R_{12}'$, $- NR_5'$ 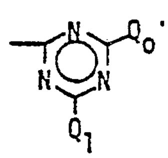 ,

$- NR_5' - COCH_2\overset{\oplus}{N}R_{13}'R_{14}'R_{15}'\ A^{\ominus}$ or

$- CO - Q_{10}' - \overset{\oplus}{N}(CH_3)_2 - (CH_2)_{1-4} - Z_1\ A^{\ominus}$,

$Q_{10}'$ $-(CH_2)_{2-6}-$, $- CH_2 - \underset{CH_3}{CH} -$ or $- CH_2 - \underset{CH_3}{CH} - CH_2 -$,

$W_0'$ $- NH -$, $- NHCONH -$, $- NHCO - CH = CH - CO - NH -$,

$- NHCO(CH_2)_{2-3} - CONH -$, $- NHCO$ 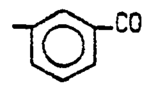 $CO - NH -$,

$- NH - CO$ 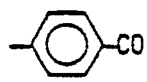 $CO - NH -$ or 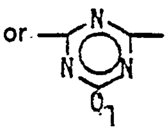

$K_1'$ is one of the groups of the formulae aa), $aa_1$), $aa_2$), $aa_3$), $aa_4$), $aa_5$), $aa_6$), $aa_7$), $aa_8$), $aa_9$), $aa_{10}$), $aa_{11}$), $aa_{12}$) or

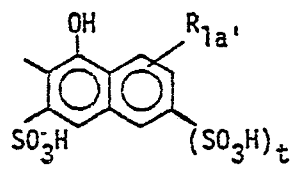

$aa_{13}$) or

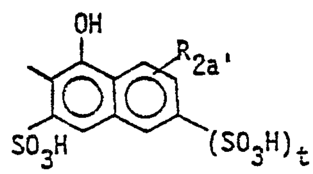

$aa_{14}$)

and KK is one of the groups of formulae $aa_1$), $aa_2$), $aa_3$), $aa_4$), $aa_5$), $aa_6$), $aa_7$), $aa_8$), $aa_9$), $aa_{10}$), $aa_{11}$), $aa_{12}$), $aa_{13}$) or $aa_{14}$),
mixtures of the compounds of formula II, their acid addition salts or salts of acids as well as 1 : 1- or 1 : 2-metal complexes thereof with the provisos,

α) the monoazo compounds b' of formula II have one and preferably two and, when the dis- and polyazo compounds of formula II are free from sulfonic acid groups [formulae $c_1$), $c_2$), $d_3$), $d_4$)], at least two water solubilizing cationic groups or at least two protonatable basic groups or at least one cationic and at least one protonatable groups, and when the compounds of formula II contain sulfonic acid groups, the number of the cationic and of the protonatable basic groups is at least one greater than the number of the sulfonic acid groups and probably additional —COOH groups and

$\beta_1$) when r is the number 2, S' is hydrogen, $R_0'$ is hydrogen, $R_1'$ is different from alkylene, $T_1$ is different from CN and the group M' is free from sulfonic acid groups.

3. Basic compounds according to Claim 1 in one of the possible tautomeric form of formula

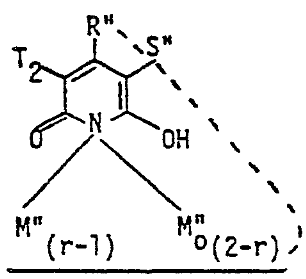

(III)

$B_2$

wherein

R″ is methyl or phenyl,

each $T_2$ independently is

- CN or i1″) 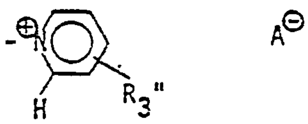

each M″ independently is $-R_1''-NH-R_0''$,

each $M_0''$ independently is $-R_1''-NH-R_0''$, hydrogen $CH_3$, $-C_2H_5$, benzyl, $-(CH_2)_{2\text{-}3}-NR_{13}''R_{14}''$ or $-(CH_2)_{2\text{-}3}-N^{\oplus}R_{13}''R_{14}''R_{15}''\ A^{\ominus}$,

S″, when r is the number 2,

a) is hydrogen

b″) is a group of formula

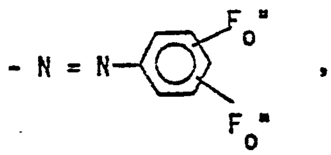

,

c1′) 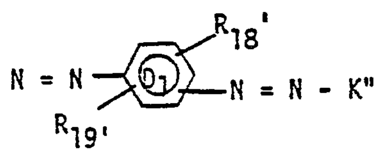

c2′) 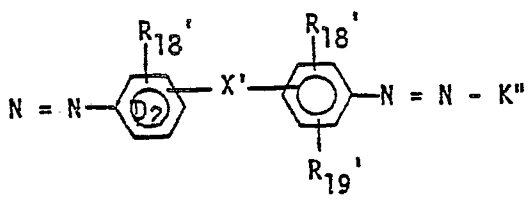

or S″, when r is the number 1,

d1′) is a group of formula

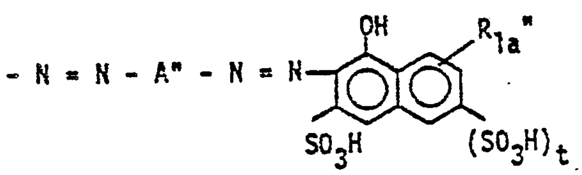

d2′) 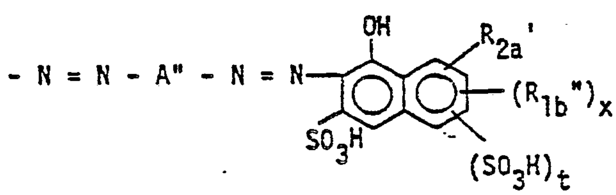

d3′) 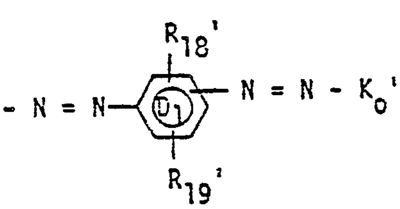

d4')
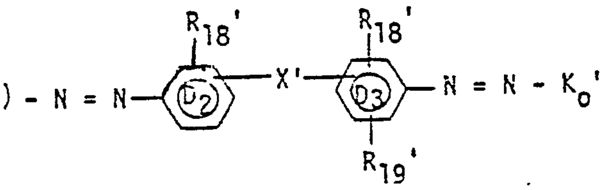

wherein

$K_0'$ is a group of formula

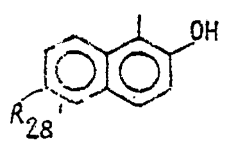

aa')

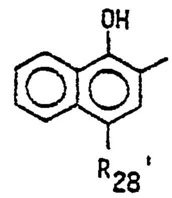

($aa_1'$)

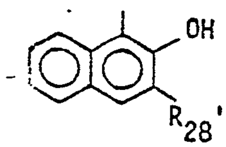

$aa_2'$)

each A" independently is a group of formula

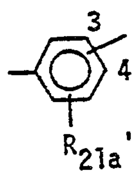

or

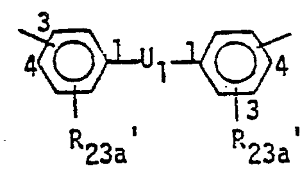

(mm)

(nm)

whereby the group (mm) is attached in positions 1,3 or 1,4 and the group (nm) is attached in positions 3,3 ; 3,4 ; or 4,4,

$R_{1b}''$, when x is 1 is a group of formula

e") —N = N—$K_1''$

f") —N = N—A"—N = N—$K_1''$

g")
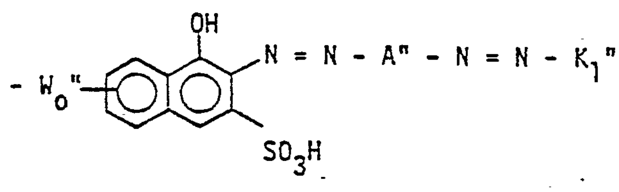

K" is a group of formula

$aa_4'$) $B_2$, wherein r is 1 or 2,

$aa_5$) is a phenol,

$aa_6'$) is
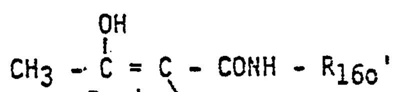

$aa_7'$) is
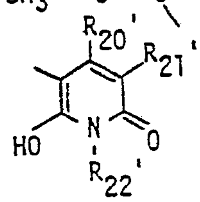

aa8') is 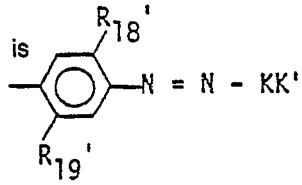

aa9') is 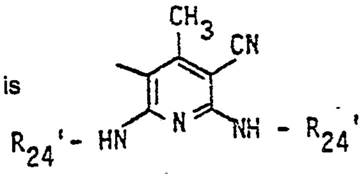

aa10') is 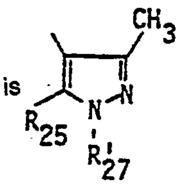

aa11') is 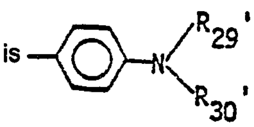

aa12') is 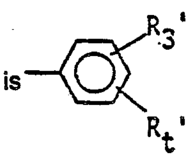

$R_1''$ is 1,2-ethylene, 1,3-propylene or meta- or para-phenylene,
$R_0''$ is a group of formula

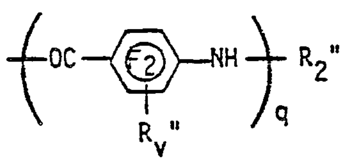 (Ic)

$R_v''$ is hydrogen, chloro or methyl,
each $R_2$ independently is a group of formula

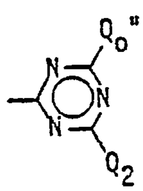 h1'') or

$- CO - CH_2 - Z_2$ h4'')

or hydrogen, wherein
$R_3''$ is hydrogen or methyl,
$Q_2$ is chloro, OH, $—NH_2$, mono (1-2C)-alkylamino, $OCH_3$, monohydroxy (2-4C)-alkylamino, dis-[hydroxy(2-4C)-alkyl] amino or $Q_0''$ and

$Q_0''$ is 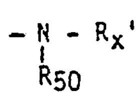

wherein

$R_x'$ is a group of formulae

$-(CH_2)_{2-3}-NR_{50}-(CH_2)_{2-3}-NR_{50}-C_2H_5$,

$-(CH_2)_{2-3}-\overset{\oplus}{N}(R_{13}'')_2-(CH_2)_{2-3}-\overset{\oplus}{N}(R_{13}'')_2-C_2H_5\ \ 2A^{\ominus}$

$-(CH_2)_{2-3}-NR_{50}-C_2H_5$,

$-(CH_2)_{2-3}-\overset{\oplus}{N}(R_{13}'')_2-C_2H_5\ \ A^{\ominus}$, $-NHCOCH_2-Z_2$,

$-CH_2-CO-NH-V'-Z_2$,

$-V'-Z_2$, 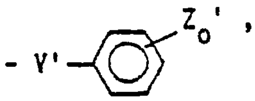 , 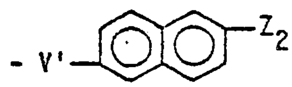 ,

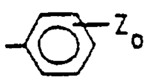 , 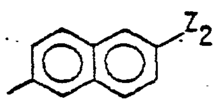 , 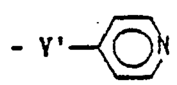 , 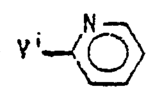

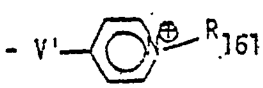 $A^{\ominus}$ 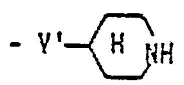 , 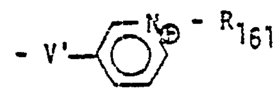 $A^{\ominus}$,

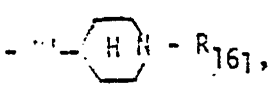 , 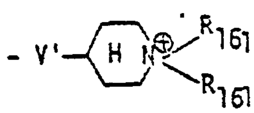 $A^{\ominus}$,

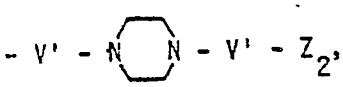 , 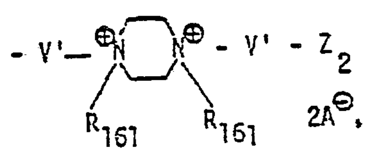 $2A^{\ominus}$,

or $R_x'$ and $R_{50}$ form together with the N-atom to which they are attached a group

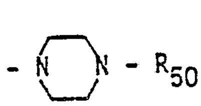 or 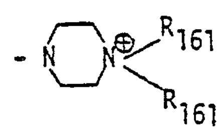 $A^{\ominus}$

$R_{161}$ is methyl or ethyl,

each V' independently is an (1-4C)-alkylene group,

$Z_0'$—$N(CH_3)_2$,

$-\overset{\oplus}{N}(CH_3)_3\ A^{\ominus}$,

or a group —CONH—$V_0'$—$Z_2$, —NH—CO—$V_0'$—$Z_2$, —CO—$V_0'$—$Z_2$, —$SO_2$NH—$V_0'$—$Z_2$, —$V_0'$—$Z_2$ or —NHNHCO$CH_2$—$Z_2$ and

$V_0'$ is an (1-4C)-alkylene group,

$R_{50}$ is hydrogen or methyl,

each $Z_2$ independently is a group —$NR_{11}''R_{12}''$ or

$-\overset{\oplus}{N}R_{13}''R_{14}''R_{15}''\ A^{\ominus}$,

each $R_{11}''$ and $R_{12}''$ independently is hydrogen, methyl or ethyl,

each $R_{13}''$ and $R_{14}''$ independently is methyl or ethyl ; or

$R_{15}''$ is methyl, ethyl or benzyl, and

the groups $R_{11}''$ and $R_{12}''$ form together with the N-atom to which they are attached a morpholine-,

piperazine- or N-methylpiperazin ring ; or the groups $R_{13}''$, $R_{14}''$ and $R_{15}''$ form together with the N-atom to which they are attached a pyridine or an α- or β-picoline ring or a N-methylmorpholine-, N-methylpiperidine-, N-methylpiperazine- or N,N'-dimethylpiperazine ring,
wherein each

$F_0''$ independently is hydrogen, $NO_2$, chloro, methyl, methoxy or chlorphenoxy,

X' when the rings $D_2$ and $D_3$ are unsubstituted stands for $X_1$, $X_5$, $X_5$, $X_7$, $X_{10}$, $X_{11}$, $X_{12}$, $X_{16}$, $X_{17}$, $X_{22}$, $X_{25}$, $X_{26}$, $X_{27}$, $X_{30}$, $X_{31}$, $X_{49}$, $X_{50}$, $X_{51}$, $X_{52}$, $X_{53}$, $X_{54}$, $X_{58}$, $X_{59}$, $X_{62}$, $X_{63}$, $X_{54}$, $X_{101}$ ($R_{43}$ = $-(CH_2)_{1\text{-}2}$, $X_{103}$ ($R_{43}$ = $-(CH_2)_{1\text{-}2}$ and $R_{45}$ = $-NH-(CH_2)_{2\text{-}3}-N(C_2H_5)_2$, $X_{104}$ ($R_{44}$ = H, g = 2 or 3), $X_{108}$ ($R_{43}$ = $-(CH_2)_{1\text{-}3}$),

$X_{70}$ $-NH-CO-NH-$, $X_{71}$ 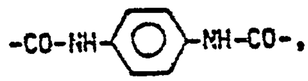

$X_{72}$ 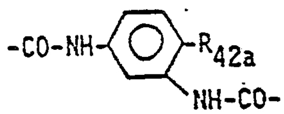

$X_{73}$ $-NH-CO-CH_2-CH_2-CO-NH-$,

$X_{74}$ $-NH-CO-CH=CH-CO-NH-$,

$X_{75}$ $-NH-CO-(CH_2)_4-CO-NH-$,

$X_{76}$ 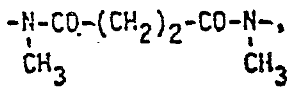

$X_{77}$ 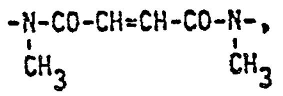

$X_{78}$ 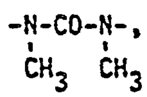

$X_{79}$ 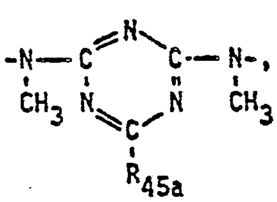 $X_{80}$ 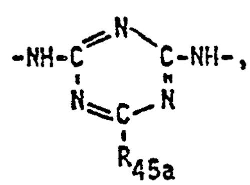

$X_{81}$ $-CH_2-$, $X_{82}$ $-(CH_2)_2-$, $X_{83}$ $-(CH_2)_3-$,

$X_{84}$ $-(CH_2)_4-$, $X_{85}$ $-CO-NH-(CH_2)_2-NH-CO-$,

$X_{86}$ $-CO-NH-(CH_2)_3-NH-CO-$, $X_{87}$ $-CO-NH-(CH_2)_4-NH-CO-$,

$X_{88}$ 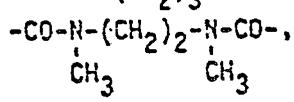 $X_{89}$ 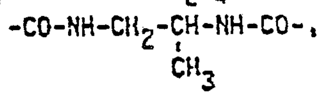

$X_{90}$ 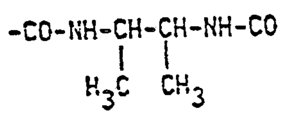

each $R_{42a}$ independently is hydrogen, $-Cl$, $-CH_3$ or $-OCH_3$ and
each $R_{45a}$ independently is

$-Cl$, $-NH-CH_2-CH_2-OH$, $OCH_3$,
$OH$, $NH_2$, $-N(CH_2-CH_2OH)_2$, $-\underset{H}{N}-(CH_2)_3N(C_2H_5)_2$, $OC_2H_5$,

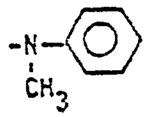 , 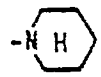 or 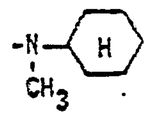 .

or
X, when the rings $D_2$ and $D_3$ are substituted, especially in ortho-position to both azo-bridges by chloro, methyl or methoxy, X stands for the direct binding $X_1$, $X_{40}$, $X_{70}$, $X_{71}$, $X_{80}$, $X_{81}$, $X_{82}$ or $X_{85}$,
each $R_s'$ and $R_t'$ independently is hydrogen, methyl methoxy, ethyl, ethoxy or butyl,
$R_{160}'$ is a group of $-(CH_2)_{v'}-Z_2$,

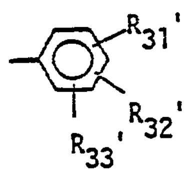 , 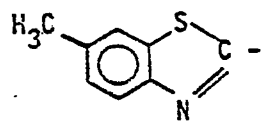 ,

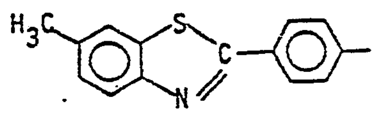 oder 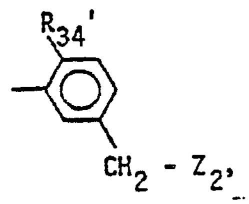

v' is 2, 3 or 4,
each $R_{18}'$ and $R_{19}'$ independently is hydrogen, chloro, $CH_3$, $C_2H_5$, $OCH_3$ or $OC_2H_5$,
$R_{20}'$ is methyl or phenyl,
$R_{21}'$ is one of the group $T_2$,
$R_{22}'$ is a group $-R_1''-NH-R_0''$, hydrogen, methyl, ethyl, benzyl, phenylethyl,

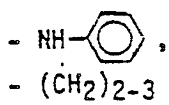 , $-(CH_2)_2-OH$, $-(CH_2)_3OCH_3$ $-(CH_2)_2CN$,
$-(CH_2)_{2-3}-N(CH_3)_2$, $-(CH_2)_{2-3}-\overset{\oplus}{N}(CH_3)_3\ A^{\ominus}$ or
$-(CH_2)_{2-3}-\overset{\oplus}{N}(CH_3)_2\ A^{\ominus}$,
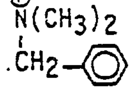

each $R_{24}'$ independently is hydrogen,

$(CH_2)_2OH$,
$-(CH_2)_3-OCH_3$, $-(CH_2)_{2-3}-N(CH_3)_2$ or
$-(CH_2)_{2-3}-\overset{\oplus}{N}(CH_3)_3\ A^{\ominus}$.

$R_{27}'$ is hydrogen, 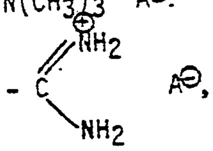

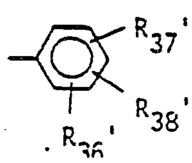

each $R_{28}'$ independently is

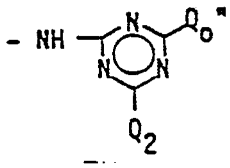 or $-NHCOCH_2-Z_2$.

$-CONH(CH_2)_{2-3}-Z_2$ or 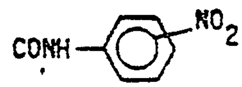

$R_{29}'$ is $CH_3$, $C_2H_5$, $C_2H_4CN$ or benzyl,
$R_{30}'$ $CH_3$, $C_2H_5$ or $-(CH_2)_{v}'-Z_2$,
each $R_{31}'$ and $R_{32}'$ independently is hydrogen, chloro, bromo, methyl, ethyl, methoxy, ethoxy, $NO_2$ or CN,
$R_{33}'$ is hydrogen,

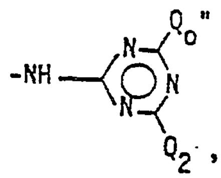 ,

$-NH-CO(CH_2)_{2-3}-Z_2$ or $-SO_2-NH(CH_2)_{2-3}-Z_2$,
$R_{34}'$ is methoxy or ethoxy,
each $R_{36}'$ and $R_{37}'$ independently is hydrogen, chloro, methyl or methoxy,
$R_{38}'$ is hydrogen,

$-CO-NH-(CH_2)_{v}'-Z_2$,

$-NHCO(CH_2)_{2-3}-Z_2$ or 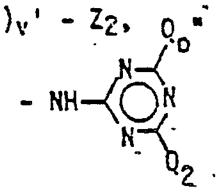

$R_{21a}'$ is H, Cl, $CH_3$, OH, $OCH_3$, $C_2H_5$ or $OC_2H_5$,
each $R_{23a}'$ independently is H, Cl, $CH_3$, OH or $OCH_3$,
$U_1$ is a direct bond, $-NHCO-$ or $-NHCONH-$,
$R_{1a}''$ is hydrogen,

$-NR_{50}-(CH_2)_{2-3}-NR_{11}''R_{12}''$,
$-NR_{50}-(CH_2)_{2-3}-\overset{\oplus}{N}R_{13}''R_{14}''R_{15}''\ A^{\ominus}$ or

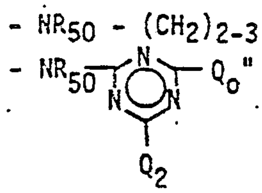

$W_0''$ is $-NH-$, $-NH-CO-CH=CH-CONH-$ or $-NHCO(CH_2)_2CONH-$,
$K_1''$ is a group of formulae aa'), $aa_1'$), $aa_2'$), $aa_3'$), $aa_4'$), $aa_5'$), $aa_6'$), $aa_7'$), $aa_8'$), $aa_9'$), $aa_{10}'$), $aa_{11}'$), $aa_{12}'$) or a group of formula

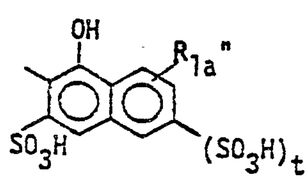 $aa_{13}'$) or

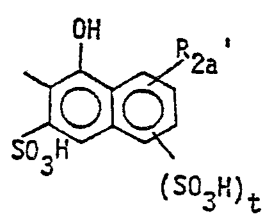 $aa_{14}'$)

and KK' is a group of formulae aa'), $aa_1'$), $aa_2'$), $aa_4'$), $aa_5'$), $aa_6'$), $aa_7'$), $aa_8'$), $aa_9'$), $aa_{10}'$), $aa_{11}'$), $aa_{12}'$), $aa_{13}'$) $aa_{14}'$),
mixtures of the compounds of formula III, their acid addition salts or salts of acids as well as 1 : 1- or 1 : 2-metal-complexes thereof with the conditions mentioned before and with the proviso,

$\beta_2$) when r is the number 2, then S" is hydrogen, $R_0$" is hydrogen, $R_1$" is different from 1, 2-ethylene, $T_1$ is different from CN and the group M" is free from sulfonic acid.

4. Metal complex compounds according to claim 1 of the formula

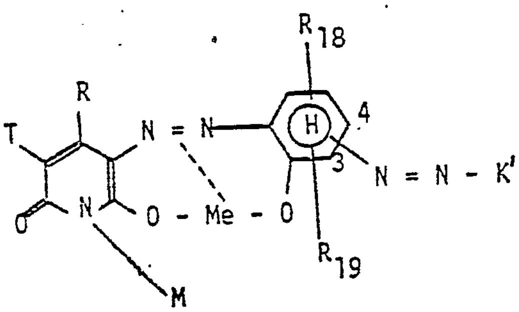

(V)

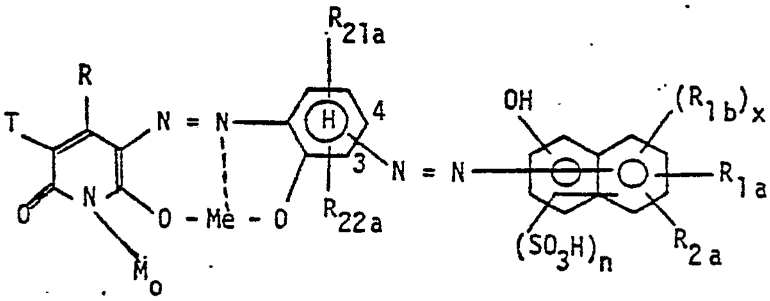

(VI)

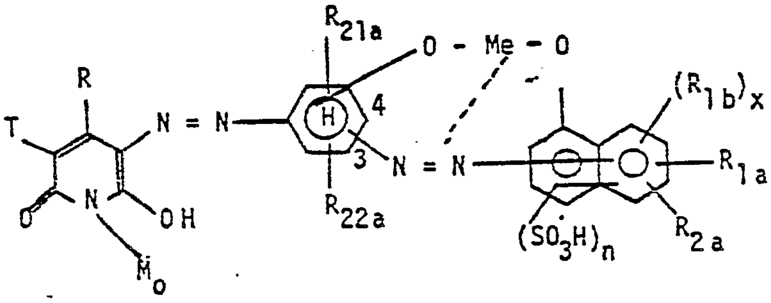

(VII)

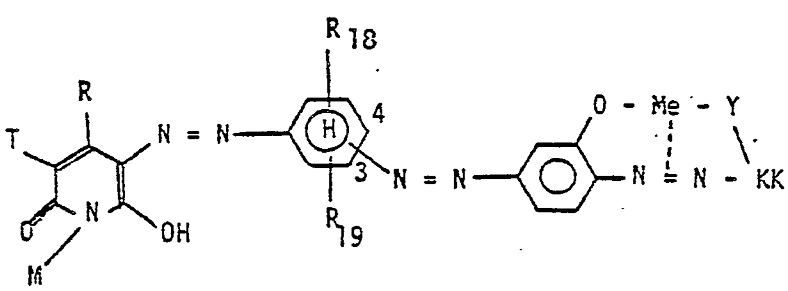

(VIII)

wherein Y is —O— or —NH—,

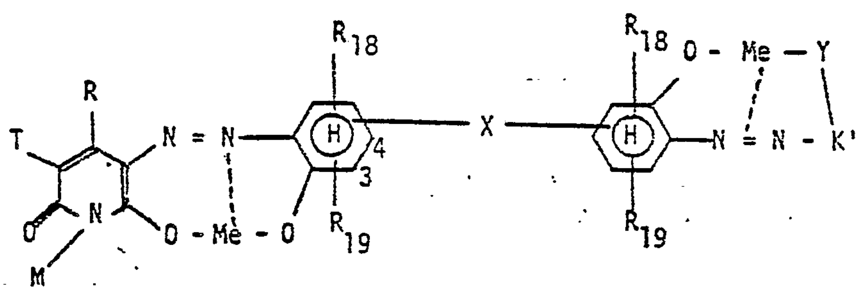

(IX)

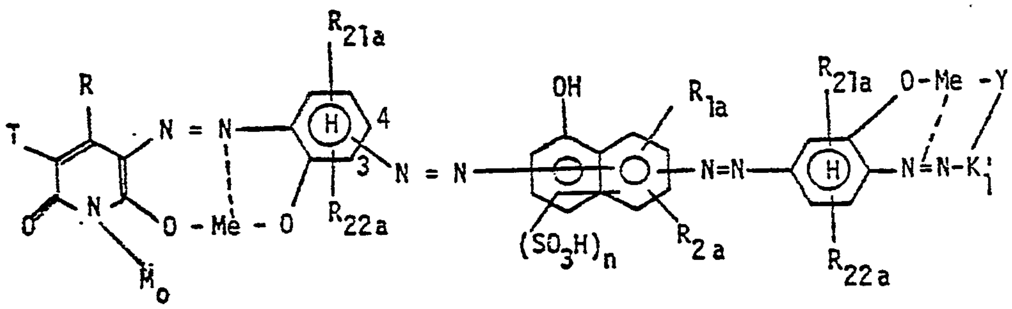

(X)

whereby the azo groups in the rings H are bound in the 3- or 4-position and

Me is a copper, chromium, cobalt, nickel or manganese atom forming 1 : 1-complexes, preferable a copper atom and

Me a chromium, cobalt or iron atom, preferably an iron atom forming 1 : 2-metal complexes.

5. A process for preparing a compound of formula I according to Claim 1, wherein S is different from hydrogen, comprising coupling the diazo compound of an amine of formula

$$DD—NH_2, \qquad XI,$$

wherein DD is a group of formula

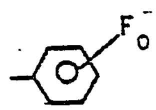

XIa)

—A—N = N—K XI b)

or

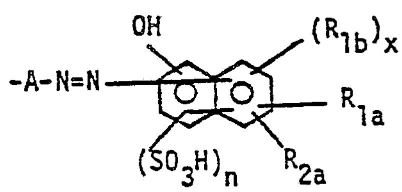

XI c)

with a compound of formula

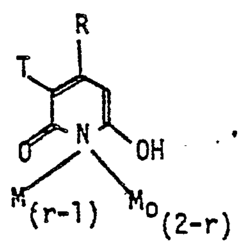

(XII)

and if required converting the resulting compounds into the corresponding 1 : 1 or 1 : 2 metal complex form.

6. A process for preparing a compound of formula I according to claim 1, wherein S is hydrogen, comprising cyclisizing a compound of formula

$$T—CH_2—CO—NH—R_1—NH—M_{00} \qquad XXVI,$$

wherein $M_{00}$ is any blocking group, especially a compound of formula

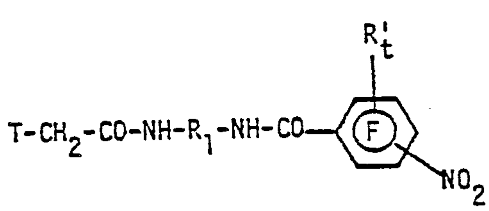

XXVII

wherein $R_t'$ is hydrogen, halogen, (1-4C)-alkyl or (1-4C)-alkoxy with an alkylester of acetic acid of formula

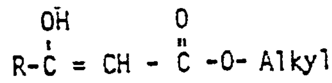

XXVIII

by known methods to a compound of formula

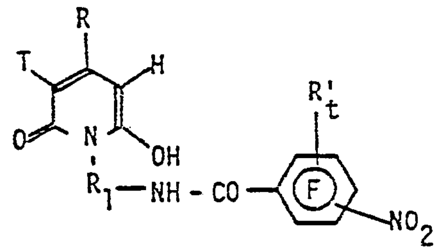

XXIX

and for compounds, wherein $R_0$ is hydrogen, this compound is saponified or for compounds, wherein $R_{00}$ is a group of formula

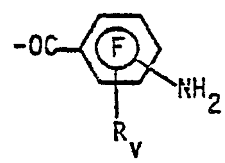

XXa

the nitro group is reduced to an amino group.

7. A process for dyeing or printing paper and leather, comprising applying to that substrate a compound of formula I, wherein S is different from hydrogen.

8. A process for dyeing, padding or printing naturale or regenerated cellulose materials, hemp, flax, sisal, jute, coir or strew comprising applying to that substrate a compound of formula I, when S is different from hydrogen.

9. The dyed and printed material according to Claim 7.

10. The dyed, padded or printed material according to Claim 8.

**Claims** (for the Contracting State AT)

1. A process for preparing a basic compound or a basic azo compound corresponding to one of the possible tautomeric forms of the formula

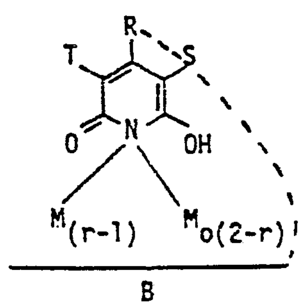

(I)

wherein

each R independently is hydrogen, a (1-4C)-alkyl, (5-6C)-cycloalkyl-, or phenyl group ; benzyl or phenylethyl,

each T independently is hydrogen,

i) CN

$i_1$) 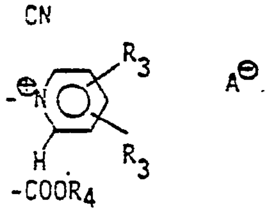

$i_2$) $-COOR_4$

i3) $-CONR_5R_6$

i4) $-SO_2NR_5R_6$

i5) 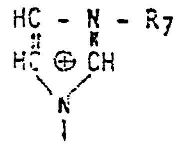 $A^{\ominus}$ · or

i6) 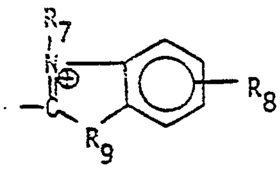 $A^{\ominus}$

r is 1 oder 2,

each M independently is a group $—R_1—NH—R_0$,

each $M_0$ independently is a group $R_1—NH—R_0$, hydrogen, $—NR_{11}R_{12}$, (1-4C)-alkyl, hydroxy(2-4C)-alkyl, (1-4C)-alkoxy-(1-4C)-alkyl, (5-6C)-cycloalkyl optionally substituted by 1 to 3 (1-4C)-alkyl radicals, phenyl(1-3C)-alkyl, $—V_1—NR_{13}R_{14}$ or $—V_2—$

$$\overset{\oplus}{N}R_{13}R_{14}R_{15}A^{\ominus},$$

$A^{\ominus}$ is an anion,

S, when r stands for the number 2, is

a) hydrogen,

b) a group of formula

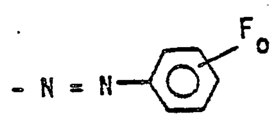 or

c) $—N = N—A—N = N—K$,

and $F_0$ is a substituent conventionally used in azo chemistry, preferably halogen, $NO_2$, $NH_2$, alkyl, alkoxy, CN, trifluoroalkyl, phenyl, anilino, benzoyl, carbamoyl, phenoxy, halogenphenoxy, dihalogenphenoxy, alkylsulfonyl, phenylsulfonyl, alkylsulfonylamino, di-N-alkylaminosulfonyl or alkylsulfonyl, or S, when r stands for the number 1, is

d) a group of formula

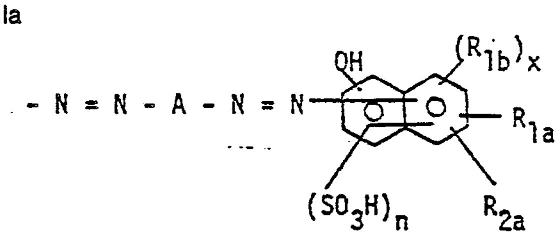

wherein

n is 0, 1 or 2,

x is 0 or 1,

each A independently is the residue of a tetrazo component,

K is the residue of any diazo- or coupling component, preferably free from sulfonic acid groups, of the pyrazolone-5, 5-aminopyrazole, aniline, phenol, acetoacetylalkyl- or arylamide, barbituric acid, dimedone, dimedonecarbonic acidester, pyridone series, e. g. the group B, wherein r is 1 or 2, or 2,6-diaminopyridine,

$R_{1b}$, when x is 1, is a group of the formula

e) $—N = N—K_1$,

f) $—N = N—A_1—N = N—K_1$,

g) 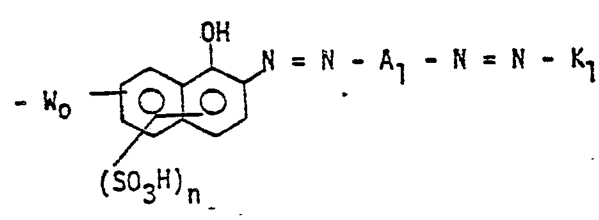

$K_1$ has one of the significances of the group K or is an α- or β-naphthol, an α- or β-aminonaphthalene or an aminonaphthol,

each $R_1$ independently is an alkylene- or alkenylene group, which may be interrupted by 1 or 2 heteroatoms or a phenylene- or cyclohexylene group,

$R_0$ is a group of formula

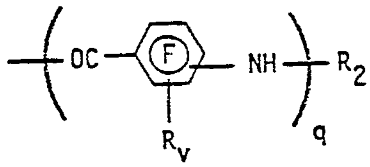

(Ia)

q is 0 or 1 and

$R_v$ is hydrogen, halogen, nitro, (1-4C)-alkyl or (1-4C)-alkoxy,

$R_2$ is a group of the formula

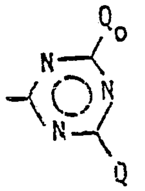

$h_1$)

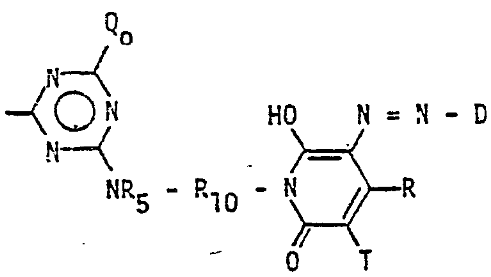

$h_3$) or

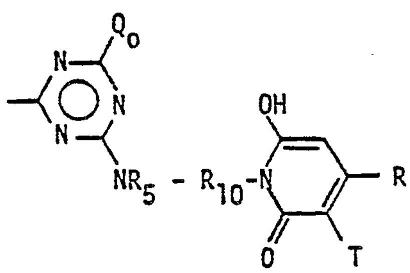

$h_2$)

$-CO-(CH_2)_{1-3}-Z$ $h_4$)

or hydrogen, wherein

each D independently is the residue of a diazo compound, which may be substituted by substituents conventionally used in the azo chemistry, preferably a group of formula

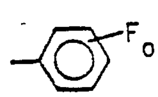

$-A-N=N-K$ or $-A-N=N-$ 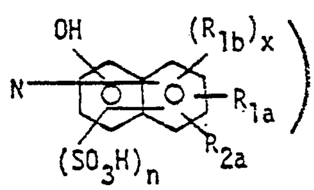

each $R_3$ independently is hydrogen, (1-4C)-alkyl, $-NR_5R_6$ or $-CONR_5R_6$,

each $R_4$ independently is (1-6C)-alkyl or phenyl-(1-3C)-alkyl,

each $R_5$ and $R_6$ independently is hydrogen or (1-4C)-alkyl,

each $R_7$ independently is (1-4C)-alkyl,

each $R_8$ independently is hydrogen or (1-4C)-alkyl,

each $R_9$ independently is —S—, —O— or

$$-\overset{|}{N}-R_5,$$

each $R_{10}$ independently is an (2-6C)-alkylene group or

$$-\overset{*}{N}HCOCH_2-$$

whereby the atom marked * is bound to the —$NR_5$— group,

$Q_0$ is an aliphatic, cycloaliphatic, aromatic or heterocyclic amino group, the N-atom of which is bound at the C-atom of the triazinyl radical,

Q is halogen, OH, —$NH_2$ (1-4C)-alkoxy, phenyl or $Q_0$,

each Z independently is a group of formula —$NR_{11}R_{12}$ or

$$-\overset{\oplus}{N}R_{13}R_{14}R_{15} \quad A^{\ominus}$$

each $R_{11}$ and $R_{12}$ independently are hydrogen, unsubstituted (1-6C)-alkyl, (2-6C)-alkyl substituted by OH, CN or halogen, phenyl-(1-3C)-alkyl, the phenyl group of which may be substituted by chloro, (1-4C)-alkyl and (1-4C)-alkoxy, unsubstituted (5-6C)-cycloalkyl or (5-6C)-cycloalkyl substituted by 1 to 3 (1-3C)-alkyl groups,

each $R_{13}$ and $R_{14}$ independently have one of the meanings of $R_{11}$ and $R_{12}$, whereby one of the groups $R_{13}$ and $R_{14}$ and preferably both radicals are different from hydrogen,

each $R_{15}$ independently is (1-4C)-alkyl optionally substituted by phenyl,

each $V_1$ independently is (1-6C)-alkylene or (2-6C)-alkenylene,

each $V_2$ independently is (2-6C)-alkylene or (2-6C)-alkenylene, the groups $R_5$ and $R_6$, $R_{11}$ and $R_{12}$, $R_{13}$ and $R_{14}$ respectively may form together with the N-atom to which they are attached a saturated heterocyclic ring and $R_{13}$, $R_{14}$ and $R_{15}$ may form together with the N-atom to which they are attached a pyridine ring or a saturated heterocyclic ring whereby the pyridine- and the heterocyclic ring may be substituted by one, two or three (1-4C)-alkyl groups,

$R_{1a}$ is hydrogen,

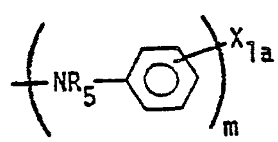

or

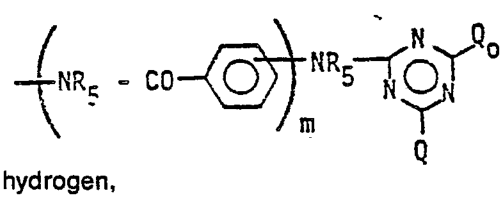

whereby, when x is 1, $R_{1a}$ is hydrogen,

$X_{1a}$ is

$$-NR_5-Q_{10}-NR_{11}R_{12},\ -COCH_2-\overset{\oplus}{N}R_{13}R_{14}R_{15}\ A^{\ominus},$$
$$-NR_5-COCH_2-NR_{11}R_{12},\ -CO-Q_{10}-\overset{\oplus}{N}R_{13}R_{14}R_{15}\ A^{\ominus},$$
$$-NR_5-Q_{10}-\overset{\oplus}{N}R_{13}R_{14}R_{15}\ A^{\ominus},\ -NR_5-COCH_2\overset{\oplus}{N}R_{13}R_{14}R_{15}A^{\ominus}$$
$$\text{or}\quad -CO-Q_{10}-\overset{\oplus}{N}(CH_3)_2-(CH_2)_4-Z\ A^{\ominus},$$

m is 0 or 1,

$Q_{10}$ is

$$-\overset{*}{N}HCOCH_2-$$

or linear or branched (2-6C)-alkylene,

$R_{2a}$ is hydrogen or, when $R_{1a}$ is hydrogen and $R_{1b}$ is a group of formulae e) or f) is also OH, $NH_2$, (1-4C)-alkylcarbonylamino, benzoylamino or phenylamino, the phenyl group of which may be substituted by 1 or 2 substituents selected from the group consisting of halogen, $NO_2$, $NH_2$ (1-4C)-alkyl and (1-4C)-alkoxy,

each $A_1$ independently is the group of a tetrazo compound or the group of a diazo- and/or coupling compound and

$W_0$ is a divalent bridge member,
mixtures of the compounds of formula I, their acid addition salts, or salts of acids and 1 : 1 or 1 : 2-metal complex thereof, with the proviso that

α) when the monoazo compounds b) of formula I are free from sulfonic acid groups, the compounds of formula b) have one, preferably two, and when the dis- and polyazo compounds of formula I are free from sulfonic acid groups, the compounds have at least two water solubilizing cationic or at least two water solubilizing protonatable basic groups or at least one cationic and at least one protonatable basic groups and, when the compounds of formula I contain sulfonic acid groups, the number of the cationic and/or protonatable basic groups present, is at least one number greater than the sum of sulpho groups or possible additional present anionic groups and

β) when r stands for the number 2, S is hydrogen and $R_0$ is hydrogen, $R_1$ is different from alkylene, T is different from CN and the group M is free from sulfonic acid groups, wherein, is different from hydrogen, comprising coupling the diazo compound of an amine of formula

$$DD—NH_2 \qquad XI,$$

wherein DD is a group of formulae

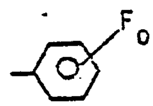

XIa)

$$—A—N = N—K \qquad XIb)$$

or

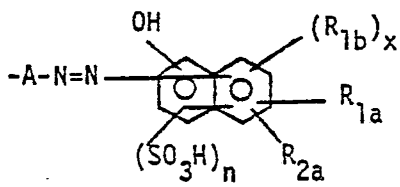

XI c)

with a compound of formula

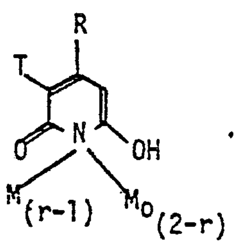

XII

and, if required, converting the resulting compounds into the corresponding metal complex form, or, when S is hydrogen, comprising cyclisizing a compound of formula

$$T—CH_2—CONHR_1—NH—M_{00} \qquad XXVI,$$

wherein $M_{00}$ is any blocking group, especially compound of formula

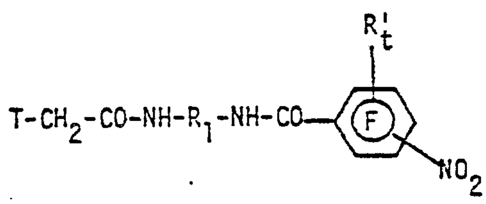

XXVII

wherein $R'_t$ is hydrogen, halogen, (1-4C)-alkyl or (1-4C)-alkoxy, with an alkylester of acetic acid of formula

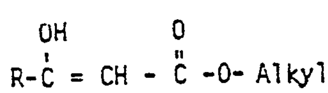

XXVIII

by known methods to a compound of formula

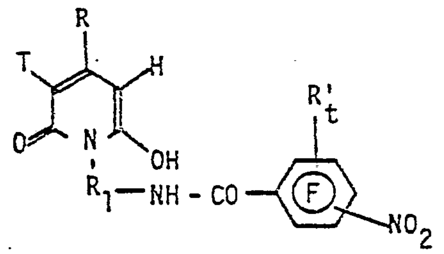

XXIX

and for compounds, wherein $R_0$ is hydrogen, this compound is saponifed or for compounds, wherein $R_{00}$ is a group of formula

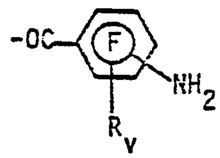

XXa

the nitro group is reduced to an amino group.

2. A process for preparing metal complex compounds of formula I according to Claim 1, wherein S is different from hydrogen, comprising a compound of formula I with metal yielding agent to convert the compounds into the corresponding 1 : 1 — or 1 : 2 — metal complex compounds.

3. Stable liquid dyeing compositions containing a compound of formula I according to Claim 1 in water soluble acid addition salt form or as a quaternary ammonium salt.

4. Water-soluble stable solid dyeing compositions containing a compound of formula I according to Claim 1 in water-soluble acid addition salt form or as a quaternary ammonium salt.

5. A process for dyeing or printing paper and leather, comprising applying to that substrate a compound of formula I wherein S is different from hydrogen.

6. A process for dyeing, padding or printing naturale or regenerated cellulose materials, hemp, flax, sisal, jute, coir or straw comprising applying to that substrate a compound of formula I, when S is different from hydrogen.

**Revendications** (pour les Etats Contractants : BE, CH, DE, FR, GB, IT, LI, NL, SE)

1. Composés basiques ou composés azoïques qui correspondent, sous une des formes tautomères possibles, à la formule

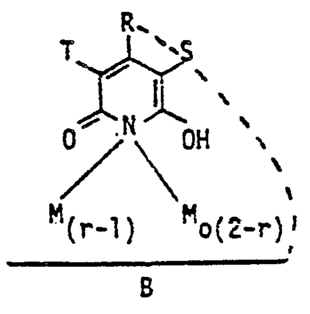

(I)

dans laquelle

R signifie indépendamment l'hydrogène, un reste alkyle en $C_1$-$C_4$, cycloalkyle en $C_5$-$C_6$ ou phényle, benzyle ou phényléthyle,

T signifie indépendamment l'hydrogène ou bien

i) -CN

$i_1$) 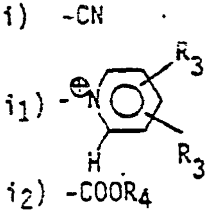 $A^{\ominus}$

$i_2$) $-COOR_4$

$i_3$) $-CONR_5R_6$

i4) $-SO_2NR_5R_6$

i5) 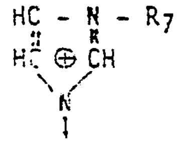 $A^{\ominus}$ ou

i6) 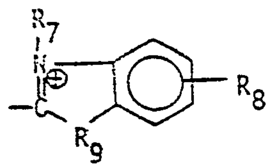 $A^{\ominus}$

r signifie 1 ou 2,

M signifie indépendamment un reste $—R_1—NH—R_0$,

$M_0$ signifie indépendamment un reste $—R_1—NH—R_0$, l'hydrogène ou un groupe $—NR_{11}R_{12}$, alkyle en $C_1$-$C_4$, hydroxyalkyle en $C_2$-$C_4$, (alcoxy en $C_1$-$C_4$)-alkyle en $C_1$-$C_4$, cycloalkyle en $C_5$-$C_6$ éventuellement substitué par 1 à 3 groupes alkyle en $C_1$-$C_4$, (phényl)-alkyle en $C_1$-$C_3$, $—V_1—NR_{13}R_{14}$ ou $—V_2—$

$\overset{\oplus}{N}R_{13}R_{14}R_{15}A^{\ominus}$

$A^{\ominus}$ signifie un anion,

S signifie, lorsque r signifie 2,

a) l'hydrogène,

b) un reste de formule

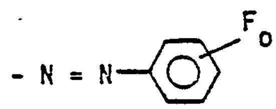 ou

c) $—N{=}N—A—N{=}N—K$

et $F_0$ signifie un substituant usuel dans la chimie des azoïques, de préférence un halogène ou un groupe $NO_2$, $NH_2$, alkyle, alcoxy, CN, trifluoroalkyle, phényle, aniline, benzoyle, carbamoyle, phénoxy, halogéno-phénoxy, dihalogéno-phénoxy, alkylsulfonyle, phénylsulfonyle, alkylsulfonylamino, di-N-alkylaminosulfonyle ou alkylsulfonyle, ou bien, lorsque r signifie 1,

d) un reste de formule

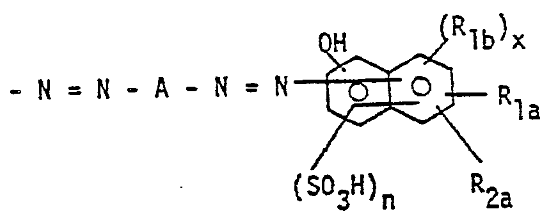

dans laquelle

n signifie 0, 1 ou 2,

x signifie 0 ou 1

A signifie indépendamment le reste d'une composante de tétrazotation,

K signifie le reste d'une composante de diazotation ou de copulation quelconque, de préférence exempte de groupes sulfo, de la série de la pyrazolone-5, du 5-aminopyrazole, de l'aniline, du phénol, de l'acétoacétylalkyl- ou arylamide, de l'acide barbiturique, de la dimédone, d'un ester de l'acide dimédone-carboxylique, de la pyridone (par exemple du reste B, dans lequel r signifie 1 ou 2) ou de la 2,6-diaminopyridine,

$R_{1b}$, lorsque x signifie 1, représente un reste de formule

e) $—N{=}N—K_1$

f) $—N{=}N—A_1—N{=}N—K_1$

g) 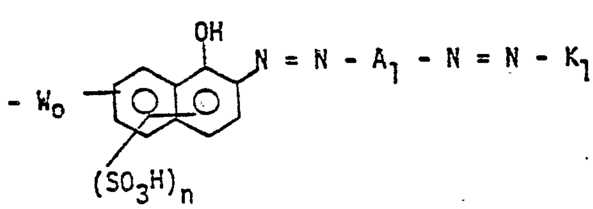

$K_1$ signifie un reste K ou un α- ou β-naphtol, un α- ou β-aminonaphtalène ou un aminonaphtol,

$R_1$ signifie indépendamment un reste alkylène ou alcénylène éventuellement interrompu par 1 ou 2 hétéroatomes, un reste phénylène ou cyclohexylène,

$R_0$ ou un reste de formule

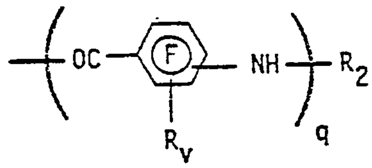

(Ia)

q signifie 0 ou 1, et

$R_1$ signifie l'hydrogène, un halogène ou un groupe nitro, alkyle en $C_1$-$C_4$ ou alcoxy en $C_1$-$C_4$,

$R_2$ représente un reste de formule

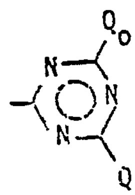

$h_1$)

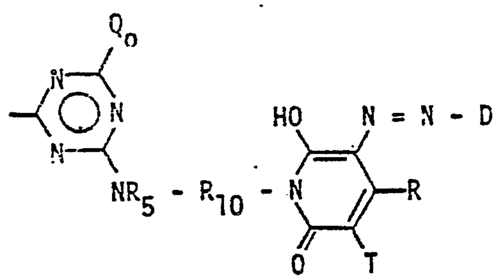

$h_3$) ou

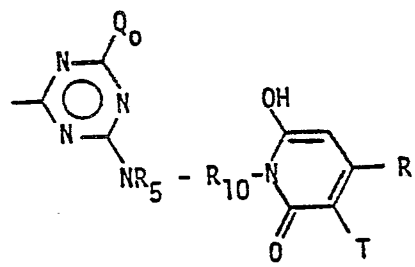

$h_2$)

$$-CO-(CH_2)_{1-3}-Z \qquad h_4)$$

ou bien l'hydrogène, où

D signifie indépendamment le reste d'une composante de diazotation pouvant être substituée par des substituants usuels dans la chimie des azoïques, de préférence un reste

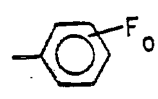

- A - N = N - K ou - A - N = 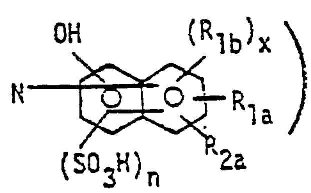

$R_3$ signifie indépendamment l'hydrogène, un reste alkyle en $C_1$-$C_4$ ou un groupe $-NR_5R_6$ ou $-CONR_5R_6$,

$R_4$ signifie indépendamment un groupe alkyle en $C_1$-$C_6$ ou (phényl)-alkyle en $C_1$-$C_3$,
$R_5$ et $R_6$, indépendamment l'un de l'autre, signifient l'hydrogène ou un groupe alkyle en $C_1$-$C_4$,
$R_7$ signifie indépendamment un groupe alkyle en $C_1$-$C_4$,
$R_8$ signifie indépendamment l'hydrogène ou un groupe alkyle en $C_1$-$C_4$,
$R_9$ signifie indépendamment un groupe —S—, —O— ou

$$-\overset{|}{N}-R_5$$

$R_{10}$ signifie indépendamment un reste alkylène en $C_2$-$C_6$ ou un groupe

$$-\overset{*}{N}HCOCH_2-$$

l'atome désigné par un * étant lié au reste $—NR_5—$,
$Q_0$ signifie un reste aminoaliphatique, cycloaliphatique, aromatique ou hétérocyclique, dans lequel l'atome d'azote est lié à l'atome de carbone du reste triazinyle,
Q signifie un halogène ou un groupe OH, $NH_2$, alcoxy en $C_1$-$C_4$, phényle ou $Q_0$,
Z signifie indépendamment un reste de formule $—NR_{11}R_{12}$ ou

$$-\overset{\oplus}{N}R_{13}R_{14}R_{15}\quad A^{\ominus}$$

$R_{11}$ et $R_{12}$, indépendamment l'un de l'autre, signifient l'hydrogène ou un groupe alkyle en $C_1$-$C_6$ non substitué, un groupe alkyle en $C_2$-$C_6$ substitué par un halogène ou par un groupe OH ou CN, un groupe (phényl)-alkyle en $C_1$-$C_3$ dont le reste phényle porte éventuellement 1 à 3 substituants choisis parmi le chlore et les groupes alkyle en $C_1$-$C_4$ et alcoxy en $C_1$-$C_4$, un groupe cycloalkyle en $C_5$-$C_6$ non substitué ou substitué par 1 à 3 groupes alkyle en $C_1$-$C_4$,
$R_{13}$ et $R_{14}$, indépendamment l'un de l'autre, ont l'une des significations données pour $R_{11}$ et $R_{12}$, un des restes $R_{13}$ et $R_{14}$, de préférence les deux restes, étant différent de l'hydrogène,
$R_{15}$ signifie indépendamment un reste alkyle en $C_1$-$C_4$ éventuellement substitué par un groupe phényle,
$V_1$ signifie indépendamment un groupe alkylène en $C_1$-$C_6$ ou alcénylène en $C_2$-$C_6$,
$V_2$ signifie indépendamment un groupe alkylène en $C_2$-$C_6$ ou alcénylène en $C_2$-$C_6$,
les restes $R_5$ et $R_6$ ou $R_{11}$ et $R_{12}$ ou encore $R_{13}$ et $R_{14}$ pouvant former ensemble, avec l'atome d'azote auquel ils sont liés, un noyau hétérocyclique saturé, et $R_{13}$, $R_{14}$ et $R_{15}$ pouvant former ensemble, avec l'atome d'azote auquel ils sont liés, un cycle pyridine ou un noyau hétérocyclique saturé, et le cycle pyridine et le noyau hétérocyclique pouvant être substitués par 1, 2 ou 3 groupes alkyle en $C_1$-$C_4$,
$R_{1a}$ signifie l'hydrogène ou un groupe

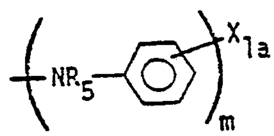

ou

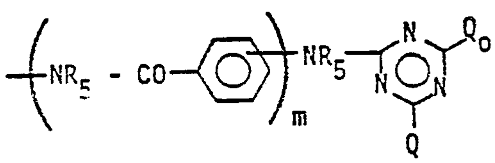

$R_{1a}$ signifiant l'hydrogène lorsque x = 1,
$X_{1a}$ représente un groupe

$$-NR_5-Q_{10}-NR_{11}R_{12},\ -COCH_2-\overset{\oplus}{N}R_{13}R_{14}R_{15}\ A^{\ominus},$$
$$-NR_5-COCH_2-NR_{11}R_{12},\ -CO-Q_{10}-\overset{\oplus}{N}R_{13}R_{14}R_{15}\ A^{\ominus},$$
$$-NR_5-Q_{10}-\overset{\oplus}{N}R_{13}R_{14}R_{15}\ A^{\ominus},\ -NR_5-COCH_2\overset{\oplus}{N}R_{13}R_{14}R_{15}A^{\ominus}$$
$$\text{ou}\ -CO-Q_{10}-\overset{\oplus}{N}(CH_3)_2-(CH_2)_4-Z\ A^{\ominus},$$

m signifie 0 ou 1,

$Q_{10}$ signifie un groupe

$$-\overset{*}{N}HCOCH_2-$$

ou un groupe alkylène en $C_2$-$C_6$ linéaire ou ramifié,

$R_{2a}$ signifie l'hydrogène ou bien, lorsque $R_{1a}$ représente l'hydrogène et $R_{1b}$ un reste de formule e) ou f), également un groupe OH, $NH_2$, (alkyl en $C_1$-$C_4$)-carbonylamino, benzoylamino ou phénylamino dont le reste phényle porte éventuellement 1 ou 2 substituants choisis parmi les halogènes et les groupes $NO_2$, $NH_2$, alkyle en $C_1$-$C_4$ et alcoxy en $C_1$-$C_4$,

$A_1$ signifie indépendamment le reste d'une composante de tétrazotation ou le reste d'une composante de diazotation/copulation et

$W_0$ signifie un pont bivalent,

mélanges des composés de formule I, leurs sels d'addition d'acides ou leurs sels d'acides ainsi que leurs complexes métallifères, tels que leurs complexes métallifères 1 : 1 ou 1 : 2, avec la condition

α) qu'ils contiennent, lorsque les composés monoazoïques b) de formule I sont exempts de groupes sulfo, un et de préférence deux et, lorsque les composés disazoïques et polyazoïques de formule I sont exempts de groupes sulfo, au moins deux groupes cationiques hydrosolubilisants ou au moins deux groupes basiques protonables hydrosolubilisants, ou au moins un groupe cationique et au moins un groupe basique protonable et, lorsque les composés de formule I contiennent des groupes sulfo, le nombre de groupes cationiques et/ou de groupes basiques protonables soit supérieur d'au moins 1 unité au nombre de groupes sulfo et de groupes anioniques supplémentaires éventuellement présents, et

β) lorsque r signifie 2, S représente l'hydrogène et $R_0$ représente l'hydrogène, $R_1$ soit différent d'un groupe alkylène, T soit différent du groupe CN et le reste M soit exempt de groupes sulfo.

2. Composés basiques selon la revendication 1 sous une des formes tautomères possibles de formule

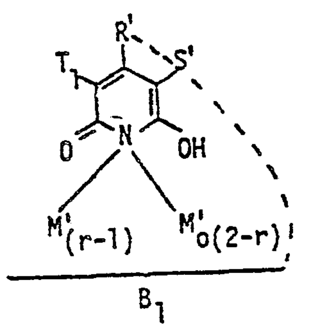

(II)

dans laquelle

R' signifie indépendamment un groupe méthyle, éthyle, phényle, benzyle ou cyclohexyle,

$T_1$ signifie indépendamment l'hydrogène,

i) - CN

$i_1'$) 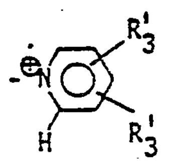 $A^{\ominus}$ ou

$i_3'$) $- CONR_5'R_6'$,

M' signifie indépendamment un reste $—R_1'—NH—R_0'$,

$M_0'$ signifie indépendamment un reste $—R_1'—NHR_0'$, l'hydrogène ou un groupe $—CH_3$, $—C_2H_5$, $—C_2H_4OH$, cyclohexyle, benzyle $—(CH_2)_{1-3}—NR_{13}'R_{14}'$ ou

$$-(CH_2)_{2-3} - \overset{\oplus}{N}R_{13}'R_{14}'R_{15}'A^{\ominus}$$

S', lorsque r signifie 2, représente

a) l'hydrogène

b') un reste de formule

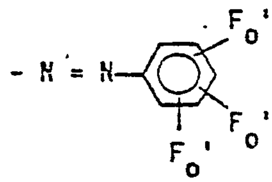

$c_1)$ 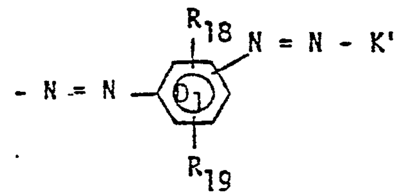 ou

$c_2)$ 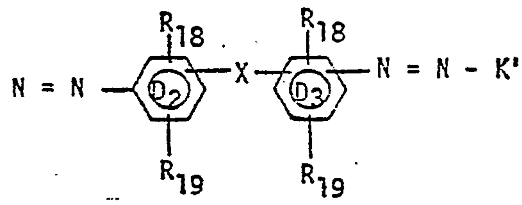

ou bien S', lorsque r signifie 1, représente

$d_1)$ un reste de formule

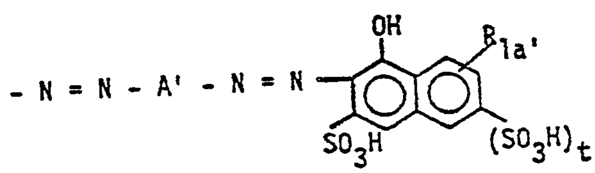

$d_2)$ 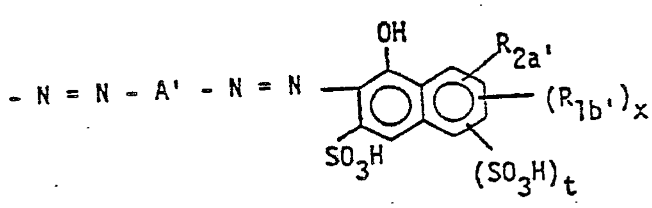

$d_3)$ 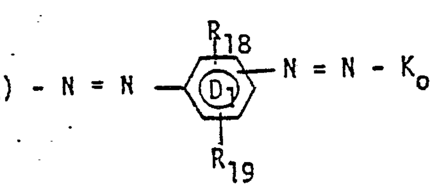 ou

$d_4)$ 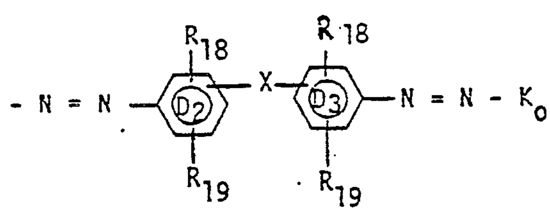

dans lesquelles

$K_0$ signifie un reste de formule

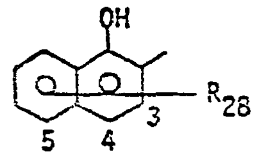

aa),

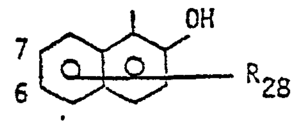

aa$_1$),

A′ signifie indépendamment un reste de formule

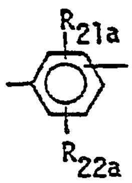

(m)

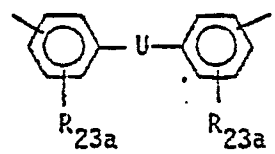

(n)

ou

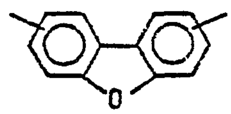

(o)

$R_{1b}$′, lorsque x signifie 1, représente un reste de formule

e′) —N=N—$K_1$′

f′) —N=N—A′—N=N—$K_1$′

g′)

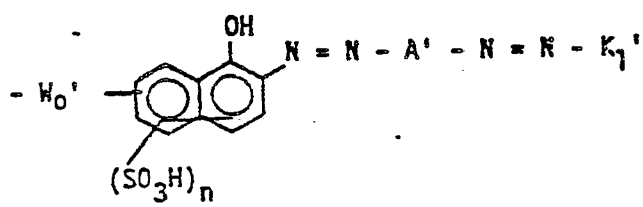

K′ signifie un reste de formule

aa$_4$) $B_1$, dans lequel r peut signifier aussi bien 1 que 2,

aa$_5$) un phénol,

aa$_6$)

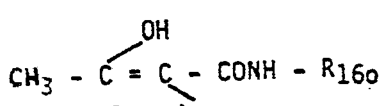

aa$_7$)

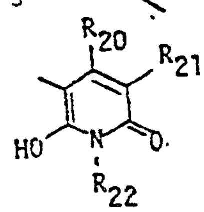

aa$_8$)

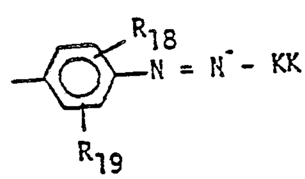

aa$_9$)

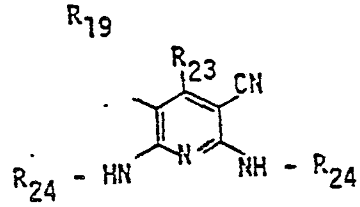

aa10) 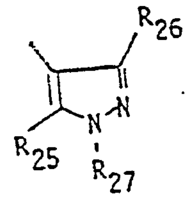

aa11) 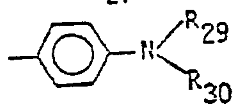

aa12) 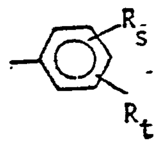

$R_1'$ signifie indépendamment un reste alkylène en $C_2$-$C_8$ linéaire ou ramifié ou un reste méta- ou paraphénylène,

$R_0'$ signifie un reste de formule

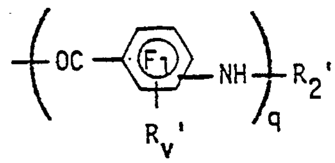 (Ib)

$R_v'$ signifie indépendamment l'hydrogène, le chlore ou un groupe nitro, méthyle ou méthoxy,

$R_2'$ signifie un reste de formule

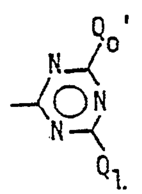 h1' ou

$-CO-(CH_2)_{1-2}-Z_1$ h4')

ou bien l'hydrogène,

$R_3'$ signifie indépendamment l'hydrogène ou un groupe méthyle, éthyle, $—NH_2$ ou $—N(CH_3)_2$,

$R_5'$ et $R_6'$, indépendamment l'un de l'autre, signifient l'hydrogène ou un groupe méthyle ou éthyle,

$Q_1$ signifie le chlore ou un groupe OH, $NH_2$

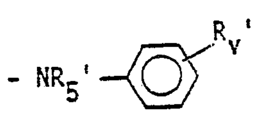 , 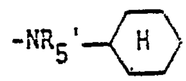 , $OCH_3$

monoalkylamino en $C_1$-$C_4$, di(alkyl en $C_1$-$C_2$)-amino, monohydroxyalkylamino en $C_2$-$C_4$, bis-[hydroxy-(alkyl en $C_2$-$C_4$)]-amino ou $Q_0'$, et

$Q_0'$ signifie un groupe

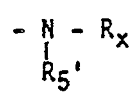

où

$R_x$ signifie un reste alkyle en $C_1$-$C_{12}$ interrompu par un et/ou plusieurs groupes de formule $—NR_5'—$ ou

$$-\overset{\oplus}{N}R_7R_7 - A^{\ominus}$$

et éventuellement substitué par un groupe OH, un groupe

$-NHCOCH_2-Z_1$, $-CH_2CONH-V-Z_1$, $-V-Z_1$, $-V$-arylène-$Z_0$, -arylène-$Z_0$, 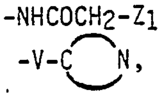,

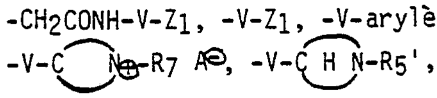

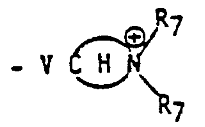 $2A^{\ominus}$, 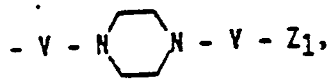

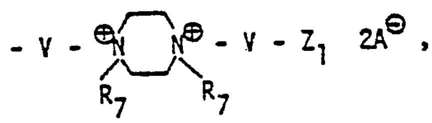

ou bien $R_x$ et $R_5'$ peuvent former ensemble, avec l'atome d'azote auquel ils sont liés, un reste

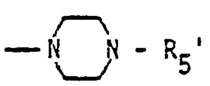 ou 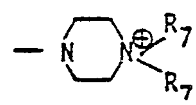 $2A^{\ominus}$

V signifie indépendamment un reste alkylène en $C_1$-$C_8$ ou un reste alcénylène en $C_3$-$C_8$,
$Z_0$ signifie un groupe $-N(CH_3)_2$, $-N(C_2H_5)_2$,

$$-\overset{\oplus}{N}(CH_3)_3\ A^{\ominus}\ -\overset{\oplus}{N}(C_2H_5)_3\ A^{\ominus}$$

ou un reste $-CONH-V_0-Z_1$, $-NHCO-V_0-Z_1$, $-CO-V_0-Z_1$, $-SO_2-NH-V_0-Z_1$, $-V_0-Z_1$ ou $-NHNHCOCH_2-Z_1$,
et le reste arylène peut être substitué par un halogène ou par un groupe OH, $NO_2$, alkyle en $C_1$-$C_4$ ou alcoxy en $C_1$-$C_4$,

$V_0$ signifie un reste alkylène en $C_1$-$C_8$ et le groupe de formule

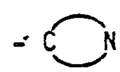 ou 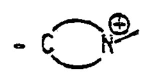

représente un hétérocycle insaturé et le groupe de formule

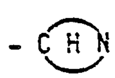 ou 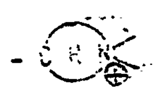

représente un hétérocycle saturé,

$Z_1$ signifie indépendamment un reste de formule

$$NR_{11}'R_{12}' \quad \text{ou} \quad -\overset{\oplus}{N}R_{13}'R_{14}'R_{15}'\ A^{\ominus}$$

$R_{11}'$ et $R_{12}'$, indépendamment l'un de l'autre, signifient l'hydrogène ou un groupe alkyle en $C_1$-$C_6$ linéaire ou ramifié, un groupe hydroxyalkyle en $C_2$-$C_3$ non ramifié, benzyle, 2-cyanoéthyle ou 2-chloroéthyle,

$R_{13}'$ et $R_{14}'$, indépendamment l'un de l'autre, signifient un groupe alkyle en $C_1$-$C_6$ linéaire ou ramifié, hydroxyalkyle en $C_2$-$C_3$ non ramifié, benzyle, 2-cyanoéthyle ou 2-chloroéthyle,

$R_{15}'$ signifie un groupe méthyle, éthyle, propyle ou benzyle,

les restes $R_5'$ et $R_6'$ ou $R_{11}'$ et $R_{12}'$ peuvent former ensemble, avec l'atome d'azote auquel ils sont liés, un cycle morpholine, pipéridine, pyrrolidine, pipérazine ou N-méthylpipérazine, ou bien les restes $R_{13}'$, $R_{14}'$ et $R_{15}'$ peuvent former ensemble, avec l'atome d'azote auquel ils sont liés, un cycle pyridine, picoline ou lutidine ou un cycle de formule

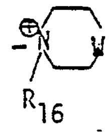 . $A^{\ominus}$

dans laquelle

$R_{16}$ signifie un groupe méthyle ou éthyle, et W signifie un groupe —$CH_2$, —O—, —S—, —$SO_2$—, —SO—,

$$-\overset{|}{N}H, \quad -\overset{|}{N}-R_{16} \quad \text{ou} \quad -\underset{\oplus}{\overset{|}{N}}(R_{16})_2\ A^{\ominus}$$

$F_0'$ signifie indépendamment l'hydrogène, un halogène ou un groupe —$NO_2$, —$NH_2$, alkyle en $C_1$-$C_4$, alcoxy en $C_1$-$C_4$, CN, trifluorométhyle, phényle, anilino, benzoyle, carbamoyle, phénoxy, halogénophénoxy, dihalogénophénoxy, alkylsulfonyle en $C_1$-$C_4$, phénylsulfonyle, alkylsulfonylamino en $C_1$-$C_4$, di-N-(alkyl en $C_1$-$C_4$)-aminosulfonyle ou alkylsulfonyle en $C_1$-$C_4$,

X, lorsque les cycles $D_2$ et $D_3$ ne sont pas substitués, signifie pour $X_1$ une liaison directe,

$X_2$ un reste alkylène linéaire ou ramifié contenant de 1 à 4 atomes de carbone,

$X_3$ -CO-, $X_4$ $-NH-\overset{S}{\overset{\|}{C}}-NH-$, $X_5$ -S-, $X_6$ -O-,

$X_7$ -CH=CH-, $X_8$ -S-S-, $X_9$ -$SO_2$-,

$X_{10}$ -NH-, $X_{11}$ -NH-CO-, $X_{12}$ $-\underset{CH_3}{\underset{|}{N}}-CO-$,

$X_{13}$ 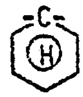 , $X_{14}$ 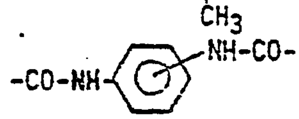 ,

$X_{15}$ 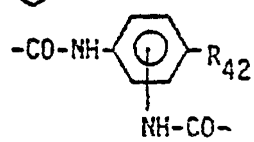 , $X_{16}$ 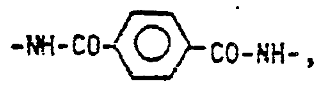

$X_{17}$ -$SO_2$-NH-, $X_{18}$ 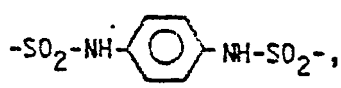

$X_{19}$ $-\underset{R_{44}}{\underset{|}{N}}-CO-R_{43}-CO-\underset{R_{44}}{\underset{|}{N}}-$, $X_{20}$ $-\underset{R_{44}}{\underset{|}{N}}-CO-CH=CH-CO-\underset{R_{44}}{\underset{|}{N}}-$,

$X_{21}$ $-\underset{R_{44}}{\underset{|}{N}}-CO-\underset{R_{44}}{\underset{|}{N}}-$, $X_{22}$ -CO-NH-NH-CO-,

$X_{23}$ $-CH_2CO-NH-NH-CO-CH_2-$, $X_{24}$ -CH=CH-CO-NH-NH-CO-CH=CH-,

$X_{25}$ 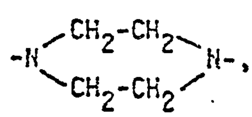 , $X_{26}$ 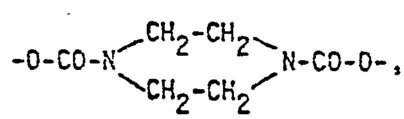

$X_{27}$ 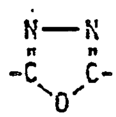 , $X_{28}$ $-O-\underset{O}{\underset{\|}{C}}-O-$, $X_{29}$ $-\underset{O}{\underset{\|}{C}}-O-$, $X_{30}$ $-\underset{O}{\underset{\|}{C}}-\underset{O}{\underset{\|}{C}}-$,

$X_{31}$ $-O-(CH_2)_9-O-$,

(Suite)

$X_{32}$ 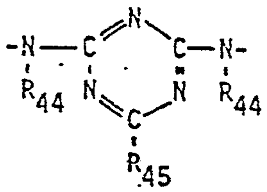 , $X_{33}$ 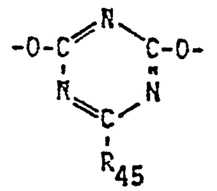 ,

$X_{34}$ 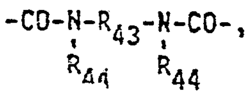 , $X_{35}$ 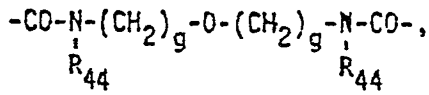 ,

$X_{36}$ 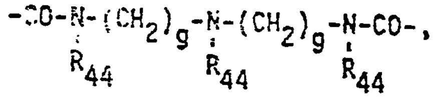 ,

$X_{37}$ 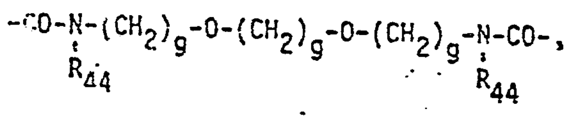 ,

$R_{38}$ 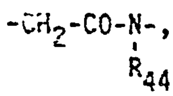 , $R_{39}$ 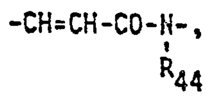 ,

$X_{40}$ $-N=N-$, $X_{41}$ $-CH_2-S-CH_2-$, $X_{42}$ $-SO-$,
$X_{43}$ $-CH_2-SO-CH_2-$,
$X_{44}$ $-CH_2-SO_2-CH_2-$, $X_{45}$ $-CH_2-NH-CO-NH-CH_2-$,
$X_{46}$ $-CH_2-NH-CS-NH-CH_2-$,

$X_{47}$ 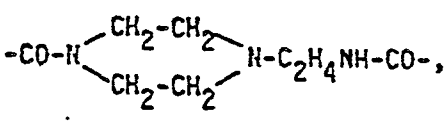 ,

$X_{48}$ 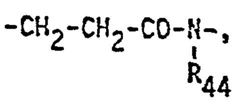 , $X_{49}$ $-CH_2-CO-CH_2-$,

$X_{50}$ $-CH=CH-CH=CH-$, $X_{51}$ 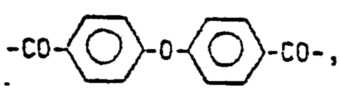 ,

$X_{52}$ 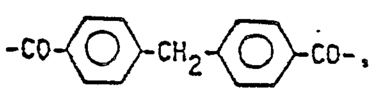 , $X_{53}$ 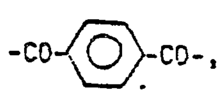 ,

$X_{54}$ 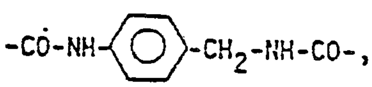 ,

$X_{55}$ 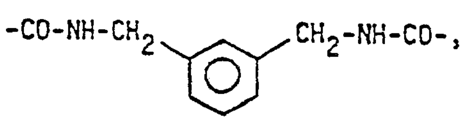 ,

(Suite)

$X_{56}$ 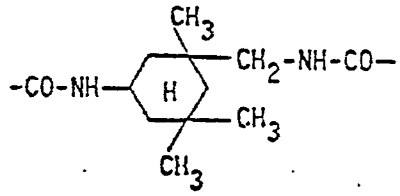,

$X_{57}$ 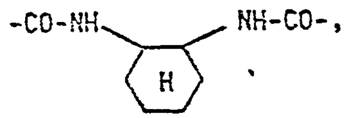, $X_{58}$ $-CH_2-CO-$,

$X_{59}$ $-CH=CH-CO-CH=CH-$, $X_{60}$ 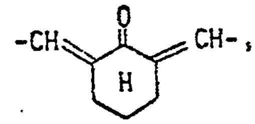

$X_{61}$ 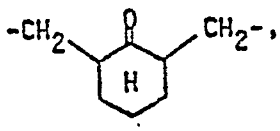, $X_{62}$ $-O-CH_2-CH_2-O-$,

$X_{63}$ $-O-CH_2-O-$, $X_{64}$ $-CO-NH-R_{43}-CO-NH-$,
$X_{100}$ $-CO-NH-R_{43}-CO-NH-R_{43}-NH-CO-$,
$X_{101}$ $-CO-NH-R_{43}-NH-CO-CH_2-CH_2-CO-NH-R_{43}-NH-CO-$,
$X_{102}$ $-CO-NH-R_{43}-NH-CO-CH=CH-CO-NH-R_{43}-NH-CO-$,

$X_{103}$ 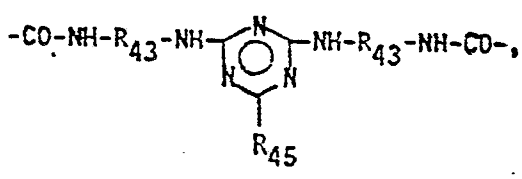

$X_{104}$ $-SO_2-NR_{44}-(CH_2)_g-NR_{44}-SO_2-$,
$X_{105}$ $-CO-NR_{44}-R_{43}-O-CO-$,

$X_{106}$ 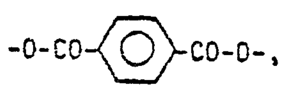

$X_{107}$ 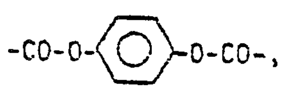

$X_{108}$ $-CONH-R_{43}-NH-CO-NH-R_{43}-NH-CO-$,

$R_{42}$ signifie indépendamment un halogène ou un groupe alkyle en $C_1$-$C_4$ ou alcoxy en $C_1$-$C_4$,
$R_{43}$ signifie indépendamment un reste alkylène en $C_1$-$C_4$ linéaire ou ramifié,
$R_{44}$ signifie indépendamment l'hydrogène ou un groupe alkyle en $C_1$-$C_4$,
$R_{45}$ signifie indépendamment un halogène ou un groupe

$-NH-CH_2-CH_2-OH$,
$-N(CH_2-CH_2-OH)_2$, $NH_2$, $OH$, $NH-(CH_2)_{2-3}N(C_2H_5)_2$,

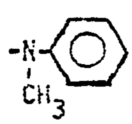, 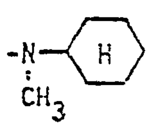, 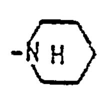, $-OCH_3$, $OC_2H_5$,

g signifie indépendamment 1, 2, 3 ou 4,

X, lorsque les cycles $D_2$ et/ou $D_3$ sont substitués, en particulier en position ortho par rapport aux deux ponts azo, représente $X_1$ une liaison directe ou $X_2$, $X_{14}$, $X_{21}$, $X_{32}$, $X_{34}$ ou $X_{40}$,

$R_s$ et $R_t$, indépendamment l'un de l'autre, représentent l'hydrogène ou un groupe alkyle en $C_1$-$C_4$ ou alcoxy en $C_1$-$C_4$,

$R_{160}$ représente un reste $-(CH_2)_v-Z_1$,

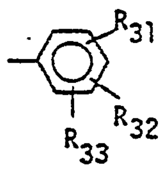 ,
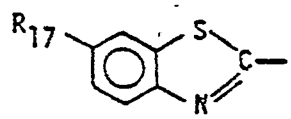 ,

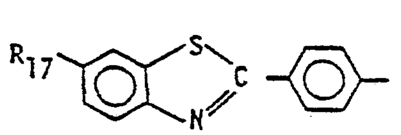 ,
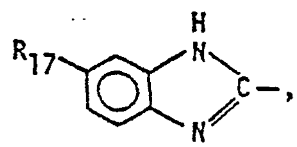

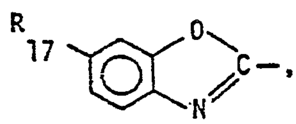
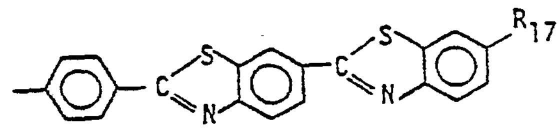

ou
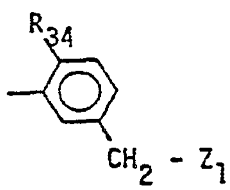

$R_{17}$ signifie indépendamment H ou un groupe méthyle,

$R_{18}$ et $R_{19}$, indépendamment l'un de l'autre, signifient l'hydrogène, un halogène ou un groupe alkyle en $C_1$-$C_4$ ou alcoxy en $C_1$-$C_4$,

$R_{20}$ signifie indépendamment H ou un groupe méthyle, éthyle, phényle, benzyle ou cyclohexyle,

$R_{21}$ signifie un reste $T_1$ ou

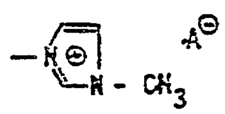

$R_{22}$ signifie un reste de formule $-R_1'-NH-R_0'$, l'hydrogène, un reste alkyle en $C_1$-$C_4$ éventuellement substitué par un groupe phényle, un groupe $-(CH_2)-CN$, $-(CH_2)_v-Z_1$, $-(CH_2)_2-OH$, $-(CH_2)_2OCH_3$ ou

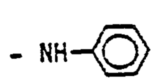

$R_{23}$ signifie un groupe alkyle en $C_1$-$C_4$ ou phényle,

$R_{24}$ signifie indépendamment l'hydrogène ou un groupe alkyle en $C_1$-$C_4$, $-(CH_2)_{2\text{-}3}-OCH_3$, $-(CH_2)_2-OH$, $-(CH_2)_{2\text{-}3}-N(CH_3)_2$ ou

$$-(CH_2)_{2\text{-}3}-\overset{\oplus}{N}(CH_3)_3\ A^{\ominus}$$

$R_{25}$ signifie un groupe OH ou $NH_2$,

$R_{26}$ signifie un groupe alkyle en $C_1$-$C_4$ ou $-COR_{35}$,

$R_{27}$ signifie l'hydrogène ou un groupe

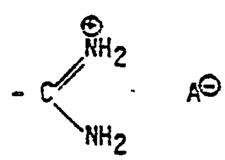

ou 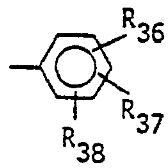

$R_{28}$ dans une des positions 3, 4, 5, 6 ou 7, signifie indépendamment l'hydrogène ou un groupe

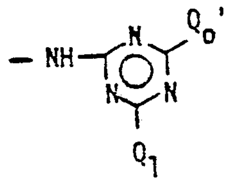 , $-NHCO(CH_2)_{1-3}-Z_1$

$—SO_2—NH—V_0—Z_1$, $—CONH—V_0—Z_1$, $—CONHNH_2$, $—NH—V_0—Z_1$, $—CH_2—Z_1$, $—NHNHCOCH_2—Z_1$ ou

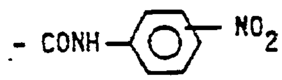

$R_{29}$ signifie un groupe alkyle en $C_1$-$C_4$,

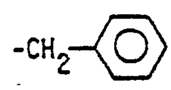

ou $—(CH_2)_2—CN$,

$R_{30}$ signifie un groupe alkyle en $C_1$-$C_4$ ou $—(CH_2)_v—Z_1$,

v signifie indépendamment 1, 2, 3, 4, 5 ou 6,

$R_{31}$ et $R_{32}$, indépendamment l'un de l'autre, signifient l'hydrogène, un halogène ou un groupe alkyle en $C_1$-$C_4$, alcoxy en $C_1$-$C_4$, $NO_2$ ou CN,

$R_{33}$ signifie l'hydrogène ou un groupe,

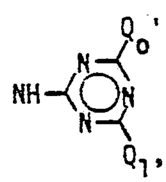

$—NHCO—(CH_2)_{2-3}—Z_1$, $—SO_2—NH—(CH_2)_{2-3}—Z_1$ ou $—CONH—V_0—Z_1$,

$R_{34}$ signifie un groupe alcoxy en $C_1$-$C_4$,

$R_{35}$ signifie un groupe $—O—R_{39}$, $—NR_{40}R_{41}$ ou $—NH—V_0—Z_1$,

$R_{36}$ et $R_{37}$, indépendamment l'un de l'autre, signifient l'hydrogène, un halogène ou un groupe alkyle en $C_1$-$C_4$, alcoxy en $C_1$-$C_4$, $—NO_2$ ou $—NH_2$,

$R_{38}$ signifie l'hydrogène ou un groupe $—CONH—V_0—Z_1$, $—SO_2NH—V_0—Z_1$, $—NHCO(CH_2)_{2-3}—Z_1$ ou

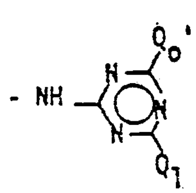

$R_{39}$ signifie un groupe alkyle en $C_1$-$C_4$,

$R_{40}$ et $R_{41}$, indépendamment l'un de l'autre, signifient l'hydrogène ou un groupe alkyle en $C_1$-$C_4$,

$R_{21a}$ signifie H, OH, un halogène ou un groupe alkyle en $C_1$-$C_4$, alcoxy en $C_1$-$C_4$, CN, $—CONH_2$, $—NHCO$-alkyle en $C_1$-$C_4$, $—NHCONH_2$, COOH ou $—SO_3H$,

$R_{22a}$ signifie H, un halogène ou un groupe alkyle en $C_1$-$C_4$ ou alcoxy en $C_1$-$C_4$ et les restes $R_{21a}$ et $R_{22a}$ se trouvent en position para l'un par rapport à l'autre,

$R_{23a}$ signifie indépendamment H, OH, un halogène ou un groupe CN, alkyle en $C_1$-$C_4$, alcoxy en $C_1$-$C_4$, —COOH ou —$SO_3H$,

U signifie une liaison directe, un groupe —$(CH_2)_{1-3}$, —O—, —S—, —$SO_2$—, —NHCO—, —NHCONH—, —$NHCO(CH_2)_{2-3}$—CONH—, —$CONH(CH_2)_{2-3}$—NHCO—, —$O(CH_2)_{2-3}$—O—, —N=N—, —CH=CH—CO—CH=CH—,

t signifie 0 ou 1,

$R_{2a}'$ signifie H, OH ou $NH_2$,

$R_{1a}'$ signifie l'hydrogène ou un groupe

$-NR_5'-Q_{10}'-NR_{11}'R_{12}'$,

$-NR_5'-Q_{10}'-N^{\oplus}R_{13}'R_{14}'R_{15}'\ A^{\ominus}$, $-NR_5'COCH_2NR_{11}'R_{12}'$,

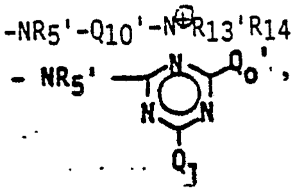,

$-NR_5'-COCH_2N^{\oplus}R_{13}'R_{14}'R_{15}'\ A^{\ominus}$ ou

$-CO-Q_{10}'-N^{\oplus}(CH_3)_2-(CH_2)_{1-4}-Z_1\ A^{\ominus}$,

$Q_{10}'$ signifie un groupe $-(CH_2)_{2-6}-$, $-CH_2-CH(CH_3)-$ ou $-CH_2-CH(CH_3)-CH_2-$,

$W_o'$ signifie un groupe -NH-, -NHCONH-, -NHCO-CH=CH-CO-NH-,

$-NHCO(CH_2)_{2-3}-CONH-$, 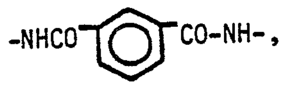,

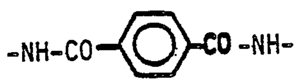 ou 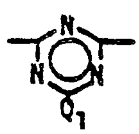,

$K_1'$ signifie un reste de formule aa), $aa_1$), $aa_2$), $aa_3$), $aa_4$), $aa_5$), $aa_6$), $aa_7$), $aa_8$), $aa_9$), $aa_{10}$), $aa_{11}$), $aa_{12}$) ou

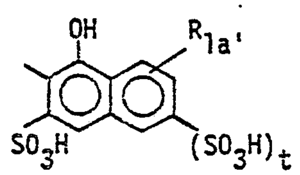

$aa_{13}$) ou

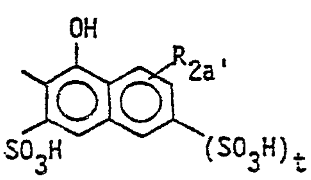

$aa_{14}$)

et KK signifie un reste de formule $aa_1$), $aa_2$), $aa_3$), $aa_4$), $aa_5$), $aa_6$), $aa_7$), $aa_8$), $aa_9$), $aa_{10}$), $aa_{11}$), $aa_{12}$), $aa_{13}$) ou $aa_{14}$),

mélanges de composés de formule II, leurs sels d'addition d'acides ou leurs sels d'acides ainsi que leurs complexes métallifères 1 : 1 ou 1 : 2, avec la condition que

$\alpha_1$) les composés monoazoïques b' de formule II contiennent un et de préférence deux et, lorsque les composés disazoïques et polyazoïques de formule II sont exempts de groupes sulfo [formules $c_1$), $c_2$), $d_3$), $d_4$)], au moins deux groupes cationiques hydrosolubilisants ou au moins deux groupes basiques protonables ou encore au moins un groupe cationique et au moins un groupe protonable et, lorsque les composés de formule II contiennent des groupes sulfo, le nombre de groupes cationiques et/ou de groupes basiques protonables soit supérieur d'au moins une unité au nombre de groupes sulfo et de groupes —COOH supplémentaires éventuellement présents, et

β₁) lorsque r signifie 2, S' représente l'hydrogène et $R_0$' représente l'hydrogène, $R_1$' soit différent d'un groupe alkylène, $T_1$ soit différent de CN et le reste M' soit exempt de groupes sulfo.

3. Composés basiques selon la revendication 1 sous une des formes tautomères possibles de formule III

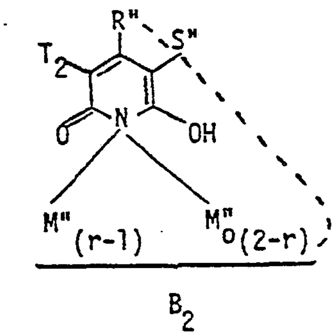

(III)

dans laquelle

R'' signifie un groupe méthyle ou phényle,

$T_2$ signifie indépendamment un groupe

- CN ou $i_1$'')

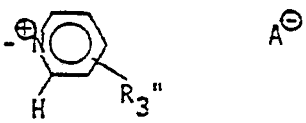

M'' signifie indépendamment un groupe $-R_1''-NH-R_0''$,

$M_0$'' signifie indépendamment l'hydrogène ou un groupe $-R_1''-NH-R_0''$, $CH_3$, $-C_2H_5$, benzyle, $-(CH_2)_{2\text{-}3}-NR_{13}''R_{14}''$ ou $-(CH_2)_{2\text{-}3}-NR_{13}''R_{14}''R_{15}''\ A^{\ominus}$,

S'', lorsque r signifie 2, représente

a) l'hydrogène,

b'') un reste de formule

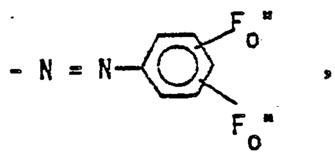

,

$c_1$')

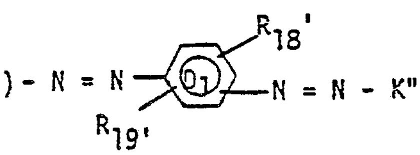

$c_2$')

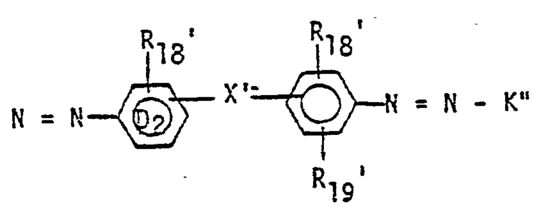

ou bien S'', lorsque r signifie 1, représente

$d_1$') un reste de formule

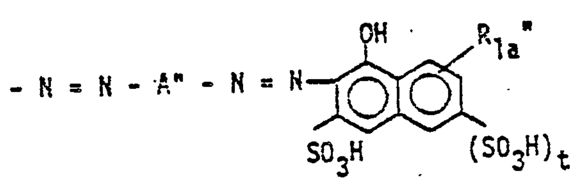

d2')
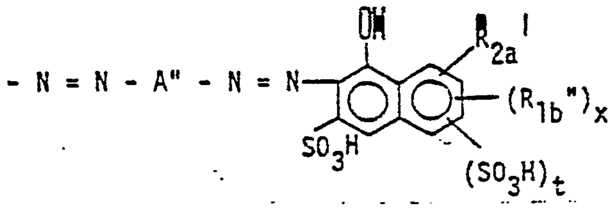

d3')
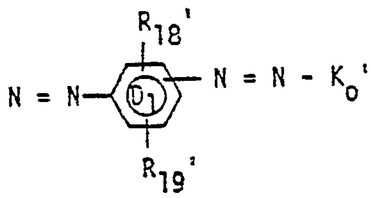

d4')
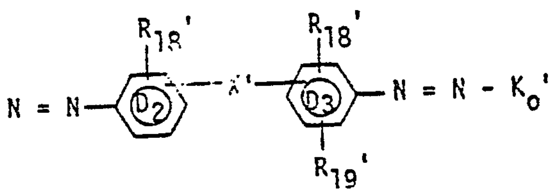

où

K$_0$' représente un reste de formule

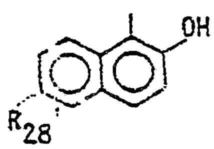

aa')

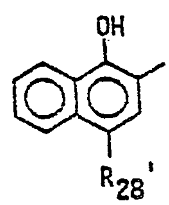

(aa$_1$')

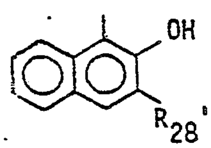

aa$_2$')

A" signifie indépendamment un reste de formule

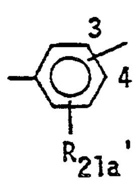

ou

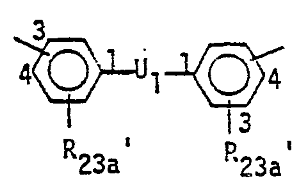

(mm) (nm)

le reste (mm) étant rattaché en position 1,3 ou 1,4, et le reste (nm) en position 3,3, 3,4 ou 4,4,

R$_{1b}$", lorsque x signifie 1, représente un reste de formule

e") —N = N—K$_1$"

f") —N = N—A"—N = N—K$_1$"

g")
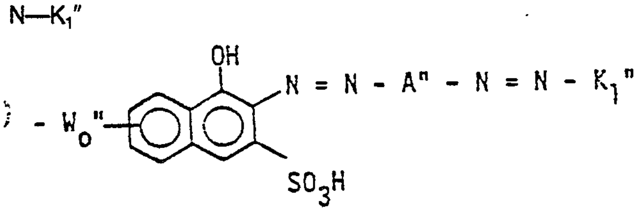

K″ signifie un reste de formule

aa$_4$′) $B_2$ dans lequel r signifie 1 ou 2,

aa$_5$) un phénol,

aa$_6$′) 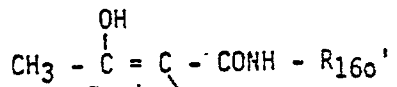

aa$_7$′) 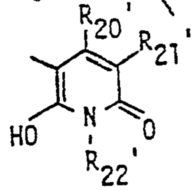

aa$_8$′) 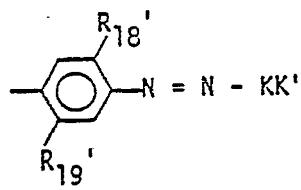

aa$_9$′) 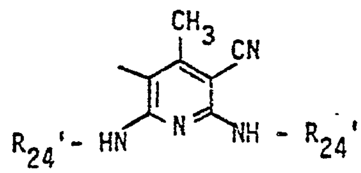

aa$_{10}$′) 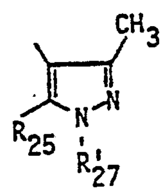

aa$_{11}$′) 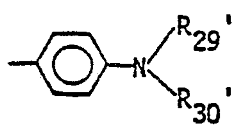

aa$_{12}$′) 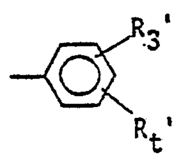

$R_1$″ signifie un groupe 1,2-éthylène, 1,3-propylène ou méta- ou para-phénylène,
$R_0$″ signifie un reste de formule

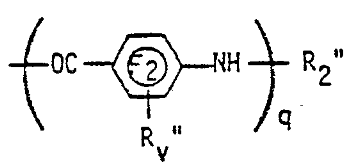

(Ic)

$R_v$″ signifie l'hydrogène, le chlore ou un groupe méthyle,
$R_2$″ signifie indépendamment l'hydrogène ou un reste de formule

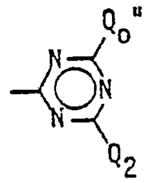

h$_1''$) ou

$- CO - CH_2 - Z_2$ h$_4''$)

où

$R_3''$ signifie l'hydrogène ou un groupe méthyle,

$Q_2$ signifie le chlore ou un groupe OH, $—NH_2$, mono-alkylamino en $C_1$-$C_2$, $OCH_3$, (monohydroxyalkyl en $C_2$-$C_4$)-amino, bis-[hydroxy-(alkyl en $C_2$-$C_4$)]-amino ou bien $Q_0''$ et

$Q_0''$ signifie

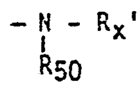

où

$R_x'$ représente un reste de formule

$- (CH_2)_{2-3} - \overset{\oplus}{N}R_{50} - (CH_2)_{2-3} - \overset{\oplus}{N}R_{50} - C_2H_5,$

$- (CH_2)_{2-3} - \overset{\oplus}{N}(R_{13}'')_2 - (CH_2)_{2-3} - \overset{\oplus}{N}(R_{13}'')_2 - C_2H_5 \quad 2A^{\ominus}$

$- (CH_2)_{2-3} - \overset{\oplus}{N}R_{50} - C_2H_5,$

$- (CH_2)_{2-3} - \overset{\oplus}{N}(R_{13}'')_2 - C_2H_5 \quad A^{\ominus}, - NHCOCH_2 - Z_2,$

$- CH_2 - CO - NH - V' - Z_2,$

$- V' - Z_2,$ 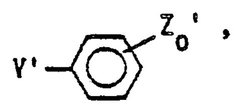 , 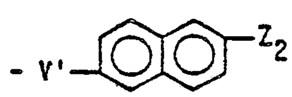 ,

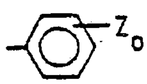 , 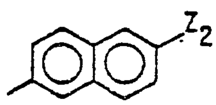 , 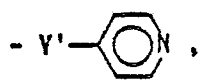 , 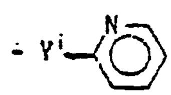

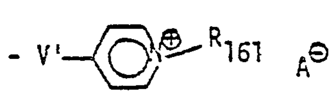 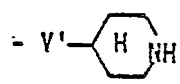 , 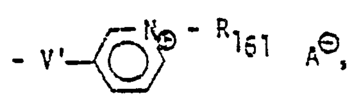

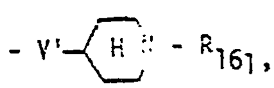 , 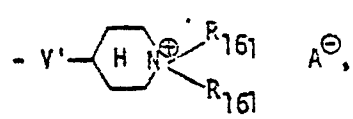

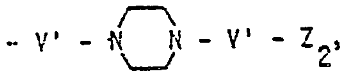 , 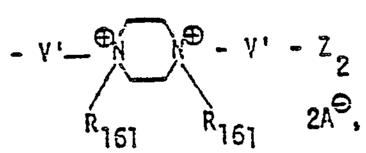

ou bien $R_x'$ et $R_{50}$ peuvent former ensemble, avec l'atome d'azote auquel ils sont liés, un reste

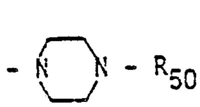 ou 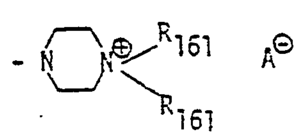

$R_{161}$ signifie un groupe méthyle ou éthyle,
V′ signifie indépendamment un reste alkylène en $C_1$-$C_4$,
$Z_0'$ signifie un groupe $—N(CH_3)_2$,

$$-\overset{\oplus}{N}(CH_3)_3\ A^{\ominus}$$

ou un reste $—CONH—V_0'—Z_2$, $—NH—CO—V_0'—Z_2$, $—CO—V_0'—Z_2$, $—SO_2NH—V_0'—Z_2$, $—V_0'—Z_2$ ou $—NHNHCOCH_2—Z_2$ et
$V_0'$ signifie un reste alkylène en $C_1$-$C_4$,
$R_{50}$ signifie l'hydrogène ou un groupe méthyle,
$Z_2$ signifie indépendamment un reste $—NR_{11}''R_{12}''$ ou

$$-\overset{\oplus}{N}R_{13}''R_{14}''R_{15}''\ A^{\ominus}$$

$R_{11}''$ et $R_{12}''$ représentent, indépendamment l'un de l'autre, l'hydrogène ou un groupe méthyle ou éthyle,
$R_{13}''$ et $R_{14}''$ représentent, indépendamment l'un de l'autre, un groupe méthyle ou éthyle,
$R_{15}''$ représente un groupe méthyle, éthyle ou benzyle,
les restes $R_{11}''$ et $R_{12}''$ peuvent former ensemble, avec l'atome d'azote auquel ils sont liés, un cycle morpholine, pipérazine ou N-méthylpipérazine, les restes $R_{13}''$, $R_{14}''$ et $R_{15}''$ peuvent former ensemble, avec l'atome d'azote auquel ils sont liés, un cycle pyridine, α-picoline ou β-picoline, N-méthylmorpholine, N-méthylpipéridine, N-méthylpipérazine ou N,N'-diméthylpipérazine,
$F_0''$ signifie indépendamment l'hydrogène, le chlore ou un groupe $NO_2$, méthyle, méthoxy ou chlorophénoxy,
X′, lorsque les cycles $D_1$ et $D_2$ sont non substitués, signifie $X_1$, $X_5$, $X_6$, $X_7$, $X_{10}$, $X_{11}$, $X_{12}$, $X_{16}$, $X_{17}$, $X_{22}$, $X_{25}$, $X_{26}$, $X_{27}$, $X_{30}$, $X_{31}$, $X_{49}$, $X_{50}$, $X_{51}$, $X_{52}$, $X_{53}$, $X_{54}$, $X_{58}$, $X_{59}$, $X_{62}$, $X_{63}$, $X_{64}$, $X_{101}$ ($R_{43} = —(CH_2)_{1\text{-}2}$), $X_{103}$ ($R_{43} = —(CH_2)_{1\text{-}2}$ et $R_{45} = —NH—(CH_2)_{2\text{-}3}—N(C_2H_5)_2$, $X_{104}$ ($R_{44}$ = H, g = 2 ou 3), $X_{108}$ ($R_{43} = —(CH_2)_{1\text{-}3}$),

$X_{70}$ $-NH-CO-NH-$, $X_{71}$ 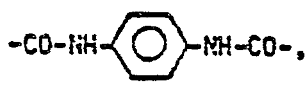

$X_{72}$ 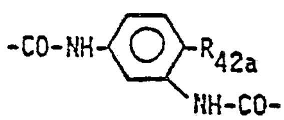

$X_{73}$ $-NH-CO-CH_2-CH_2-CO-NH-$,
$X_{74}$ $-NH-CO-CH=CH-CO-NH-$,
$X_{75}$ $-NH-CO-(CH_2)_4-CO-NH-$,
$X_{76}$ 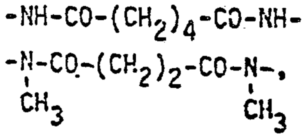

$X_{77}$ 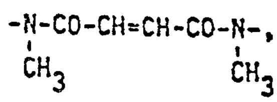

$X_{78}$ 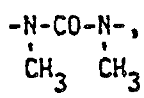

$X_{79}$ 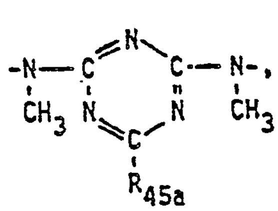 $X_{80}$ 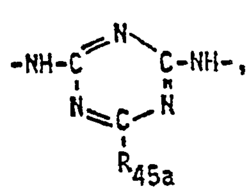

$X_{81}$ $-CH_2-$, $X_{82}$ $-(CH_2)_2-$, $X_{83}$ $-(CH_2)_3-$,
$X_{84}$ $-(CH_2)_4-$, $X_{85}$ $-CO-NH-(CH_2)_2-NH-CO-$,
$X_{86}$ $-CO-NH-(CH_2)_3-NH-CO-$, $X_{87}$ $-CO-NH-(CH_2)_4-NH-CO-$,
$X_{88}$ $-CO-N(CH_3)-(CH_2)_2-N(CH_3)-CO-$, $X_{89}$ $-CO-NH-CH_2-CH(CH_3)-NH-CO-$,

$X_{90}$ $-CO-NH-CH(CH_3)-CH(CH_3)-NH-CO-$

$R_{42a}$ signifie indépendamment l'hydrogène ou $-Cl$, $-CH_3$ ou $-OCH_3$, et
$R_{45a}$ signifie indépendamment

$-Cl$, $-NH-CH_2-CH_2-OH$, $OCH_3$,
$OH$, $NH_2$, $-N(CH_2-CH_2OH)_2$, $-NH-(CH_2)_3N(C_2H_5)_2$, $OC_2H_5$,

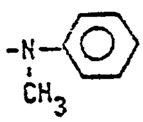 , 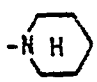 ou 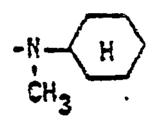 .

ou bien

X, lorsque les cycles $D_2$ et $D_3$ sont substitués, en particulier en position ortho par rapport aux deux ponts azo par le chlore ou par un groupe méthyle ou méthoxy, signifie une liaison directe $X_1$, $X_{40}$, $X_{70}$, $X_{71}$, $X_{80}$, $X_{81}$, $X_{82}$ ou $X_{85}$,

$R_s'$ et $R_t'$, indépendamment l'un de l'autre, représentent l'hydrogène ou un groupe méthyle, méthoxy, éthyle, éthoxy ou butyle,

$R_{160}'$ signifie un reste $-(CH_2)_{v'}-Z_2$,

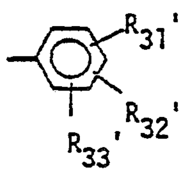 , 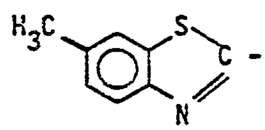 ,

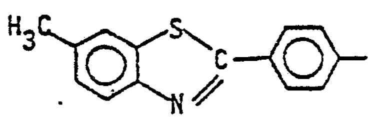 ou 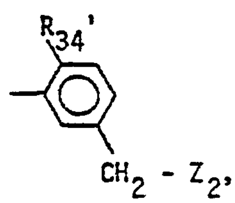

v' signifie 2, 3 ou 4,

$R_{18}'$ et $R_{19}'$, indépendamment l'un de l'autre, représentent l'hydrogène, le chlore ou un groupe $CH_3$, $C_2H_5$, $OCH_3$ ou $OC_2H_5$,

$R_{20}'$ signifie un groupe méthyle ou phényle,

$R_{21}'$ signifie un des restes de $T_2$,

$R_{22}'$ signifie un reste $-R_1''-NH-R_0''$, l'hydrogène, un groupe méthyle, éthyle, benzyle, phényléthyle,

- NH-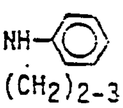, $-(CH_2)_2-OH$, $-(CH_2)_3OCH_3$ $-(CH_2)_2CN$,
$-(CH_2)_{2-3}-N(CH_3)_2$, $-(CH_2)_{2-3}-\overset{\oplus}{N}(CH_3)_3\ A^{\ominus}$ ou
$-(CH_2)_{2-3}-\overset{\oplus}{N}(CH_3)_2\ A^{\ominus}$,
$CH_2$-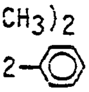

$R_{24}'$ signifie indépendamment l'hydrogène ou un groupe

$$-(CH_2)_2OH,\quad -(CH_2)_3-OCH_3,\ -(CH_2)_{2-3}-N(CH_3)_2 \quad \text{ou} \quad -(CH_2)_{2-3}-\overset{\oplus}{N}(CH_3)_3\ A^{\ominus}.$$

$R_{27}'$ signifie l'hydrogène ou un groupe

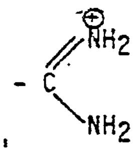 $A^{\ominus}$,

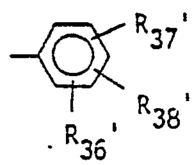

$R_{28}'$ signifie indépendamment un groupe

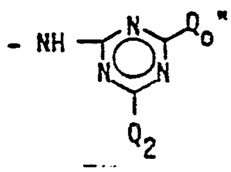 ou $-NHCOCH_2-Z_2$.

$-CONH(CH_2)_{2-3}-Z_2$ ou $-CONH-$ 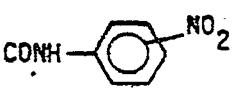

$R_{29}'$ signifie un groupe $CH_3$, $C_2H_5$, $C_2H_4CN$ ou benzyle,

$R_{30}'$ signifie un groupe $CH_3$, $C_2H_5$ ou $-(CH_2)_{v}'-Z_2$,

$R_{31}'$ et $R_{32}'$, indépendamment l'un de l'autre, représentent l'hydrogène, le chlore, le brome ou un groupe méthyle, éthyle, méthoxy, éthoxy, $NO_2$ ou CN,

$R_{33}'$ signifie l'hydrogène ou un groupe

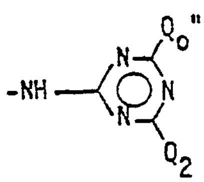

$-NH-CO(CH_2)_{2-3}-Z_2$ ou $-SO_2-NH(CH_2)_{2-3}-Z_2$,

$R_{34}'$ signifie un groupe méthoxy ou éthoxy,

$R_{36}'$ et $R_{37}'$, indépendamment l'un de l'autre, représentent l'hydrogène, le chlore ou un groupe méthyle ou méthoxy,

$R_{38}'$ signifie l'hydrogène ou un groupe

$-CO-NH-(CH_2)_{v}'-Z_2$,

$-NHCO(CH_2)_{2-3}-Z_2$ ou 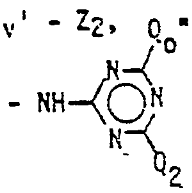

$R_{21a}'$ signifie H, Cl, $CH_3$, OH, $OCH_3$, $C_2H_5$ ou $OC_2H_5$,

$R_{23a}$ signifie indépendamment H, Cl, $CH_3$, OH ou $OCH_3$,

$U_1$ signifie une liaison directe, $-NHCO-$ ou $-NHCONH-$,

$R_{1a}''$ signifie l'hydrogène,

$- NR_{50} - (CH_2)_{2-3} - NR_{11}''R_{12}''$

$- NR_{50} - (CH_2)_{2-3} - NR_{13}''R_{14}''R_{15}'' A^{\ominus}$ ou

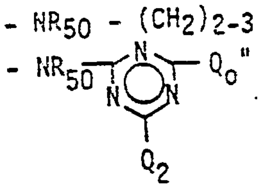

$W_p''$ signifie —NH—, —NH—CO—CH = CH—CONH— ou —NHCO$(CH_2)_2$CONH—,

$K_1''$ signifie un reste de formule aa'), $aa_1'$), $aa_2'$), $aa_3'$), $aa_4'$), $aa_5'$), $aa_6'$), $aa_7'$), $aa_8'$), $aa_9'$, $aa_{10}'$), $aa_{11}'$), $aa_{12}'$) ou un reste de formule

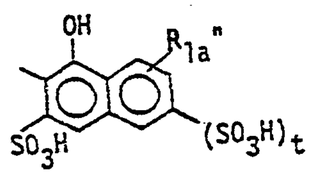

$aa_{13}'$) ou

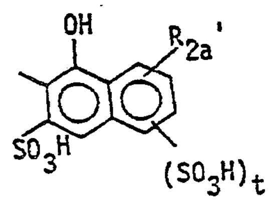

$aa_{14}'$)

et KK' représente un reste de formule aa'), $aa_1'$), $aa_2'$), $aa_4'$), $aa_5'$), $aa_6'$), $aa_7'$), $aa_8'$), $aa_9'$), $aa_{10}'$), $aa_{11}'$), $aa_{12}'$), $aa_{13}'$), $aa_{14}'$),
mélanges de composés de formule III, leurs sels d'addition d'acides et leurs sels d'acides, ainsi que leurs complexes métallifères 1 : 1 ou 1 : 2, avec les conditions énoncées précédemment et avec la condition que

$\beta_2$) lorsque r signifie 2, S'' représente l'hydrogène, $R_0''$ représente l'hydrogène, $R_1''$ soit différent d'un groupe 1,2-éthylène, $T_1$ soit différent d'un groupe CN et le reste M'' soit exempt de groupes sulfo.

4. Composés à complexe métallifère selon la revendication 1, correspondant aux formules

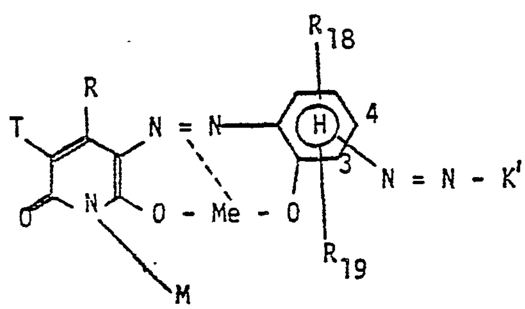

(V)

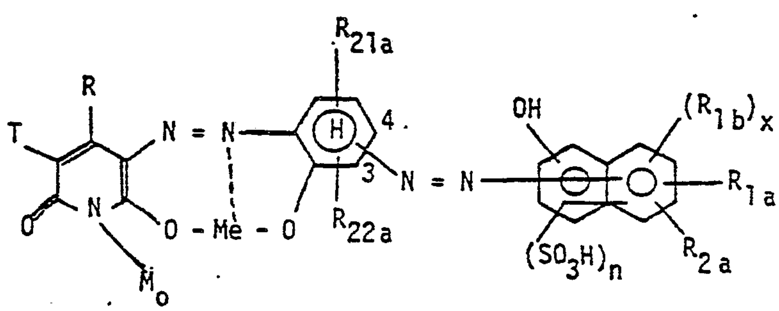

(VI)

(Suite)

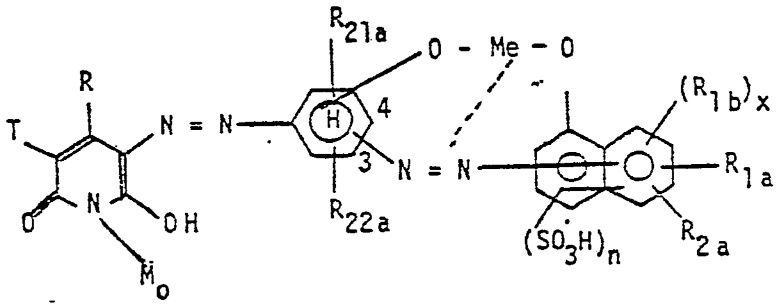

(VII)

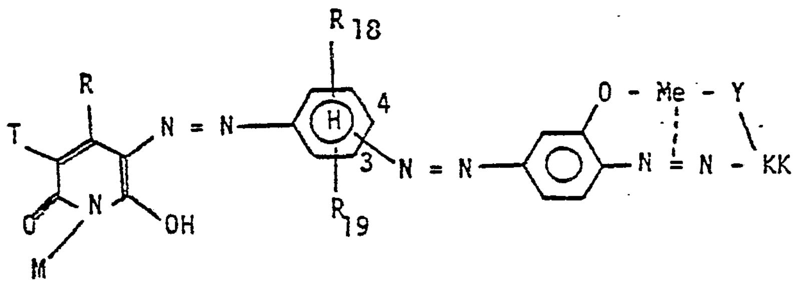

(VIII)

où Y signifie —O— ou —NH—,

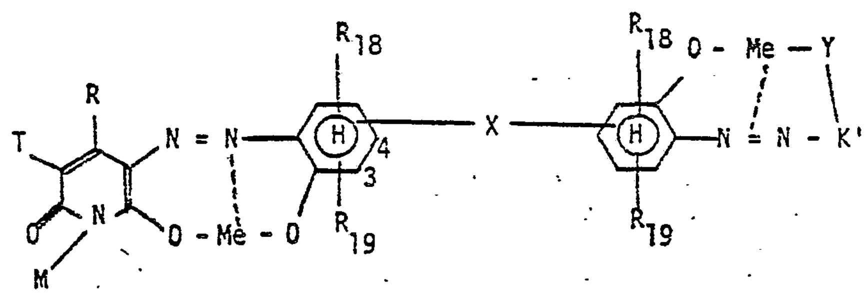

(IX)

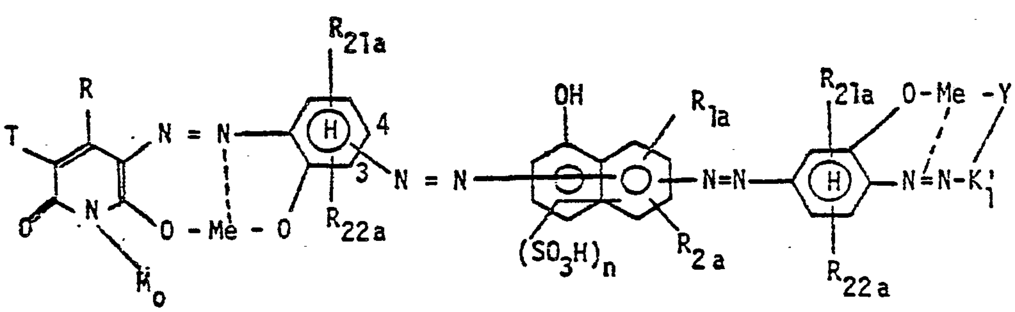

(X)

les restes azo dans les cycles H étant liés en position 3 ou 4, et

Me représentant pour les complexes métallifères 1 : 1 un atome de cuivre, de chrome, de cobalt, de nickel ou de manganèse, avantageusement un atome de cuivre, et

Me représentant pour les complexes métallifères 1 : 2 un atome de chrome, de cobalt ou de fer, avantageusement un atome de fer.

5. Procédé de préparation des composés de formule I selon la revendication 1, où S est différent de l'hydrogène, caractérisé en ce que l'on copule le diazoïque d'une amine de formule

$$DD—NH_2, \qquad XI,$$

dans laquelle DD représente un reste

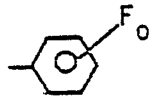

XIa)

—A—N = N—K XI b)

ou

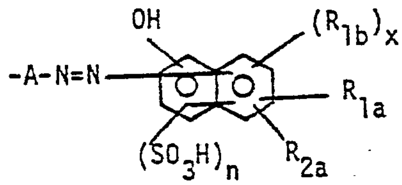

XI c)

avec un composé de formule

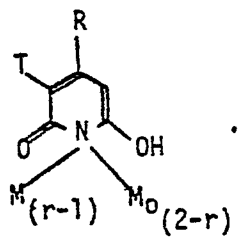

(XII)

et on transforme éventuellement les composés obtenus en complexes métallifères.

6. Procédé de préparation des composés de formule I selon la revendication 1, où S représente l'hydrogène, caractérisé en ce qu'on cyclise selon les méthodes connues un composé de formule

$$T—CH_2—CO—NH—R_1—NH—M_{00} \qquad XXVI$$

dans laquelle $M_{00}$ signifie un groupe protecteur quelconque, avantageusement un composé de formule

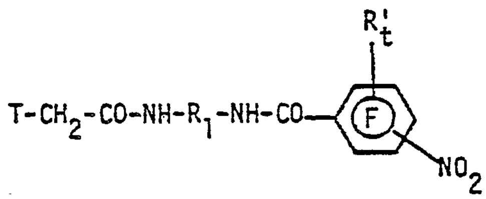

XXVII

dans laquelle $R_t'$ signifie l'hydrogène, un halogène ou un groupe alkyle en $C_1$-$C_4$ ou alcoxy en $C_1$-$C_4$, avec un acétate d'alkyle de formule

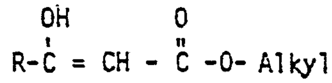

XXVIII

en un composé de formule

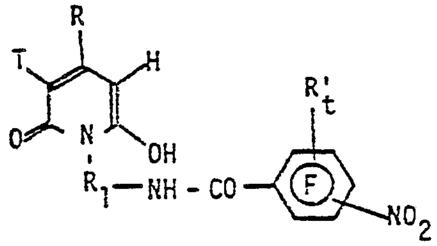

XXIX

et pour les composés dans lesquels $R_0$ signifie l'hydrogène, on saponifie ces composés, ou bien pour les composés dans lesquels $R_{00}$ signifie un reste de formule

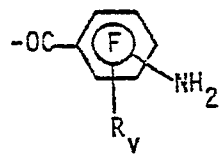

XXa

on réduit le groupe nitro en groupe amino.

7. Utilisation des composés de formule I selon la revendication 1, où S est différent de l'hydrogène, pour la teinture ou l'impression du papier et du cuir.

8. Utilisation des composés de formule I selon la revendication 1, où S est différent de l'hydrogène, pour la teinture, le foulardage ou l'impression de la cellulose naturelle ou régénérée ainsi que des fibres libériennes telles que le chanvre, le lin, le sisal, le jute, la fibre de coco ou la paille.

9. Les substrats teints ou imprimés selon la revendication 7.

10. Les substrats teints, foulardés ou imprimés selon la revendication 8.

**Revendications** (Pour l'Etat contractant AT)

1. Procédé de préparation de composés ou de composés azoïques qui correspondent, sous une des formes tautomères possibles, à la formule

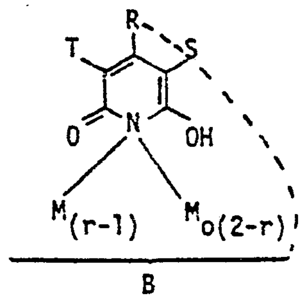

(I)

dans laquelle

R signifie indépendamment l'hydrogène, un reste alkyle en $C_1$-$C_4$, cycloalkyle en $C_5$-$C_6$ ou phényle, benzyle ou phényléthyle,

T signifie indépendamment l'hydrogène ou bien

i) -CN

$i_1$) 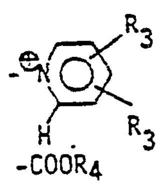 $A^{\ominus}$

$i_2$) $-COOR_4$

$i_3$) $-CONR_5R_6$

$i_4$) $-SO_2NR_5R_6$

$i_5$) 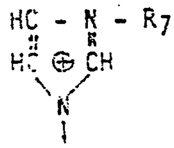 $A^{\ominus}$ ou

$i_6$) 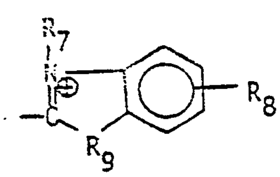 $A^{\ominus}$

r signifie 1 ou 2,

M signifie indépendamment un reste $—R_1—NH—R_0$,

$M_0$ signifie indépendamment un reste $—R_1—NH—R_0$, l'hydrogène ou un groupe $—NR_{11}R_{12}$, alkyle en $C_1$-$C_4$, hydroxyalkyle en $C_2$-$C_4$, (alcoxy en $C_1$-$C_4$)-alkyle en $C_1$-$C_4$, cycloalkyle en $C_5$-$C_6$ éventuellement substitué par 1 à 3 groupes alkyle en $C_1$-$C_4$, (phényl)-alkyle en $C_1$-$C_3$, $—V_1—NR_{13}R_{14}$ ou $—V_2—$

$$\overset{\oplus}{N}R_{13}R_{14}R_{15}A^{\ominus}$$

$A^{\ominus}$ signifie un anion,

S signifie, lorsque r signifie 2,

a) l'hydrogène,

b) un reste de formule

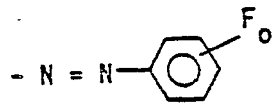 ou

c) $—N=N—A—N—=N—K$

et $F_0$ signifie un substituant usuel dans la chimie des azoïques, de préférence un halogène ou un groupe $NO_2$, $NH_2$, alkyle, alcoxy, CN, trifluoroalkyle, phényle, aniline, benzoyle, carbamoyle, phénoxy, halogéno-phénoxy, dihalogéno-phénoxy, alkylsulfonyle, phénylsulfonyle, alkylsulfonylamino, di-N-alkylaminosulfonyle ou alkylsulfonyle, ou bien, lorsque r signifie 1,

d) un reste de formule

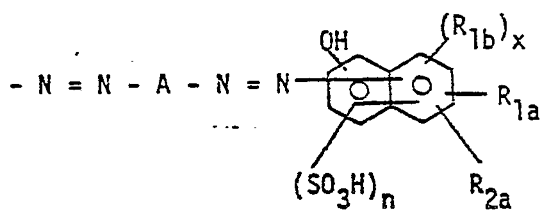

dans laquelle

n signifie 0, 1 ou 2,

x signifie 0 ou 1,

A signifie indépendamment le reste d'une composante de tétrazotation,

K signifie le reste d'une composante de diazotation ou de copulation quelconque, de préférence exempte de groupes sulfo, de la série de la pyrazolone-5, du 5-aminopyrazole, de l'aniline, du phénol, de l'acétoacétylalkyl- ou arylamide, de l'acide barbiturique, de la dimédone, d'un ester de l'acide dimédone-carboxylique, de la pyridone (par exemple du reste B, dans lequel r signifie 1 ou 2) ou de la 2,6-diaminopyridine,

$R_{1b}$, lorsque x signifie 1, représente un reste de formule

e) $—N=N—K_1$

f) $—N=N—A_1—N=N—K_1$

g) 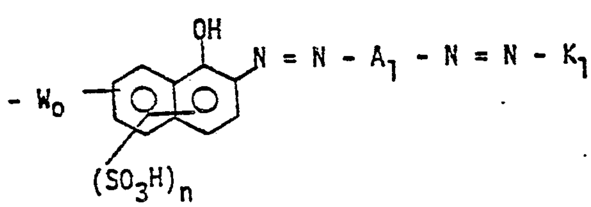

$K_1$ signifie un reste K ou un α- ou β-naphtol, un α- ou β-aminonaphtalène ou un aminonaphtol,

$R_1$ signifie indépendamment un reste alkylène ou alcénylène éventuellement interrompu par 1 ou 2 hétéroatomes, un reste phénylène ou cyclohexylène,

$R_0$ ou un reste de formule

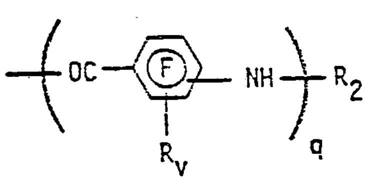 (Ia)

q signifie 0 ou 1, et
$R_1$ signifie l'hydrogène, un halogène ou un groupe nitro, alkyle en $C_1$-$C_4$ ou alcoxy en $C_1$-$C_4$,
$R_2$ représente un reste de formule

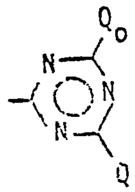

h1)

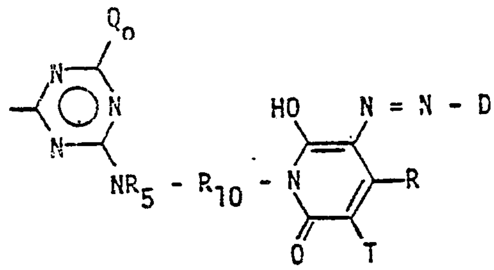

h3) ou

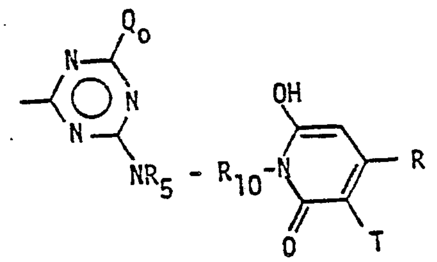

h2)

$$- CO - (CH_2)_{1-3} - Z$$ h4)

ou bien l'hydrogène,

D signifie indépendamment le reste d'une composante de diazotation pouvant être substituée par des substituants usuels dans la chimie des azoïques, de préférence un reste

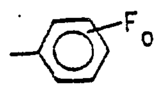

$- A - N = N - K$ ou $- A - N = N$ 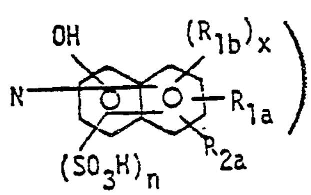

$R_3$ signifie indépendamment l'hydrogène ou un reste alkyle en $C_1$-$C_4$ ou un groupe $—NR_5R_6$ ou $—CONR_5R_6$,
$R_4$ signifie indépendamment un groupe alkyle en $C_1$-$C_6$ ou (phényl)-alkyle en $C_1$-$C_3$,
$R_5$ et $R_6$, indépendamment l'un de l'autre, signifient l'hydrogène ou un groupe alkyle en $C_1$-$C_4$,
$R_7$ signifie indépendamment un groupe alkyle en $C_1$-$C_4$,
$R_8$ signifie indépendamment l'hydrogène ou un groupe alkyle en $C_1$-$C_4$,
$R_9$ signifie indépendamment un groupe —S—, —O— ou

$$- \overset{|}{N} - R_5$$

$R_{10}$ signifie indépendamment un reste alkylène en $C_2$-$C_6$ ou un groupe

$$-NHCOCH_2-$$

l'atome désigné par un * étant lié au reste $—NR_5—$,

$Q_0$ signifie un reste amino aliphatique, cycloaliphatique, aromatique ou hétérocyclique, dans lequel l'atome d'azote est lié à l'atome de carbone du reste triazinyle,

Q signifie un halogène ou un groupe OH, $NH_2$, alcoxy en $C_1$-$C_4$, phényle ou $Q_0$,

Z signifie indépendamment un reste de formule $—NR_{11}R_{12}$ ou

$$- \overset{\oplus}{N}R_{13}R_{14}R_{15} \quad A^{\ominus}$$

$R_{11}$ et $R_{12}$, indépendamment l'un de l'autre, signifient l'hydrogène ou un groupe alkyle en $C_1$-$C_6$ non substitué, un groupe alkyle en $C_2$-$C_6$ substitué par un halogène ou par un groupe OH ou CN, un groupe (phényl)-alkyle en $C_1$-$C_3$, dont le reste phényle porte éventuellement 1 à 3 substituants choisis parmi le chlore et les groupes alkyle en $C_1$-$C_4$ et alcoxy en $C_1$-$C_4$, un groupe cycloalkyle en $C_5$-$C_6$ non substitué ou substitué par 1 à 3 groupes alkyle en $C_1$-$C_4$,

$R_{13}$ et $R_{14}$, indépendamment l'un de l'autre, ont l'une des significations données pour $R_{11}$ et $R_{12}$, un des restes $R_{13}$ et $R_{14}$, de préférence les deux restes, étant différent de l'hydrogène,

$R_{15}$ signifie indépendamment un reste alkyle en $C_1$-$C_4$ éventuellement substitué par un groupe phényle,

$V_1$ signifie indépendamment un groupe alkylène en $C_1$-$C_6$ ou alcénylène en $C_2$-$C_6$,

$V_2$ signifie indépendamment un groupe alkylène en $C_2$-$C_6$ ou alcénylène en $C_2$-$C_6$,

les restes $R_5$ et $R_6$ ou $R_{11}$ et $R_{12}$ ou encore $R_{13}$ et $R_{14}$ pouvant former ensemble, avec l'atome d'azote auquel ils sont liés, un noyau hétérocyclique saturé, et $R_{13}$, $R_{14}$ et $R_{15}$ pouvant former ensemble, avec l'atome d'azote auquel ils sont liés, un cycle pyridine ou un noyau hétérocyclique saturé, et le cycle pyridine et le noyau hétérocyclique pouvant être substitués par 1, 2 ou 3 groupes alkyle en $C_1$-$C_4$,

$R_{1a}$ signifie l'hydrogène ou un groupe

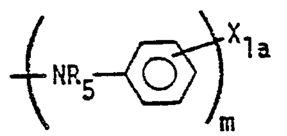

ou

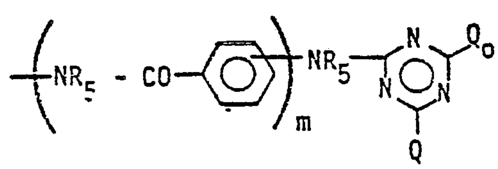

$R_{1a}$ signifiant l'hydrogène lorsque x = 1,

$X_{1a}$ représente un groupe

$$- NR_5 - Q_{10} - NR_{11}R_{12}, \; - COCH_2 - \overset{\oplus}{N}R_{13}R_{14}R_{15} \; A^{\ominus},$$
$$- NR_5 - COCH_2 - NR_{11}R_{12}, \; - CO - Q_{10} - \overset{\oplus}{N}R_{13}R_{14}R_{15} \; A^{\ominus},$$
$$- NR_5 - Q_{10} - \overset{\oplus}{N}R_{13}R_{14}R_{15} \; A^{\ominus}, \; -NR_5-COCH_2\overset{\oplus}{N}R_{13}R_{14}R_{15}A^{\ominus}$$
$$\text{ou} \quad -CO-Q_{10}-\overset{\oplus}{N}(CH_3)_2-(CH_2)_4-Z \; A^{\ominus},$$

m signifie 0 ou 1,

$Q_{10}$ signifie un groupe

$$-\overset{*}{N}HCOCH_2-$$

ou un groupe alkylène en $C_2$-$C_6$ linéaire ou ramifié,

$R_{2a}$ signifie l'hydrogène ou bien, lorsque $R_{1a}$ représente l'hydrogène et $R_{1b}$ un reste de formule e) ou f), également un groupe OH, $NH_2$, (alkyl en $C_1$-$C_4$)-carbonylamino, benzoylamino ou phénylamino, dont le reste phényle porte éventuellement 1 ou 2 substituants choisis parmi les halogènes et les groupes $NO_2$, $NH_2$, alkyle en $C_1$-$C_4$ et alcoxy en $C_1$-$C_4$,

$A_1$ signifie indépendamment le reste d'une composante de tétrazotation ou le reste d'une composante de diazotation/copulation et

$W_0$ signifie un pont bivalent,

des mélanges des composés de formule I, de leurs sels d'addition d'acides ou de leurs sels d'acides ainsi

que de leurs complexes métallifères, tels que leurs complexes métallifères 1 : 1 ou 1 : 2, avec la condition

α) qu'ils contiennent, lorsque les composés monoazoïques b) de formule I sont exempts de groupes sulfo, un et de préférence deux et, lorsque les composés disazoïques et polyazoïques de formule I sont exempts de groupes sulfo, au moins deux groupes cationiques hydrosolubilisants ou au moins deux groupes basiques protonables hydrosolubilisants, ou au moins un groupe cationique et au moins un groupe basique protonable et, lorsque les composés de formule I contiennent des groupes sulfo, le nombre de groupes cationiques et/ou de groupes basiques protonables soit supérieur d'au moins 1 unité au nombre de groupes sulfo et de groupes anioniques supplémentaires éventuellement présents, et

β) lorsque r signifie 2, S représente l'hydrogène et $R_0$ représente l'hydrogène, $R_1$ soit différent d'un groupe alkylène, T soit différent du groupe CN et le reste M soit exempt de groupes sulfo,

caractérisé en ce que, lorsque S est différent de l'hydrogène, on copule le diazoïque d'une amine de formule

$$DD—NH_2, \quad XI,$$

dans laquelle DD représente un reste

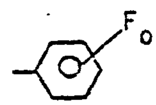

XIa)

—A—N = N—K XI b)

ou

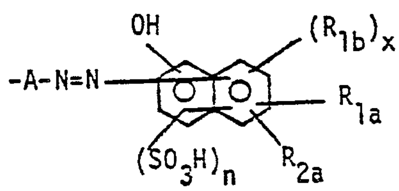

XIc)

avec un composé de formule

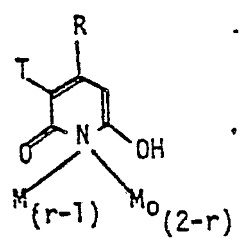

(XII)

et on transforme éventuellement les composés obtenus en complexes métallifères, ou, lorsque S' signifie l'hydrogène, on cyclise selon les méthodes connues un composé de formule

$$T—CH_2—CO—NH—R_1—NH—M_{00} \quad XXVI$$

dans laquelle $M_{00}$ signifie un groupe protecteur quelconque, avantageusement un composé de formule

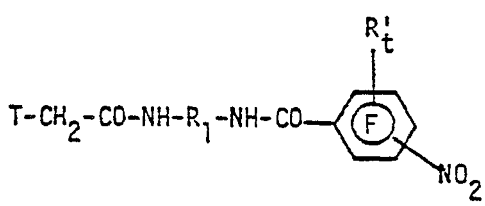

XXVII

dans laquelle $R_t'$ représente l'hydrogène, un halogène ou un groupe alkyle en $C_1$-$C_4$ ou alcoxy en $C_1$-$C_4$, avec un acétate d'alkyle de formule

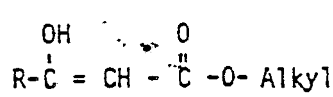

XXVIII

en un composé de formule

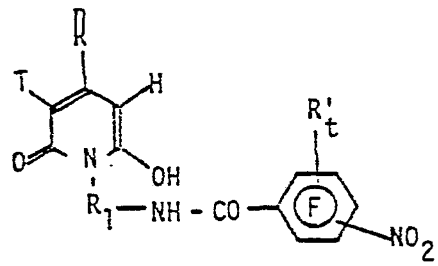

XXIX

et pour les composés dans lesquels $R_0$ représente l'hydrogène, on saponifie ces composés, ou bien pour les composés dans lesquels $R_{00}$ représente un reste de formule

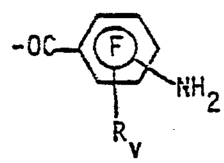

XXa

on réduit le groupe nitro en groupe amino.

2. Procédé de préparation des composés à complexe métallifère de formule I selon la revendication 1, où S est différent de l'hydrogène, caractérisé en ce qu'on fait réagir un composé de formule I avec des composés donneurs de métal tels que des complexes métallifères 1 : 1 ou des complexes métallifères 1 : 2 sont obtenus.

3. Préparations tinctoriales liquides, stables au stockage, contenant un composé de formule I selon la revendication 1, sous forme d'un sel d'addition d'acide hydrosoluble ou d'un sel d'ammonium quaternaire.

4. Préparations tinctoriales solides granulées, hydrosolubles, contenant un composé de formule I selon la revendication 1, sous forme d'un sel d'addition d'acide hydrosoluble ou d'un sel d'ammonium quaternaire.

5. Utilisation des composés de formule I selon la revendication 1, pour la teinture ou l'impression du papier et du cuir.

6. Utilisation des composés de formule I selon la revendication 1, pour la teinture, le foulardage ou l'impression de la cellulose naturelle ou régénérée ainsi que des fibres libériennes telles que le chanvre, le lin, le sisal, le jute, la fibre de coco ou la paille.